(19) Europäisches Patentamt
European Patent Office
Office européen des brevets

(11) EP 3 964 588 B1

(12) # EUROPÄISCHE PATENTSCHRIFT

(45) Veröffentlichungstag und Bekanntmachung des Hinweises auf die Patenterteilung:
**17.04.2024 Patentblatt 2024/16**

(21) Anmeldenummer: **21000252.3**

(22) Anmeldetag: **02.09.2021**

(51) Internationale Patentklassifikation (IPC):
***C12Q 1/6883*** *(2018.01)* ***G16B 99/00*** *(2019.01)*

(52) Gemeinsame Patentklassifikation (CPC):
**C12Q 1/6883; G16B 20/20;** C12Q 2600/118; C12Q 2600/156

(54) **VERFAHREN ZUR PROGNOSE DER SCHWERE DES VERLAUFS EINER COVID-19-ERKRANKUNG**

METHOD FOR PREDICTING THE SEVERITY OF THE PROGRESSION OF A COVID-19 INFECTION

PROCÉDÉ DE PRÉVISION DE LA GRAVITÉ DE L'ÉVOLUTION DE LA MALADIE COVID-19

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2020 DE 102020005556**

(43) Veröffentlichungstag der Anmeldung:
**09.03.2022 Patentblatt 2022/10**

(73) Patentinhaber: **Gesellschaft für Individualisierte Medizin mbH (IndyMed)
18055 Rostock (DE)**

(72) Erfinder: **Thiesen, Hans-Jürgen
18055 Rostock (DE)**

(56) Entgegenhaltungen:
- **D. ELLINGHAUS ET AL.: "Genomewide Association Study of Severe Covid-19 with Respiratory Failure", N ENGL J MED, Bd. 383, 17. Juni 2020 (2020-06-17), Seiten 1522-1534, XP055845288,**
- **Ellinghaus D. ET AL: "Genomewide Association Study of Severe Covid-19 with Respiratory Failure", GWAS Catalog, 17. Juni 2020 (2020-06-17), XP055881283, Gefunden im Internet: URL:https://www.ebi.ac.uk/gwas/publications/32558485 [gefunden am 2022-01-19] & Ellinghaus D ET AL: "Genomewide Association Study of Severe Covid-19 with Respiratory Failure.", GWAS Catalog, 17. Juni 2020 (2020-06-17), Seiten 1-1, XP055881297, Gefunden im Internet: URL:https://www.ebi.ac.uk/gwas/publications/32558485 [gefunden am 2022-01-19]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die Erfindung betrifft ein Verfahren zur Prognose der Schwere des Verlaufs einer Covid-19-Erkrankung unter Verwendung von Einzelnukleotid-Polymorphismus(SNP)-Markern.

Coronaviren stellen eine ernsthafte Bedrohung für die globale Gesundheit dar, wie das schwere akute respiratorische Syndrom (SARS), das nahöstliche respiratorische Syndrom (MERS) und Covid-19 belegen. Das SARS-Coronavirus (SARS-CoV), das MERS-Coronavirus (MERS-CoV) und das neuartige SARS-CoV-2-Coronavirus sind die Erreger von SARS-, MERS- und Covid-19-Krankheitsausbrüchen.

Beim SARS-Cov-2-Coronavirus ist das Spike(S)-Glycoprotein auf der Virushülle entscheidend für die Übertragung und Infektion und bestimmt den Tropismus des Virus- und Wirtszelleneintritts. Tropismus bezeichnet in der Virologie die Fähigkeit eines Virus, eine bestimmte Sorte von Zellen oder bestimmte Gewebe zu infizieren und sich dort zu vermehren. SARS-CoV-2 bindet über eine Domäne (die sog. "Receptor-binding Domaine, RBD) der S1-Untereinheit des S-Proteins an den ACE2-Rezeptor (ACE2 = "Angiotensine-Converting Enzyme 2") von Zellen und verwendet dann seine C-terminale Transmembranuntereinheit (S2) zur Fusion mit der Wirtszellmembran. Aufgrund dieser lebenswichtigen funktionellen Eigenschaft und der festgestellten Antigenität ist das S-Protein ein wichtiges Ziel für die Impfstoffentwicklung.

Es werden aber nicht nur sichere Impfstoffe benötigt, die schnell wirksame und lang anhaltende virusspezifische Immunantworten gegen diese Infektionserreger auslösen, sondern es ist aus der Sicht des behandelnden Arztes außerdem sehr wünschenswert, den zu erwartenden Verlauf bzw. die Schwere einer Covid-19-Erkrankung prognostizieren und so rechtzeitig geeignete Maßnahmen wie die Verlegung auf eine Intensivstation mit Möglichkeit zur künstlichen Beatmung ergreifen zu können bzw. sobald Impfstoffe zur Verfügung stehen, eine entsprechende Impfung bei Risikopatienten vorzunehmen. Dies ist in Anbetracht der aktuellen und nach wie vor um sich greifenden Covid-19-Pandemie und der begrenzten Kapazitäten der Intensivstationen besonders wichtig.

Aufgabe der Erfindung ist die Bereitstellung eines Verfahrens, das die Prognostizierung der Schwere des Verlaufs einer Covid-19-Erkrankung ermöglicht.

Die Erfindung basiert auf der Verwendung von 381 Einzelnukleotid-Polymorphismen (SNPs), die in dem erfindungsgemäßen Verfahren als Marker zur Prognose der Schwere des Verlaufs einer Covid-19-Erkrankung dienen.

Die Erfindung betrifft somit ein Verfahren zur Prognose der Schwere des Verlaufs einer Covid-19-Erkrankung mit den in Anspruch 1 definierten Merkmalen.

Bevorzugte und/oder vorteilhafte Ausführungsformen der Erfindung sind Gegenstand der Unteransprüche.

Dem Fachmann ist klar, dass das erfindungsgemäße Verfahren sich sowohl zur Prognose der Schwere des Verlaufs einer bestehenden Covid-19-Erkrankung eignet als auch zur Prognose der Schwere des Verlaufs einer möglicherweise zukünftigen SARS-Corona-Erkrankung, bzw. sich sogar mit der auf dem individuellen Genom beruhenden unterschiedlichen Ansprechbarkeiten von Medikamenten und Impfstoffklassen (wie u.a. Tot-, Vektor- und DNA-, bzw. RNA-Impfstoffen) kombinieren läßt, so dass zukünftig der bestmögliche Impfstoff ausgewählt werden kann. Solche impfrelevanten Polymorphismen sind für die Gene MTHFR, IL1R, und Il1A bekannt und werden u.a. von der Firma Tellmegen bestimmt (www.tellmegen.com).

Mit dem Begriff Einzelnukleotid-Polymorphismus (SNP, engl. *Single Nucleotide Polymorphism*; im Laborjargon gesprochen: "Snip") wird eine Variation eines einzelnen Basenpaares in einem komplementären DNA-Doppelstrang bezeichnet. SNPs sind geerbte und vererbbare genetische Varianten und stellen circa 90 % aller genetischen Varianten im menschlichen Genom dar. SNPs können die Expression und/oder Funktion von Genen über Änderungen posttranslationaler epigenetischer Modikationen (PTM) verändern und dadurch beeinflußen oder bestimmen, wie Menschen Krankheiten entwickeln und auf Krankheitserreger, Chemikalien, Medikamente, Impfstoffe und andere Wirkstoffe reagieren, siehe auch die Webseiten der Firmen 23andme (www.23andme.com) und tellmegen (www.tellmegen.com) am 23.08.2020. Ihre wissenschaftliche Bedeutung, z.B. auch im Hinblick auf die sog. "personalisierte Medizin", liegt in der komplexen Kombinatorik ihres Auftreten und ihrer hohen Variabilität zwischen unterschiedlichen Populationen (Ethnien). Außerdem sind sie sehr schnell und einfach zu bestimmen.

Zur Detektion von Polymorphismen in einer Population wurden unterschiedliche Verfahren entwickelt, die eine Durchmusterung (engl. *Screening*) mit hohem Durchsatz ermöglichen (engl. *Highthroughput Screening*). Heute werden dazu überwiegend sogenannte "SNP-Arrays" eingesetzt. SNP-Arrays sind ein bekannter Typ von DNA-Arrays (DNA-Arrays werden auch als "DNA-Chips", "Microarrays" oder "DNA Bio-Chips" bezeichnet). Die DNA-Array-Technologie nutzt unter anderem Techniken der Halbleitertechnik (daher auch die Bezeichnung "Chip"), um Nukleotidsequenzen auf ein etwa fingernagelgroßes Plastik- oder Glasplättchen, den Microarray, als Gensonden aufzubringen und dort zu immobilisieren. Bei einem SNP-Array weist der Array immobilisierte allel-spezifische Oligonukleotide als Sonden auf. Jedes Testfeld auf einem solchen Microarray trägt eine definierte Sequenz. Nach der Inkubation mit (z.B.) Fluoreszenz-markierterZiel-DNA in Lösung werden die Stellen mit durch Hybridisierung gebundener DNA auf dem Microarray mit einem geeigneten Detektionssystem aufgezeichnet und das Hybridisierungssignal interpretiert. So kann der betreffende SNP spezifisch und eindeutig nachgewiesen werden.

DNA-Arrays mit mehreren hundertausend Nukleotidsequenzen als Gensonden für SNPs sind kommerziell erhältlich, z.B. von Illumina, Inc., San Diego, California, USA. Es gibt auch kommerzielle Hersteller, die nach Kundenvorgaben maßgeschneiderte DNA-Arrays produzieren. Allgemein einsetzbare Verfahren zur Detektion und Analyse von SNPs mit DNA-Arrays sind im Stand der Technik bekannt, beispielsweise aus EP 2 423 329 A1, EP 2 423 330 A1 und EP 2 331 709 B1. Häufig stellt auch der DNA-Array-Hersteller Benutzungsempfehlungen und Protokolle zur Verfügung.

Dem Fachmann ist klar, das an Stelle eines DNA-Arrays auch beliebige andere geeignete Techniken zur SNP-Detektion und -Analyse eingesetzt werden können (z.B. DNA-Sequenzierung) und er kann diese nach Maßgabe der praktischen Gegebenheiten, z.B. den im Labor etablierten Techniken und vorhandenen Analyse-Geräten, auswählen. Die hier in Anspruch 1 genannten 381 SNPs werden bisher noch nicht auf den oben erwähnten Illumina-DNA-Arrays zur Verfügung gestellt, sondern können bisher nur im Rahmen einer gezielten DNA-Sequenzierung erhalten werden.

Durch die Verwendung der angegebenen 381 SNPs in dem erfindungsgemäßen Verfahren ist es möglich, individuell (personalisiert) für einen Patienten vorherzusagen, ob z.B. ein asymptomatischer, ein milder, ein moderater oder ein schwerer Covid-19-Erkrankungsverlauf zu erwarten ist. Durch diese Differenzierungsmöglichkeit kann der Arzt frühzeitig entscheiden, ob geeignete Gegenmaßnahmen wie z.B bestimmte Abstands- und Kontaktregelungen in Coronavirus-Endemiegebieten, bzw. eine vorzeitige Verlegung eines Patienten auf eine Intensivstation oder eine sofortige bevorzugte Immunisierung (sobald ein Impfstoff zur Verfügung steht) durch eine Impfung einzuleiten sein werden.

Die erfindungsgemäß verwendeten SNPs sind an sich bekannt. Die in den folgenden Tabellen und in den Patentansprüchen angegebenen Referenz-SNP-Codes (rs#) für jeden erfindungsgemäß verwendeten SNP beziehen sich auf entsprechende Einträge in der *Single Nucleotide Polymorphismen Database (dbSNP)* des *National Center for Biotechnology Information (NCBI)* (https://www.ncbi.nlm.nih.gov/snp/). Diese Referenz-SNP-Codes sind für jeden SNP eindeutig und unveränderlich. Die dbSNP-Datenbank enthält sämtliche relevanten Daten zu einem bekannten SNP wie z.B. das betroffene Allel, die Position auf dem Chromosom, die Genbezeichnung, die Nukleotidsequenz des SNPs, etc.. Anhand der in der dbSNP-Datenbank angegebenen Nukleotidsequenzen für SNPs kann mit Standardmethoden der DNA-Arraytechnologie ohne weiteres ein maßgeschneiderter Array mit Gensonden für die Detektion und die Analyse der relevanten SNPs hergestellt werden. Es wurde der kommerzielle Array der Firma tellmegen /www.tellmegebn.com verwendet, dessen SNP-Zusammenstellung dem Erfinder zur Verfügung stand, da jedem Kunden seine Daten aufgrund der europäischen Gendiagnostik-Gesetzgebung zur Verfügung zu stellen sind.

Die in dem erfindungsgemäßen Verfahren zur Bestimmung des Schweregrades einer Covid-19-Erkrankung verwendeten 381 mit Covid-19 assoziierten SNPs stammen aus der Publikation Ellinghaus et al., 2020. Diese SNPs wurden vom Erfinder in Beziehung zu den vollständigen Genomsequenzen (2504 Genome aus dem sog. "1000 Genomes Project") gesetzt, indem diese Genome über https://www.internationalgenome.org heruntergeladen und hinsichtlich der 381 SNPs analysiert wurden. Im Vergleich: "GWAS Catalog - The NHGRI-EBI Catalog of human genome-wide association studies" gibt eine Übersicht übergenomweit assoziierte Studien. Die Liste der 381 SNPs wurde extrahiert aus den Daten von Ellinghaus D, Degenhardt F, Bujanda L, et al. Genomewide Association Study of Severe Covid-19 with Respiratory Failure [published online ahead of print, 2020 Jun 17]. N Engl J Med. 2020;NEJMoa2020283. doi:10.1056/NEJMoa2020283, und zwar über https://www.ebi.ac.uk/gwas; accession numbers: GCST90000255 and GCST90000256. Die vom Erfinder extrahierte Genliste der 381 SNPs wurde erstellt und genutzt, um aufgrund einer beschriebenen Assoziation zu der Schwere von Covid-19-Erkrankten das individuelle Risiko, an Covid-19 schwer oder leicht zu erkranken, bestimmen zu können, siehe auch Zhao J, Yang Y, Huang H, et al. Relationship between the ABO Blood Group and the Covid-19 Susceptibility [published online ahead of print, 2020 Aug 4]. Clin Infect Dis. 2020;ciaa1150. doi:10.1093/cid/ciaa1150. Die letztere Publikation weiß darauf hin, dass Blutproben eines Menschen auch mit der Schwere einer Covid-19-Erkrankung assoziiert sein mögen. Die erfindungsgemäß durchgeführten bioinformatischen Analysen zeigen in überraschender Art und Weise, dass das individuelle Risiko an Covid-19 zu erkranken anhand von 32 SNPs in Verbindung mit den genannten 2504 Genomen bestimmbar ist, noch bevor es zur Erkrankung kommt. Die Genliste in Tabelle 1b dieser Beschreibung (nach Gordon DE, Jang GM, Bouhaddou M, et al. A SARS-CoV-2 protein interaction map reveals targets for drug repurposing. Nature. 2020;583(7816):459-468. doi:10.1038/s41586-020-2286-9) gibt 332 Proteine an, die mit CoV2 Proteinen interagieren. Die erfindungsgemäß durchgeführten bioinformatischen Analysen führten dazu, dass diesen 332 Genorten SNPs zugewiesen (Tabelle 1c) und diese dann mit den p-Werten der Ellinghaus-Publikation assoziiert werden konnten, siehe Tabelle 3a (isTMG). Laut Tabelle 1c wurde das Protein FYCO1 als Gen mit seinen SNPs rs7652331 rs1545985, rs13079478, rs33910087, rs4683158, rs17214952 als kausaler Marker für die Interaktion mit dem CoV2-Virus identifiziert. Dieses Gen FYCO1 Gen und seine SNPs sind aufgrund der erfindungsgemäß durchgeführten Analysen gleichzeitig an der Pathogenese und der Schwere der Covid-19-Erkrankung beteiligt, da zwei dieser SNPs rs13079478 und rs17214952 sowohl in Tabelle 1b und Tabelle 1c dieser Beschreibung gefunden werden.

Die folgenden Tabellen offenbaren die erfindungsgemäß verwendeten SNPs und die mit dem erfindungsgemäßen Verfahren erhaltenen Ergebnisse.

Tabelle 1a zeigt die 381 mit einer Covid-19-Erkrankung assoziierten SNPs, die erfindungsgemäß zur Bestimmung des Schweregrades einer Covid-19-Erkrankung verwendet werden, und zwar unter Einbeziehung von vollständigen Genomsequenzen (2504 Genome aus dem "1000 Genomes Project").

Jedem SNP-Effekt-Allel ist ein Signifikanzwert für seine Beteiligung an der Schwere einer Covid-19-Erkrankung zugeordnet. Die Anzahl der SNPs mit seinem homozygoten Effekt- (2), seinem heterozygoten (1) und seinem homozygoten anderen Allel (1) ergibt für jeden Patienten einen spezifischen Referenzwert, der dann zur Unterscheidung von schweren und leichten Covid-19 Erkrankungen verwandt wird, wobei jeder Referenzwert mit einem bekannten Schweregrad der Covid-19-Erkrankung assoziiert ist und das Vorliegen unterschiedlicher SNP-Allelmuster zwischen Patienten, die vergleichbare Schweregrade der Covid-19-Erkrankung aufweisen, noch über die Bestimmung ähnlicher SNP-Muster-Verteilungen in Covid-19-Gruppen subklassifiziert wird. Prognostische Aussagen, zu welchem Schweregrad bei einem Menschen, der sich möglicherweise mit SARS-CoV2 infiziert, sich eine Covid-19-Erkrankung entwickelt, umfassen letztendlich 123 Möglichkeiten der Schweregrad-Gruppierung, die dann zur PCA-Klassifizierung gesetzt wird, und zu Impfempfehlungen führt.

Tabelle 1b zeigt die 32 SNPs, die zur Prognose des Schweregrads einer Covid-19-Erkrankung besonders gut geeignet sind.

Diese 32 SNPs definieren die zugeordneten Covid-19 Schweregrade ausgehend von ursprünglich 381 SNPs. Aufgrund ihrer Effekt-Allel-Verteilungen wird diesen SNPs die höchste Priorität zugewiesen, um Ähnlichkeiten zwischen Genomen herzustellen, deren SNP Sequenzen über Anspruch 1 mit der Schwere einer Covid-19 definiert sind.

Tabelle 1c zeigt SNPs in Genen, die direkt mit einzelnen Poteinen des CoV2-Viruses interagieren, vgl. Gordon DE, Jang GM, Bouhaddou M, et al. A SARS-CoV-2 protein interaction map reveals targets for drug repurposing. Nature. 2020;583(7816):459-468. doi:10.1038/s41586-020-2286-9. in dieser Publikation werden Proteine identifiziert, die direkt mit CoV2 interagieren, bzw. deren Aktionspartner wiederum mit weiteren Proteinen in der menschlichen Zelle. Beispielhaft seien genannt kodierende SNPs der Proteine, die direkt mit CoV2 interagieren, bzw. die SNPs, die die Expressionshöhe der RNA-Expression, bzw. die der Proteinexpression beeinflussen, d.h. SNPs dieser Gene beeinflußen somit direkt bzw. indirekt die Replikationsrate des SARS-CoV2 Virus, Im Rahmen der Erfindung durchgeführte Analysen haben ergeben, dass bestimmte SNPs des FYCO1-Proteins mit der Schwere einer Covid-19 Erkrankung assoziiert sind und gleichzeitig mit dem NSP13-Protein von CoV2 interagieren, Es zeigt sich jetzt, dass sich diese SNPs rs13079478 und rs17214952 des Gens FYCO1 sowohl in der Tabelle 1b als auch in der Tabelle 1c befinden. Damit ist nachgewiesen, dass zwei unabhängige wissenschaftliche Ansätze das gleiche Gen in Verbindung mit der Covid-19-Erkrankung bringen, so dass hier ein kausaler Zusammenhang zur Pathogenese der Covid-19 -Erkrankung hergestellt werden kann, was erfindungsgemäß erstmalig hier beschrieben wird. Laut Tabelle 3b zeigt sich, dass sich die 2504 Genome des "1000 Genomes Project" mit Hilfe dieser 2 SNPs in 2 Gruppen unterteilen lassen.

Tabellen 1d und 1e zeigen krankheitsverstärkende SNPs, die Auskunft darüber geben, ob im Leben mit zunehmendem Alter das einzelne Individuum aufgrund seiner individuellen SNPs ein höheres oder geringeres individuelles Risiko für das Auftreten bestimmter Volkskrankheiten ausweist. Dieser Zusammenhang wird dann in Verbindung mit einer SARS-CoV2-Infektion mit zunehmendem Alter lebensbedrohlicher. Wären die über 80-zig Jährigen an Covid-19 Verstorbenen 40 Jahre jünger, dann würden diese mit 40 Jahren nicht an Covid-19 versterben, aber gegebenenfalls krankenhauspflichtig werden. Die internationalen Daten der Johns-Hopkins-University (www.worldometers.info/coronavirus) zeigen, dass im Durchschnitt jeder 30-zigste an Covid-19 Erkrankte verstirbt, wenn keine besonderen Maßnahmen zur Prävention bzw. in der Behandlung der Covid-19-Erkrankung unternommen werden.

In den Tabellen 1a bis 1e bedeuten die Spaltenüberschriften folgendes:

"coord": SNP Koordinaten im menschlichen Genom, kodiert nach HG19/GRCH37

"DBSNP.ID": RS Nummer laut SNP Datenbank dbSNP

"dbsnp gene strand": Hier wird angegeben, ob das Gen in kodierender 5'-3'-Richtung vorliegt oder auf dem Gegenstrang in entgegengesetzter Form

"dbsnp gene symbol": Hier sind die offiziellen Gensymbole zu den Gen-Namen genannt.

"dbsnp Ref": Hier wird das Nukleotid des Referenz-Genomes genannt

"dbsnp Alt": Hier wird das Nukleotid der Genvariante genannt, divergent von "dbsnp Ref"

"COV EA": Hier wird das Covid-19-Effekt- (krankheitsassoziiertes) Allel genannt.

"COV OA": Hier wird das andere Allel genannt, welches protektiv sein könnte.

"Pval main": Hier ist das Signifikanzmaß für die Abhängig der SNPs zur Covid-19-Erkrankung angegeben.

"Pval_corr sex_age": Hier wurde von Ellinghaus et al., 2020 eine geschlechts- und altersbezogene Korrektur des Signifikanzmaßes genannt.

"Pval min": Hier wurde erfindungsgemäß immer mit den geringsten P-Werten gearbeitet

"isTMG": Diese SNPs sind zur Zeit auf dem kommerziellen DNA-Array der Firma Tellmegen vorhanden und werden auch in Tabelle 1b ausgewiesen. Dieser DNA-Array umfaßt insgesamt 754525 unterschiedliche SNPs.

"Merkmal" (in Tabelle 1d): beschreibt die SNPs, die mit Covid-19 assoziierten Komorbiditäten im unterschiedlichen Maße vergesellschaftet sind. Diese SNPs beschreiben und vorhersagen mit unterschiedlicher Signifikanz das Auftreten bestimmter Krankheiten in Abhängigkeit vom individuellen Genom.

Erfindungsgemäß können neben den oben genannten 381 bzw. 32 SNPs zusätzlich mindestens einer der in Tabelle 1d bzw. 1e angegebenen SNPs, die mit den dort genannten Erkrankungen assoziiert sind, bestimmt werden, um den Einfluß von krankheitsassoziierten SNPs in die Covid-19-Risikobetrachtung integrieren zu können. Für jedes Genom werden die homozygoten Affekt- (2), die heterozygoten (1), und die homozygoten anderes Allel bestimmt und in Beziehung zum klinischen Bild und der Familienanamnese bzw. zum Alter, Geschlecht, BMI und klinisch bekannten Komorbiditäten gesetzt. Aufgrund der hier dargestellten Komplexität ist ein gezieltes abgestuftes Vorgehen erforderlich. Nachdem die Analyseergebnisse nach dem Verfahren gemäß Anspruch 1 und Anspruch 2, bzw. entsprechende Analysen nach dem Verfahren gemäß Anspruch 4 vorliegen, findet eine vergleichende Analysen dieser Datensätze mit den Effekt-Allelen der in Anspruch 5 definierten SNPs statt. Eine gezielte strukturierte Einbindung dieser nach dem Verfahren gemäß Anspruch 5 gewonnenen Daten wird vom Fachmann genauso fortgeführt wie in dem Verfahren gemäß Anspruch 3 für FYCO1 durchgeführt und dokumentiert, siehe Tabelle 3b. Es ist das Ziel, für jede in Anspruch 5 definierte Erkrankung die Affekt-Allele der SNPs zu nutzen, die entweder als Haplotyp protektiv (0) oder als Haplotyp (2) die Schwere einer Covid-19 Erkrankung innerhalb eine Population definieren.

Tabellen 2a und 2b zeigen beispielhaft die Untersuchungsergebnisse von 100 anonymisierten Patientenproben, die mit dem erfindungsgemäßen Verfahren unter Verwendung der 381 bzw. 32 SNPs gewonnen wurden und in Beziehung zu den insgesamt 2573 komplett-sequenzierten Genomen aus dem "1000 Genomes Projekt" und von Complete Genomics (CG69) gesetzt werden, siehe auch Figuren 1 und 2.

In den Tabellen 2a und 2b bedeuten die Spaltenbeschriftungen folgendes:

1. Kohorte:

    1 kG = Patientenproben aus dem "1000 Genomes"-Projekt,
    vgl. https://www.internationalgenome.org
    CG69 = Es handelt sich um komplett sequenzierte Genome, die die Firma Complete Genomics seinen Kunden für wissenschaftliche Zwecke zur Verfügung gestellt hat, bevor die Firma von BGI aufgekauft wurde.
    TMG: Die Firma Tellmegen hat dem Erfinder anonymisierte Daten von 50 spanischen und 50 italienischen Klienten zur Verfügung gestellt, um nachweisen zu können, dass es möglich ist, mit 32 SNPs anstatt 381 SNPs zu arbeiten.

2. Proben-ID:
Identifikations-Nr. der anonymisierten Patientenprobe

3. 32 $p<10^{-5}$ [tmg]:
Unterspalten "0" bis "2": Anzahl der erfindungsgemäß bevorzugten 32 SNPs. Die 32 SNPs, die hier analysiert werden, sind definiert über p-Werte von $< 10^{-5}$. Dieser Wert gibt die Signifikanz für eine Beteiligung eines der 32 SNPs an der Schwere einer Covid-19-Erkrankung im Vergleich zum anderen Allel an. Unterspalte "0" bezieht sich auf das entsprechende homozygote andere Allele (d.h. nicht mit Covid-19 assoziierte Allele), Unterspalte "1" bezieht sich auf das partiell relevante heterozygote Allele, Unterspalte "2" bezieht sich auf relevante mit Covid-19 assoziierte (d.h. mit einer Erkrankung verbundene) homozygote Allele.

4. 381 $p<10^{-5}$ [all]:
Unterspalten "0" bis "2": Gesamtzahl der erfindungsgemäß verwendeten 381 SNPs, die mit einem p-Wert von $< 10^{-5}$angegeben werden, die jeweiligen SNPs tragen zur Schwere der Covid-19-Erkrankung bei. Unterspalte "0" bezieht sich auf das homozygote andere Allele (d.h. nicht mit Covid-19 assoziierte Allele), Unterspalte "1" bezieht sich auf partiell relevante heterozygote Allele, Unterspalte "2" bezieht sich auf das relevante mit Covid-19 assoziierte (d.h. mit einer Erkrankung verbundene) homozygote Allele.

5. Schweregrad - 1000 (Tabelle 2a):
Der Schweregrad 1000 bezieht sich auf die Verwendung kompletter Genomsequenzen menschlicher Genome, dort werden die 381 SNPs identifiziert und das Covid-19 relevante Allel bestimmt, welches mit der Zahl 2 versehen wird. Es werden dann pro Genom alle SNPs der 381 SNPS gezählt, die eine 2 aufweisen. Der höchste Wert, der hier erzielt wurde, beträgt 190 SNPs im Genom HG0349. Je höher der Schweregrad ist, desto wahrscheinlicher wird man schwer an Covid-19 erkranken. Wichtig ist zu wissen, dass 80% bis 90% aller SARS-CoV2 Infizierten keinen Arzt benötigen, siehe Figur 1 und Figur 2. Die Individuen mit den Genomen der Segmente G10, G11, und G12 sollten mit größter Wahrscheinlichkeit nicht in Abhängigkeit zu den genannten 381 SNPs erkranken, sie gehören der Gruppe der asymptomatischen Infizierten an.

Die Bewertung (engl. *Scoring*) des Schweregrades einer Covid-19-Erkrankung erfolgte nach folgenden Kriterien ("X" bedeutet hier den betreffenden Wert in Spalte "381 p<$10^{-5}$ [all]", Unterspalte "2".

| Wert "X" in Unterspalte "2" | Schweregrad Covid-19 | Impfempfehlung 381 SNPS |
|---|---|---|
| X < 100 | Asymptomatisch | 5. Freiwillig |
| 100 ≤ X < 1.30 | Gering | 4. Später |
| 130 ≤ X < 140 | Moderat-Gering | 3. Baldig |
| 140 ≤ X < 160 | Moderat | 2. Dringend |
| X ≥ 160 | Hoch | 1. Umgehend |

Impfempfehlung: Es ist offensichtlich, dass bei begrenzten Ressourcen an Impfstoffen, die Probanden mit den höheren auf SNPs basierenden Schwergraden zuerst geimpft werden müssten. Es werden 5 Grade der Impfempfehlung basierend auf 381 SNPs ermittelt. Wichtig ist, dass diese genombasierte Einteilung um medizinische Parameter wie Alter und Gewicht bzw. klinisch dokumentierte Vorerkrankungen zu ergänzen sein wird, indem auch weitere krankheitsassoziierte SNP-Bestimmungen aus Tabelle 1d und 1e zur genbasierten Bestimmung des zu erwartenden Schweregrades einer Covid-19 Erkrankung eingebunden werden können.

6. TMG-32 (Schweregrad - TMG-32 in Tabelle 2a):

Die Bewertung (engl. *Scoring*) des Schweregrades einer Covid-19-Erkrankung erfolgte nach folgenden Kriterien ("X" bedeutet hier den betreffenden Wert in Spalte "32p<$10^{-5}$ [tmg]", Unterspalte "2".

| Wert "X" in Unterspalte "2" | Schweregrad Covid-19 | Impfempfehlung TMG32 |
|---|---|---|
| X ≤ 5 | Asymptomatisch | E - Freiwillig |
| 6 ≤ X < 9 | Gering | D - Später |
| 9 ≤ X < 12 | Moderat-Gering | C - Baldig |
| 12 ≤ X < 15 | Moderat | B - Dringend |
| X > 15 | Hoch | A - Umgehend |

Hier werden max. 32 SNPs erfaßt. Bisher wiesen die Genomdaten einen Maximal-Wert von 19 SNPs auf, die sich auch mittels der SNP Analysen der Firma Tellmegen bestimmen ließen. Die mit dem erfindungsgemäßen Verfahren gewonnenen Daten zeigen, dass diese 32 SNPs, siehe Figur 2, ausreichen, um die schwersten und leichtesten Covid-19 Fälle bestimmen zu können. Mit den 32 SNPs lassen sich die SNP Muster nutzen, um Patienten mit ähnlichen SNP-Muster zusammenzuführen. SNP Daten, die nur 32/381 SNPs umfassen, reichen zu einer Ähnlichkeitsbestimmung aus, siehe Figur 3. Mit diesem Vorgehen können Covid-19-Erkenntnisse von ehemals Covid-19-Rekonvaleszenten aufgrund ähnlicher SNP-Muster für prädiktive Aussagen bisher noch nicht infizierter Menschen getätigt und sozio-ökonomisch genutzt werden.

7. Allelfrequenz: Hier wird geprüft, wie die Verteilung von homozygot gesunden (O) zu heterozygoten (1) und homozygoten Covid19-Allelen (2) ist. Zur Allelfrequenzbestimmung werden die vollständigen Genomsequenzen von Individuen verwandt, die aus der gleichen Region stammen und dergleichen Ethnie angehören.

anfällig: (2) ist größer (0)
gleichrangig: 0 und 2 haben die gleichen Werte
protektiv: 0 ist größer 2
asymptomatisch: 1 ist größer 0 und 1 ist größer 2

8. IndyMed Gruppe: Dieser Wert nimmt 51 Werte (T1-T51) an und ergibt sich aus einer Zusammenfassung der Intervallwerte aus der Homozygotie von 381 SNPs und von TMG32 SNPs, der Verteilung von Effekt- und Nicht-Effekt-Allelen von 72 SNPs auf Chromosom 3 (Tabelle 2c) und der Verteilung der unterschiedlichen SNP-Muster bei gleicher oder ähnlicher Zahl eines TMG32 Schweregrades, siehe Tabelle 2b.

9. Covid-19 IndyMed-Klassifikation/Gruppe: Hier handelt es sich um eine erweitere Subklassifikation, die 4 Stufen beinhaltet (M0 bis M03). Hierzu wurden die 32 SNPs in zwei Blöcke a16 SNPs aufgeteilt, die 16 häufigsten SNPs bilden eine Gruppe und die letzten 16 häufigsten SNPS die zweite SNP Gruppe. Die zwei SNP Gruppen sind definiert, indem die ersten 16 SNPs in Abbildung 2b häufiger Effektallele aufweisen als die letzten 16 SNPs. M0 trifft zu, wenn in beiden Gruppen weniger als 5 SNPs mit Effektallel auftreten. M1 trifft zu, wenn 5 SNPs weniger in SNP-Gruppe 2 als in SNP-Gruppe 1 auftreten. M02 beschreibt den umgekehrten Fall. M03 bedeutet, beide SNP-Gruppen haben mehr als 5 SNPs in beiden SNP-Gruppen.

10. IndyMed Score: Dieser ist eine untergeordnete Klassifikation, die 32 TMG SNPs $p<10^{-5}$ mit Werten von 1-19 in Intervalle A, B, C, D und E zusammenfaßt. Es werden mit dem IndyMed Score ähnliche TMG-SNP Muster zusammengefasst und absteigend mit A, B, C, D, E klassifiziert.

11. Impfempfehlung: Diese ergibt sich aus der Anzahl der homozygoten Effektallelen 381 $p<10^{-5}$ mit den Werten von 1-5, bzw. aus der Anzahl der homozygoten Effektallele mit 32 $p<10^{-5}$ mit den Werten A, B, C, D, und E. Höchste Impfempfehlung ist 1a und am wichtigsten könnte die Aussage zur Impfempfehlung 5e sein. Hier sind die Menschen angesprochen, die mit größter Wahrscheinlichkeit keine oder nur geringe Symptome einer Covid-19 Erkrankung zeigen werden und daher Impfungen nicht notwendigerweise als erste Priorität gesehen werden müssen.

12. rs# in Tabelle 2b: Die Referenz-SNP-Codes (rs#) umfassen 32 SNPs mit "32$p<10^{-5}$ [tmg]" mit in der Unterspalte "2" verwendeten SNPs der *Single Nucleotide Polymorphismen Database (dbSNP)* des *National Center for Biotechnology Information (NCBI)* (https://www.ncbi.nlm.nih.gov/snp/). Die Ziffer "2" bedeutet, dass es sich um sich relevante mit Covid-19 assoziierte (d.h. mit einer Erkrankung verbundene) homozygote Allele handelt.

13. Gruppe: Hier handelt es sich um eine Klassifikation T1-T51 der Anmelderin, siehe auch Covid-19 IndyMed-Klassifikation in Tabelle 2a, bzw. siehe Tabelle 2c, die Gruppendefinitionen beschreibend.

14: Muster: Nach Ermittlung der 32 SNPs eines jeden neuen Probanden wird eine ihm ähnliche Genomstruktur aus den 2573 Genomen zugeordnet, siehe Tabelle 2b.
Für die 32 SNPs ergeben die Werte mit 0, 1 und 2. In Tabelle 2c sind die erstellten Wertetabellen der 2573 Genome mit ihren 51 Gruppen und 4 unterschiedlichen Covid-19 Untergruppen M0 bis M03 dargestellt, siehe Beschreibung der Covid-19 IndyMed-Klassifikation von zuvor. Es ergeben sich insgesamt 123 Gruppierungen mit den 32 SNPs und vier Untergruppen mit den Mustern M0, M01, M02, M03. Die Gruppierungen basieren auf der ursprünglichen Verteilung der 32 SNPs, gezeigt in Tabelle 2b. Aus PCA-Grafiken in Figur 1 und Figur 2 geht hervor, zu welchen der 381 SNPs basierten Kategorien der PCA Klassen G1 bis G15 das entsprechende individuelle 32 SNP Genom am ähnlichsten ist, siehe auch Figur 3. Aufgrund dieser Daten kann erfindungsgemäß dann eine Impfempfehlung erteilt werden und den Impfkandidaten unterschiedliche Impfprioritäten empfohlen werden.

15. PCA: Principal Component Analysis:
Hier werden die 381 SNPs mit den Werten (0) für homozygot anderes Allel, mit (1) für heterozygot und mit (2) für homozygot /schwere Covid19-Erkrankung mittels Principal Component Analyse angegeben. Die 1. Principal Component (x Achse) trennt die 2573 Genome nach der höchsten Varianz zwischen den Genomen, die 2. Principal Component die Genome nach der 2. höchsten Varianz.

16. Impfempfehlungen /Tabelle 2b ergeben sich wie beschrieben in
siehe 5. Schweregrad - 1000 (Tabelle 2a) und 6. TMG-32 (Schweregrad - TMG-32 in Tabelle 2a):

Tabelle 2c:
Für jede der 51 "IndyMed-Gruppen" T01-T51 ist angegeben, wie ein Patient in eine der Gruppen zugeordnet werden soll. Dazu sind 5 Zählungen nötig:

- Die Anzahl der homozygoten Effekt-SNPs innerhalb der 32 TMG-SNPs
- Die Anzahl der homozygoten Effekt-SNPs innerhalb der 381 WGS-SNPs
- Die Anzahl der homozygoten Nicht-Effekt-SNPs innerhalb der 72 Chr3-SNPs
- Die Anzahl der heterozygoten SNPs innerhalb der 72 Chr3-SNPs
- Die Anzahl der homozygoten Effekt-SNPs innerhalb der 72 Chr3-SNPs Jeder Gruppe ist für jeden dieser 5 Werte ein Intervall zugeordnet. Ein Patient gehört zu einer bestimmten Gruppe, sofern alle Werte im angegeben Bereich in einer Gruppe liegen. Ist kein Intervall angegeben, so ist dieser Wert nicht relevant für die Gruppenzuordnung.

Tabelle 3a:

Unter den 32 SNPs befinden sich zwei SNPs, die in menschlichen Populationen zwei Haplotypen des FYCO1-Gens unterscheiden. In Tabelle 3a gehören alle Haplotypen entweder zur SNP-Gruppe A, bzw. zur SNP-Gruppe B. Alle SNPs in Gruppe A verhalten sich wie rs13079478, bzw. in Gruppe B wie rs17214952.

Da dieses Protein FYCO1 mit NSP13 vom SARS-CoV2 interagiert, handelt es sich hier um einen SNP-Marker für schwere oder leichte SARS-CoV-Infektionen und assoziiert mit der Schwere einer Covid-19-Erkrankung.

Die SNPs des Gens FYC01 können als kausale Marker für die Effizienz der SARS-CoV2-Pathogenese dienen und diese haplotyp-spezifisch beschreiben, bzw. Aussagen zur Schwere einer Covid-19-Erkrankung definieren.

Tabelle 3b:
Mit dem SNP rs13079478 sind 27 weitere SNPs vergesellschaftet in SNP-Klasse A, das heißt, diese kommen gleichzeitig gemeinsam homozygot als Affektallel vor, gleiches gilt für SNP rs13079478 in der SNP Klasse B. Die Verteilung zwischen Homozygot 0 und Homozygot 2 ergibt, dass der SNP rs17214952 größtenteils an der Schwere bzw. an der Pathogenese des SARS-CoV2 beteiligt ist, während SNP rs13079478 protektiv sein sollte. Diese Aussagen sollten auch für die anderen SNPs in dieser Gruppe gelten. In mehr als 2000/2504 Fällen kommen die beiden Allele-Varianten SNP rs13079478 protektiv und SNP rs17214952 Covid-19 assoziiert gemeinsam vor.

Tabelle 4:

TMG-Probe: Hier werden anonymisierte Kunden der Firma Tellmegen kodiert, von denen die 32 bei TellmeGen gemessenen SNPs der TMG-Proben (Daten der Firma Tellmegen) gemessen wurden.

Ähnlichkeitsindex: Der Index errechnet sich aus 64 (2 x 32: da 32 SNPs betrachtet werden und jeder SNP max. eine Differenz von 2 haben kann) minus der Summe der absoluten Differenzen innerhalb dieser SNPs. D.h. ein Index von "0" bedeutet maximale Unähnlichkeit (jeder SNP ist anders), 64 bedeutet, dass alle 32 SNPs identisch sind.

32 SNP-basierte Impfempfehlung: Impf-Ziel ist es, Genome aus dem "1000 Genomes Project" zu identifizieren, die zu diesen 32 SNPs in TMG-Probe am ähnlichsten sind, d.h. die dort erstellten Impfempfehlungen sollten für diese Genome vergleichbar ähnlich sein wie die Impfempfehlungen zu den 2504 Genomen. Die Werte in Klammern geben die Anzahl ähnlicher Genome an.

Ähnlichste Patienten aus "1000 Genomes": Hier sind die Patienten aus dem "1000 Genomes Project" gelistet, deren Impfempfehlungen zuvor in Tabelle 2b gelistet wurden.

Für den Fachmann ist es nun offensichtlich, dass bei Vorliegen von klinischen Daten auch diese in die hier vorgestellte Erstellung eines Ähnlichkeitsindexes zu integrieren sein werden. Falls Covid-19-Rekonvaleszente im Geschlecht, Alter, Body Mass Index, bzw. ähnliche Komorbiditäten aufweisen sollten, dann ergeben sich Kohorten, die auch auf der Genomebene weitere Ähnlichkeiten im SNP-Muster aufweisen sollten, siehe Tabelle 1d.

Die Erstellung des Covid-19 basierten Ähnlicheitsmaßes beruht auf die Verwendung von 32 SNPs und deren Zuordnung zu weiteren Covid-19 assoziierten SNPs (n=381), deren Effektallele in komplett sequenzierten Genomen bestimmbar sind. Wie in Tabelle 4 zusammengefaßt und in Figur 3 visualisiert, kann jetzt dokumentiert werden, wie ähnlich die Genome von Covid-19-Schwerkrankten im gegenseitigen Vergleich sind, bzw. wie sie sich in 123 unterschiedliche Covid-19 Gruppierungen klassifizieren lassen. Für den Fachmann ist offensichtlich: Es können erweiterte

Ähnlichkeitsindices für diese Genompaare (SNP-Datensatz versus Komplettgenomen) erstellt werden, indem die vollständigen SNP-Datensätze mit den Komplettgenomen in den Bereichen verglichen werden, die aus klinischer Sicht mit den Komorbiditäten assoziiert sein sollten. Aus diesen Daten ergibt sich dann auch, ob eine ergänzende Sequenzierung erforderlich sein könnte, um eine noch genauere vergleichende Kartierung genomischer Marker erreichen zu können, bzw. um kausale Zusammenhänge zwischen Genom des Menschen und der Biologie des SARS-CoV2 Virus und seinen Varianten sich erschließen zu können, einschließlich Medikamentenentwicklung und des Monitorings von Impfantworten

Tabelle 1a: 381 SNPs zur Covid-19 Erkrankung

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 1:88993151 | rs75558547 | | | C | T | T | C | 4,49E-06 | 2,064E-06 | 2,064E-06 | |
| 1:89003816 | rs306313 | - | PKN2-AS1 | C | T | T | c | 7,569E-06 | 0,0000027 | 0,0000027 | X |
| 2:15724310 | rs2080811 | | | G | A | A | G | 1,115 E-06 | 0,0000113 | 1,115E-06 | |
| 2:15726493 | rs12619108 | | | G | A | A | G | 1,676E-06 | 1,767E-05 | 1,676E-06 | X |
| 2:15727121 | rs12619874 | | | G | C | C | G | 1,462E-06 | 2,627E-05 | 1,462E-06 | |
| 2:15733969 | rs62120724 | + | DDX1 | G | A | A | G | 9.728E-06 | 7.947E-05 | 9.728E-06 | |
| 2:15735648 | rs2302929 | + | DDX1 | T | C | T | C | 9,544E-06 | 0,0001304 | 9,544E-06 | |
| 2:15736697 | rs2032778 | + | DDX1 | G | A | A | G | 9,48E-06 | 8,039E-05 | 9,48E-06 | |
| 2:15744984 | rs79808866 | + | DDX1 | T | G | T | G | 8,588E-06 | 0,0005177 | 8,588E-06 | |
| 2:15746220 | rs41264195 | + | DDX1 | C | T | T | C | 8,49E-06 | 0,0005125 | 8,49E-06 | |
| 2:15749324 | rs3770462 | + | DDX1 | T | G | T | G | 6,737E-06 | 0,000398 | 6,737E-06 | |
| 2:15750131 | rs4668941 | + | DDX1 | T | G | T | G | 8,511E-06 | 0,0005172 | 8,511E-06 | |
| 2:15753279 | rs2302934 | + | DDX1 | C | G | C | G | 8,105E-06 | 0,0002959 | 8,105E-06 | |
| 2:15755622 | rs12616567 | + | DDX1 | A | C | A | C | 6,847E-06 | 0,0002742 | 6,847E-06 | |
| 2:15758612 | rs76880770 | + | DDX1 | G | A | A | G | 6,623E-06 | 0,0002374 | 6,623E-06 | |
| 2:15758798 | rs76860840 | + | DDX1 | C | G | C | G | 3,658E-06 | 0,0001792 | 3,658E-06 | |
| 2:15761966 | rs62122279 | + | DDX1 | T | C | T | C | 6,906E-06 | 7,039E-05 | 6,906E-06 | |
| 2:15764618 | rs4668946 | + | DDX1 | A | G | A | G | 4,715E-06 | 0,0000466 | 4,715E-06 | |
| 2:15765038 | rs12614308 | + | DDX1 | G | A | A | G | 4,715E-06 | 0,0000466 | 4,715E-06 | |
| 2:15767952 | rs807636 | + | DDX1 | C | T | T | C | 2,254E-06 | 0,0001264 | 2.254E-06 | |
| 2:15769607 | rs807635 | + | DDX1 | C | T | T | C | 2,852E-06 | 0,0001395 | 2,852E-06 | |
| 2:15778300 | rs807628 | | | A | G | A | G | 5,044E-06 | 5,338E-05 | 5,044E-06 | |
| 2:15779689 | rs807627 | | | T | C | T | C | 4,792E-06 | 4,741E-05 | 4,792E-06 | |
| 2:15782591 | rs807623 | | | C | T | T | C | 0,0000043 | 4,805E-05 | 0,0000043 | |
| 2:15782755 | rs807622 | | | A | G | A | G | 4,427E-06 | 4,892E-05 | 4,427E-06 | |
| 2:15783136 | rs807620 | | | C | T | T | C | 0,0000043 | 0,0000481 | 0,0000043 | |
| 2:15787060 | rs807618 | | | G | C | C | G | 6,02E-06 | 9,863E-05 | 6,02E-06 | |
| 2:15788502 | rs807615 | | | A | G | A | G | 5.506E-06 | 5,883 E-05 | 5.506E-06 | |
| 2:15789567 | rs807613 | | | C | T | T | C | 5,596E-06 | 6,638E-05 | 5,596E-06 | |
| 2:15789981 | rs807611 | | | G | T | T | G | 4,507E-06 | 7,008E-05 | 4,507E-06 | |
| 2:15790176 | rs12185539 | | | A | G | A | G | 4,309E-06 | 7,076E-05 | 4,309E-06 | |
| 2:15790180 | rs12185540 | | | A | G | A | G | 6,189E-06 | 8,739E-05 | 6,189E-06 | |
| 2:15790564 | rs807610 | | | C | G | C | G | 4,637E-06 | 0,0000736 | 4,637E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 2:15790578 | rs34779292 | | | A | AG | A | AG | 4,928E-06 | 0,0000798 | 4,928E-06 | |
| 2:15791347 | rs807607 | | | G | C | C | G | 5,519E-06 | 8,814E-05 | 5,519E-06 | |
| 2:15791726 | rs807606 | | | C | A | A | C | 7,554E-06 | 0,0001073 | 7,554E-06 | |
| 2:15792252 | rs807605 | | | G | A | A | G | 9,377E-06 | 0,0001437 | 9,377E-06 | |
| 2:15792508 | rs807604 | | | T | A | A | T | 7,641E-06 | 0,0001253 | 7,641E-06 | |
| 2:15792609 | rs807602 | | | | C | A | A | C | 8,601E-06 | 0,0001301 | 8,601E-06 |
| 2:15793114 | rs807599 | | | | T | C | T | C | 4,791E-06 | 0,0001096 | 4,791E-06 |
| 2:15793219 | rs62122281 | | | | G | C | C | G | 8,606E-06 | 0,0001374 | 8,606E-06 |
| 2:15793649 | rs807598 | | | | G | A | A | G | 6,744E-06 | 0,0001218 | 6,744E-06 |
| 2:15793904 | rs807597 | | | | T | C | T | C | 4,802E-06 | 0,0000978 | 4,802E-06 |
| 2:15794581 | rs807596 | | | | G | A | A | G | 9,695E-06 | 0,0001572 | 9,695E-06 |
| 2:15794674 | rs807595 | | | | C | T | T | C | 9,522E-06 | 0,0001563 | 9,522E-06 |
| 2:15795055 | rs807594 | | | | C | T | T | C | 9,435E-06 | 0,000154 | 9,435E-06 |
| 2:15797202 | rs807592 | | | | T | C | T | c | 5,82E-06 | 0,0004216 | 5,82E-06 |
| 2:15797925 | rs807590 | | | | G | T | T | G | 6,114E-06 | 0,0001338 | 6,114E-06 |
| 2:15798785 | rs807589 | | | | C | A | A | C | 3,476E-06 | 0,0001647 | 3,476E-06 |
| 2:15799980 | rs807587 | | | | C | G | C | G | 3.757E-06 | 0.000145 | 3.757E-06 |
| 2:15800128 | rs807586 | | | | T | C | T | C | 8,764E-06 | 0,0001547 | 8,764E-06 |
| 2:15800566 | rs807585 | | | | A | T | A | T | 8,961E-06 | 0,0001598 | 8,961E-06 |
| 2:15800721 | rs807583 | | | | G | A | A | G | 9,005E-06 | 0,0001601 | 9,005E-06 |
| 2:15802351 | rs807581 | | | | T | G | T | G | 4,511E-06 | 0,0006292 | 4,511E-06 |
| 2:15803584 | rs807579 | | | | T | C | T | C | 8,878E-06 | 0,0001566 | 8,878E-06 |
| 2:15803640 | rs807578 | | | | G | C | C | G | 8,811E-06 | 0,0001547 | 8,811E-06 |
| 2:15804394 | rs807577 | | | | C | G | C | G | 6,282E-06 | 0,0001579 | 6,282E-06 |
| 2:15804456 | rs807576 | | | | T | C | T | C | 6,145 E-06 | 0,0001605 | 6,145E-06 |
| 2:130468648 | rs35344266 | | | | A | T | A | T | 7,071E-05 | 1,133 E-07 | 1,133 E-07 |
| 2:130476707 | rs34508855 | | | | G | T | T | G | 0,00265 | 3,333E-06 | 3,333E-06 |
| 2:130488515 | rs12989715 | | | T | C | T | C | 0,00171 | 2,007E-06 | 2,007E-06 | |
| 3:8253480 | rs9882319 | | | A | G | A | G | 0,001249 | 6,945E-06 | 6,945E-06 | |
| 3:45841938 | rs35896106 | + | LOC107986082 | C | T | T | C | 1,733E-08 | 7,881E-09 | 7,881E-09 | |
| 3:45843242 | rs13071258 | + | LOC107986082 | G | A | A | G | 1,11E-09 | 2,285E-10 | 2,285E-10 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:45843315 | rs17763537 | + | LOC107986082 | C | T | T | C | 9,116E-10 | 2,558E-10 | 2,558E-10 | |
| 3:45844198 | rs34668658 | + | LOC107986082 | A | C | A | C | 1,437E-09 | 3,195E-10 | 3,195E-10 | |
| 3:45845748 | rs17078346 | + | LOC107986082 | A | C | A | c | 8,777E-07 | 2,284E-06 | 8,777E-07 | |
| 3:45846769 | rs17763742 | + | LOC107986082 | A | G | A | G | 1,194E-09 | 2,168E-10 | 2,168 E-10 | |
| 3:45847241 | rs17078348 | + | LOC107986082 | A | G | A | G | 1,063E-06 | 3,098E-06 | 1,063E-06 | X |
| 3:45850783 | rs72893671 | | | T | A | A | T | 1,546E-08 | 7,133 E-09 | 7,133E-09 | |
| 3:45859651 | rs17713054 | + | LOC107986083 | G | A | A | G | 3,742E-10 | 5,515E-11 | 5,515E-11 | |
| 3:45861932 | rs13078854 | + | LOC107986083 | G | A | A | G | 2.963E-10 | 4,91E-11 | 4.91E-11 | |
| 3:45862952 | rs71325088 | + | LOC107986083 | T | C | T | C | 2,937E-10 | 4,893E-11 | 4,893E-11 | |
| 3:45864732 | rs10490770 | - | LZTFL1 | T | C | T | C | 2,784E-10 | 4,585E-11 | 4,585E-11 | |
| 3:45867440 | rs35624553 | - | LZTFL1 | A | G | A | G | 3,365E-10 | 5,571E-11 | 5,571E-11 | |
| 3:45871908 | rs67959919 | - | LZTFL1 | G | A | A | G | 2,615E-10 | 4,69E-11 | 4,69E-11 | |
| 3:45880481 | rs35508621 | - | LZTFL1 | T | C | T | C | 2,308E-10 | 4,868E-11 | 4,868E-11 | |
| 3:45888690 | rs34288077 | - | LZTFL1 | A | G | A | G | 3,135E-10 | 5,965E-11 | 5,965E-11 | |
| 3:45889921 | rs35081325 | - | LZTFL1 | A | T | A | T | 2,488E-10 | 5,361E-11 | 5,361E-11 | |
| 3:45889949 | rs35731912 | - | LZTFL1 | C | T | T | C | 1,915E-10 | 3,984E-11 | 3,984E-11 | |
| 3:45899651 | rs34326463 | - | LZTFL1 | A | G | A | G | 3,455E-10 | 8,194E-11 | 8,194E-11 | |
| 3:45900634 | rs76374459 | - | LZTFL1 | G | C | C | G | 6,758E-10 | 7,533E-10 | 6,758E-10 | |
| 3:45901089 | rs73064425 | - | LZTFL1 | C | T | T | C | 2,942E-10 | 7,504E-11 | 7,504E-11 | |
| 3:45908116 | rs13081482 | - | LZTFL1 | A | T | A | T | 3,781E-10 | 1,167E-10 | 1,167E-10 | |
| 3:45908514 | rs35652899 | - | LZTFL1 | C | G | C | G | 2,595E-10 | 8,204E-11 | 8,204E-11 | |
| 3:45909024 | rs35044562 | - | LZTFL1 | A | G | A | G | 4,806E-10 | 1,25E-10 | 1,25E-10 | |
| 3:45909528 | rs73064431 | - | LZTFL1 | C | T | T | C | 2,268E-09 | 1,44E-09 | 1,44E-09 | |
| 3:45909644 | rs13092887 | - | LZTFL1 | C | A | A | C | 1,759E-09 | 1,457E-09 | 1,457E-09 | |
| 3:45910870 | rs2191031 | - | LZTFL1 | G | A | A | G | 6,804E-06 | 0,0001038 | 6,804E-06 | |
| 3:45916786 | rs34901975 | - | LZTFL1 | G | A | A | G | 3,003E-07 | 1,216E-06 | 3,003E-07 | |
| 3:45918215 | rs17764831 | - | LZTFL1 | G | A | A | G | 4,108E-07 | 1,481E-06 | 4,108E-07 | |
| 3:45920298 | rs34518147 | - | LZTFL1 | T | C | T | C | 4,352E-07 | 1,528E-06 | 4,352E-07 | |
| 3:45926043 | rs74586549 | - | LZTFL1 | C | T | T | C | 7,335E-06 | 2,573E-05 | 7,335E-06 | |
| 3:45928769 | rs34338823 | - | LZTFL1 | G | A | A | G | 3.568E-07 | 1,261E-06 | 3,568E-07 | |
| 3:45932407 | rs71325091 | - | LZTFL1 | G | A | A | G | 4,728E-07 | 1,499E-06 | 4,728E-07 | |
| 3:45937833 | rs3774641 | - | LZTFL1 | G | T | T | G | 5,947E-06 | 0,0001061 | 5,947E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:45939016 | rs17764980 | - | LZTFL1 | G | A | A | G | 5,517E-07 | 1,882E-06 | 5,517E-07 | |
| 3:45939802 | rs17714101 | - | LZTFL1 | G | A | A | G | 5,407E-07 | 1,886E-06 | 5,407E-07 | |
| 3:45945132 | rs17714228 | +;;- | LZTFL1 | A | C | A | C | 5,646E-07 | 1,93E-06 | 5,646E-07 | |
| 3:45947018 | rs71325092 | - | LZTFL1 | A | G | A | G | 6,468E-07 | 2,294E-06 | 6,468E-07 | |
| 3:45951647 | rs35280891 | - | LZTFL1 | G | A | A | G | 5,624E-06 | 1,443E-05 | 5,624E-06 | |
| 3:45954339 | rs34068335 | - | LZTFL1 | C | T | T | C | 2,244E-06 | 6,023E-06 | 2,244E-06 | |
| 3:45960420 | rs1994491 | - | FYCO1 | G | C | C | G | 5,981E-06 | 4,638E-06 | 4,638E-06 | |
| 3:45960646 | rs1994492 | - | FYCO1 | T | C | T | C | 3,364E-06 | 7,802E-06 | 3,364E-06 | |
| 3:45960700 | rs1994493 | - | FYCO1 | C | T | T | C | 3,364E-06 | 7,644E-06 | 3.364E-06 | |
| 3:45962603 | rs75928798 | - | FYCO1 | A | G | A | G | 4,304E-06 | 1,114E-05 | 4,304E-06 | |
| 3:45968043 | rs2373087 | - | FYCO1 | T | G | T | G | 3,244E-06 | 7,599E-06 | 3,244E-06 | |
| 3:45970391 | rs35831747 | - | FYCO1 | G | A | A | G | 3,072E-06 | 7,729E-06 | 3,072E-06 | |
| 3:45970944 | rs13099120 | - | FYCO1 | C | G | C | G | 2,822E-06 | 5,982E-06 | 2,822E-06 | |
| 3:45974092 | rs35477280 | - | FYCO1 | G | A | A | G | 2,938E-06 | 7,582E-06 | 2,938E-06 | |
| 3:45975443 | rs13066516 | - | FYCO1 | C | T | T | C | 3,285E-06 | 7,688E-06 | 3,285E-06 | |
| 3:45976662 | rs77902290 | - | FYCO1 | C | T | T | C | 3,086E-06 | 7,51E-06 | 3,086E-06 | |
| 3:45981171 | rs2171531 | +;;- | CXCR6;; FYCO1 | C | T | T | C | 4,609E-06 | 1,041E-05 | 4,609E-06 | |
| 3:45983476 | rs55920693 | +;;- | CXCR6;; FYCO1 | T | A | A | T | 4,661E-06 | 0,0000108 | 4,661E-06 | |
| 3:45985347 | rs6785091 | +;;- | CXCR6;; FYCO1 | C | G | C | G | 4,913E-06 | 6,643E-06 | 4,913E-06 | |
| 3:45989873 | rs56332428 | +;;- | CXCR6;; FYCO1 | C | T | T | C | 8,317E-06 | 6.324E-06 | 6,324E-06 | |
| 3:45989921 | rs71325095 | +;;- | CXCR6;; FYCO1 | G | T | T | G | 7,851E-06 | 5,997E-06 | 5,997E-06 | |
| 3:45993645 | rs35501575 | - | FYCO1 | C | T | T | C | 4,552E-06 | 0,0000105 | 4,552E-06 | |
| 3:45995748 | rs34381952 | - | FYCO1 | T | C | T | C | 8,108E-06 | 6,101E-06 | 6,101E-06 | |
| 3:45996501 | rs4388012 | - | FYCO1 | A | G | A | G | 8,066E-06 | 6,101E-06 | 6,101E-06 | |
| 3:45997263 | rs41289616 | - | FYCO1 | T | C | T | C | 8,039E-06 | 6,101E-06 | 6,101E-06 | |
| 3:45999209 | rs34000569 | - | FYCO1 | A | G | A | G | 4,819E-06 | 1,083E-05 | 4,819E-06 | |
| 3:46000728 | rs34324101 | - | FYCO1 | T | G | T | G | 4,802E-06 | 1,086E-05 | 4,802E-06 | |
| 3:46000870 | rs13069079 | - | FYCO1 | G | A | A | G | 4,844E-06 | 1,088E-05 | 4,844E-06 | |
| 3:46001227 | rs76597151 | - | FYCO1 | G | A | A | G | 8,018E-06 | 6,065E-06 | 6,065E-06 | |
| 3:46001367 | rs34849862 | - | FYCO1 | C | A | A | C | 4,844E-06 | 1,088E-05 | 4,844E-06 | |
| 3:46001720 | rs35827997 | - | FYCO1 | T | G | T | G | 8,039E-06 | 6,065E-06 | 6,065E-06 | |
| 3:46003496 | rs35209528 | - | FYCO1 | T | C | T | C | 4,642E-06 | 1,061E-05 | 4,642E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:46003537 | rs36039366 | - | FYCO1 | G | A | A | G | 7,761E-06 | 5,967E-06 | 5,967E-06 | |
| 3:46006239 | rs35855315 | - | FYCO1 | A | G | A | G | 5,036E-06 | 4,71E-06 | 4,71E-06 | |
| 3:46006269 | rs35525815 | - | FYCO1 | C | T | T | C | 6,012E-06 | 5,176E-06 | 5,176E-06 | |
| 3:46006341 | rs17280623 | - | FYCO1 | C | G | C | G | 5,085E-06 | 4,713E-06 | 4,713E-06 | |
| 3:46007488 | rs13078739 | - | FYCO1 | G | A | A | G | 5,015E-06 | 4,769E-06 | 4,769E-06 | |
| 3:46007823 | rs13079478 | - | FYCO1 | G | T | T | G | 3,01E-06 | 8,545E-06 | 3,01E-06 | X |
| 3:46007825 | rs13059238 | - | FYCO1 | T | C | T | C | 5,015E-06 | 4,769E-06 | 4,769E-06 | |
| 3:46008087 | rs13079869 | - | FYCO1 | G | A | A | G | 3,01E-06 | 8,545E-06 | 3,01E-06 | |
| 3:46009487 | rs33910087 | - | FYCO1 | G | A | A | G | 2,967E-06 | 8,514E-06 | 2,967E-06 | |
| 3:46010007 | rs13071283 | - | FYCO1 | T | C | T | C | 4.923E-06 | 4.739E-06 | 4.739E-06 | |
| 3:46011436 | rs17214952 | - | FYCO1 | A | G | A | G | 4,808E-06 | 4,707E-06 | 4,707E-06 | X |
| 3:46012279 | rs17215008 | - | FYCO1 | T | C | T | C | 2,884E-06 | 8,335E-06 | 2,884E-06 | |
| 3:46012391 | rs71325098 | - | FYCO1 | A | G | A | G | 2,877E-06 | 8,37E-06 | 2,877E-06 | |
| 3:46013832 | rs71325100 | - | FYCO1 | C | T | T | C | 2,55E-06 | 7,604E-06 | 2,55E-06 | |
| 3:46014545 | rs41289622 | - | FYCO1 | T | G | T | G | 2,518E-06 | 7,559E-06 | 2,518E-06 | |
| 3:46015569 | rs71325101 | - | FYCO1 | T | C | T | C | 4,524E-06 | 4,335E-06 | 4,335E-06 | |
| 3:46015933 | rs71325102 | - | FYCO1 | T | C | T | C | 4, 753 E-06 | 4,619E-06 | 4,619E-06 | |
| 3:46018344 | rs13066062 | - | FYCO1 | G | A | A | G | 2,809E-06 | 8,183E-06 | 2,809E-06 | |
| 3:46022833 | rs36122610 | - | FYCO1 | G | A | A | G | 2,175E-06 | 6,059E-06 | 2,175E-06 | |
| 3:46024529 | rs34442130 | - | FYCO1 | T | A | A | T | 2,161E-06 | 6,023E-06 | 2,161E-06 | |
| 3:46025048 | rs13075758 | - | FYCO1 | G | A | A | G | 2,189E-06 | 6,095E=06 | 2,189E-06 | |
| 3:46035097 | rs17330872 | - | FYCO1 | A | G | A | G | 1,741E-06 | 4,783E-06 | 1,741E-06 | |
| 3:46036521 | rs71327003 | - | FYCO1 | C | T | T | C | 2,026E-06 | 5,715 E-06 | 2,026E-06 | |
| 3:46039060 | rs36023124 | - | FYCO1 | G | C | C | G | 2,013E-06 | 6,15E-06 | 2,013E-06 | |
| 3:46039868 | rs71615438 | + | LOC105377063 | A | G | A | G | 2,041E-06 | 6,15E-06 | 2,041E-06 | |
| 3:46041837 | rs34754340 | + | LOC105377063 | C | T | T | C | 1,938E-06 | 6,071E-06 | 1.938E-06 | |
| 3:46046459 | rs34836513 | | | G | A | A | G | 1,963E-06 | 6,128E-06 | 1,963E-06 | |
| 3:46047658 | rs34283240 | | | G | C | C | G | 2,095E-06 | 6,375E-06 | 2,095E-06 | |
| 3:46049764 | rs76281521 | | | G | A | A | G | 2,951E-06 | 6,971E-06 | 2,951E-06 | |
| 3:46049765 | rs13433997 | | | T | C | T | C | 1,189 E-05 | 5,861E-06 | 5,861E-06 | |
| 3:46049827 | rs67937868 | | | G | T | T | G | 1,79E-06 | 4,419E-06 | 1,79E-06 | |
| 3:46051702 | rs55875328 | | | G | A | A | G | 1.287E-06 | 2.142E-06 | 1.287E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:46051774 | rs67200151 | | | G | A | A | G | 1,282E-06 | 2,135E-06 | 1,282E-06 | |
| 3:46052263 | rs13089855 | | | G | C | C | G | 1,034E-06 | 1,982E-06 | 1,034E-06 | |
| 3:46052800 | rs34493660 | | | G | A | A | G | 1,044E-06 | 2,042E-06 | 1,044E-06 | |
| 3:46053581 | rs3851346 | | | A | T | A | T | 1,705E-06 | 3,207E-06 | 1,705E-06 | |
| 3:46053968 | rs35883205 | | | A | G | A | G | 1,427E-06 | 0,000003 | 1,427E-06 | |
| 3:46054156 | rs61650989 | | | G | T | T | G | 1,068E-06 | 2,046E-06 | 1,068E-06 | |
| 3:46055250 | rs35669129 | | | G | A | A | G | 1,081E-06 | 2,029E-06 | 1,081E-06 | |
| 3:46055716 | rs13081151 | | | G | A | A | G | 1,38E-06 | 2,631E-06 | 1,38E-06 | |
| 3:46055909 | rs13081213 | | | C | T | T | C | 1,084E-06 | 2,033E-06 | 1,084E-06 | |
| 3:46056162 | rs34168660 | | | G | A | A | G | 1,057E-06 | 1,985 E-06 | 1,057E-06 | |
| 3:46056173 | rs35751180 | | | C | T | T | C | 1,417E-06 | 3.55E-06 | 1,417E-06 | |
| 3:46056911 | rs4362758 | | | T | A | A | T | 1,665E-06 | 2,889E-06 | 1,665E-06 | |
| 3:46057867 | rs73833520 | | | A | G | A | G | 1,69E-06 | 2,907E-06 | 1,69E-06 | |
| 3:46058908 | rs71327006 | - | XCR1 | G | A | A | G | 2,768E-06 | 4,748E-06 | 2,768E-06 | |
| 3:46059249 | rs71327007 | - | XCR1 | C | T | T | C | 2,51E-06 | 4,303E-06 | 2,51E-06 | |
| 3:46059484 | rs35454877 | - | XCR1 | T | C | T | c | 1.69E-06 | 2,975E-06 | 1,69E-06 | |
| 3:46061218 | rs35334665 | - | XCR1 | T | C | T | C | 2,699E-06 | 4,571E-06 | 2,699E-06 | |
| 3:46061304 | rs36040135 | - | XCR1 | A | G | A | G | 1,69E-06 | 2,907E-06 | 1,69E-06 | |
| 3:46061840 | rs13074382 | - | XCR1 | A | G | A | G | 1,69E-06 | 2,925E-06 | 1,69E-06 | |
| 3:46061997 | rs13097556 | - | XCR1 | T | C | T | C | 1,69E-06 | 2,907E-06 | 1,69E-06 | |
| 3:46063329 | rs2230322 | - | XCR1 | T | C | T | C | 1,679E-06 | 2,957E-06 | 1,679E-06 | |
| 3:46063753 | rs876668 | - | XCR1 | T | C | T | C | 1.679E-06 | 2.957E-06 | 1,679E-06 | |
| 3:46064618 | rs60019065 | - | XCR1 | G | C | C | G | 1,616E-06 | 2,848E-06 | 1,616E-06 | |
| 3:46064626 | rs71327009 | - | XCR1 | C | G | C | G | 3,188E-06 | 5,204E-06 | 3,188E-06 | |
| 3:46065763 | rs35930050 | - | XCR1 | G | A | A | G | 1,665E-06 | 2,956E-06 | 1,665E-06 | |
| 3:46065963 | rs34584867 | - | XCR1 | G | A | A | G | 1,665E-06 | 2,92E-06 | 1,665E-06 | |
| 3:46067507 | rs35161099 | - | XCR1 | T | G | T | G | 2,828E-06 | 4,591E-06 | 2,828E-06 | |
| 3:46068473 | rs35159820 | - | XCR1 | A | G | A | G | 1,762E-06 | 2,856E-06 | 1,762E-06 | |
| 3:46068835 | rs71327010 | - | XCR1 | G | T | T | G | 2,828E-06 | 4,572E-06 | 2,828E-06 | |
| 3:46069573 | rs13090194 | - | XCR1 | A | C | A | C | 1,772E-06 | 2,892E-06 | 1,772E-06 | |
| 3:46070337 | rs59166269 | - | XCR1 | C | G | C | G | 1,772E-06 | 2,892E-06 | 1,772E-06 | |
| 3:46070353 | rs57437758 | - | XCR1 | A | C | A | C | 1,772E-06 | 2,892E-06 | 1,772E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:46071081 | rs34562820 | - | XCR1 | G | A | A | G | 2,874E-06 | 4,599E-06 | 2,874E-06 | |
| 3:46071763 | rs13082697 | - | XCR1 | T | C | T | C | 1,782 E-06 | 2,892E-06 | 1,782E-06 | |
| 3:46071868 | rs13060287 | - | XCR1 | A | G | A | G | 1,789E-06 | 2,91E-06 | 1,789E-06 | |
| 3:46072519 | rs34619093 | - | XCR1 | G | A | A | G | 2,885E-06 | 4,583E-06 | 2,885E-06 | |
| 3:46072630 | rs68087193 | - | XCR1 | C | T | T | C | 1,761E-06 | 2,874E-06 | 1,761E-06 | |
| 3:46072704 | rs2102055 | - | XCR1 | G | A | A | G | 2,825E-06 | 5,472E-06 | 2,825E-06 | |
| 3:46072768 | rs2102056 | - | XCR1 | G | A | A | G | 1,784E-06 | 2,894E-06 | 1,784E-06 | |
| 3:46072839 | rs2088690 | - | XCR1 | C | T | T | C | 1,784E-06 | 2,889E-06 | 1,784E-06 | |
| 3:46073314 | rs34774687 | - | XCR1 | T | C | T | C | 1,795 E-06 | 2,91E-06 | 1,795E-06 | |
| 3:46073923 | rs35814488 | - | XCR1 | A | G | A | G | 1,772E-06 | 2,917E-06 | 1,772E-06 | |
| 3:46074711 | rs6790866 | - | XCR1 | G | A | A | G | 1,795E-06 | 2,91E-06 | 1,795E-06 | |
| 3:46074858 | rs6791016 | - | XCR1 | G | A | A | G | 0,0000018 | 2,916E-06 | 0,0000018 | |
| 3:46075090 | rs6780028 | - | XCR1 | A | C | A | C | 0,0000018 | 2,916E-06 | 0,0000018 | |
| 3:46077332 | rs2088692 | - | XCR1 | A | G | A | G | 1,959E-06 | 2,831E-06 | 1,959E-06 | |
| 3:46081306 | rs34438204 | - | XCR1 | T | C | T | C | 3,125E-06 | 4,555E-06 | 3,125E-06 | |
| 3:46082481 | rs13063033 | - | XCR1 | G | A | A | G | 4,115E-06 | 6,275E-06 | 4,115E-06 | |
| 3:46087992 | rs34679077 | - | XCR1 | G | A | A | G | 3,125E-06 | 4,583E-06 | 3,125E-06 | |
| 3:46094063 | rs34863575 | - | XCR1 | A | G | A | G | 4,532E-06 | 8,374E-06 | 4,532E-06 | |
| 3:46095104 | rs35772789 | - | XCR1 | A | G | A | G | 4,52E-06 | 8,332E-06 | 4,52E-06 | |
| 3:46096043 | rs34340587 | - | XCR1 | G | A | A | G | 4,631E-06 | 8,395E-06 | 4,631E-06 | |
| 3:46096798 | rs77399277 | - | XCR1 | T | C | T | C | 4,25E-06 | 6,889E-06 | 4,25E-06 | |
| 3:46098526 | rs34718164 | - | XCR1 | C | G | C | G | 4,25E-06 | 6,93E-06 | 4,25E-06 | |
| 3:46099154 | rs13096741 | - | XCR1 | C | T | T | C | 4,273E-06 | 6,984E-06 | 4,273E-06 | |
| 3:46100138 | rs35420565 | - | XCR1 | C | G | C | G | 4,262E-06 | 6,889E-06 | 4,262E-06 | |
| 3:46100491 | rs34047915 | - | XCR1 | C | T | T | C | 4,288E-06 | 6,93E-06 | 4,288E-06 | |
| 3:46101466 | rs71327014 | - | XCR1 | C | T | T | C | 4,25E-06 | 7,037E-06 | 4,25E-06 | |
| 3:46102173 | rs71327015 | - | XCR1 | G | C | C | G | 3,566E-06 | 5,743E-06 | 3,566E-06 | |
| 3:46103680 | rs34127208 | - | XCR1 | G | T | T | G | 4,314E-06 | 7,012E-06 | 4,314E-06 | |
| 3:46107498 | rs35516580 | - | XCR1 | A | G | A | G | 4,131E-06 | 6,907E-06 | 4,131E-06 | |
| 3:46107601 | rs34766614 | - | XCR1 | A | G | A | G | 6,994E-06 | 1,325E-05 | 6,994E-06 | |
| 3:46111213 | rs71327017 | - | XCR1 | T | C | T | C | 3,798E-06 | 5,99E-06 | 3,798E-06 | |
| 3:46116010 | rs13072267 | - | XCR1 | T | A | A | T | 4,567E-06 | 6,812E-06 | 4,567E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 3:46117096 | rs36057789 | - | XCR1 | A | G | A | G | 4,363E-06 | 6,72E-06 | 4,363E-06 | |
| 3:46117960 | rs76647202 | - | XCR1 | A | G | A | G | 4,595E-06 | 6,852E-06 | 4.595E-06 | |
| 3:46120517 | rs71327021 | - | XCR1 | A | G | A | G | 4,611E-06 | 6,852E-06 | 4,611E-06 | |
| 3:46123043 | rs13091868 | - | XCR1 | A | G | A | G | 4,718E-06 | 6,933E-06 | 4,718E-06 | |
| 3:46123055 | rs13092030 | - | XCR1 | C | T | T | C | 4,66E-06 | 6,812E-06 | 4,66E-06 | |
| 3:46123333 | rs9838450 | - | XCR1 | A | C | A | C | 2,889E-05 | 9,117E-06 | 9,117E-06 | X |
| 3:46124418 | rs17215981 | - | XCR1 | A | G | A | G | 4,473E-06 | 6,458E-06 | 4,473E-06 | |
| 3:46127557 | rs13089554 | - | XCR1 | G | A | A | G | 4,611E-06 | 6,779E-06 | 4,611E-06 | |
| 3:46128542 | rs13095717 | - | XCR1 | G | A | A | G | 4,583E-06 | 6,743E-06 | 4,583E-06 | |
| 3:46128709 | rs13075528 | - | XCR1 | T | C | T | C | 4,63E-06 | 6,761E-06 | 4,63E-06 | |
| 3:46128713 | rs13095602 | - | XCR1 | A | G | A | G | 4,599E-06 | 6,76E-06 | 4,599E-06 | |
| 3:46131225 | rs71327023 | | | C | G | C | G | 5,188E-06 | 7,264E-06 | 5,188E-06 | |
| 3:46135690 | rs13063527 | | | G | A | A | G | 4,509E-06 | 6,651E-06 | 4,509E-06 | |
| 3:46136438 | rs13068570 | | | A | G | A | G | 4,608E-06 | 6,73E-06 | 4,608E-06 | |
| 3:46138107 | rs1491950 | | | G | A | A | G | 5,545E-06 | 7,524E-06 | 5,545E-06 | |
| 3:46138455 | rs4373103 | | | G | A | A | G | 5,417E-06 | 7,336E-06 | 5,417E-06 | |
| 3:46138842 | rs34492478 | | | A | T | A | T | 4,945E-06 | 6,17E-06 | 4,945E-06 | |
| 3:46140073 | rs71327024 | | | G | T | T | G | 5,371E-06 | 7,361E-06 | 5,371E-06 | |
| 3:46141844 | rs1491951 | | | G | A | A | G | 7,297E-06 | 8,542E-06 | 7,297E-06 | |
| 3:46142464 | rs34460587 | | | C | T | T | C | 4,931E-06 | 6,714E-06 | 4,931E-06 | |
| 3:46143187 | rs34452002 | | | C | T | T | C | 4,949E-06 | 6,731E-06 | 4,949E-06 | |
| 3:46144981 | rs34093271 | | | G | T | T | G | 4,893E-06 | 6,753E-06 | 4,893E-06 | |
| 3:46146314 | rs13093179 | | | G | T | T | G | 4,914E-06 | 6,714E-06 | 4,914E-06 | |
| 3:46147504 | rs71327025 | | | A | G | A | G | 4.962E-06 | 6.794E-06 | 4.962E-06 | |
| 3:46154272 | rs35539222 | | | T | C | T | C | 4,962E-06 | 6,754E-06 | 4,962E-06 | |
| 3:46154457 | rs35110864 | | | G | A | A | G | 4,992E-06 | 6,794E-06 | 4,992E-06 | |
| 3:46170897 | rs35581648 | + | LOC105377067 | G | T | T | G | 0,0000106 | 9,394E-06 | 9,394E-06 | |
| 4:180344873 | rs1455662 | | | A | G | A | G | 9,599E-06 | 0,003648 | 9,599E-06 | |
| 4:180346230 | rs1455663 | | | A | G | A | G | 9,868E-06 | 0,002934 | 9,868E-06 | |
| 4:180346325 | rs1455664 | | | T | C | T | C | 9,774E-06 | 0,002902 | 9,774E-06 | |
| 6:20314719 | rs911360 | + | LOC101928573 | C | T | T | C | 4,085E-06 | 0,00157 | 4,085E-06 | |
| 6:20314890 | rs6930922 | + | LOC101928573 | C | T | T | C | 4,856E-06 | 0,00168 | 4,856E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 6:43845981 | rs13190738 | + | LOC107986598 | C | T | T | C | 0,0000475 | 6,513E-06 | 6,513E-06 | |
| 6:44507083 | rs55839388 | | | G | A | A | G | 0,006246 | 6,113E-06 | 6,113E-06 | |
| 6:44517310 | rs60328892 | - | LOC105375075 | A | T | A | T | 0,002609 | 2,227E-06 | 2.227E-06 | |
| 6:44518128 | fs73434199 | - | LOC105375075 | G | A | A | G | 0,002591 | 2,173E-06 | 2,173E-06 | |
| 6:44518763 | rs73434201 | - | LOC105375075 | C | T | T | C | 0,002921 | 4,421E-06 | 4,421E-06 | |
| 7:123006727 | rs12706520 | | | G | A | A | G | 9,632E-06 | 0,0001935 | 9,632E-06 | |
| 7:158003981 | rs4506155 | - | PTPRN2 | G | A | A | G | 8,619E-06 | 0,002905 | 8,619E-06 | |
| 7:158007923 | rs4595081 | - | PTPRN2 | G | T | T | G | 6,62E-06 | 0,002431 | 6,62E-06 | |
| 7:158015457 | rs6948273 | - | PTPRN2 | T | C | T | C | 6,989E-06 | 0,002823 | 6,989E-06 | |
| 7:158016777 | rs10156036 | - | PTPRN2 | A | C | A | C | 4,963E-06 | 0,00214 | 4,963E-06 | |
| 7:158020147 | rs6970487 | - | PTPRN2 | A | G | A | G | 4,889E-06 | 0,002275 | 4,889E-06 | |
| 7:158020284 | rs6974918 | - | PTPRN2 | T | G | T | G | 5,549E-06 | 0,002458 | 5,549E-06 | |
| 7:158020287 | rs6951312 | - | PTPRN2 | C | A | A | C | 5,475E-06 | 0,002443 | 5,475E-06 | |
| 7:158020293 | rs6974924 | - | PTPRN2 | T | C | T | C | 5.475E-06 | 0,002448 | 5,475E-06 | X |
| 7:158022103 | rs2335848 | - | PTPRN2 | A | G | A | G | 5,355E-06 | 0,0025 | 5,355E-06 | |
| 7:158023589 | rs6963124 | - | PTPRN2 | T | A | A | T | 5,051E-06 | 0,002578 | 5,051E-06 | |
| 7:158023815 | rs3965316 | - | PTPRN2 | C | T | T | C | 6,025E-06 | 0,002919 | 6,025E-06 | |
| 7:158025026 | rs10276960 | - | PTPRN2 | T | C | T | C | 5,934E-06 | 0,002463 | 5,934E-06 | |
| 7:158025121 | rs10273930 | - | PTPRN2 | A | G | A | G | 7,002E-06 | 0,002755 | 7,002E-06 | |
| 7:158025328 | rs6949642 | - | PTPRN2 | C | A | A | C | 8,017E-06 | 0,002972 | 8,017E-06 | |
| 8:22044324 | rs117184073 | + | BMP1 | G | C | C | G | 4,272E-05 | 7,367E-06 | 7,367E-06 | |
| 8:22050435 | rs76670680 | + | BMP1 | C | T | T | C | 4,785E-05 | 6,428E-06 | 6,428E-06 | |
| 8:80568972 | rs174S2856 | + | STMN2 | T | G | T | G | 0,0005882 | 9,681E-06 | 9,681E-06 | |
| 8:80644873 | rs2440652 | | | G | T | T | G | 0,0002323 | 5,879E-06 | 5,879E-06 | |
| 8:80649860 | rs2461037 | | | G | A | A | G | 0,0003125 | 9,569E-06 | 9,569E-06 | |
| 8:122818621 | rs10088118 | | | G | C | C | G | 6,792E-06 | 0,001085 | 6,792E-06 | |
| 8:122828601 | rs4389947 | | | G | A | A | G | 4,907E-06 | 0,0007286 | 4,907E-06 | |
| 8:122848563 | rs10087789 | | | C | G | C | G | 6,995E-06 | 0,001053 | 6,995E-06 | |
| 8:122848829 | rs10091098 | | | G | A | A | G | 4,554E-06 | 0,000828 | 4,554E-06 | X |
| 8:122858181 | rs13263570 | | | G | A | A | G | 8.495E-06 | 0,001263 | 8,495E-06 | |
| 8:126952467 | rs28730361 | - | LINC00861 | T | A | A | T | 8,113E-06 | 0,002401 | 8,113E-06 | |
| 9:136137065 | rs687621 | | | A | G | A | G | 7,632E-08 | 5,887E-07 | 7,632E-08 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 9:136137106 | rs687289 | | | G | A | A | G | 8,138E-08 | 6,234E-07 | 8,138E-08 | X |
| 9:136139265 | rs657152 | | | C | A | A | C | 4,951E-08 | 5,354E-07 | 4,951E-08 | X |
| 9:136142217 | rs644234 | | | T | G | T | G | 5,293E-08 | 6,683E-07 | 5,293E-08 | X |
| 9:136142355 | rs643434 | | | G | A | A | G | 5.331E-08 | 6.73E-07 | 5,331E-08 | |
| 9:136143212 | rs544873 | | | G | A | A | G | 5,293E-08 | 6,657E-07 | 5,293E-08 | X |
| 9:136143372 | rs545971 | | | C | T | T | C | 9,583E-08 | 8,158E-07 | 9,583E-08 | X |
| 9:136143442 | rs612169 | | | A | G | A | G | 1,083E-07 | 9,819E-07 | 1,083E-07 | |
| 9:136144427 | rs8176663 | | | T | C | T | C | 1,05E-07 | 9,257E-07 | 1,05E-07 | x |
| 9:136144873 | rs491626 | | | C | T | T | C | 1,051E-07 | 9,278E-07 | 1,051E-07 | |
| 9:136144960 | rs492488 | | | G | A | A | G | 1,02E-07 | 9,029E-07 | 1,02E-07 | |
| 9:136144994 | rs493246 | | | G | A | A | G | 1,011E-07 | 9,029E-07 | 1,011E-07 | x |
| 9:136145118 | rs494242 | | | C | T | T | C | 5,392E-08 | 6,749E-07 | 5,392E-08 | |
| 9:136145240 | rs495203 | | | C | T | T | C | 8,521E-08 | 7,096E-07 | 8,521E-08 | x |
| 9:136145471 | rs582118 | | | A | G | A | G | 1,019E-07 | 9,293E-07 | 1,019E-07 | |
| 9:136145484 | rs582094 | | | A | T | A | T | 1,036E-07 | 9,164E-07 | 1,036E-07 | X |
| 9:136145974 | rs2769071 | | | A | G | A | G | 1,173E-07 | 9,223E-07 | 1,173E-07 | X |
| 9:136146046 | rs677355 | | | G | A | A | G | 1,123E-07 | 8,91E-07 | 1,123E-07 | X |
| 9:136146077 | rs676996 | | | T | G | T | G | 1,168E-07 | 9,223E-07 | 1,168E-07 | X |
| 9:136146227 | rs676457 | | | A | T | A | T | 1,208E-07 | 9,324E-07 | 1,208E-07 | |
| 9:136146431 | rs527210 | | | C | T | T | C | 1,357E-07 | 1,04E-06 | 1,357E-07 | |
| 9:136146597 | rs550057 | | | C | T | T | C | 1,861E-06 | 3,115E-06 | 1,861E-06 | x |
| 9:136146664 | rs674302 | | | T | A | A | T | 1,257E-07 | 9,513E-07 | 1,257E-07 | X |
| 9:136147160 | rs554833 | | | C | T | T | C | 1,128E-07 | 9,117E-07 | 1,128E-07 | x |
| 9:136147553 | rs660340 | | | A | G | A | G | 7,974E-07 | 1,001E-06 | 7,974E-07 | |
| 9:136147702 | rs581107 | | | C | T | T | C | 7,874E-07 | 1,002E-06 | 7,874E-07 | |
| 9:136147823 | rs659104 | | | T | G | T | G | 7,974E-07 | 9.99E-07 | 7,974E-07 | |
| 9:136148000 | rs647800 | | | A | G | A | G | 2,411E-07 | 4,347E-07 | 2,411E-07 | |
| 9:136148035 | rs473533 | | | T | C | T | C | 7,887E-07 | 1,014E-06 | 7,887E-07 | |
| 9:136148231 | rs475419 | | | C | T | T | C | 6,321E-07 | 1,157E-06 | 6,321E-07 | |
| 9:136148368 | rs476410 | | | G | C | C | G | 8,956E-07 | 1,199E-06 | 8,956E-07 | |
| 9:136148409 | rs645982 | | | A | G | A | G | 9,022E-07 | 1,197E-06 | 9,022E-07 | |
| 9:136148647 | rs500498 | | | T | C | T | C | 9,061E-07 | 1,195E-06 | 9,061E-07 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 9:136149229 | rs505922 | | | T | C | T | C | 3,205E-07 | 2,186E-06 | 3,205E-07 | X |
| 9:136149500 | rs529565 | | | T | C | T | c | 2,435E-07 | 1,515E-06 | 2,435E-07 | X |
| 9:136155444 | rs633862 | | | C | T | T | C | 8,789E-07 | 8,022E-07 | 8,022E-07 | |
| 11:745884 | rs7936322 | + | TALDO1 | C | T | T | C | 0,005853 | 3,129E-06 | 3,129E-06 | |
| 11:749452 | rs3901233 | + | TALDO1 | T | A | A | T | 0,005907 | 6.029E-06 | 6.029E-06 | |
| 11:750849 | rs7945912 | + | TALDO1 | A | T | A | T | 0,004788 | 2,827E-06 | 2,827E-06 | |
| 11:762441 | rs4963163 | + | TALDO1 | A | C | A | C | 0,003827 | 1,168E-06 | 1,168E-06 | |
| 11:770007 | rs7940065 | - | GATD1 | C | T | T | C | 0,00319 | 1,986E-06 | 1,986E-06 | |
| 11:771034 | rs7930569 | - | GATD1 | A | G | A | G | 0,003295 | 2.005E-06 | 2,005E-06 | |
| 11:771716 | rs3934992 | - | GATD1 | A | C | A | C | 0,0004561 | 8,725E-07 | 8,725E-07 | |
| 11:772490 | rs12224894 | - | GATD1 | G | A | A | G | 0,003299 | 3,655E-06 | 3,655E-06 | |
| 11:772701 | rs12223324 | - | GATD1 | A | G | A | G | 0,003746 | 2,362E-06 | 2,362E-06 | X |
| 11:774568 | rs7951926 | - | GATD1 | C | T | T | C | 0,003622 | 4,018E-06 | 4,018E-06 | |
| 11:774608 | rs7952569 | - | GATD1 | G | A | A | G | 0,003959 | 2,486E-06 | 2,486E-06 | |
| 11:774982 | rs7952095 | - | GATD1 | C | A | A | C | 0,004077 | 1,378E-06 | 1,378E-06 | |
| 11:775275 | rs7942564 | - | GATD1 | A | G | A | G | 0,003966 | 2,446E-06 | 2,446E-06 | |
| 11:775423 | rs7948070 | - | GATD1 | C | T | T | C | 0,003843 | 2,365E-06 | 2,365E-06 | |
| 11:775651 | rs7948539 | + | GATD1 | T | A | A | T | 0,004143 | 2,474E-06 | 2,474E-06 | |
| 11:5691474 | rs12796811 | - | TRIMS | C | G | C | G | 3,622E-06 | 2,295E-05 | 3,622E-06 | |
| 11:87561583 | rs78243302 | - | RAB38 | G | A | A | G | 3,958E-05 | 8,042E-06 | 8,042E-06 | |
| 12:62422567 | rs10506436 | - | TAFA2 | T | A | A | T | 2,841E-06 | 0,001144 | 2,841E-06 | |
| 12:62435946 | rs10784278 | - | TAFA2 | T | A | A | T | 2,318E-06 | 0,00112 | 2,318E-06 | |
| 12:62437797 | rs10784279 | - | TAFA2 | C | T | T | C | 3,502E-06 | 0,001848 | 3,502E-06 | |
| 12:62459512 | rs9739956 | - | TAFA2 | T | C | T | C | 9,449E-07 | 0,0002553 | 9,449E-07 | |
| 12:62463979 | rs10877786 | - | TAFA2 | T | G | T | G | 6,887E-07 | 0,0002475 | 6,887E-07 | |
| 12:62465910 | rs11174294 | - | TAFA2 | C | T | T | C | 1,586E-06 | 0,000275 | 1,586E-06 | |
| 12:62466762 | rs10877787 | - | TAFA2 | A | G | A | G | 1.58E-06 | 0,0002722 | 1,58E-06 | |
| 12:62469458 | rs10747951 | - | TAFA2 | C | T | T | C | 1,578E-06 | 0,0002721 | 1,578E-06 | |
| 12:62470897 | rs10506432 | - | TAFA2 | A | C | A | C | 1,58E-06 | 0,0002725 | 1,58E-06 | |
| 12:62471458 | rs11174296 | - | TAFA2 | C | G | C | G | 1,746E-06 | 0,0003511 | 1,746E-06 | |
| 12:62472571 | rs9738190 | - | TAFA2 | G | A | A | G | 1,51E-06 | 0,0002515 | 1,51E-06 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | IsTMG |
|---|---|---|---|---|---|---|---|---|---|---|---|
| 12: 127179868 | rs12814401 | + | LINC02824 | A | T | A | T | 0,0009084 | 4,068E-06 | 4,068E-06 | |
| 13:63021420 | rs9592188 | | | C | T | T | C | 0,003426 | 8,184 E-06 | 8,184E-06 | |
| 13:63042159 | rs9598460 | | | G | T | T | G | 0,003607 | 5,984E-06 | 5,984E-06 | |
| 14:32422346 | rs11627388 | | | A | C | A | C | 3,19E-06 | 0,0003814 | 3,19E-06 | |
| 14:32422746 | rs7152677 | | | G | C | C | G | 1,524E-06 | 0,0005329 | 1,524E-06 | |
| 15:74420226 | rs12904268 | + | ISLR2 | T | G | T | G | 0,0001292 | 7,137E-06 | 7,137E-06 | |
| 15:74421010 | rs2279379 | + | ISLR2 | C | G | C | G | 0,0001479 | 6,254E-06 | 6,254E-06 | |
| 15:74434196 | rs8038527 | + | ISLR2 | A | G | A | G | 0,0001943 | 0,0000072 | 0,0000072 | X |
| 16:74936031 | rs2059266 | - | WDR59 | A | G | A | G | 4,007E-06 | 0,0001579 | 4,007E-06 | |
| 16:82334366 | rs7197718 | | | G | A | A | G | 6,486E-06 | 1,633E-05 | 6,486E-06 | |
| 16:82338406 | rs114093749 | | | C | T | T | C | 3,859E-06 | 1,726E-05 | 3,859E-06 | |
| 16:82378755 | rs73606907 | | | C | G | C | G | 9,287E-06 | 2,586E-06 | 2,586E-06 | |
| 16:82382762 | rs79407403 | | | C | T | T | C | 9,774E-06 | 6,164E-06 | 6,164E-06 | |
| 16:82384779 | rs73606932 | | | C | G | C | G | 1,342E-05 | 3,763E-06 | 3,763E-06 | |
| 16:82386997 | rs79289897 | | | A | G | A | G | 1,352E-05 | 9,004E-06 | 9,004E-06 | |
| 16:82391321 | rs7198537 | | | T | C | T | C | 9, 843 E-06 | 1,747E-06 | 1,747E-06 | |
| 16:82391520 | rs79300914 | | | T | C | T | C | 4, 305 E-06 | 1,567E-06 | 1,567E-06 | |
| 16:82393449 | rs60395048 | | | A | G | A | G | 5,367E-06 | 1,S88E-06 | 1,588E-06 | |
| 16:82395196 | rs16957124 | | | T | C | T | C | 5,257E-06 | 1,596E-06 | 1,596E-06 | |
| 16:82395484 | rs76668502 | | | G | A | A | G | 5,256E-06 | 1,591E-06 | 1,591E-06 | |
| 16:82395530 | rs16957126 | | | C | T | T | C | 5,256E-06 | 1,591E-06 | 1,591E-06 | |
| 16:82395695 | rs77595609 | | | C | T | T | C | 5,203E-06 | 1,594E-06 | 1,594E-06 | |
| 16:82396097 | rs4569267 | | | G | T | T | G | 7,785E-06 | 1,298E-05 | 7,785E-06 | X |
| 17:10376558 | rs117438562 | + | MYHAS | G | T | T | G | 7,494E-06 | 0,04264 | 7,494E-06 | |
| 18:3724602 | rs13381043 | - | DLGAP1 | G | A | A | G | 1,133E-07 | 1,822E-05 | 1,133E-07 | |
| 18:3725189 | rs4797120 | - | DLGAP1 | G | C | C | G | 1,906E-06 | 1,865E-05 | 1,906E-06 | |
| 18:6725321 | rs76187417 | | | T | G | T | G | 0,0005568 | 9,503E-06 | 9,503E-06 | X |
| 18:22624707 | rs56132597 | - | LOC107985141 | G | A | A | G | 8,935E-06 | 0,0001125 | 8,935E-06 | X |
| 19:4717672 | rs12610495 | - | DPP9 | A | G | A | G | 5,204E-06 | 7,161E-06 | 5,204E-06 | X |
| 22:28128191 | rs134130 | | | T | C | T | C | 0,0001091 | 9,145E-06 | 9,145E-06 | |

Tabelle 1b: 32 SNPs zur Covid-19 Erkrankung

| coord | DBSNP.ID | dbsnp gene strand | dbsnp gene symbol | dbsnp Ref | dbsnp Alt | COV EA | COV OA | Pval main | Pval_corr sex_age | Pval min |
|---|---|---|---|---|---|---|---|---|---|---|
| 9: 136139265 | rs657152 | | | C | A | A | C | 4,951E-08 | 5,354E-07 | 4,95E-08 |
| 9: 136142217 | rs644234 | | | T | G | T | G | 5,293E-08 | 6,683E-07 | 5,29E-08 |
| 9: 136143212 | rs544873 | | | G | A | A | G | 5,293E-08 | 6,657E-07 | 5,29E-08 |
| 9: 136137065 | rs687621 | | | A | G | A | G | 7,632E-08 | 5,887E-07 | 7,63E-08 |
| 9: 136137106 | rs687289 | | | G | A | A | G | 8,138E-08 | 6,234E-07 | 8,14E-08 |
| 9: 136145240 | rs495203 | | | C | T | T | C | 8,521E-08 | 7,096E-07 | 8,52E-08 |
| 9: 136143372 | rs545971 | | | C | T | T | C | 9,583E-08 | 8,158E-07 | 9,58E-08 |
| 9: 136144994 | rs493246 | | | G | A | A | G | 1,011E-07 | 9,029E-07 | 1,01E-07 |
| 9: 136145484 | rs582094 | | | A | T | A | T | 1,036E-07 | 9,164E-07 | 1,04E-07 |
| 9: 136144427 | rs8176663 | | | T | C | T | C | 1,05E-07 | 9,257E-07 | 1,05E-07 |
| 9: 136146046 | rs677355 | | | G | A | A | G | 1,123E-07 | 8,91E-07 | 1,12E-07 |
| 9: 136147160 | rs554833 | | | C | T | T | C | 1,128E-07 | 9,117E-07 | 1,13E-07 |
| 9: 136146077 | rs676996 | | | T | G | T | G | 1,168E-07 | 9,223E-07 | 1,17E-07 |
| 9: 136145974 | rs2769071 | | | A | G | A | G | 1,173E-07 | 9,223E-07 | 1,17E-07 |
| 9: 136146664 | rs674302 | | | T | A | A | T | 1,257E-07 | 9,513E-07 | 1.26E-07 |
| 9: 136149500 | rs529565 | | | T | C | T | C | 2,435E-07 | 1,515E-06 | 2,44E-07 |
| 9: 136149229 | rs505922 | | | T | C | T | C | 3,205E-07 | 2,186E-06 | 3,21E-07 |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min |
|---|---|---|---|---|---|---|---|---|---|---|
| 3:45847241 | rs17078348 | + | LOC107986082 | A | G | A | G | 1,063E-06 | 3,098E-06 | 1,06E-06 |
| 2:15726493 | rs12619108 | | | G | A | A | G | 1,676E-06 | 1,767E-05 | 1,68E-06 |
| 9: 136146597 | rs550057 | | | C | T | T | C | 1,861E-06 | 3,115E-06 | 1.86E-06 |
| 11:772701 | rs12223324 | - | GATD1 | A | G | A | G | 0,003746 | 2,362E-06 | 2,36E-06 |
| 1:89003816 | rs306313 | - | PKN2-AS1 | C | T | T | C | 7,569E-06 | 0,0000027 | 2,7E-06 |
| 3:46007823 | rs13079478 | - | FYCO1 | G | T | T | G | 3,01E-Q6 | 8,545E-06 | 3,01E-06 |
| 8: 122848829 | rs10091098 | | | G | A | A | G | 4,554E-06 | 0,000828 | 4,55E-06 |
| 3:46011436 | rs17214952 | - | FYCO1 | A | G | A | G | 4,808E-06 | 4,707E-06 | 4,71E-06 |
| 19:4717672 | rs12610495 | - | DPP9 | A | G | A | G | 5,204E-06 | 7,161E-06 | 5,2E-06 |
| 7: 158020293 | rs6974924 | - | PTPRN2 | T | C | T | C | 5,475E-06 | 0,002448 | 5,48E-06 |
| 15: 74434196 | rs8038527 | + | ISLR2 | A | G | A | G | 0,0001943 | 0,0000072 | 7,2E-06 |
| 16: 82396097 | rs4569267 | | | G | T | T | G | 7,785E-06 | 1,298E-05 | 7,79E-06 |
| 18: 22624707 | rs56132597 | - | LOC107985141 | G | A | A | G | 8,935E-06 | 0,0001125 | 8,94E-06 |
| 3:46123333 | rs9838450 | - | XCR1 | A | C | A | C | 2,889E-05 | 9,117E-06 | 9,12E-06 |
| 18:6725321 | rs76187417 | | | T | G | T | G | 0,0005568 | 9,503E-06 | 9,5E-06 |

Tabelle 1c: SNPs-SARS-CoV2 assoziiert

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 1:1733219 | rs10907185 | - | GNB1 | A | G | A | G | 0,001566 | 0,04648 | 0,001566 | X |
| 1:1804302 | rs3855951 | - | GNB1 | C | T | T | C | 0,02259 | 0,04027 | 0,02259 | |
| 1:40547950 | rs3131660 | - | PPT1 | C | T | T | C | 0,264 | 0,04536 | 0,04536 | |
| 1:53235599 | rs34517448 | + | ZYG11B | T | C | T | C | 0,01726 | 0,01787 | 0,01726 | X |
| 1:87477786 | rs4233338 | + | HS2ST1 | G | C | A | G | 0,02225 | 0,003759 | 0,003759 | |
| 1:87477786 | rs4233338 | + | HS2ST1 | G | C | A | G | 0,02225 | 0,003759 | 0,003759 | |
| 1:87520970 | rs4610985 | + | HS2ST1 | A | T | A | G | 0,01026 | 0,002744 | 0,002744 | |
| 1:87520970 | rs4610985 | + | HS2ST1 | A | T | A | G | 0,01026 | 0,002744 | 0,002744 | |
| 1:87562677 | rs191000784 | + | HS2ST1 | A | G | A | G | 0,2222 | 0,01123 | 0,01123 | X |
| 1:97279692 | rs273885 | + | PTBP2 | C | T | T | C | 0,02499 | 0,0713 | 0,02499 | X |
| 1: 101382999 | rs76861341 | + | SLC30A7 | A | C | A | C | 0,008887 | 0,0007756 | 0,0007756 | X |
| 1: 109487904 | rs67008022 | - | CLCC1 | G | A | A | G | 0,3102 | 0,04193 | 0,04193 | X |
| 1: 150466925 | rs12116970 | + | TARS2 | C | T | T | C | 0,05642 | 0,04679 | 0,04679 | X |
| 1: 150466925 | rs12116970 | + | TARS2 | C | T | T | C | 0,05642 | 0,04679 | 0,04679 | X |
| 1: 179995975 | rs7521948 | + | CEP350 | G | A | A | G | 0,1858 | 0,0355 | 0,0355 | |
| 1: 180037987 | rs6692394 | + | CEP350 | A | G | A | G | 0,352 | 0,02488 | 0,02488 | |
| 1: 184662181 | rs1061508 | - | EDEM3 | C | T | T | C | 0,01743 | 0,1876 | 0,01743 | X |
| 1: 184662181 | rs1061508 | - | EDEM3 | C | T | T | C | 0,01743 | 0,1876 | 0,01743 | X |
| 1: 184673317 | rs12079454 | - | EDEM3 | G | A | A | G | 0,1646 | 0,04395 | 0,04395 | X |
| 1: 184677464 | rs3736757 | - | EDEM3 | G | A | A | G | 0,002425 | 0,04749 | 0,002425 | |
| 1: 184693647 | rs67337751 | - | EDEM3 | G | A | A | G | 0,03893 | 0,03624 | 0,03624 | X |
| 1: 184721398 | rs60400761 | - | EDEM3 | A | G | A | G | 0,04723 | 0,08381 | 0,04723 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 1: 220721754 | rs73093505 | + | MARK1 | T | G | T | G | 0,01143 | 0,06838 | 0,01143 | X |
| 1: 220776140 | rs76309804 | + | MARK1 | G | A | A | G | 0,003433 | 0,03372 | 0,003433 | X |
| 1: 220801974 | rs10495150 | + | MARK1 | A | C | A | C | 0,002439 | 0,0063 | 0,002439 | X |
| 1: 236380077 | rs6428948 | - | ERO1B | G | A | A | G | 0,02667 | 0,1293 | 0,02667 | X |
| 1: 236418610 | rs79779531 | - | ERO1B | C | T | T | C | 0,1811 | 0,009158 | 0,009158 | X |
| 2:10710730 | rs9287719 | - | NOL10 | C | T | T | C | 0,0845 | 0,01873 | 0,01873 | X |
| 2:10758859 | rs6714413 | - | NOL10 | C | T | T | C | 0,03487 | 0,009626 | 0,009626 | X |
| 2:10758859 | rs6714413 | - | NOL10 | C | T | T | C | 0,03487 | 0,009626 | 0,009626 | X |
| 2:10800347 | rs72777326 | - | NOL10 | T | C | T | C | 0,03265 | 0,3161 | 0,03265 | X |
| 2:10827157 | rs11684157 | - | NOL10 | C | G | A | C | 0,09598 | 0,02842 | 0,02842 | |
| 2:10827157 | rs11684157 | - | NOL10 | C | G | A | C | 0,09598 | 0,02842 | 0,02842 | |
| 2:29371387 | rs3099559 | + | CLIP4 | C | G | A | C | 0,1406 | 0,02947 | 0,02947 | X |
| 2:29371387 | rs3099559 | + | CLIP4 | C | G | A | C | 0,1406 | 0,02947 | 0,02947 | X |
| 2:29372369 | rs3112916 | + | CLIP4 | A | T | A | G | 0,1806 | 0,04036 | 0,04036 | |
| 2:29372369 | rs3112916 | + | CLIP4 | A | T | A | G | 0,1806 | 0,04036 | 0,04036 | |
| 2:29380922 | rs116686919 | + | CLIP4 | A | C | A | C | 0,1182 | 0,02112 | 0,02112 | X |
| 2:29381291 | rs4666170 | + | CLIP4 | A | T | A | G | 0,3238 | 0,03503 | 0,03503 | X |
| 2:29381291 | rs4666170 | + | CLIP4 | A | T | A | G | 0,3238 | 0,03503 | 0,03503 | X |
| 2:55321517 | rs34656234 | - | RTN4 | G | A | A | G | 0,002485 | 0,01254 | 0,002485 | X |
| 2:65299298 | rs2723088 | + | CEP68 | C | A | A | C | 0,005872 | 0,2051 | 0,005872 | |
| 2:65299300 | rs2723089 | + | CEP68 | G | C | C | G | 0,005838 | 0,2052 | 0,005838 | X |
| 2:65328694 | rs10195328 | - | RAB1A | G | C | A | G | 0,01781 | 0,1997 | 0,01781 | X |
| 2:65328694 | rs10195328 | - | RAB1A | G | C | A | G | 0,01781 | 0,1997 | 0,01781 | X |
| 2:65338759 | rs62140404 | - | RAB1A | A | C | A | C | 0,0304 | 0,182 | 0,0304 | X |
| 2:85787341 | rs12714145 | - | GGCX | C | T | T | C | 0,24 | 0,02935 | 0,02935 | X |
| 2: 153605828 | rs2346553 | + | ARL6IP6 | A | C | A | C | 0,01029 | 0,07135 | 0,01029 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 2: 178296772 | rs6720483 | + | AGPS | A | G | A | G | 0,009301 | 0,2796 | 0,009301 | X |
| 2: 178358281 | rs12469386 | + | AGPS | C | T | T | C | 0,04363 | 0,4125 | 0,04363 | |
| 2: 233441702 | rs74453786 | + | EIF4E2 | A | C | A | C | 0,0346 | 0,1018 | 0,0346 | X |
| 2: 233660846 | rs2305138 | + | GIGYF2 | G | A | A | G | 0,01188 | 0,1446 | 0,01188 | X |
| 2: 233722093 | rs3738904 | + | GIGYF2 | C | T | T | C | 0,009808 | 0,1093 | 0,009808 | X |
| 3:45962752 | rs7652331 | - | FYCO1 | T | C | T | C | 0,1173 | 0,04034 | 0,04034 | X |
| 3:45962752 | rs7652331 | - | FYCO1 | T | C | T | C | 0,1173 | 0,04034 | 0,04034 | X |
| 3:46005478 | rs1545985 | - | FYCO1 | G | A | A | G | 0,0989 | 0,02478 | 0,02478 | X |
| 3:46007823 | rs13079478 | - | FYCO1 | G | T | T | G | 3,01E-06 | 8,545E-06 | 3,01E-06 | X |
| 3:46009487 | rs33910087 | - | FYCO1 | G | T | A | G | 2,967E-06 | 8,514E-06 | 2,967E-06 | |
| 3:46009487 | rs33910087 | - | FYCO1 | G | T | A | G | 2,967E-06 | 8,514E-06 | 2,967E-06 | |
| 3:46010077 | rs4683158 | - | FYCO1 | C | T | T | C | 0,01484 | 0,1439 | 0,01484 | |
| 3:46010077 | rs4683158 | - | FYCO1 | C | T | T | C | 0,01484 | 0,1439 | 0,01484 | |
| 3:46010077 | rs4683158 | - | FYCO1 | C | T | T | C | 0,01484 | 0,1439 | 0,01484 | |
| 3:46011436 | rs17214952 | - | FYCO1 | A | G | A | G | 4,808E-06 | 4,707E-06 | 4,707E-06 | x |
| 3:48787219 | rs6791542 | - | PRKAR2A | A | G | A | G | 0.04798 | 0,06668 | 0,04798 | |
| 3:48821036 | rs34037363 | - | PRKAR2A | C | T | T | C | 0,06033 | 0,009487 | 0,009487 | x |
| 3:48833347 | rs7647812 | - | PRKAR2A | G | A | A | G | 0,04254 | 0,04804 | 0,04254 | x |
| 3:49064110 | rs11706052 | - | IMPDH2 | A | G | A | G | 0,06667 | 0,01277 | 0,01277 | X |
| 3:49406095 | rs11716445 | - | RHOA | G | A | A | G | 0,1418 | 0,01466 | 0,01466 | x |
| 3: 100112271 | rs80073654 | - | TOMM70 | G | A | A | G | 0,24 | 0,0296 | 0,0296 | X |
| 3: 179419433 | rs114691754 | + | USP13 | C | T | T | C | 0,1323 | 0,02067 | 0,02067 | x |
| 4:6126549 | rs16838298 | - | JAKMIP1 | G | T | T | G | 0,2897 | 0,02483 | 0,02483 | X |
| 4:7061952 | rs114453087 | - | GRPEL1 | T | G | T | G | 0,03089 | 0,1893 | 0,03089 | x |
| 4:42042876 | rs12510574 | + | SLC30A9 | G | A | A | G | 0,3874 | 0,04279 | 0,04279 | x |
| 4:56820412 | rs77591659 | + | CEP135 | G | A | A | G | 0,00862 | 0,0769 | 0,00862 | x |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 4:57362827 | rs10015634 | + | SRP72 | G | A | A | G | 0,04294 | 0,03693 | 0,03693 | |
| 4:77040424 | rs6532203 | - | NUP54 | T | C | T | C | 0,02921 | 0,4229 | 0,02921 | |
| 4:77044258 | rs6532208 | - | NUP54 | C | T | T | C | 0,04079 | 0,4601 | 0,04079 | |
| 4:99197839 | rs10003496 | + | RAP1GDS1 | A | G | A | G | 0,01566 | 0,04515 | 0,01566 | |
| 4:99231913 | rs7655622 | + | RAP1GDS1 | C | T | T | C | 0,029 | 0,0686 | 0,029 | X |
| 4:99237439 | rs10516428 | + | RAP1GDS1 | T | G | T | G | 0,2273 | 0,03908 | 0,03908 | X |
| 4:99302166 | rs17027526 | + | RAP1GDS1 | T | C | T | C | 0,01198 | 0,04037 | 0,01198 | |
| 5:60291420 | rs116034902 | + | NDUFAF2 | A | G | A | G | 0,0453 | 0,07236 | 0,0453 | X |
| 5:64265857 | rs78248692 | + | CWC27 | G | T | T | G | 0,1109 | 0,01974 | 0,01974 | X |
| 5:71515616 | rs6831 | - | MRP527 | A | G | A | G | 0,0002578 | 0,00264 | 0,0002578 | |
| 5:71519664 | rs3209157 | - | MRPS27 | C | T | T | C | 0,07316 | 0,03662 | 0,03662 | X |
| 5:71521551 | rs6889892 | - | MRP527 | A | G | A | G | 0,002383 | 0.01568 | 0.002383 | X |
| 5:71525636 | rs72647244 | - | MRPS27 | C | T | T | C | 0,004199 | 0,06543 | 0,004199 | X |
| 5:77574504 | rs62364494 | - | AP3B1 | G | A | A | G | 0,01634 | 0,3568 | 0,01634 | X |
| 5: 112196595 | rs28460815 | + | SRP19 | A | G | A | G | 0,04279 | 0,03834 | 0,03834 | X |
| 5: 112235182 | rs72787387 | - | REEPS | T | C | T | C | 0,3493 | 0,04805 | 0,04805 | X |
| 5: 127609633 | rs2291628 | - | FBN2 | G | A | A | G | 0,135 | 0,03842 | 0,03842 | X |
| 5: 127637529 | rs12523609 | - | FBN2 | G | A | A | G | 0,1518 | 0,0299 | 0,0299 | X |
| 5: 127699375 | rs374748 | - | FBN2 | G | A | A | G | 0,4167 | 0,04429 | 0,04429 | |
| 5: 127744469 | rs28763954 | - | FBN2 | G | A | A | G | 0,1217 | 0,02167 | 0,02167 | X |
| 5: 127770805 | rs331079 | - | FBN2 | G | C | C | G | 0,05496 | 0,02077 | 0,02077 | X |
| 5: 138406078 | rs7701116 | - | SIL1 | C | T | T | C | 0,01466 | 0,06824 | 0,01466 | X |
| 5: 138410922 | rs7724108 | - | SIL1 | G | A | A | G | 0,01342 | 0,09443 | 0,01342 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 5: 138461547 | rs11951084 | - | SIL1 | C | T | T | C | 0,0318 | 0,09143 | 0,0318 | X |
| 5: 138468344 | rs1549203 | - | SIL1 | C | T | T | C | 0,01382 | 0,09886 | 0,01382 | |
| 5: 138486543 | rs11958425 | - | SIL1 | T | C | T | C | 0,02297 | 0,1629 | 0,02297 | X |
| 5: 151177514 | rs72800300 | + | G3BP1 | A | G | A | G | 0,1211 | 0,0436 | 0,0436 | X |
| 5: 151186145 | rs116200464 | + | G3BP1 | A | T | A | G | 0,04767 | 0,2347 | 0,04767 | X |
| 5: 151186145 | rs116200464 | + | G3BP1 | A | T | A | G | 0,04767 | 0,2347 | 0,04767 | X |
| 5: 162938043 | rs17061784 | + | MAT2B | A | G | A | G | 0,03155 | 0,466 | 0,03155 | X |
| 5: 172313211 | rs12652763 | + | ERGIC1 | G | A | A | G | 0,03588 | 0,02435 | 0,02435 | X |
| 5: 172344808 | rs72814105 | + | ERGIC1 | G | A | A | G | 0,0461 | 0,104 | 0,0461 | X |
| 5: 172370094 | rs79931282 | + | ERGIC1 | A | G | A | G | 0,11 | 0,02709 | 0,02709 | X |
| 6:3086920 | rs9392453 | + | RIPK1 | C | T | T | C | 0,03274 | 0,02827 | 0,02827 | |
| 6:3088987 | rs17513153 | + | RIPK1 | G | A | A | G | 0,08729 | 0,008471 | 0,008471 | X |
| 7:16730542 | rs17169592 | + | BZW2 | C | T | T | C | 0,007909 | 0,05678 | 0,007909 | X |
| 7:16740554 | rs1459558 | + | BZW2 | A | G | A | G | 0,05429 | 0,01601 | 0,01601 | X |
| 7:34997422 | rs1649229 | - | DPY19L1 | T | G | T | G | 0,008052 | 0,07127 | 0,008052 | X |
| 7:35020843 | rs328902 | - | DPY19L1 | C | T | T | C | 0,0789 | 0,01703 | 0,01703 | X |
| 7:35059967 | rs34949146 | - | DPY19L1 | A | C | A | C | 0,1095 | 0,03629 | 0,03629 | X |
| 7:35071808 | rs79787993 | - | DPY19L1 | G | T | T | G | 0,03832 | 0,07128 | 0,03832 | X |
| 7:39687771 | rs117718957 | + | RALA | C | T | T | C | 0,09641 | 0,03178 | 0,03178 | X |
| 7:39687771 | rs117718957 | + | RALA | C | T | T | C | 0,09641 | 0,03178 | 0,03178 | X |
| 7:75570557 | rs78906654 | + | POR | C | T | T | C | 0,6484 | 0,03751 | 0,03751 | X |
| 7:75589903 | rs2868177 | + | POR | A | G | A | G | 0,02988 | 0,09614 | 0,02988 | X |
| 7:75590808 | rs2868180 | + | POR | T | C | T | C | 0,02808 | 0,1065 | 0,02808 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 7:75595682 | rs7804806 | + | POR | A | G | A | G | 0,02099 | 0,1172 | 0,02099 | X |
| 7:91718874 | rs56295910 | + | AKAP9 | A | T | A | G | 0,01673 | 0,05369 | 0,01673 | X |
| 7:91718874 | rs56295910 | + | AKAP9 | A | T | A | G | 0,01673 | 0,05369 | 0,01673 | X |
| 7: 102936527 | rs3757508 | + | PMPCB | G | T | T | G | 0,658 | 0,03131 | 0,03131 | X |
| 7: 106686448 | rs2395833 | + | PRKAR2B | G | T | T | G | 0,7241 | 0,03004 | 0,03004 | X |
| 7: 106799997 | rs257376 | + | PRKAR2B | G | A | A | G | 0,4612 | 0,02357 | 0,02357 | |
| 8:63940364 | rs16930092 | - | GGH | T | C | T | C | 0,04198 | 0,08282 | 0,04198 | X |
| 8:74861484 | rs7830335 | - | ELOC | G | A | A | G | 0,02743 | 0,005514 | 0,005514 | X |
| 8:74878696 | rs12547746 | - | ELOC | T | C | T | C | 0,03129 | 0,05982 | 0,03129 | X |
| 8:74878696 | rs12547746 | - | ELOC | T | C | T | C | 0,03129 | 0,05982 | 0,03129 | X |
| 8:74884255 | rs4738406 | - | ELOC | C | T | T | C | 0,05227 | 0,04179 | 0,04179 | |
| 8:96164643 | rs73264571 | + | PLEKHF2 | C | A | A | C | 0,02695 | 0,7735 | 0,02695 | X |
| 9:33924136 | rs116990828 | - | UBAP2 | G | A | A | G | 0,01547 | 0,1123 | 0,01547 | X |
| 9:35087090 | rs72722967 | - | PIGO | G | A | A | G | 0,4632 | 0,0474 | 0,0474 | X |
| 9:84228593 | rs7865343 | - | TLE1 | G | A | A | G | 0,03467 | 0,01874 | 0,01874 | X |
| 9:84230322 | rs7028704 | - | TLE1 | T | C | T | C | 0,03382 | 0,009377 | 0,009377 | X |
| 9: 102846630 | rs16918878 | - | ERP44 | C | T | T | C | 0,04092 | 0,04644 | 0,04092 | X |
| 9: 123205698 | rs117351867 | - | CDK5RAP2 | C | T | T | C | 0,03872 | 0,001908 | 0,001908 | X |
| 9: 123878315 | rs34680486 | + | CNTRL | A | G | A | G | 0,02485 | 0,03721 | 0,02485 | X |
| 9: 124112339 | rs10985215 | - | STOM | G | A | A | G | 0,01063 | 0,02618 | 0,01063 | X |
| 9: 132585058 | rs2296793 | - | TOR1A | G | A | A | G | 0,04489 | 0,1809 | 0,04489 | |
| 9: 133569200 | rs148203698 | + | EXOSC2 | C | T | C | G | 0,0353 | 0,1041 | 0,0353 | |
| 9: 133569200 | rs148203698 | + | EXOSC2 | C | T | C | G | 0,0353 | 0,1041 | 0,0353 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 10:944496 | rs7077992 | - | LARP4B | A | C | A | C | 0,2826 | 0,03813 | 0,03813 | |
| 10:954957 | rs6560859 | - | LARP4B | A | T | A | G | 0,1069 | 0,02042 | 0,02042 | X |
| 10:954957 | rs6560859 | - | LARP4B | A | T | A | G | 0,1069 | 0,02042 | 0,02042 | X |
| 10:954957 | rs6560859 | - | LARP4B | A | T | A | G | 0,1069 | 0,02042 | 0,02042 | X |
| 10: 27812418 | rs10829268 | + | RAB18 | G | T | A | G | 0,4581 | 0,04784 | 0,04784 | |
| 10: 27812418 | rs10829268 | + | RAB18 | G | T | A | G | 0,4581 | 0,04784 | 0,04784 | |
| 10: 27812418 | rs10829268 | + | RAB18 | G | T | A | G | 0,4581 | 0,04784 | 0,04784 | |
| 10: 135111870 | rs11101684 | - | TUBGCP2 | T | C | T | C | 0,04484 | 0,05983 | 0,04484 | X |
| 11:993907 | rs11538725 | + | AP2A2 | C | T | C | G | 0,01516 | 0,01752 | 0,01516 | |
| 11:993907 | rs11538725 | + | AP2A2 | C | T | C | G | 0,01516 | 0,01752 | 0,01516 | |
| 11:3708725 | rs7937799 | - | NUP98 | T | G | T | G | 0,2816 | 0,007147 | 0,007147 | X |
| 11:3717120 | rs2957865 | - | NUP98 | G | A | A | G | 0,03516 | 0,7065 | 0,03516 | |
| 11: 63620253 | rs10897453 | + | MARK2 | A | T | A | G | 0,03701 | 0,1559 | 0,03701 | x |
| 11: 63620253 | rs10897453 | + | MARK2 | A | T | A | G | 0,03701 | 0,1559 | 0,03701 | X |
| 11: 63639881 | rs928948 | + | MARK2 | A | G | A | G | 0,04207 | 0,15 | 0,04207 | |
| 11: 77815059 | rs17825668 | - | ALG8 | A | G | A | G | 0,02403 | 0,2466 | 0,02403 | X |
| 11: 77837761 | rs548907 | - | ALG8 | C | T | T | C | 0,198 | 0,01061 | 0,01061 | X |
| 11: 77837761 | rs548907 | - | ALG8 | C | T | T | C | 0,198 | 0,01061 | 0,01061 | x |
| 11: 78282716 | rs117341758 | - | NARS2 | G | C | A | G | 0,02447 | 0,1272 | 0,02447 | X |
| 11: 78282716 | rs117341758 | - | NARS2 | G | C | A | G | 0,02447 | 0,1272 | 0,02447 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 11: 110048680 | rs74924161 | - | RDX | C | A | A | C | 0,04875 | 0,02212 | 0,02212 | X |
| 12:1112055 | rs61917258 | + | ERC1 | A | T | A | G | 0,03439 | 0,005261 | 0,005261 | X |
| 12:1112055 | rs61917258 | + | ERC1 | A | T | A | G | 0,03439 | 0,005261 | 0,005261 | X |
| 12:1153596 | rs7311276 | + | ERC1 | A | G | A | G | 0,02389 | 0,0161 | 0,0161 | X |
| 12:1153596 | rs7311276 | + | ERC1 | A | G | A | G | 0,02389 | 0,0161 | 0,0161 | X |
| 12:1163115 | rs11061620 | + | ERC1 | T | C | T | C | 0,01929 | 0,00175 | 0,00175 | X |
| 12:1163518 | rs12812965 | + | ERC1 | T | G | T | G | 0,01808 | 0,002984 | 0,002984 | |
| 12:1201546 | rs2887533 | + | ERC1 | A | G | A | G | 0,01784 | 0,002701 | 0,002701 | X |
| 12:1216632 | rs3924361 | + | ERC1 | G | A | A | G | 0,05349 | 0,01964 | 0,01964 | |
| 12:1256950 | rs117086722 | + | ERC1 | C | T | T | C | 0,04914 | 0,08727 | 0,04914 | X |
| 12:1278294 | rs73041063 | + | ERC1 | A | T | A | G | 0,02146 | 0,6421 | 0,02146 | X |
| 12:1278294 | rs73041063 | + | ERC1 | A | T | A | G | 0,02146 | 0,6421 | 0,02146 | X |
| 12:1298657 | rs11061660 | + | ERC1 | G | A | A | G | 0,1572 | 0,0397 | 0,0397 | X |
| 12:1388465 | rs73029107 | + | ERC1 | A | C | A | C | 0,02205 | 0,5904 | 0,02205 | X |
| 12:1402861 | rs73029190 | + | ERC1 | T | C | T | C | 0,04521 | 0,6914 | 0,04521 | X |
| 12:1422858 | rs56262248 | + | ERC1 | A | T | A | AT | 0,03739 | 0,7084 | 0,03739 | X |
| 12:1425657 | rs73034919 | + | ERC1 | C | A | A | C | 0,02098 | 0,5621 | 0,02098 | X |
| 12:1429798 | rs144000701 | + | ERC1 | T | C | T | C | 0,02635 | 0,3909 | 0,02635 | X |
| 12:1432337 | rs10848459 | + | ERC1 | A | G | A | G | 0,006497 | 0,03793 | 0,006497 | X |
| 12:1463229 | rs73038672 | + | ERC1 | G | A | A | G | 0,02546 | 0,6483 | 0,02546 | X |
| 12:1463373 | rs77082078 | + | ERC1 | A | G | A | G | 0,06148 | 0,04546 | 0,04546 | X |
| 12:1493559 | rs6489283 | + | ERC1 | T | G | T | G | 0,04141 | 0,7005 | 0,04141 | X |
| 12:1493559 | rs6489283 | + | ERC1 | T | G | T | G | 0,04141 | 0,7005 | 0,04141 | X |
| 12:1497540 | rs118186282 | + | ERC1 | C | T | T | C | 0,01797 | 0,6374 | 0,01797 | X |
| 12:1510592 | rs73597943 | + | ERC1 | G | A | A | G | 0,04562 | 0,2728 | 0,04562 | X |
| 12:1513206 | rs111449649 | + | ERC1 | T | G | T | G | 0,02072 | 0,6602 | 0,02072 | X |
| 12:1526547 | rs75321406 | + | ERC1 | T | C | T | C | 0,01444 | 0,6022 | 0,01444 | X |
| 12:1534427 | rs76653618 | + | ERC1 | G | A | A | G | 0,01079 | 0,4591 | 0,01079 | X |
| 12:1550888 | rs12313774 | + | ERC1 | A | G | A | G | 0,04123 | 0,1974 | 0,04123 | |
| 12:1551524 | rs7975502 | + | ERC1 | G | A | A | G | 0,03468 | 0,1797 | 0,03468 | X |
| 12:1600160 | rs79833769 | + | ERC1 | G | A | A | G | 0,08367 | 0,04872 | 0,04872 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 12: 19281902 | rs78925317 | + | PLEKHA5 | C | T | T | C | 0,03827 | 0,1367 | 0,03827 | X |
| 12: 19281902 | rs78925317 | + | PLEKHA5 | C | T | T | C | 0,03827 | 0,1367 | 0,03827 | X |
| 12: 19299390 | rs10770437 | + | PLEKHA5 | C | T | T | C | 0,002818 | 0,0005255 | 0,0005255 | X |
| 12: 19303772 | rs1514831 | + | PLEKHA5 | T | G | T | C | 0,02732 | 0,00651 | 0,00651 | X |
| 12: 19303772 | rs1514831 | + | PLEKHA5 | T | G | T | C | 0,02732 | 0,00651 | 0,00651 | X |
| 12: 19306444 | rs7133858 | + | PLEKHA5 | C | T | T | C | 0,154 | 0,00554 | 0,00554 | |
| 12: 19306444 | rs7133858 | + | PLEKHA5 | C | T | T | C | 0,154 | 0,00554 | 0,00554 | |
| 12: 19317082 | rs10841162 | + | PLEKHA5 | T | C | T | C | 0,03847 | 0,01043 | 0,01043 | |
| 12: 19317082 | rs10841162 | + | PLEKHA5 | T | C | T | C | 0,03847 | 0,01043 | 0,01043 | |
| 12: 19336630 | rs10770446 | + | PLEKHA5 | G | A | A | G | 0,1677 | 0,006117 | 0,006117 | X |
| 12: 19338608 | rs7962879 | + | PLEKHA5 | C | T | T | C | 0,0284 | 0,07062 | 0,0284 | |
| 12: 19381632 | rs4497470 | + | PLEKHA5 | A | G | A | G | 0,03037 | 0,01196 | 0,01196 | X |
| 12: 19384213 | rs16915248 | + | PLEKHA5 | G | A | A | G | 0,03477 | 0,1656 | 0,03477 | X |
| 12: 19402989 | rs11044463 | + | PLEKHA5 | A | G | A | G | 0,02238 | 0,01653 | 0,01653 | X |
| 12: 19439080 | rs7965006 | + | PLEKHA5 | C | T | T | C | 0,01292 | 0,04521 | 0,01292 | X |
| 12: 19439080 | rs7965006 | + | PLEKHA5 | C | T | T | C | 0,01292 | 0,04521 | 0,01292 | X |
| 12: 19490585 | rs10841210 | + | PLEKHA5 | C | T | T | C | 0,04224 | 0,2963 | 0,04224 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 12: 19522884 | rs79072570 | + | PLEKHA5 | G | A | A | G | 0,0141 | 0,04792 | 0,0141 | X |
| 12: 29305799 | rs10843330 | + | FAR2 | C | A | A | C | 0,003461 | 0,04473 | 0,003461 | |
| 12: 29359264 | rs1496218 | + | FAR2 | G | A | A | G | 0,03175 | 0,2137 | 0,03175 | X |
| 12: 29373899 | rs10843346 | + | FAR2 | G | C | A | G | 0,007694 | 0,08694 | 0,007694 | X |
| 12: 29373899 | rs10843346 | + | FAR2 | G | C | A | G | 0,007694 | 0,08694 | 0,007694 | X |
| 12: 29426607 | rs116789485 | + | FAR2 | G | A | A | G | 0,02214 | 0,3367 | 0,02214 | X |
| 12: 32972940 | rs1454934 | - | PKP2 | C | T | T | C | 0,0224 | 0,1548 | 0,0224 | X |
| 12: 32974218 | rs7967264 | - | PKP2 | T | C | T | C | 0,0464 | 0,107 | 0,0464 | X |
| 12: 32983457 | rs11052270 | - | PKP2 | T | C | T | C | 0,02363 | 0,04233 | 0,02363 | X |
| 12: 33037790 | rs7979825 | - | PKP2 | T | C | T | C | 0,06934 | 0,04524 | 0,04524 | X |
| 12: 58106836 | rs701006 | + | OS9 | A | G | A | G | 0,02836 | 0,1467 | 0,02836 | X |
| 12: 58108052 | rs1633360 | + | OS9 | C | T | T | C | 0,0282 | 0,105 | 0,0282 | X |
| 12: 64849716 | rs41292019 | + | TBK1 | T | C | T | C | 0,0144 | 0,02751 | 0,0144 | X |
| 12: 64864660 | rs78948244 | + | TBK1 | T | C | T | C | 0,1127 | 0,005422 | 0,005422 | X |
| 12: 120130853 | rs61068064 | - | CIT | A | G | A | G | 0,154 | 0,01448 | 0,01448 | X |
| 12: 120149272 | rs77411677 | - | CIT | G | A | A | G | 0,1073 | 0,008324 | 0,008324 | X |
| 12: 120263003 | rs55707601 | - | CIT | G | C | C | G | 0,1603 | 0,01639 | 0,01639 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 12: 120280652 | rs10774517 | - | CIT | T | C | T | C | 0,04391 | 0,08976 | 0,04391 | X |
| 12: 120293690 | rs280590 | - | CIT | C | T | T | C | 0,04361 | 0,02757 | 0,02757 | X |
| 12: 125317504 | rs57620361 | - | SCARB1 | A | G | A | G | 0,03334 | 0,05022 | 0,03334 | X |
| 13: 25911888 | rs73168527 | + | NUP58 | A | G | A | G | 0,04538 | 0,1879 | 0,04538 | X |
| 13: 25919996 | rs9507502 | + | NUP58 | C | T | T | C | 0,5495 | 0,04163 | 0,04163 | X |
| 13: 25919996 | rs9507502 | + | NUP58 | C | T | T | C | 0,5495 | 0,04163 | 0,04163 | X |
| 13: 36916985 | rs7335635 | - | SPART | G | A | A | G | 0,1278 | 0,03944 | 0,03944 | X |
| 13: 77794549 | rs117936226 | - | MYCBP2 | C | T | A | C | 0,04166 | 0,4837 | 0,04166 | X |
| 13: 77794549 | rs117936226 | - | MYCBP2 | C | T | A | C | 0,04166 | 0,4837 | 0,04166 | X |
| 13: 77849275 | rs9544446 | - | MYCBP2 | C | T | T | C | 0,03809 | 0,1298 | 0,03809 | X |
| 13: 77871440 | rs11617489 | - | MYCBP2 | T | C | T | C | 0,02592 | 0,7325 | 0,02592 | X |
| 13: 96468781 | rs78768204 | - | UGGT2 | A | G | A | G | 0,03576 | 0,1062 | 0,03576 | X |
| 13: 96638651 | rs12863903 | - | UGGT2 | C | T | T | C | 0,04034 | 0,074 | 0,04034 | |
| 13: 96638651 | rs12863903 | - | UGGT2 | C | T | T | C | 0,04034 | 0,074 | 0,04034 | |
| 14: 23984843 | rs222670 | + | THTPA | T | C | T | C | 0,1734 | 0,02813 | 0,02813 | X |
| 14: 23985973 | rs222671 | + | THTPA | A | G | A | G | 0,2517 | 0,02266 | 0,02266 | X |
| 14: 37424934 | rs568318446 | - | SLC25A21 | G | T | G | GT | 0,1084 | 0,02148 | 0,02148 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 14: 37429530 | rs10137084 | - | SLC25A21 | G | T | A | G | 0,8966 | 0,01757 | 0,01757 | X |
| 14: 37429530 | rs10137084 | - | SLC25A21 | G | T | A | G | 0,8966 | 0,01757 | 0,01757 | X |
| 14: 37430306 | rs7150942 | - | SLC25A21 | T | C | T | C | 0,3471 | 0,04039 | 0,04039 | |
| 14: 51206145 | rs12717411 | - | NIN | A | T | A | T | 0,03672 | 0,2355 | 0,03672 | |
| 14: 51206145 | rs12717411 | - | NIN | A | T | A | T | 0,03672 | 0,2355 | 0,03672 | |
| 14: 70347348 | rs11158820 | + | SMOC1 | A | G | A | G | 0,193 | 0,03518 | 0,03518 | X |
| 14: 70399679 | rs117146868 | + | SMOC1 | G | T | T | G | 0,009374 | 0,02352 | 0,009374 | X |
| 14: 92376372 | rs2498827 | - | FBLNS | G | A | A | G | 0,04569 | 0,007066 | 0,007066 | |
| 14: 92377131 | rs2498825 | - | FBLNS | A | C | A | C | 0,04348 | 0,2135 | 0,04348 | X |
| 14: 92415320 | rs2474030 | - | FBLNS | A | G | A | G | 0,009787 | 0,02006 | 0,009787 | X |
| 14: 103881753 | rs77743349 | + | MARK3 | T | G | T | G | 0,007422 | 0,01751 | 0,007422 | X |
| 14: 103889181 | rs12885234 | + | MARK3 | A | G | A | G | 0,05837 | 0,02955 | 0,02955 | |
| 15: 48830657 | rs62011404 | - | FBN1 | A | C | A | C | 0,03906 | 0,2994 | 0,03906 | X |
| 15: 48873292 | rs78141811 | - | FBN1 | C | T | T | C | 0,2375 | 0,04477 | 0,04477 | X |
| 15: 48903221 | rs76249401 | - | FBN1 | C | T | T | C | 0,2738 | 0,01603 | 0,01603 | X |
| 15: 50482304 | rs8182064 | + | SLC27A2 | C | T | A | C | 0,02141 | 0,0307 | 0,02141 | X |
| 15: 50482304 | rs8182064 | + | SLC27A2 | C | T | A | C | 0.02141 | 0,0307 | 0,02141 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 15: 57353034 | rs2703580 | + | TCF12 | C | T | T | C | 0,1173 | 0,04921 | 0,04921 | |
| 15: 57382759 | rs116915857 | + | TCF12 | C | T | T | C | 0,07008 | 0,0388 | 0,0388 | X |
| 15: 57453423 | rs76089422 | + | TCF12 | T | C | T | C | 0,02777 | 0,2408 | 0,02777 | X |
| 15: 57540626 | rs118031436 | + | TCF12 | A | G | A | G | 0,03449 | 0,769 | 0,03449 | X |
| 15: 76562738 | rs8042654 | - | ETFA | T | C | T | C | 0,004638 | 0,00429 | 0,00429 | |
| 15: 91083353 | rs8033595 | + | CRTC3 | G | A | A | G | 0,005123 | 0,02151 | 0,005123 | |
| 15: 91143801 | rs76029207 | + | CRTC3 | C | T | T | C | 0,1859 | 0,03896 | 0,03896 | X |
| 15: 91143801 | rs76029207 | + | CRTC3 | C | T | T | C | 0,1859 | 0,03896 | 0,03896 | X |
| 15: 101869519 | rs62021260 | - | PCSK6 | T | C | T | C | 0,04713 | 0,01443 | 0,01443 | X |
| 15: 101922323 | rs1058260 | - | PCSK6 | A | G | A | G | 0,04423 | 0,02726 | 0,02726 | |
| 15: 101924546 | rs1135911 | - | PCSK6 | C | T | T | C | 0,03065 | 0,2281 | 0,03065 | X |
| 15: 101924838 | rs2277589 | - | PCSK6 | C | T | T | C | 0,3898 | 0,02979 | 0,02979 | X |
| 15: 101936490 | rs3784471 | - | PCSK6 | A | G | A | G | 0,1885 | 0,0393 | 0,0393 | X |
| 15: 101939682 | rs1982906 | - | PCSK6 | G | A | A | G | 0,004061 | 0,01385 | 0,004061 | X |
| 15: 101949927 | rs12901252 | - | PCSK6 | A | C | A | C | 0,03395 | 0,06043 | 0,03395 | X |
| 15: 101964921 | rs7168655 | - | PCSK6 | G | A | A | G | 0,009951 | 0,204 | 0,009951 | X |
| 16: 16060394 | rs215095 | + | ABCC1 | G | A | A | G | 0,01771 | 0,1377 | 0,01771 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 16: 16076883 | rs215059 | + | ABCC1 | T | G | T | C | 0,01805 | 0,1442 | 0,01805 | |
| 16: 16076883 | rs215059 | + | ABCC1 | T | G | T | C | 0,01805 | 0,1442 | 0,01805 | |
| 16: 16079117 | rs215066 | + | ABCC1 | G | A | A | G | 0,001688 | 0,05687 | 0,001688 | X |
| 16: 16086833 | rs119774 | + | ABCC1 | C | T | T | C | 0,0008307 | 0,01832 | 0,0008307 | X |
| 16: 16091237 | rs215069 | + | ABCC1 | C | T | T | C | 0,001235 | 0,01595 | 0,001235 | X |
| 16: 16095952 | rs6498595 | + | ABCC1 | G | C | C | G | 0,1533 | 0,04124 | 0,04124 | X |
| 16: 16095952 | rs6498595 | + | ABCC1 | G | C | C | G | 0,1533 | 0,04124 | 0,04124 | X |
| 16: 16101875 | rs8187843 | + | ABCC1 | G | T | A | G | 0,04903 | 0,4782 | 0,04903 | X |
| 16: 16101875 | rs8187843 | + | ABCC1 | G | T | A | G | 0,04903 | 0,4782 | 0,04903 | X |
| 16: 16101875 | rs8187843 | + | ABCC1 | G | T | A | G | 0,04903 | 0,4782 | 0,04903 | X |
| 16: 16106119 | rs4780589 | + | ABCC1 | A | T | A | G | 0,1544 | 0,04274 | 0,04274 | |
| 16: 16106119 | rs4780589 | + | ABCC1 | A | T | A | G | 0,1544 | 0,04274 | 0,04274 | |
| 16: 16108894 | rs1967120 | + | ABCC1 | G | C | A | G | 0,1367 | 0,03458 | 0,03458 | X |
| 16: 16108894 | rs1967120 | + | ABCC1 | G | C | A | G | 0,1367 | 0,03458 | 0,03458 | X |
| 16: 16110891 | rs3784862 | + | ABCC1 | G | C | A | G | 0,02552 | 0,0518 | 0,02552 | X |
| 16: 16110891 | rs3784862 | + | ABCC1 | G | C | A | G | 0,02552 | 0,0518 | 0,02552 | X |
| 16: 16110955 | rs3784863 | + | ABCC1 | G | T | T | G | 0,02552 | 0,0518 | 0,02552 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 16: 16117271 | rs152033 | + | ABCC1 | T | C | T | C | 0,08671 | 0,02907 | 0,02907 | X |
| 16: 16138313 | rs2230669 | + | ABCC1 | G | A | A | G | 0,01293 | 0,2827 | 0,01293 | X |
| 16: 16159061 | rs35599 | + | ABCC1 | T | C | T | C | 0,1011 | 0,03211 | 0,03211 | X |
| 16: 16168608 | rs35621 | + | ABCC1 | C | T | T | C | 0,04201 | 0,0117 | 0,0117 | X |
| 16: 16170615 | rs35626 | + | ABCC1 | G | T | T | G | 0,04357 | 0,1563 | 0,04357 | X |
| 16: 16199656 | rs11861085 | + | ABCC1 | G | A | A | G | 0,04456 | 0,1072 | 0,04456 | |
| 16: 16199670 | rs11861115 | + | ABCC1 | G | A | A | G | 0,03467 | 0,101 | 0,03467 | X |
| 16: 16205501 | rs3887893 | + | ABCC1 | T | C | T | C | 0,03793 | 0,03112 | 0,03112 | X |
| 16: 16220126 | rs212079 | + | ABCC1 | C | T | T | C | 0,03582 | 0,1383 | 0,03582 | X |
| 16: 16220126 | rs212079 | + | ABCC1 | C | T | T | C | 0,03582 | 0,1383 | 0,03582 | X |
| 16: 16220858 | rs2299670 | + | ABCC1 | C | T | T | C | 0,01772 | 0,04331 | 0,01772 | X |
| 16: 16221164 | rs212080 | + | ABCC1 | A | G | A | G | 0,001572 | 0,06364 | 0,001572 | X |
| 16: 69459591 | rs246141 | + | CYBSB | C | T | T | C | 0,0596 | 0,02226 | 0,02226 | |
| 16: 83849664 | rs12932079 | + | HSBP1 | G | A | A | G | 0,02275 | 0,211 | 0,02275 | |
| 16: 88639749 | rs9935402 | + | ZC3H18 | G | A | A | G | 0,1989 | 0,01828 | 0,01828 | X |
| 16: 88656638 | rs4782505 | + | ZC3H18 | G | T | T | G | 0,0271 | 0,008733 | 0,008733 | |
| 16: 88659990 | rs8053676 | + | ZC3H18 | C | T | T | C | 0,04093 | 0,01799 | 0,01799 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 16: 88659990 | rs8053676 | + | ZC3H18 | C | T | T | C | 0,04093 | 0,01799 | 0,01799 | X |
| 16: 88668051 | rs7199004 | + | ZC3H18 | A | T | A | G | 0,05594 | 0,00211 | 0,00211 | |
| 16: 88668051 | rs7199004 | + | ZC3H18 | A | T | A | G | 0,05594 | 0,00211 | 0,00211 | |
| 16: 88669216 | rs4782377 | + | ZC3H18 | T | C | T | C | 0,05793 | 0,007261 | 0,007261 | |
| 16: 88677118 | rs11646212 | + | ZC3H18 | C | T | T | C | 0,03022 | 0,05293 | 0,03022 | X |
| 16: 88688832 | rs3751675 | + | ZC3H18 | A | G | A | G | 0,1085 | 0,008265 | 0,008265 | |
| 16: 88695376 | rs4782307 | + | ZC3H18 | A | G | A | G | 0,04728 | 0,01252 | 0,01252 | X |
| 17: 36889559 | rs2879097 | + | CISD3 | C | T | T | C | 0,03617 | 0,06111 | 0,03617 | |
| 17: 63713663 | rs9892443 | - | CEP112 | T | C | T | C | 0,04196 | 0,08619 | 0,04196 | X |
| 17: 63734166 | rs7215873 | - | CEP112 | A | G | A | G | 0,1389 | 0,03928 | 0,03928 | |
| 17: 63785418 | rs117859736 | - | CEP112 | A | C | A | C | 0,1366 | 0,0331 | 0,0331 | X |
| 17: 63850547 | rs746628 | - | CEP112 | T | C | T | C | 0,2389 | 0,02748 | 0,02748 | |
| 17: 64185208 | rs77584326 | - | CEP112 | A | G | A | G | 0,02422 | 0,04815 | 0,02422 | X |
| 18: 19349109 | rs74917169 | + | MIB1 | A | G | A | G | 0,1444 | 0,03782 | 0,03782 | X |
| 18: 19352099 | rs75584356 | + | MIB1 | T | C | T | C | 0,05247 | 0,02449 | 0,02449 | X |
| 18: 19358947 | rs3931634 | + | MIB1 | A | T | A | G | 0,1209 | 0,03524 | 0,03524 | X |
| 18: 19358947 | rs3931634 | + | MIB1 | A | T | A | G | 0,1209 | 0,03524 | 0,03524 | X |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 18: 19358947 | rs3931634 | + | MIB1 | A | T | A | G | 0,1209 | 0,03524 | 0,03524 | X |
| 19: 10714058 | rs7253253 | + | SLC44A2 | G | T | T | G | 0,2233 | 0,02012 | 0,02012 | X |
| 19: 11625280 | rs17001450 | - | ECSIT | G | A | A | G | 0,05811 | 0,02504 | 0,02504 | X |
| 19: 14204228 | rs75973396 | - | PRKACA | G | A | A | G | 0,006113 | 0,1912 | 0,006113 | X |
| 19: 15397160 | rs2079014 | - | BRD4 | C | T | T | C | 0,03176 | 0,1301 | 0,03176 | |
| 19: 15397160 | rs2079014 | - | BRD4 | C | T | T | C | 0,03176 | 0,1301 | 0,03176 | |
| 19: 38872634 | rs3760884 | + | PSMD8 | T | C | T | C | 0,02955 | 0,03241 | 0,02955 | X |
| 19: 40862281 | rs7507651 | + | PLD3 | T | G | T | G | 0,02616 | 0,07996 | 0,02616 | X |
| 19: 41211056 | rs3865452 | - | COQ8B | T | C | T | C | 0,006443 | 0,1879 | 0,006443 | |
| 20: 25434139 | rs17857107 | - | NINL | C | T | T | C | 0,04451 | 0,05595 | 0,04451 | |
| 20: 25434139 | rs17857107 | - | NINL | C | T | T | C | 0,04451 | 0,05595 | 0,04451 | |
| 20: 25434139 | rs17857107 | - | NINL | C | T | T | C | 0,04451 | 0,05595 | 0,04451 | |
| 20: 25470056 | rs431579 | - | NINL | T | C | T | C | 0,02688 | 0,1139 | 0,02688 | |
| 20: 25470056 | rs431579 | - | NINL | T | C | T | C | 0,02688 | 0,1139 | 0,02688 | |
| 20: 25566485 | rs6115218 | - | NINL | A | G | A | G | 0,02177 | 0,05332 | 0,02177 | |
| 20: 55929881 | rs4811837 | + | RAE1 | T | C | T | C | 0,04025 | 0,1953 | 0,04025 | |
| 21: 47766113 | rs34500739 | + | PCNT | T | G | T | G | 0,0337 | 0,1507 | 0,0337 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 21: 47766650 | rs2839217 | + | PCNT | G | T | A | G | 0,02451 | 0,347 | 0,02451 | |
| 21: 47766650 | rs2839217 | + | PCNT | G | T | A | G | 0,02451 | 0,347 | 0,02451 | |
| 21: 47766650 | rs2839217 | + | PCNT | G | T | A | G | 0,02451 | 0,347 | 0,02451 | |
| 21: 47818298 | rs2073382 | + | PCNT | T | C | T | C | 0,02359 | 0,3876 | 0,02359 | |
| 21: 47831509 | rs35940413 | + | PCNT | A | G | A | G | 0,003348 | 0,008313 | 0,003348 | |
| 21: 47836122 | rs2839245 | + | PCNT | T | G | T | C | 0,01835 | 0,06447 | 0,01835 | |
| 21: 47836122 | rs2839245 | + | PCNT | T | G | T | C | 0,01835 | 0,06447 | 0,01835 | |
| 21: 47836395 | rs1044998 | + | PCNT | T | G | T | G | 0,01826 | 0,06445 | 0,01826 | |
| 21: 47836547 | rs35346764 | + | PCNT | T | C | T | C | 0,01851 | 0,06503 | 0,01851 | |
| 21: 47838123 | rs2839246 | + | PCNT | A | G | A | G | 0,01842 | 0,06477 | 0,01842 | |
| 21: 47841941 | rs7277175 | + | PCNT | A | G | A | G | 0,01846 | 0,06494 | 0,01846 | |
| 21: 47848459 | rs2839256 | + | PCNT | G | T | A | G | 0,01812 | 0,06408 | 0,01812 | |
| 21: 47848459 | rs2839256 | + | PCNT | G | T | A | G | 0,01812 | 0,06408 | 0,01812 | |
| 21: 47851636 | rs743346 | + | PCNT | G | A | A | G | 0,02603 | 0,09518 | 0,02603 | |
| 22: 19952132 | rs4646316 | + | COMT | C | T | T | C | 0,2811 | 0,02851 | 0,02851 | X |
| 22: 19952132 | rs4646316 | + | COMT | C | T | T | C | 0,2811 | 0,02851 | 0,02851 | X |
| 22: 39148483 | rs2072798 | - | SUN2 | G | A | A | G | 0,09613 | 0,03124 | 0,03124 | |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | inTMG |
|---|---|---|---|---|---|---|---|---|---|---|---|
| 22: 43024871 | rs11704825 | - | CYB5R3 | T | C | T | C | 0,0419 | 0,03534 | 0,03534 | X |

**Tabelle 1d: Komorbiditäten**

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 1:11033082 | rs12565727 | C1orf127 | Glatze | A | G | A | G | 0,5257 | 0,1286 | 0,1286 |
| 21:27269932 | rs63750847 | APP | Alzheimer Erkrankung | C | T | T | C | 0,5582 | 0,9116 | 0,5582 |
| 1:11854476 | rs1801131 | MTHFR | Anaphylaxie nach Impfungen | T | G | T | G | 0,8352 | 0,2664 | 0,2664 |
| 1:11856378 | rs1801133 | MTHFR | Anaphylaxie nach Impfungen | G | A | A | G | 0,0778 | 0,01363 | 0,01363 |
| 2:102769786 | rs949963 | IL1R1 | Anaphylaxie nach Impfungen | C | T | T | C | 0,5507 | 0,6842 | 0,5507 |
| 2:102781649 | rs2228139 | IL1R1 | Anaphylaxie nach Impfungen | C | G | C | G | 0,289 | 0,1513 | 0,1513 |
| 2:113527906 | rs4848300 | | Anaphylaxie nach Impfungen | T | C | T | C | 0,2703 | 0,521 | 0,2703 |
| 2:113537223 | rs17561 | IL1A | Anaphylaxie nach Impfungen | C | A | A | C | 0,1987 | 0,4608 | 0,1987 |
| 2:113542960 | rs1800587 | IL1A | Anaphylaxie nach Impfungen | G | C | A | G | 0,1911 | 0,4378 | 0,1911 |
| 2:227660544 | rs1801278 | IRS1 | Antidiabeticum Sulphonylharnstoff | C | T | T | C | 0,8373 | 0,5774 | 0,5774 |
| 3:12393125 | rs1801282 | PPARG | Antidiabeticum Sulphonylharnstoff | C | G | C | G | 0,2556 | 0,7044 | 0,2556 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 10:11475834 9 | rs7903146 | TCF7L2 | Antidiabeticum Sulphonylharnstoff | C | T | T | C | 0,03868 | 0,4714 | 0,03868 |
| 10:11480890 2 | rs12255372 | TCF7L2 | Antidiabeticum Sulphonylharnstoff | G | T | T | G | 0,1568 | 0,9093 | 0,1568 |
| 11:17408630 | rs5215 | KCNJ11 | Antidiabeticum Sulphonylharnstoff | C | T | T | C | 0,2626 | 0,3634 | 0,2626 |
| 11:17409572 | rs5219 | KCNJ11 | Antidiabeticum Sulphonylharnstoff | T | C | T | C | 0,2539 | 0,3321 | 0,2539 |
| 11:17418477 | rs757110 | ABCC8 | Antidiabeticum Sulphonylharnstoff | C | T | A | C | 0,2024 | 0,1696 | 0,1696 |
| 11:17419279 | rs1799859 | ABCC8 | Antidiabeticum Sulphonylharnstoff | C | T | T | C | 0,8133 | 0,9182 | 0,8133 |
| 5:142661490 | rs6196 | NR3C1 | Antipsychotika und Gewichtszunahme | A | G | A | G | 0,762 | 0,5471 | 0,5471 |
| 18:57851097 | rs17782313 | | Antipsychotika und Gewichtszunahme | T | C | T | C | 0,5245 | 0,5219 | 0,5219 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 18:57882787 | rs489693 | | Antipsychotika und Gewichtszunahme | C | T | A | C | 0,6403 | 0,5528 | 0,5528 |
| 1:11854476 | rs1801131 | MTHFR | Antipsychotika und Gewichtszunahme | T | G | T | G | 0,8352 | 0,2664 | 0,2664 |
| 1:11905981 | rs5067 | NPPA | Asthma | A | T | A | G | 0,02902 | 0,105 | 0,02902 |
| 1:186650846 | rs689465 | PTGS2 | Asthma | T | C | T | C | 0,8043 | 0,6269 | 0,6269 |
| 1:203155882 | rs4950928 | CHI3L1 | Asthma | G | T | C | G | 0,4781 | 0,6666 | 0,4781 |
| 5:148206440 | rs1042713 | ADRB2 | Asthma | G | A | A | G | 0,458 | 0,7438 | 0,458 |
| 11:67352689 | rs1695 | GSTP1 | Asthma | A | G | A | G | 0,8389 | 0,2814 | 0,2814 |
| 17:38057197 | rs8069176 | | Asthma | G | T | A | G | 0,6913 | 0,7276 | 0,6913 |
| 17:38069949 | rs7216389 | GSDMB | Asthma | C | T | T | C | 0,524 | 0,9106 | 0,524 |
| 5:149581321 | rs13153971 | SLC6A7 | Asthma | T | C | A | G | 0,7762 | 0,8809 | 0,7762 |
| 7:8007876 | rs37973 | GLCCI1 | Asthma Inhalation Corticosteroide | G | C | A | G | 0,5149 | 0,6044 | 0,5149 |
| 17:45810919 | rs2240017 | TBX21 | Asthma Inhalation Corticosteroide | C | G | C | G | 0,01101 | 0,06398 | 0,01101 |
| 1:152259078 | rs11204971 | | Atopische Dermatitis | A | G | A | G | 0,72 | 0,9728 | 0,72 |
| 1:203155882 | rs4950928 | CHI3L1 | Atopisches Asthma | G | T | C | G | 0,4781 | 0,6666 | 0,4781 |
| 2:113590390 | rs1143634 | IL1B | Belastungsbedingte Muskelschäden | G | A | A | G | 0,2151 | 0,1149 | 0,1149 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 3:123512627 | rs28497577 | MYLK | Belastungsbedingte Muskelschäden | G | T | T | G | 0,975 | 0,3837 | 0,3837 |
| 6:31543031 | rs1800629 | TNF | Belastungsbedingte Muskelschäden | G | A | A | G | 0,6323 | 0,9298 | 0,6323 |
| 6:160113872 | rs4880 | SOD2 | Belastungsbedingte Muskelschäden | A | G | A | G | 0,9923 | 0,8354 | 0,8354 |
| 7:22766645 | rs1800795 | IL6 | Belastungsbedingte Muskelschäden | C | G | C | G | 0,9948 | 0,7795 | 0,7795 |
| 8:118184783 | rs13266634 | SLC30A8 | Belastungsbedingte Muskelschäden | C | T | T | C | 0,3997 | 0,02096 | 0,02096 |
| 11:66328095 | rs1815739 | ACTN3 | Belastungsbedingte Muskelschäden | T | C | T | C | 0,2485 | 0,3292 | 0,2485 |
| 3:12393125 | rs1801282 | PPARG | Belastungsglukose Test | C | G | C | G | 0,2556 | 0,7044 | 0,2556 |
| 3:169492101 | rs10936599 | MYNN | Biologisches Altern | C | T | T | C | 0,4076 | 0,9149 | 0,4076 |
| 5:1286516 | rs2736100 | TERT | Biologisches Altern | C | A | A | C | 0,07519 | 0,2332 | 0,07519 |
| 17:7579472 | rs1042522 | TP53 | Biologisches Altern | G | T | C | G | 0,4632 | 0,9689 | 0,4632 |
| 19:19361735 | rs1064395 | NCAN | Bipolare Störung | G | A | T | C | 0,3075 | 0,4266 | 0,3075 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 1:239436542 | rs2820037 | | Bluthochdruck | A | T | A | T | 0,9095 | 0,9021 | 0,9021 |
| 3:148459988 | rs5186 | AGTR1 | Bluthochdruck | A | C | A | C | 0,2868 | 0,04728 | 0,04728 |
| 4:2906707 | rs4961 | ADD1 | Bluthochdruck | G | T | T | G | 0,9661 | 0,4994 | 0,4994 |
| 4:148463840 | rs5335 | EDNRA | Bluthochdruck | G | C | C | G | 0,6133 | 0,7306 | 0,6133 |
| 6:12296255 | rs5370 | EDN1 | Bluthochdruck | G | T | T | G | 0,773 | 0,7957 | 0,773 |
| 8:140180024 | rs6997709 | | Bluthochdruck | G | T | T | G | 0,9995 | 0,2957 | 0,2957 |
| 12:24981611 | rs7961152 | BCAT1 | Bluthochdruck | A | C | A | C | 0,8361 | 0,8975 | 0,8361 |
| 12:10204221 3 | rs11110912 | MYBPC1 | Bluthochdruck | C | G | C | G | 0,7718 | 0,5606 | 0,5606 |
| 13:68035371 | rs1937506 | | Bluthochdruck | G | A | A | G | 0,5881 | 0,4268 | 0,4268 |
| 15:96830550 | rs2398162 | NR2F2-AS1 | Bluthochdruck | A | G | A | G | 0,854 | 0,563 | 0,563 |
| 16:20365654 | rs13333226 | UMOD | Bluthochdruck | A | G | A | G | 0,2641 | 0,5202 | 0,2641 |
| 1:11906068 | rs5065 | NPPA | Bluthochdrucktherapie | A | G | A | G | 0,02964 | 0,1061 | 0,02964 |
| 4:45175691 | rs13130484 | | Body Mass Index | C | T | T | C | 0,8852 | 0,6633 | 0,6633 |
| 7:128363287 | rs6971091 | FAM71F1 | Body Mass Index | G | A | A | G | 0,7515 | 0,6615 | 0,6615 |
| 11:8123499 | rs2272383 | TUB | Body Mass Index | G | C | A | G | 0,8004 | 0,7626 | 0,7626 |
| 11:8149753 | rs1528133 | RIC3 | Body Mass Index | T | G | T | G | 0,4337 | 0,2346 | 0,2346 |
| 16:53809247 | rs1121980 | FTO | Body Mass Index | G | C | A | G | 0,8921 | 0,1877 | 0,1877 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 18:57851763 | rs10871777 | | Body Mass Index | A | G | A | G | 0,6425 | 0,6633 | 0,6425 |
| 18:57884750 | rs12970134 | | Body Mass Index | G | A | A | G | 0,4484 | 0,5476 | 0,4484 |
| 18:58039276 | rs2229616 | MC4R | Body Mass Index | C | T | | | | | |
| | rs2272382 | | Body Mass Index | | | | | | | |
| 1:66105944 | rs1892534 | | C-reactives Protein | C | T | T | C | 0,178 | 0,1888 | 0,178 |
| 15:78806023 | rs8034191 | HYKK | Chronisch-obstruktive Lungenerkran-kung (COPD) | T | C | T | C | 0,6024 | 0,2506 | 0,2506 |
| 4:89883979 | rs7671167 | FAM13A | Chronisch-obstruktive Lungenerkran-kung (COPD) | C | T | A | G | 0,02869 | 0,4265 | 0,02869 |
| 6:55142337 | rs2653349 | HCRTR2 | Cluster Kopfschmerz | A | T | A | G | 0,4208 | 0,7779 | 0,4208 |
| 10:10128776 4 | rs10883365 | LINC01475 | Colitis ulcerosa | G | A | T | G | 0,09376 | 0,8494 | 0,09376 |
| 6:32433167 | rs2395185 | | Colitis ulcerosa | G | T | A | G | 0,4044 | 0,6992 | 0,4044 |
| 3:49701983 | rs9858542 | BSN | Colitis ulcerosa | G | A | T | C | 0,008248 | 0,2736 | 0,008248 |
| 1:67705958 | rs11209026 | IL23R | Colitis Ulcerosa | G | A | A | G | 0,999 | 0,9629 | 0,9629 |
| 1:109818530 | rs646776 | CELSR2 | Coronare Herzerkranku ng | C | T | T | C | 0,09439 | 0,06964 | 0,06964 |
| 1:109822166 | rs599839 | PSRC1 | Coronare Herzerkranku ng | G | C | A | G | 0,2332 | 0,282 | 0,2332 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 3:108147728 | rs3900940 | MYH15 | Coronare Herzerkranku ng | T | C | T | C | 0,5396 | 0,7347 | 0,5396 |
| 4:169677486 | rs7439293 | PALLD | Coronare Herzerkranku ng | G | A | A | G | 0,5767 | 0,6621 | 0,5767 |
| 5:99948982 | rs383830 | | Coronare Herzerkranku ng | A | T | A | T | 0,05572 | 0,1458 | 0,05572 |
| 6:151252985 | rs6922269 | MTHFD1L | Coronare Herzerkranku ng | G | A | A | G | 0,5672 | 0,3711 | 0,3711 |
| 6:160961137 | rs3798220 | LPA | Coronare Herzerkranku ng | T | C | T | C | 0,7928 | 0,9901 | 0,7928 |
| 7:150690079 | rs2070744 | NOS3 | Coronare Herzerkranku ng | C | T | T | C | 0,4597 | 0,6086 | 0,4597 |
| 9:22098574 | rs4977574 | CDKN2B-AS1 | Coronare Herzerkranku ng | A | T | A | G | 0,723 | 0,3374 | 0,3374 |
| 9:22125347 | rs1333048 | | Coronare Herzerkranku ng | A | C | A | C | 0,5058 | 0,2165 | 0,2165 |
| 9:22125503 | rs1333049 | | Coronare Herzerkranku ng | G | C | C | G | 0,9712 | 0,5548 | 0,5548 |
| 10:44753867 | rs501120 | | Coronare Herzerkranku ng | T | C | T | C | 0,9409 | 0,6905 | 0,6905 |
| 11:11664891 7 | rs964184 | ZPR1 | Coronare Herzerkranku ng | G | C | C | G | 0,7848 | 0,9727 | 0,7848 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 11:13075064 2 | rs2298566 | SNX19 | Coronare Herzerkrankung | A | T | A | C | 0,04477 | 0,3822 | 0,04477 |
| 12:11188460 8 | rs3184504 | SH2BS | Coronare Herzerkrankung | T | G | | C | 0,7665 | 0,3291 | 0,3291 |
| 15:67458639 | rs17228212 | SMAD3 | Coronare Herzerkrankung | T | C | T | C | 0,3211 | 0,1722 | 0,1722 |
| 16:83212398 | rs8055236 | CDH13 | Coronare Herzerkrankung | G | T | T | G | 0,4073 | 0,2411 | 0,2411 |
| 19:30064396 | rs7250581 | | Coronare Herzerkrankung | A | G | A | G | 0,4056 | 0,7457 | 0,4056 |
| 19:45422946 | rs4420638 | APOC1 | Coronare Herzerkrankung | A | G | A | G | 0,000125 5 | 0,01072 | 0,000125 5 |
| 22:26689635 | rs688034 | SEZ6L | Coronare Herzerkrankung | C | T | T | C | 0,6524 | 0,9639 | 0,6524 |
| 6:35607571 | rs1360780 | FKBP5 | Depression | T | C | T | C | 0,07709 | 0,1271 | 0,07709 |
| 12:84564068 | rs1545843 | LOC107984536 | Depression | G | A | A | G | 0,9932 | 0,3265 | 0,3265 |
| 12:84920334 | rs1031681 | | Depression | T | C | T | C | 0,8152 | 0,4866 | 0,4866 |
| 1:66558759 | rs4655595 | PDE4B | Diabetes Mellitus, Tvpe 2 | A | G | A | G | 0.7645 | 0,822 | 0.7645 |
| 2:161214175 | rs6718526 | RBMS1 | Diabetes Mellitus, Type 2 | T | C | T | C | 0,1905 | 0,3702 | 0,1905 |
| 3:12393125 | rs1801282 | PPARG | Diabetes Mellitus, Type 2 | C | G | C | G | 0,2556 | 0,7044 | 0,2556 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 3:55313400 | rs358806 | | Diabetes Mellitus, Type 2 | A | T | A | C | 0,4318 | 0,6128 | 0,4318 |
| 4:122665514 | rs7659604 | | Diabetes Mellitus, Type 2 | C | T | T | C | 0,8069 | 0,9271 | 0,8069 |
| 6:20717255 | rs9465871 | CDKAL1 | Diabetes Mellitus, Type 2 | T | C | T | C | 0,1673 | 0,09751 | 0,09751 |
| 7:44229068 | rs1799884 | GCK | Diabetes Mellitus, Type 2 | C | T | T | C | 0,265 | 0,07909 | 0,07909 |
| 8:118184783 | rs13266634 | SLC30A8 | Diabetes Mellitus, Type 2 | C | T | T | C | 0,3997 | 0,02096 | 0,02096 |
| 9:22134094 | rs10811661 | | Diabetes Mellitus, Type 2 | T | C | T | C | 0,7311 | 0,3151 | 0,3151 |
| 10:44068558 | rs9326506 | ZNF239 | Diabetes Mellitus, Type 2 | A | C | A | C | 0,115 | 0,1801 | 0,115 |
| 10:11475604 1 | rs4506565 | TCF7L2 | Diabetes Mellitus, Type 2 | A | T | A | T | 0,1442 | 0,5531 | 0,1442 |
| 10:11475834 9 | rs7903146 | TCF7L2 | Diabetes Mellitus, Type 2 | C | T | T | C | 0,03868 | 0,4714 | 0,03868 |
| 10:11480890 2 | rs12255372 | TCF7L2 | Diabetes Mellitus, Type 2 | G | T | T | G | 0,1568 | 0,9093 | 0,1568 |
| 11:17409572 | rs5219 | KCNJ11 | Diabetes Mellitus, Type 2 | T | C | T | C | 0,2539 | 0,3321 | 0,2539 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 11:41915366 | rs9300039 | | Diabetes Mellitus, Type 2 | C | A | A | C | 0,668 | 0,6949 | 0,668 |
| 11:92708710 | rs10830963 | MTNR1B | Diabetes Mellitus, Type 2 | C | G | C | G | 0,1388 | 0,01251 | 0,01251 |
| 12:51357542 | rs12304921 | SLC11A2 | Diabetes Mellitus, Type 2 | A | G | A | G | 0,8369 | 0,7923 | 0,7923 |
| 12:71577101 | rs1495377 | | Diabetes Mellitus, Type 2 | G | C | C | G | 0,7475 | 0,4215 | 0,4215 |
| 15:74604834 | rs2930291 | CCDC33 | Diabetes Mellitus, Type 2 | G | C | A | G | 0,2473 | 0,5998 | 0,2473 |
| 15:80413384 | rs2903265 | ZFAN D6 | Diabetes Mellitus, Type 2 | A | G | A | G | 0,5223 | 0,6589 | 0,5223 |
| 16:53816275 | rs8050136 | FTO | Diabetes Mellitus, Type 2 | C | A | A | C | 0,5887 | 0,3078 | 0,3078 |
| 17:17715101 | rs11868035 | SREBF1 | Diabetes Mellitus, Type 2 | G | A | A | G | 0,7196 | 0,5419 | 0,5419 |
| 17:17734740 | rs1889018 | SREBF1 | Diabetes Mellitus, Type 2 | G | A | A | G | 0,8683 | 0,3419 | 0,3419 |
| 20:368905 | rs2295490 | TRIB3 | Diabetes Mellitus, Type 2 | A | T | A | G | 0,6213 | 0,7779 | 0,6213 |
| 5:149357747 | rs10489391 9 | SLC26A2 | Diastophische Dysplasia | C | T | | | | | |
| 5:149359991 | rs10489391 5 | SLC26A2 | Diastophische Dysplasia | C | T | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 5.149361113 | rs10489392 4 | SLC26A2 | Diaslophische Dysplasia | T | A | | | | | |
| 1:161193683 | rs5082 | APOA2 | Diätwirkung | G | A | A | G | 0,1206 | 0,2059 | 0,1206 |
| 3:12393125 | rs1801282 | PPARG | Diätwirkung | C | G | C | G | 0,2556 | 0,7044 | 0,2556 |
| 3:186559460 | rs17300539 | ADIPOQ | Diätwirkung | G | A | A | G | 0,7849 | 0,9065 | 0,7849 |
| 11:11327082 8 | rs1800497 | ANKK1 | Diätwirkung | G | A | A | G | 0,03244 | 0,03708 | 0,03244 |
| 11:11666370 7 | rs662799 | APOA5 | Diätwirkung | G | A | A | G | 0,7079 | 0,8299 | 0,7079 |
| 7:37989095 | rs16879765 | EPDR1 | Dupuytren'sche Erkrankung | C | T | A | G | 0,4556 | 0,923 | 0,4556 |
| 13:92203813 | rs16946160 | GPC5 | Erworbenes nephrotisches Syndrom | G | A | A | G | 0,8458 | 0,9612 | 0,8458 |
| 2:21229068 | rs12713559 | APOB | Familiäre Hypercholesterolämie Type B | G | A | | | | | |
| 2:21229160 | rs5742904 | APOB | Familiäre Hypercholesterolämie Type B | C | T | | | | | |
| 2:21229161 | rs14446787 3 | APOB | Familiäre Hypercholesterolämie Type B | G | A | | | | | |
| 2:179650726 | rs28933405 | TTN | Familiäre Hypertrophische Kardiomyopathie | C | T | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 7:151265836 | rs28938173 | PRKAG2 | Familiäre Hypertrophische Kardiomyopathie | G | T | | | | | |
| 12:11135209 1 | rs10489436 9 | MYL2 | Familiäre Hypertrophische Kardiomyopathie | C | T | | | | | |
| 12:11135694 9 | rs10489437 0 | MYL2 | Familiäre Hypertrophische Kardiomyopathie | A | G | | | | | |
| 14:23898488 | rs3218714 | MYH7 | Familiäre Hypertrophische Kardiomyopathie | G | C | | | | | |
| 14:23900677 | rs3218713 | MYH7 | Familiäre Hypertrophische Kardiomyopathie | C | T | | | | | |
| 15:63353098 | rs10489450 3 | TPM1 | Familiäre Hypertrophische Kardiomyopathie | G | A | | | | | |
| 15:63353114 | rs10489450 2 | TPM1 | Familiäre Hypertrophische Kardiomyopathie | A | T | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 3:12393125 | rs1801282 | PPARG | Fettgewebebi ldung | C | G | C | G | 0,2556 | 0,7044 | 0,2556 |
| 16:28873398 | rs4788102 | SH2B1 | Fettsuch | G | A | T | C | 0,5954 | 0,6393 | 0,5954 |
| 2:634905 | rs6548238 | | Fettsuch | T | C | T | C | 0,3291 | 0,8544 | 0,3291 |
| 3:185834290 | rs7647305 | | Fettsuch | T | C | T | G | 0,3384 | 0,1441 | 0,1441 |
| 11:27667202 | rs925946 | BDNF-AS | Fettsuch | T | G | T | G | 0,701 | 0,3379 | 0,3379 |
| 1:161193683 | rs5082 | APOA2 | Fettsucht | G | A | A | G | 0,1206 | 0,2059 | 0,1206 |
| 3:186559460 | rs17300539 | ADIPOQ | Fettsucht | G | A | A | G | 0,7849 | 0,9065 | 0,7849 |
| 4:37904089 | rs35859249 | TBC1D1 | Fettsucht | C | T | T | C | 0,578 | 0,2641 | 0,2641 |
| 5:95751785 | rs6232 | PCSK1 | Fettsucht | T | C | T | C | 0,004604 | 0,00552 7 | 0,004604 |
| 7:128363287 | rs6971091 | FAM71F1 | Fettsucht | G | A | A | G | 0,7515 | 0,6615 | 0,6615 |
| 11:11666370 7 | rs662799 | APOA5 | Fettsucht | G | A | A | G | 0,7073 | 0,8299 | 0,7073 |
| 16:53813367 | rs17817449 | FTO | Fettsucht | T | G | T | G | 0,5877 | 0,3129 | 0,3129 |
| 18:57851097 | rs17782313 | | Fettsucht | T | C | T | C | 0,5245 | 0,5219 | 0,5219 |
| 18:58039276 | rs2229616 | MC4R | Fettsucht | C | T | | | | | |
| 7:44229068 | rs1799884 | GCK | Geburtsgewic ht | C | T | T | C | 0,265 | 0,07909 | 0,07909 |
| 10:11475834 9 | rs7903146 | TCF7L2 | Geburtsgewic ht | C | T | T | C | 0,03868 | 0,4714 | 0,03868 |
| 15:78894339 | rs1051730 | CHRNA3 | Geburtsgewic ht | G | A | A | G | 0,2409 | 0,4063 | 0,2409 |
| X:153761240 | rs76723693 | G6PD | Glucose-6-phosphate-dehydrogena se-mangel | A | G | | | | | |
| X:153761811 | rs13785231 8 | G6PD | Glucose-6-phosphate-dehydrogena se-mangel | C | T | | | | | |
| X:153762634 | rs5030868 | G6PD | Glucose-6-phosphate-dehydrogena se-mangel | G | A | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| X:153764217 | rs1050828 | G6PD | Glucose-6-phosphate-dehydrogenase-mangel | C | T | | | | | |
| 11:11889598 1 | rs80356491 | SLC37A4 | Glycogen Speicher Krankheit 1B | AG | | | | | | |
| 11:11889600 9 | rs80356490 | SLC37A4 | Glycogen Speicher Krankheit 1B | C | A | | | | | |
| 11:11889893 3 | rs8356483 | SLC37A4 | Glycogen Speicher Krankheit 1B | A | G | | | | | |
| 1:100346885 | rs11399412 9 | AGL | Glycogen Speicher Krankheit Typ 3 | G | A | | | | | |
| 1:100350168 | rs11399413 0 | AGL | Glycogen Speicher Krankheit Typ 3 | C | T | | | | | |
| 1:100368332 | rs11399413 1 | AGL | Glycogen Speicher Krankheit Typ 3 | C | T | | | | | |
| 11:64525298 | rs11910325 1 | PYGM | Glycogen Speicherkrankheit Typ 5 | C | T | | | | | |
| 11:64527223 | rs11698755 2 | PYGM | Glycogen Speicherkrankheit Typ 5 | G | A | | | | | |
| 11:76299194 | rs2155219 | | Graspollen-Allergie und Heufieber | G | T | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 1:109818530 | rs646776 | CELSR2 | Herzinfarkt | C | T | T | C | 0,09439 | 0,06964 | 0,06964 |
| 1:222823529 | rs17465637 | MIA3 | Herzinfarkt | A | T | A | C | 0,6574 | 0,2949 | 0,2949 |
| 6:169617726 | rs8089 | THBS2 | Herzinfarkt | A | C | A | C | 0,5723 | 0,5529 | 0,5529 |
| 9:22083404 | rs1333040 | CDKN2B-AS1 | Herzinfarkt | C | T | T | C | 0,9447 | 0,6587 | 0,6587 |
| 9:22115026 | rs2383206 | CDKN2B-AS1 | Herzinfarkt | A | G | A | G | 0,4879 | 0,2549 | 0,2549 |
| 9:22115959 | rs2383207 | CDKN2B-AS1 | Herzinfarkt | A | G | A | G | 0,6223 | 0,2859 | 0,2859 |
| 9:22124477 | rs10757278 | | Herzinfarkt | A | G | A | G | 0,941 | 0,6124 | 0,6124 |
| 9:120470054 | rs1927911 | TLR4 | Herzinfarkt | A | G | A | G | 0,2266 | 0,2605 | 0,2266 |
| 10:44753867 | rs501120 | | Herzinfarkt | T | C | T | C | 0,9409 | 0,6905 | 0,6905 |
| 11:11666370 7 | rs662799 | APOA5 | Herzinfarkt | G | A | A | G | 0,7079 | 0,8299 | 0,7079 |
| 15:67458639 | rs17228212 | SMAD3 | Herzinfarkt | T | C | T | C | 0,3211 | 0,1722 | 0,1722 |
| 20:44640225 | rs17576 | MMP9 | Herzinfarkt | A | G | A | G | 0,3827 | 0,9441 | 0,3827 |
| 12:11711048 9 | rs3782886 | BRAP | Herzinfarkt | T | C | A | G | 0,3392 | 0,4993 | 0,3392 |
| 22:37972628 | rs7291467 | LGALS2 | Herzinfarkt | G | A | A | G | 0,08157 | 0,02816 | 0,02816 |
| 21:44483184 | rs5742905 | CBS | Homocystinurie, Pyridoxin abhängig | A | G | | | | | |
| 10:10471909 6 | rs12413409 | CNNM2 | Hypertension | G | A | A | C | 0,7166 | 0,811 | 0,7166 |
| 15:75077367 | rs1378942 | CSK | Hypertension | C | T | A | T | 0,8698 | 0,134 | 0,134 |
| 4:81184341 | rs16998073 | | Hypertension | A | T | A | G | 0,5324 | 0,4946 | 0,4946 |
| 1:11862778 | rs17367504 | MTHFR | Hypertension | A | G | T | C | 0,8359 | 0,8693 | 0,8359 |
| 2:169015914 | rs3754777 | STK39 | Hypertension | C | T | A | G | 0,7631 | 0,3728 | 0,3728 |
| 11:11664891 7 | rs964184 | ZPR1 | Hypertriglycer idemie | G | C | C | G | 0,7848 | 0,9727 | 0,7848 |
| 1:201022666 | rs28930068 | CACNA1S | Hypokalemische Periodische Lähmung | C | T | | | | | |
| 1:201022667 | rs28930069 | CACNA1S | Hypokalemische Periodische Lähmung | G | C | | | | | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 1:201035411 | rs80338779 | CACNA1S | Hypokalemische Periodische Lähmung | C | A | | | | | |
| 1:201047043 | rs80338777 | CACNA1S | Hypokalemische Periodische Lähmung | C | T | | | | | |
| 17:62022968 | rs12190855 5 | SCN4A | Hypokalemische Periodische Lähmung | G | A | | | | | |
| 17:62036629 | rs80338788 | SCN4A | Hypokalemische Periodische Lähmung | C | T | | | | | |
| 17:62036638 | rs80338784 | SCN4A | Hypokalemische Periodische Lähmung | C | T | | | | | |
| 1:21890632 | rs12191800 7 | ALPL | Hypophosphatasie | G | C | | | | | |
| 1:21900176 | rs12191800 2 | ALPL | Hypophosphatasie | A | C | | | | | |
| 1:114377568 | rs2476601 | PTPN22 | Hypothyroidismus | A | G | A | G | 0,9894 | 0,3625 | 0,3625 |
| 9:100549013 | rs7850258 | PTCSC2 | Hypothyroidismus | A | G | A | G | 0,8357 | 0,9476 | 0,8357 |
| 12:11188460 8 | rs3184504 | SH2B3 | Hypothyroidismus | T | G | T | C | 0,7665 | 0,3291 | 0,3291 |
| 6:31018407 | rs2517532 | | Hypothyroidismus | A | G | A | G | 5,204E-06 | 7,16E-06 | 5,204E-06 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min | |
|---|---|---|---|---|---|---|---|---|---|---|---|
| 19:4717672 | rs12610495 | DPP9 | Idiopathische Lungenfibrose | A | T | A | G | 0,002945 | 0,00258 2 | 0,002582 | |
| 13:11353662 7 | rs1278769 | ATP11A | Idiopathische Lungenfibrose | A | G | A | G | 0,7251 | 0,6793 | 0,6793 | |
| 10:10565416 4 | rs1980653 | STN1 | Idiopathische Lungenfibrose | A | G | A | G | 0,06052 | 0,04513 | 0,04513 | |
| 17:44056767 | rs1981997 | MAPT | Idiopathische Lungenfibrose | G | A | A | G | 0,1992 | 0,5138 | 0,1992 | |
| 15:40717302 | rs2034650 | IVD | Idiopathische Lungenfibrose | G | A | T | C | 0,3022 | 0,5525 | 0,3022 | |
| 3:169518455 | rs6793295 | LRRC34 | Idiopathische Lungenfibrose | T | G | T | C | 0,08175 | 0,02151 | 0,02151 | |
| 11:1093945 | rs7934606 | MUC2 | Idiopathische Lungenfibrose | T | G | | | | | | |
| 6:32605884 | rs2187668 | HLA-DQA1 LOC107986589 | ;; | Idiopathische membranöse Nephroapthie | C | T | A | G | 0,8901 | 0,8971 | 0,8901 |
| 2:160917497 | rs4664308 | LOC105373717 PLA2R1 | ;; | Idiopathische membranöse Nephroapthie | A | G | T | C | 0,5555 | 0,5131 | 0,5131 |
| 6:32681631 | rs9275596 | | | IgA Nephropathie | C | T | A | G | 0,1891 | 0,4173 | 0,1891 |
| 20:10220496 | rs363039 | SNAP25 | | Intelligenz Messung | G | A | A | G | 0,8542 | 0,3386 | 0,3386 |
| 20:10234257 | rs363050 | SNAP25 | | Intelligenz Messung | G | A | A | G | 0.814 | 0.3539 | 0.3539 |
| 3:138713704 | rs1511412 | | | Keloidbildung | A | T | T | C | 0,2126 | 0,2887 | 0,2126 |
| 15:56194877 | rs8032158 | NEDD4 | | Keloidbildung | T | C | T | C | 0,3531 | 0,3379 | 0,3379 |
| 1:222271767 | rs873549 | | | Keloidbildung | C | T | T | C | 0,7489 | 0,6727 | 0,6727 |
| 3:138841593 | rs940187 | BPESC1 | | Keloidbildung | T | C | T | C | 0,2894 | 0,0262 | 0,0262 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min | |
|---|---|---|---|---|---|---|---|---|---|---|---|
| 3:15216429 | rs7639618 | COL6A4P1 | | Knie Osteoarthrose | C | T | A | G | 0,3175 | 0,05619 | 0,05619 |
| 1:186725003 | rs4140564 | | | Knie Osteoarthrose bei Frauen | G | A | T | G | 0,2263 | 0,4991 | 0,2263 |
| 22:24837301 | rs5751876 | ADORA2A | | Koffein Genuss | T | C | T | C | 0,5927 | 0,806 | 0,5927 |
| 7:17287269 | rs6968865 | LOC101927609 | | Koffein Genuss | A | T | A | T | 0,03252 | 0,1722 | 0,03252 |
| 15:75027880 | rs2472297 | | | Koffein Genuss | C | T | T | C | 0,3936 | 0,4193 | 0,3936 |
| 19:11202306 | rs6511720 | LDLR | | Koronare Herz-Erkrankung | G | T | A | G | 0,3101 | 0,00995 4 | 0,009951 |
| 6:11774583 | rs6903956 | ADTRP | | Koronare Herz-Erkrankung | A | G | A | G | 0,3813 | 0,4124 | 0,3813 |
| 3:58082709 | rs9834312 | FLNB | | Körpergröße | G | A | A | G | 0,2489 | 0,1209 | 0,1209 |
| 6:152297100 | rs2179922 | ESR1 | | Körpergröße | A | T | A | G | 0,3827 | 0,6312 | 0,3827 |
| 12:66358347 | rs1042725 | HMGA2 | | Körpergröße | C | T | T | C | 0,08361 | 0,409 | 0,08361 |
| 20:33907161 | rs6060369 | UQCC1 | | Körpergröße | T | C | T | C | 0,9393 | 0,7868 | 0,7868 |
| | rs8038652 | | | Körpergröße | | | | | | | |
| 6:108896215 | rs2802288 | FOXO3 | | Langlebigkeit | A | G | A | G | 0,4276 | 0,7832 | 0,4276 |
| 6:108908518 | rs2802292 | FOXO3 | | Langlebigkeit | G | T | T | G | 0,4215 | 0,7647 | 0,4215 |
| 6:108934461 | rs2764264 | FOXO3 | | Langlebigkeit | C | T | T | C | 0,4553 | 0,736 | 0,4553 |
| 9:21998035 | rs2811712 | CDKN2B-AS1 | | Langlebigkeit | G | A | A | G | 0,09936 | 0,4263 | 0,09936 |
| 15:99451976 | rs34516635 | IGF1R | | Langlebigkeit | G | A | | | | | |
| 15:65494212 | rs2073711 | CILP | | Lumbaler Bandscheibenvorfall | A | G | A | G | 0,5614 | 0,5088 | 0,5088 |
| 5:1286516 | rs2736100 | TERT | | Lungenfibrose | C | A | A | C | 0,07519 | 0,2332 | 0,07519 |
| 6:7563232 | rs2076295 | DSP | | Lungenfibrose | T | G | T | G | 0,4797 | 0,4968 | 0,4797 |
| 8:11349186 | rs13277113 | | | Lupus (Systemic Lupus Erythematosus) | G | A | | | | | |
| 6:32605884 | rs2187668 | HLA-DQA1<br>LOC107986589 | | Lupus (Systemic Lupus Erythematosus) | C | T | T | G | 0,8839 | 0,1057 | 0,1057 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min | |
|---|---|---|---|---|---|---|---|---|---|---|---|
| 1:173191475 | rs2205960 | TNFSF4 | | Lupus (Systemic Lupus Erythematosus) | G | T | T | C | 0,07256 | 0,1517 | 0,07256 |
| 16:31313253 | rs9888739 | ITGAM | | Lupus (Systemic Lupus Erythematosus) | C | T | T | C | 0,2599 | 0,05511 | 0,05511 |
| 2:239694631 | rs9287638 | | | Männliche Glatze | C | A | A | C | 0,4403 | 0,3673 | 0,3673 |
| 6:150358012 | rs9479482 | | | Männliche Glatze | T | C | T | C | 0,09704 | 0,1578 | 0,09704 |
| 7:18877874 | rs2073963 | HDAC9 | | Männliche Glatze | T | G | T | G | 0,6187 | 0,7523 | 0,6187 |
| 7:68611960 | rs6945541 | LOC105375343 | | Männliche Glatze | C | T | T | C | 0,008078 | 0,1506 | 0,008078 |
| 18:42800148 | rs10502861 | SLC14A2 | | Männliche Glatze | C | T | T | C | 0,8214 | 0,7388 | 0,7388 |
| 18:42814156 | rs8085664 | SLC14A2 | | Männliche Glatze | C | T | A | C | 0,6519 | 0,6038 | 0,6038 |
| 20:21853100 | rs2180439 | | | Männliche Glatze | C | T | T | C | 0,2696 | 0,2279 | 0,2279 |
| 20:22037575 | rs6047844 | LINC01432 | | Männliche Glatze | T | C | T | C | 0,4244 | 0,5163 | 0,4244 |
| 20:22050503 | rs1160312 | LINC01432 | | Männliche Glatze | A | G | A | G | 0,2895 | 0,5641 | 0,2895 |
| X:65824986 | rs1385699 | EDA2R | | Männliche Glatze | C | T | | | | | |
| X:66570171 | rs2223841 | | | Männliche Glatze | T | C | | | | | |
| X:66765627 | rs6152 | AR | | Männliche Glatze | G | A | | | | | |
| 1:11854476 | rs1801131 | MTHFR | | Metfromin Wirkung | T | G | T | G | 0,8352 | 0,2664 | 0,2664 |
| 1:11856378 | rs1801133 | MTHFR | | Metfromin Wirkung | G | A | A | G | 0.0778 | 0.01363 | 0.01363 |
| 6:160551204 | rs683369 | SLC22A1 | | Metfromin Wirkung | G | T | C | G | 0,9704 | 0,1315 | 0,1315 |
| 6:160575837 | rs34059508 | SLC22A1 | | Metfromin Wirkung | G | C | A | G | 0,7981 | 0,5056 | 0,5056 |
| 11:10828316 1 | rs11212617 | C11orf65 | | Metfromin Wirkung | C | A | A | C | 0,4845 | 0,3971 | 0,3971 |
| 1:11856378 | rs1801133 | MTHFR | | Methotrexat nd Nebenwirkung bei der Rheumatoid Arthritis | G | A | A | G | 0,0778 | 0,01363 | 0,01363 |
| 1:3083712 | rs2651899 | LOC105378606 PRDM16 | ;; | Migräne | T | C | A | G | 0,02532 | 0,06459 | 0,02532 |
| 10:64445564 | rs10761659 | | | Morbus Crohn | A | G | T | C | 0,3663 | 0,854 | 0,3663 |
| 10:10129159 3 | rs11190140 | LINC01475;; NKX2-3 | | Morbus Crohn | T | C | T | G | 0,01096 | 0,06562 | 0,01096 |
| 5:40401509 | rs17234657 | | | Morbus Crohn | T | G | A | G | 0,4273 | 0,2803 | 0,2803 |
| 18:12809340 | rs1893217 | PTPN2 | | Morbus Crohn | A | G | T | C | 0,8064 | 0,7651 | 0,7651 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min | |
|---|---|---|---|---|---|---|---|---|---|---|---|
| 16:50745926 | rs2066844 | NOD2 | | Morbus Crohn | C | T | C | G | 0,6521 | 0,7906 | 0,6521 |
| 16:50756540 | rs2066845 | NOD2 | | Morbus Crohn | G | T | | | | | |
| | rs2066847 | | Morbus Crohn | | | A | G | 0,3985 | 0,2764 | 0,2764 | |
| 2:234183368 | rs2241880 | ATG16L1;; SCARNA5 | Morbus Crohn | A | G | A | G | 0,3539 | 0,6487 | 0,3539 | |
| 3:49721532 | rs3197999 | APEH ;; MST1 | Morbus Crohn | G | A | T | G | 0,8808 | 0,9742 | 0,8808 | |
| 3:182760073 | rs10513789 | MCCC1 | Morbus Parkinson | T | G | T | G | 0,05897 | 0,3356 | 0,05897 | |
| 3:105912130 | rs12487066 | LOC105374027 | Multiple Sclerose | T | C | T | C | 0,2596 | 0,2201 | 0,2201 | |
| 5:35874575 | rs6897932 | IL7R | Multiple Sclerose | C | T | T | C | 0,2924 | 0,3822 | 0,2924 | |
| 6:32336187 | rs3129934 | TSBP1 | Multiple Sclerose | T | C | T | C | 0,5185 | 0,9013 | 0,5185 | |
| 6:32413051 | rs3135388 | HLA-DRA | Multiple Sclerose | A | G | A | G | 0,9633 | 0,6546 | 0,6546 | |
| 13:90365922 | rs7326018 | | Multiple Sclerose | A | T | A | T | 0,9396 | 0,7982 | 0,7982 | |
| | rs725613 | | Multiple Sclerose | | | | | | | | |
| 14:23002684 | rs1154155 | | Narcolepsie | T | A | A | G | 0,4012 | 0,586 | 0,4012 | |
| 19:10226052 | rs2305795 | EIF3G ;; P2RY11 | Narcolepsie | G | C | | | | | | |
| 2:113594867 | rs16944 | IL1B | Osteoarthrose der Hüfte | A | G | A | G | 0,009067 | 0,215 | 0,009067 | |
| 6:32373312 | rs10947262 | BTNL2 | Osteoarthrose der Knie | C | T | T | C | 0,9854 | 0,8219 | 0,8219 | |
| 7:106938420 | rs3815148 | COG5 | Osteoarthrose der Knie | A | C | A | C | 0,3404 | 0,09473 | 0,09473 | |
| 13:11369450 9 | rs11842874 | MCF2L | Osteoarthrose der Knie | A | G | A | G | 0,997 | 0,4657 | 0,4657 | |
| 20:34025983 | rs143383 | GDF5 | Osteoarthrose der Knie | A | G | A | G | 0,9511 | 0,9968 | 0,9511 | |
| 7:121033121 | rs7776725 | FAM3C | Osteoporose | T | C | T | C | 0,1578 | 0,5018 | 0,1578 | |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 11:68201295 | rs3736228 | LRP5 | Osteoporose | C | T | T | C | 0,8167 | 0,8837 | 0,8167 |
| 12:48239835 | rs1544410 | VDR | Osteoporose | C | T | T | C | 0,2047 | 0,7082 | 0,2047 |
| 2:160175310 | rs174230 | BAZ2B | Plötzlicher Herztod | T | G | T | G | 0,3048 | 0,8443 | 0,3048 |
| 17:78090815 | rs1800312 | GAA | Pompe Erkankung 1 & 2 | G | C | | | | | |
| 17:78086721 | rs28940868 | GAA | Pompe Erkrankung 1 & 2 | C | T | | | | | |
| 6:32655218 | rs2856683 | | Primäre biliäre Zirrhose | T | G | A | G | 0,3475 | 0,9611 | 0,3475 |
| 1:67685387 | rs7530511 | IL23R | Psoriasis | T | C | T | C | 0,5112 | 0,7308 | 0,5112 |
| 1:67705958 | rs11209026 | IL23R | Psoriasis | G | A | A | G | 0,999 | 0,9629 | 0,9629 |
| 5:158742950 | rs3212227 | IL12B | Psoriasis | T | G | T | G | 0,2265 | 0,1345 | 0,1345 |
| 5:158822645 | rs6887695 | | Psoriasis | G | C | C | G | 0,5369 | 0,2323 | 0,2323 |
| 6:31274555 | rs10484554 | LOC112267902 | Psoriasis | C | T | T | C | 0,8265 | 0,7969 | 0,7969 |
| 1:17657534 | rs11203366 | PADI4 | Rheumatoide Arthritis | G | A | T | G | 0,3211 | 0,2768 | 0,2768 |
| 2:61136129 | rs13031237 | REL | Rheumatoide Arthritis | G | T | A | C | 0,9198 | 0,7014 | 0,7014 |
| 20:44742064 | rs1569723 | | Rheumatoide Arthritis | C | A | A | G | 0,2419 | 0,7863 | 0,2419 |
| 6:137973068 | rs2327832 | | Rheumatoide Arthritis | A | G | A | G | 0,7764 | 0,4492 | 0,4492 |
| 1:67694202 | rs2201841 | IL23R | Rheumatoide Arthritis | A | T | A | G | 0,3413 | 0,6263 | 0,3413 |
| 1:67725120 | rs10889677 | IL23R | Rheumatoide Arthritis | C | A | A | C | 0,3795 | 0,7249 | 0,3795 |
| 1:80572058 | rs11162922 | | Rheumatoide Arthritis | A | G | A | G | 0,5021 | 0,8768 | 0,5021 |
| 1:114303808 | rs6679677 | PHTF1 | Rheumatoide Arthritis | C | A | A | C | 0,9213 | 0,3672 | 0,3672 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 2:187521260 | rs3738919 | ITGAV | Rheumatoide Arthritis | C | G | A | C | 0,9065 | 0,9587 | 0,9065 |
| 2:191964633 | rs7574865 | STAT4 | Rheumatoide Arthritis | T | G | T | G | 0,5145 | 0,405 | 0,405 |
| 2:204738919 | rs3087243 | CTLA4 | Rheumatoide Arthritis | G | A | A | G | 0,4019 | 0,6746 | 0,4019 |
| 4:25417244 | rs3816587 | ANAPC4 | Rheumatoide Arthritis | C | T | T | C | 0,5478 | 0,8512 | 0,5478 |
| 6:31583931 | rs2269475 | AIF1 | Rheumatoide Arthritis | C | T | T | C | 0,3809 | 0,2739 | 0,2739 |
| 6:32282854 | rs6910071 | TSBP1 | Rheumatoide Arthritis | A | G | A | G | 0,6704 | 0,5187 | 0,5187 |
| 6:32663851 | rs6457617 | | Rheumatoide Arthritis | C | T | T | C | 0,8585 | 0,6962 | 0,6962 |
| 6:138002637 | rs10499194 | | Rheumatoide Arthritis | C | T | T | C | 0,3404 | 0,3506 | 0,3404 |
| 6:138006504 | rs6920220 | | Rheumatoide Arthritis | G | A | A | G | 0,4237 | 0,8867 | 0,4237 |
| 7:128568960 | rs729302 | | Rheumatoide Arthritis | A | C | A | C | 0,1299 | 0,1673 | 0,1299 |
| 7:128578301 | rs2004640 | IRF5 | Rheumatoide Arthritis | G | T | T | G | 0,3716 | 0,2603 | 0,2603 |
| 7:128594183 | rs10488631 | TNPO3 | Rheumatoide Arthritis | T | C | T | C | 0,8225 | 0,8396 | 0,8225 |
| 9:123690239 | rs3761847 | TRAF1 | Rheumatoide Arthritis | G | A | A | G | 0,9839 | 0,7778 | 0,7778 |
| 9:123705087 | rs10818488 | C5-OT1 | Rheumatoide Arthritis | A | G | A | G | 0,9781 | 0,7674 | 0,7674 |
| 10:6099045 | rs2104286 | IL2RA | Rheumatoide Arthritis | T | C | T | C | 0,422 | 0,9505 | 0,422 |
| 13:20950092 | rs9550642 | | Rheumatoide Arthritis | G | A | A | G | 0,6324 | 0,833 | 0,6324 |
| 21:42511918 | rs2837960 | | Rheumatoide Arthritis | T | G | T | G | 0,7717 | 0,9734 | 0,7717 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| | rs1953126 | | Rheumatoide Arthritis | | | | | | | |
| 1:2546229 | rs6684865 | MMEL1 | Rheumatoide Arthritis | G | A | A | G | 0,2733 | 0,3967 | 0,2733 |
| 1:2553624 | rs3890745 | MMEL1 | Rheumatoide Arthritis | T | C | T | C | 0,2703 | 0,3782 | 0,2703 |
| 10:81926702 | rs1049550 | ANXA11 | Sarcoidose | G | C | A | G | 0,8441 | 0,4362 | 0,4362 |
| 12:783484 | rs12425791 | | Schalganfall | G | C | C | G | 0,08439 | 0,01251 | 0,01251 |
| 11:92698427 | rs10830962 | | Schangersch aftsdiabetes (GDM) | C | T | | | | | |
| 3:185511687 | rs4402960 | IGF2BP2 | Schwangersc haftsdiabetis | G | T | T | G | 0,249 | 0,5633 | 0,249 |
| 7:44229068 | rs1799884 | GCK | Schwangersc haftsdiabetis | C | T | T | C | 0,265 | 0,07909 | 0,07909 |
| 10:11475834 9 | rs7903146 | TCF7L2 | Schwangersc haftsdiabetis | C | T | T | C | 0,03868 | 0,4714 | 0,03868 |
| 10:11480890 2 | rs12255372 | TCF7L2 | Schwangersc haftsdiabetis | G | T | T | G | 0,1568 | 0,9093 | 0,1568 |
| 11:92708710 | rs10830963 | MTNR1B | Schwangersc haftsdiabetis | C | G | C | G | 0,1388 | 0,01251 | 0,01251 |
| 6:32605884 | rs2187668 | HLA-DQA1 | Selective IgA Mangel | C | T | T | C | 0,7448 | 0,6481 | 0,6481 |
| 2:163124051 | rs1990760 | IFIH1 | Selective IgA Mangel | C | T | A | G | 0,6252 | 0,7827 | 0,6252 |
| 6:32586854 | rs9271366 | | Selective IgA Mangel | G | A | A | G | 0,4273 | 0,2803 | 0,2803 |
| 1:67670213 | rs1004819 | IL23R | Spondylitis ankylosans | G | A | A | G | 0,6833 | 0,6509 | 0,6509 |
| 1:67688349 | rs10489629 | IL23R | Spondylitis ankylosans | T | C | T | C | 0,9555 | 0,7215 | 0,7215 |
| 1:67694202 | rs2201841 | IL23R | Spondylitis ankylosans | A | T | A | G | 0,3413 | 0,6263 | 0,3413 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 1:67702526 | rs11465804 | IL23R | Spondylitis ankylosans | T | G | T | G | 0,9125 | 0,9025 | 0,9025 |
| 1:67705958 | rs11209026 | IL23R | Spondylitis ankylosans | G | A | A | G | 0,999 | 0,9629 | 0,9629 |
| 1:67719129 | rs1343151 | IL23R | Spondylitis ankylosans | G | A | A | G | 0,9273 | 0,8844 | 0,8844 |
| 1:67725120 | rs10889677 | IL23R | Spondylitis ankylosans | C | A | A | C | 0,3795 | 0,7249 | 0,3795 |
| 1:67740092 | rs11209032 | | Spondylitis ankylosans | G | A | A | G | 0,3617 | 0,6698 | 0,3617 |
| 2:62551472 | rs10865331 | | Spondylitis ankylosans | A | G | A | G | 0,8624 | 0,5389 | 0,5389 |
| 5:96118866 | rs17482078 | ERAP1 | Spondylitis ankylosans | C | T | T | C | 0,3303 | 0,4519 | 0,3303 |
| 5:96122210 | rs10050860 | ERAP1 | Spondylitis ankylosans | C | T | T | C | 0,2741 | 0,4598 | 0,2741 |
| 5:96129535 | rs2287987 | ERAP1 | Spondylitis ankylosans | T | C | T | C | 0,2945 | 0,4699 | 0,2945 |
| 5:96350710 | rs2303138 | LNPEP | Spondylitis ankylosans | G | A | A | G | 0,7454 | 0,1762 | 0,1762 |
| 8:6735423 | rs1800972 | DEFB1 | Tendinopathien obere Extremität | C | T | C | G | 0,4286 | 0,2757 | 0,2757 |
| 9:117835899 | rs1138545 | TNC | Tendinopathien obere Extremität | C | T | T | C | 0,255 | 0,3223 | 0,255 |
| 17:48277749 | rs1800012 | COL1A1 | Tendinopathien obere Extremität | C | A | A | C | 0,009991 | 0,03158 | 0,009991 |
| 9:1.17808785 | rs2104772 | TNC | Tendinopathien untere Extremität | T | A | A | T | 0,9621 | 0,8069 | 0,8069 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 9:137734416 | rs12722 | COL5A1 | Tendinopathien untere Extremität | C | T | T | C | 0,00303 | 0,01065 | 0,00303 |
| 10:72518009 | rs4747096 | ADAMTS14 | Tendinopathien untere Extremität | A | T | A | G | 0,818 | 0,3667 | 0,3667 |
| 11:10271362 0 | rs679620 | MMP3 | Tendinopathien untere Extremität | T | C | T | C | 0,1421 | 0,05979 | 0,05979 |
| 17:48277749 | rs1800012 | COL1A1 | Tendinopathien untere Extremität | C | A | A | C | 0,009991 | 0,03158 | 0,009991 |
| 17:76924275 | rs4789932 | | Tendinopathien untere Extremität | G | C | A | G | 0,6017 | 0,8457 | 0,6017 |
| 20:34025983 | rs143383 | GDF5 | Tendinopathien untere Extremität | A | G | A | G | 0,9511 | 0,9968 | 0,9511 |
| 1:169519049 | rs6025 | F5 | Tiefe Venenthrombose | T | C | | | | | |
| 1:169701060 | rs5361 | SELE | Tiefe Venenthrombose | T | G | T | G | 0,8169 | 0,5763 | 0,5763 |
| 8:19813529 | rs268 | LPL | Tiefe Venenthrombose | A | G | A | G | 0,8868 | 0,9871 | 0,8868 |
| 18:12809340 | rs1893217 | PTPN2 | Typ 1 Diabetes | A | G | T | C | 0,7448 | 0,6481 | 0,6481 |
| 2:163124051 | rs1990760 | IFIH1 | Typ 1 Diabetes | C | T | A | G | 0,06884 | 0,205 | 0,06884 |
| 11:2169774 | rs3741208 | IGF2 | Typ 1 Diabetes | A | T | A | C | 0,4085 | 0,9945 | 0,4085 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 6:32626272 | rs9273363 | HLA-DQB1-AS1 | Typ 1 Diabetes | C | A | T | C | 0,7118 | 0,7908 | 0,7118 |
| 4:6293350 | rs10012946 | WFS1 | Typ 2 Diabetes | T | C | | | | | |
| | rs1111875 | | Typ 2 Diabetes | | | T | C | 0,1693 | 0,00815 9 | 0,008159 |
| 11:92673828 | rs1387153 | | Typ 2 Diabetes | C | T | T | C | 0,03624 | 0,01747 | 0,01747 |
| 11:2839751 | rs2237892 | KCNQ1 | Typ 2 Diabetes | C | T | A | G | 0,8301 | 0,4121 | 0,4121 |
| 9:22132076 | rs2383208 | | Typ 2 Diabetes | A | T | A | G | 0,2308 | 0,1923 | 0,1923 |
| 6:20657564 | rs4712523 | CDKAL1 | Typ 2 Diabetes | A | G | | | | | |
| | i3002432 | | Venöse Thromboembolie | | | | | | | |
| 4:72608383 | rs2282679 | GC | Vitamin D Spiegel | T | G | T | G | 0,4205 | 0,2585 | 0,2585 |
| 11:14914878 | rs10741657 | CYP2R1 | Vitamin D Spiegel | A | G | A | G | 0,009544 | 0,07337 | 0,009544 |
| 17:41055964 | rs1801175 | G6PC | Von Gierke Erkrankung | C | T | | | | | |
| 17:41055965 | rs1801176 | G6PC | Von Gierke Erkrankung | G | A | | | | | |
| 17:41063017 | rs80356484 | G6PC | Von Gierke Erkrankung | G | T | | | | | |
| 17:41063408 | rs80356487 | G6PC | Von Gierke Erkrankung | C | T | | | | | |
| | rs80356479 | G6PC | Von Gierke Erkrankung | | | | | | | |
| 4:111710169 | rs2200733 | | Vorhof Flimmern | C | T | T | C | 0,7378 | 0,5377 | 0,5377 |

(fortgesetzt)

| coord | DBSNP.ID | dbsnp gene symbol | Merkmal | dbsn p Ref | dbsn p Alt | COV EA | COV OA | Pval maln | Pval_cor r sex_age | Pval min |
|---|---|---|---|---|---|---|---|---|---|---|
| 4:111720761 | rs10033464 | | Vorhof Flimmern | T | G | T | G | 0,4753 | 0,1738 | 0,1738 |
| 10:11580505 6 | rs1801253 | ADRB1 | Wirkung von β-Blockern | G | C | C | G | 0,6586 | 0,2896 | 0,2896 |

**Tabelle 1e: Liste krankheitsassoziierter SNPs**

| | | | |
|---|---|---|---|
| rs63750847 | Alzheimer Erkrankung | rs28930068 | Hypokalemische Periodische Lähmung |
| rs1801131 | Anaphylaxie nach Impfungen | rs28930069 | Hypokalemische Periodische Lähmung |
| rs1801133 | Anaphylaxie nach Impfungen | rs80338779 | Hypokalemische Periodische Lähmung |
| rs949963 | Anaphylaxie nach Impfungen | rs80338777 | Hypokalemische Periodische Lähmung |
| rs2228139 | Anaphylaxie nach Impfungen | rs121908555 | Hypokalemische Periodische Lähmung |
| rs4848300 | Anaphylaxie nach Impfungen | rs80338788 | Hypokalemische Periodische Lähmung |
| rs17561 | Anaphylaxie nach Impfungen | rs80338784 | Hypokalemische Periodische Lähmung |
| rs1800587 | Anaphylaxie nach Impfungen | rs121918007 | Hypophosphatasie |
| rs1801278 | Antidiabeticum Sulphonylharnstoff | rs121918002 | Hypophosphatasie |
| rs1801282 | Antidiabeticum Sulphonylharnstoff | rs2476601 | Hypothyroidismus |
| rs7903146 | Antidiabeticum Sulphonylharnstoff | rs7850258 | Hypothyroidismus |
| rs12255372 | Antidiabeticum Sulphonylharnstoff | rs3184504 | Hypothyroidismus |
| rs5215 | Antidiabeticum Sulphonylharnstoff | rs2517532 | Hypothyroidismus |
| rs5219 | Antidiabeticum Sulphonylharnstoff | rs12610495 | Idiopathische Lungenfibrose |
| rs757110 | Antidiabeticum Sulphonylharnstoff | rs1278769 | Idiopathische Lungenfibrose |
| rs1799859 | Antidiabeticum Sulphonylharnstoff | rs1980653 | Idiopathische Lungenfibrose |
| rs6196 | Antipsychotika und Gewichtszunahme | rs1981997 | Idiopathische Lungenfibrose |
| rs17782313 | Antipsychotika und Gewichtszunahme | rs2034650 | Idiopathische Lungenfibrose |
| rs489693 | Antipsychotika und Gewichtszunahme | rs6793295 | Idiopathische Lungenfibrose |
| rs1801131 | Antipsychotika und Gewichtszunahme | rs7934606 | Idiopathische Lungenfibrose |
| rs5067 | Asthma | rs2187668 | Idiopathische membranöse Nephroapthie |
| rs689465 | Asthma | rs4664308 | Idiopathische membranöse Nephroapthie |
| rs4950928 | Asthma | rs9275596 | IgA Nephropathie |
| rs1042713 | Asthma | rs363039 | Intelligenz Messung |
| rs1695 | Asthma | rs363050 | Intelligenz Messung |
| rs8069176 | Asthma | rs1511412 | Keloidbildung |
| rs7216389 | Asthma | rs8032158 | Keloidbildung |
| rs13153971 | Asthma | rs873549 | Keloidbildung |
| rs37973 | Asthma Inhalation Corticosteroide | rs940187 | Keloidbildung |
| rs2240017 | Asthma Inhalation Corticosteroide | rs7639618 | Knie Osteoarthrose |
| rs11204971 | Atopische Dermatitis | rs4140564 | Knie Osteoarthrose bei Frauen |
| rs4950928 | Atopisches Asthma | rs5751876 | Koffein Genuss |
| rs1143634 | Belastungsbedingte Muskelschäden | rs6968865 | Koffein Genuss |
| rs28497577 | Belastungsbedingte Muskelschäden | rs2472297 | Koffein Genuss |
| rs1800629 | Belastungsbedingte Muskelschäden | rs6511720 | Koronare Herz-Erkrankung |
| rs4880 | Belastungsbedingte Muskelschäden | rs6903956 | Koronare Herz-Erkrankung |
| rs1800795 | Belastungsbedingte Muskelschäden | rs9834312 | Körpergröße |
| rs13266634 | Belastungsbedingte Muskelschäden | rs2179922 | Körpergröße |
| rs1815739 | Belastungsbedingte Muskelschäden | rs1042725 | Körpergröße |
| rs1801282 | Belastungsglukose Test | rs6060369 | Körpergröße |
| rs10936599 | Biologisches Altern | rs8038652 | Körpergröße |
| rs2736100 | Biologisches Altern | rs2802288 | Langlebigkeit |
| rs1042522 | Biologisches Altern | rs2802292 | Langlebigkeit |
| rs1064395 | Bipolare Störung | rs2764264 | Langlebigkeit |
| rs2820037 | Bluthochdruck | rs2811712 | Langlebigkeit |
| rs5186 | Bluthochdruck | rs34516635 | Langlebigkeit |
| rs4961 | Bluthochdruck | rs2073711 | Lumbaler Bandscheibenvorfall |

(fortgesetzt)

| | | | |
|---|---|---|---|
| rs5335 | Bluthochdruck | rs2736100 | Lungenfibrose |
| rs5370 | Bluthochdruck | rs2076295 | Lungenfibrose |
| rs6997709 | Bluthochdruck | rs13277113 | Lupus (Systemic Lupus Erythematosus) |
| rs7961152 | Bluthochdruck | rs2187668 | Lupus (Systemic Lupus Erythematosus) |
| rs11110912 | Bluthochdruck | rs2205960 | Lupus (Systemic Lupus Erythematosus) |
| rs1937506 | Bluthochdruck | rs9888739 | Lupus (Systemic Lupus Erythematosus) |
| rs2398162 | Bluthochdruck | rs9287638 | Männliche Glatze |
| rs13333226 | Bluthochdruck | rs9479482 | Männliche Glatze |
| rs5065 | Bluthochdrucktherapie | rs2073963 | Männliche Glatze |
| rs13130484 | Body Mass Index | rs6945541 | Männliche Glatze |
| rs6971091 | Body Mass Index | rs10502861 | Männliche Glatze |
| rs2272383 | Body Mass Index | rs8085664 | Männliche Glatze |
| rs1528133 | Body Mass Index | rs2180439 | Männliche Glatze |
| rs1121980 | Body Mass Index | rs6047844 | Männliche Glatze |
| rs10871777 | Body Mass Index | rs1160312 | Männliche Glatze |
| rs12970134 | Body Mass Index | rs1385699 | Männliche Glatze |
| rs2229616 | Body Mass Index | rs2223841 | Männliche Glatze |
| rs2272382 | Body Mass Index | rs6152 | Männliche Glatze |
| rs1892534 | C-reactives Protein | rs1801131 | Metfromin Wirkung |
| rs8034191 | Chronisch-obstruktive Lungenerkrankung (COPD) | rs1801133 | Metfromin Wirkung |
| rs7671167 | Chronisch-obstruktive Lungenerkrankung (COPD) | rs683369 | Metfromin Wirkung |
| rs2653349 | Cluster Kopfschmerz | rs34059508 | Metfromin Wirkung |
| rs10883365 | Colitis ulcerosa | rs11212617 | Metfromin Wirkung |
| rs2395185 | Colitis ulcerosa | rs1801133 | Methotrexat nd Nebenwirkung bei der Rheumatoid Arth-ritis |
| rs9858542 | Colitis ulcerosa | rs2651899 | Migräne |
| rs11209026 | Colitis Ulcerosa | rs10761659 | Morbus Crohn |
| rs646776 | Coronare Herzerkrankung | rs11190140 | Morbus Crohn |
| rs599839 | Coronare Herzerkrankung | rs17234657 | Morbus Crohn |
| rs3900940 | Coronare Herzerkrankung | rs1893217 | Morbus Crohn |
| rs7439293 | Coronare Herzerkrankung | rs2066844 | Morbus Crohn |
| rs383830 | Coronare Herzerkrankung | rs2066845 | Morbus Crohn |
| rs6922269 | Coronare Herzerkrankung | rs2066847 | Morbus Crohn |
| rs3798220 | Coronare Herzerkrankung | rs2241880 | Morbus Crohn |
| rs2070744 | Coronare Herzerkrankung | rs3197999 | Morbus Crohn |
| rs4977574 | Coronare Herzerkrankung | rs10513789 | Morbus Parkinson |
| rs1333048 | Coronare Herzerkrankung | rs12487066 | Multiple Sclerose |
| rs1333049 | Coronare Herzerkrankung | rs6897932 | Multiple Sclerose |
| rs501120 | Coronare Herzerkrankung | rs3129934 | Multiple Sclerose |
| rs964184 | Coronare Herzerkrankung | rs3135388 | Multiple Sclerose |
| rs2298566 | Coronare Herzerkrankung | rs7326018 | Multiple Sclerose |
| rs3184504 | Coronare Herzerkrankung | rs725613 | Multiple Sclerose |
| rs17228212 | Coronare Herzerkrankung | rs1154155 | Narcolepsie |
| rs8055236 | Coronare Herzerkrankung | rs2305795 | Narcolepsie |
| rs7250581 | Coronare Herzerkrankung | rs16944 | Osteoarthrose der Hüfte |
| rs4420638 | Coronare Herzerkrankung | rs10947262 | Osteoarthrose der Knie |
| rs688034 | Coronare Herzerkrankung | rs3815148 | Osteoarthrose der Knie |
| rs1360780 | Depression | rs11842874 | Osteoarthrose der Knie |
| rs1545843 | Depression | rs143383 | Osteoarthrose der Knie |

(fortgesetzt)

| | | | |
|---|---|---|---|
| rs1031681 | Depression | rs7776725 | Osteoporose |
| rs4655595 | Diabetes Mellitus, Type 2 | rs3736228 | Osteoporose |
| rs6718526 | Diabetes Mellitus, Type 2 | rs1544410 | Osteoporose |
| rs1801282 | Diabetes Mellitus, Type 2 | rs174230 | Plötzlicher Herztod |
| rs358806 | Diabetes Mellitus, Type 2 | rs1800312 | Pompe Erkankung 1 & 2 |
| rs7659604 | Diabetes Mellitus, Type 2 | rs28940868 | Pompe Erkrankung 1 & 2 |
| rs9465871 | Diabetes Mellitus, Type 2 | rs2856683 | Primäre biliäre Zirrhose |
| rs1799884 | Diabetes Mellitus, Type 2 | rs7530511 | Psoriasis |
| rs13266634 | Diabetes Mellitus, Type 2 | rs11209026 | Psoriasis |
| rs10811661 | Diabetes Mellitus, Type 2 | rs3212227 | Psoriasis |
| rs9326506 | Diabetes Mellitus, Type 2 | rs6887695 | Psoriasis |
| rs4506565 | Diabetes Mellitus, Type 2 | rs10484554 | Psoriasis |
| rs7903146 | Diabetes Mellitus, Type 2 | rs11203366 | Rheumatoide Arthritis |
| rs12255372 | Diabetes Mellitus, Type 2 | rs13031237 | Rheumatoide Arthritis |
| rs5219 | Diabetes Mellitus, Type 2 | rs1569723 | Rheumatoide Arthritis |
| rs9300039 | Diabetes Mellitus, Type 2 | rs2327832 | Rheumatoide Arthritis |
| rs10830963 | Diabetes Mellitus, Type 2 | rs2201841 | Rheumatoide Arthritis |
| rs12304921 | Diabetes Mellitus, Type 2 | rs10889677 | Rheumatoide Arthritis |
| rs1495377 | Diabetes Mellitus, Type 2 | rs11162922 | Rheumatoide Arthritis |
| rs2930291 | Diabetes Mellitus, Type 2 | rs6679677 | Rheumatoide Arthritis |
| rs2903265 | Diabetes Mellitus, Type 2 | rs3738919 | Rheumatoide Arthritis |
| rs8050136 | Diabetes Mellitus, Type 2 | rs7574865 | Rheumatoide Arthritis |
| rs11868035 | Diabetes Mellitus, Type 2 | rs3087243 | Rheumatoide Arthritis |
| rs1889018 | Diabetes Mellitus, Type 2 | rs3816587 | Rheumatoide Arthritis |
| rs2295490 | Diabetes Mellitus, Type 2 | rs2269475 | Rheumatoide Arthritis |
| rs104893919 | Diastophische Dysplasia | rs6910071 | Rheumatoide Arthritis |
| rs104893915 | Diastophische Dysplasia | rs6457617 | Rheumatoide Arthritis |
| rs104893924 | Diastophische Dysplasia | rs10499194 | Rheumatoide Arthritis |
| rs5082 | Diätwirkung | rs6920220 | Rheumatoide Arthritis |
| rs1801282 | Diätwirkung | rs729302 | Rheumatoide Arthritis |
| rs17300539 | Diätwirkung | rs2004640 | Rheumatoide Arthritis |
| rs1800497 | Diätwirkung | rs10488631 | Rheumatoide Arthritis |
| rs662799 | Diätwirkung | rs3761847 | Rheumatoide Arthritis |
| rs16879765 | Dupuytren’sche Erkrankung | rs10818488 | Rheumatoide Arthritis |
| rs16946160 | Erworbenes nephrotisches Syndrom | rs2104286 | Rheumatoide Arthritis |
| rs12713559 | Familiäre Hypercholesterolämie Type B | rs9550642 | Rheumatoide Arthritis |
| rs5742904 | Familiäre Hypercholesterolämie Type B | rs2837960 | Rheumatoide Arthritis |
| rs144467873 | Familiäre Hypercholesterolämie Type B | rs1953126 | Rheumatoide Arthritis |
| rs28933405 | Familiäre Hypertrophische Kardiomyopathie | rs6684865 | Rheumatoide Arthritis |
| rs28938173 | Familiäre Hypertrophische Kardiomyopathie | rs3890745 | Rheumatoide Arthritis |
| rs104894369 | Familiäre Hypertrophische Kardiomyopathie | rs1049550 | Sarcoidose |
| rs104894370 | Familiäre Hypertrophische Kardiomyopathie | rs12425791 | Schalganfall |
| rs3218714 | Familiäre Hypertrophische Kardiomyopathie | rs10830962 | Schangerschaftsdiabetes (GDM) |

(fortgesetzt)

| | | | |
|---|---|---|---|
| rs3218713 | Familiäre Hypertrophische Kardiomyopathie | rs4402960 | Schwangerschaftsdiabetis |
| rs104894503 | Familiäre Hypertrophische Kardiomyopathie | rs1799884 | Schwangerschaftsdiabetis |
| rs104894502 | Familiäre Hypertrophische Kardiomyopathie | rs7903146 | Schwangerschaftsdiabetis |
| rs1801282 | Fettgewebebildung | rs12255372 | Schwangerschaftsdiabetis |
| rs4788102 | Fettsucht | rs10830963 | Schwangerschaftsdiabetis |
| rs6548238 | Fettsucht | rs2187668 | Selective IgA Mangel |
| rs7647305 | Fettsucht | rs1990760 | Selective IgA Mangel |
| rs925946 | Fettsucht | rs9271366 | Selective IgA Mangel |
| rs5082 | Fettsucht | rs1004819 | Spondylitis ankylosans |
| rs17300539 | Fettsucht | rs10489629 | Spondylitis ankylosans |
| rs35859249 | Fettsucht | rs2201841 | Spondylitis ankylosans |
| rs6232 | Fettsucht | rs11465804 | Spondylitis ankylosans |
| rs6971091 | Fettsucht | rs11209026 | Spondylitis ankylosans |
| rs662799 | Fettsucht | rs1343151 | Spondylitis ankylosans |
| rs17817449 | Fettsucht | rs10889677 | Spondylitis ankylosans |
| rs17782313 | Fettsucht | rs11209032 | Spondylitis ankylosans |
| rs2229616 | Fettsucht | rs10865331 | Spondylitis ankylosans |
| rs1799884 | Geburtsgewicht | rs17482078 | Spondylitis ankylosans |
| rs7903146 | Geburtsgewicht | rs10050860 | Spondylitis ankylosans |
| rs1051730 | Geburtsgewicht | rs2287987 | Spondylitis ankylosans |
| rs12565727 | Glatze | rs2303138 | Spondylitis ankylosans |
| rs76723693 | Glucose-6-phosphate-dehydrogenasemangel | rs1800972 | Tendinopathien obere Extremität |
| rs137852318 | Glucose-6-phosphate-dehydrogenasemangel | rs1138545 | Tendinopathien obere Extremität |
| rs5030868 | Glucose-6-phosphate-dehydrogenasemangel | rs1800012 | Tendinopathien obere Extremität |
| rs1050828 | Glucose-6-phosphate-dehydrogenasemangel | rs2104772 | Tendinopathien untere Extremität |
| rs80356491 | Glycogen Speicher Krankheit 1B | rs12722 | Tendinopathien untere Extremität |
| rs80356490 | Glycogen Speicher Krankheit 1B | rs4747096 | Tendinopathien untere Extremität |
| rs80356489 | Glycogen Speicher Krankheit 1B | rs679620 | Tendinopathien untere Extremität |
| rs113994129 | Glycogen Speicher Krankheit Typ 3 | rs1800012 | Tendinopathien untere Extremität |
| rs113994130 | Glycogen Speicher Krankheit Typ 3 | rs4789932 | Tendinopathien untere Extremität |
| rs113994131 | Glycogen Speicher Krankheit Typ 3 | rs143383 | Tendinopathien untere Extremität |
| rs119103251 | Glycogen Speicherkrankheit Typ 5 | rs6025 | Tiefe Venenthrombose |
| rs116987552 | Glycogen Speicherkrankheit Typ 5 | rs5361 | Tiefe Venenthrombose |
| rs2155219 | Graspollen-Allergie und Heufieber | rs268 | Tiefe Venenthrombose |
| rs646776 | Herzinfarkt | rs1893217 | Typ 1 Diabetes |
| rs17465637 | Herzinfarkt | rs1990760 | Typ 1 Diabetes |
| rs8089 | Herzinfarkt | rs3741208 | Typ 1 Diabetes |
| rs1333040 | Herzinfarkt | rs9273363 | Typ 1 Diabetes |
| rs2383206 | Herzinfarkt | rs10012946 | Typ 2 Diabetes |
| rs2383207 | Herzinfarkt | rs1111875 | Typ 2 Diabetes |
| rs10757278 | Herzinfarkt | rs1387153 | Typ 2 Diabetes |
| rs1927911 | Herzinfarkt | rs2237892 | Typ 2 Diabetes |
| rs501120 | Herzinfarkt | rs2383208 | Typ 2 Diabetes |
| rs662799 | Herzinfarkt | rs4712523 | Typ 2 Diabetes |

(fortgesetzt)

| | | | |
|---|---|---|---|
| rs17228212 | Herzinfarkt | i3002432 | Venöse Thromboembolie |
| rs17576 | Herzinfarkt | rs2282679 | Vitamin D Spiegel |
| rs3782886 | Herzinfarkt | rs10741657 | Vitamin D Spiegel |
| rs7291467 | Herzinfarkt | rs1801175 | Von Gierke Erkrankung |
| rs5742905 | Homocystinurie, Pyridoxin abhängig | rs1801176 | Von Gierke Erkrankung |
| rs12413409 | Hypertension | rs80356484 | Von Gierke Erkrankung |
| rs1378942 | Hypertension | rs80356487 | Von Gierke Erkrankung |
| rs16998073 | Hypertension | rs80356479 | Von Gierke Erkrankung |
| rs17367504 | Hypertension | rs2200733 | Vorhof Flimmern |
| rs3754777 | Hypertension | rs10033464 | Vorhof Flimmern |
| rs964184 | Hypertriglyceridemie | rs1801253 | Wirkung von β-Blockern |

**Tabelle 2a: Covid-19 Impfempfehlung**

| Kohorte Proben-ID | 32 $p<10^5$ [tmg] | | | 381 [all] | $p<10^5$ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| | **0** | **1** | **2** | **0** | **1** | **2** | | | | | | | | |
| CG69 GS12886 | 12 | 1 | 19 | 197 | 14 | 170 | hoch | hoch | anfällig | T04 | M03 | A | 1A: Umgehend | |
| TMG | GL36724711RQ | 10 | 5 | 19 | | | | | | | | M03 | | |
| TMG | GL23757411XK | 11 | 4 | 19 | | | | | | | | M03 | | |
| TMG | GL52779612GN | 13 | 2 | 19 | | | | | | | | M01 | | |
| TMG | W2N2T4B5U5U 7 | 13 | 3 | 18 | | | | | | | | M03 | | |
| 1kG | HG02391 | 14 | | 18 | 201 | 14 | 166 | hoch | hoch | anfällig | T04 | M03 | A | 1A: Umgehend |
| 1kG | NA20505 | 13 | 1 | 18 | 191 | 24 | 166 | hoch | hoch | anfällig | T04 | M03 | A | 1A: Umgehend |
| 1kG | NA20530 | 12 | 2 | 18 | 188 | 27 | 166 | hoch | hoch | anfällig | T04 | M03 | A | 1A: Umgehend |
| 1kG | HG01583 | 13 | 1 | 18 | 191 | 25 | 165 | hoch | hoch | anfällig | T04 | M03 | A | 1A: Umgehend |
| 1kG | HG00739 | 12 | 2 | 18 | 164 | 72 | 145 | moderat | hoch | anfällig | T07 | M03 | A | 2A: Dringend |
| 1kG | HG02185 | 13 | 1 | 18 | 188 | 20 | 173 | hoch | hoch | anfällig | T04 | M03 | A | 1A: Umgehend |
| 1kG | HG00346 | 13 | 1 | 18 | 180 | 34 | 167 | hoch | hoch | anfällig | T04 | M03 | A | 1A: Umgehend |
| 1kG | HG00356 | 13 | 1 | 18 | 184 | 33 | 164 | hoch | hoch | anfällig | T04 | M03 | A | 1A: Umgehend |
| 1kG | NA12046 | 12 | 2 | 18 | 187 | 25 | 169 | hoch | hoch | anfällig | T04 | M03 | A | 1A: Umgehend |
| TMG | H6E4T8E0H3D1 | 13 | 3 | 3 18 | | | | | | | | M03 | | |
| 1kG | NA19190 | 13 | 1 | 18 | 184 | 51 | 146 | moderat | hoch | anfällig | T15 | M03 | A | 2A: Dringend |
| TMG | GL22377811YA | 10 | 4 | 18 | | | | | | | | M03 | | |
| 1kG | NA10847 | 13 | 1 | 18 | 191 | 22 | 168 | hoch | hoch | anfällig | T04 | M03 | A | 1A: Umgehend |
| TMG | GL32292210QG | 11 | 3 | 18 | | | | | | | | M03 | | |

(fortgesetzt)

| Kohorte Proben-ID | 32 $p<10^5$ [tmg] | | | 381 [all] | $p<10^5$ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| TMG | GL64456713ZJ | 13 | 3 | 18 | | | | | | | | M03 | | |
| TMG | GL39282510FX | 11 | 4 | 18 | | | | | | | | M01 | | |
| TMG | GL24222510XX | 13 | 2 | 18 | | | | | | | | M01 | | |
| TMG | GL44792211DV | 15 | 1 | 18 | | | | | | | | M01 | | |
| TMG | GL98739411WZ | 14 | 1 | 18 | | | | | | | | M01 | | |
| TMG | GL55694410KN | 11 | 6 | 17 | | | | | | | | M03 | | |
| 1kG | HG01603 | 13 | 2 | 17 | 184 | 33 | 164 | hoch | hoch | anfällig | T04 | M03 | A | 1A: Umgehend |
| 1kG | HG01615 | 13 | 2 | 17 | 190 | 26 | 165 | hoch | hoch | anfällig | T04 | M03 | A | 1A: Umgehend |
| 1kG | NA18619 | 14 | 1 | 17 | 171 | 61 | 149 | moderat | hoch | anfällig | T07 | M03 | A | 2A: Dringend |
| 1kG | | 12 | 3 | 17 | 186 | 25 | 170 | hoch | hoch | anfällig | T04 | M03 | A | 1A: Umgehend |
| 1kG | HG00285 | 11 | 4 | 17 | 186 | 27 | 168 | hoch | hoch | anfällig | T04 | M03 | A | 1A: Umgehend |
| 1kG | NA18625 | 12 | 3 | 17 | 161 | 80 | 140 | moderat | hoch | anfällig | T07 | M03 | A | 2A: Dringend |
| 1kG | HG01868 | 12 | 3 | 17 | 158 | 85 | 138 | moderat_gering | hoch | anfällig | T24 | M03 | A | 3A: Baldig |
| 1kG | HG01507 | 13 | 2 | 17 | 184 | 28 | 169 | hoch | hoch | anfällig | T04 | M03 | A | 1A: Umgehend |
| 1kG | NA12760 | 14 | 1 | 17 | 193 | 27 | 161 | hoch | hoch | anfällig | T04 | M03 | A | 1A: Umgehend |
| 1kG | NA19722 | 12 | 3 | 17 | 181 | 44 | 156 | moderat | hoch | anfällig | T07 | M03 | A | 2A: Dringend |
| 1kG | HG01623 | 13 | 2 | 17 | 186 | 24 | 171 | hoch | hoch | anfällig | T04 | M03 | A | 1A: Umgehend |
| 1kG | HG01437 | 13 | 2 | 17 | 177 | 42 | 162 | hoch | hoch | anfällig | T04 | M03 | A | 1A: Umgehend |
| 1kG | NA18983 | 14 | 1 | 17 | 183 | 18 | 180 | hoch | hoch | anfällig | T04 | M03 | A | 1A: Umgehend |
| 1kG | NA19774 | 14 | 1 | 17 | 187 | 19 | 175 | hoch | hoch | anfällig | T04 | M03 | A | 1A: Umgehend |

(fortgesetzt)

| Kohorte Proben-ID | | 32 $p<10^5$ [tmg] | | 381 [all] | $p<10^5$ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| TMG | GL22283511TB | 12 | 4 | 17 | | | | | | | | M03 | | |
| TMG | GL48992215KV | 10 | 5 | 17 | | | | | | | | M03 | | |
| 1kG | HG00376 | 13 | 2 | 17 | 190 | 27 | 164 | hoch | hoch | anfällig | T04 | M03 | A | 1A: Umgehend |
| 1kG | HG03947 | 13 | 2 | 17 | 192 | 25 | 164 | hoch | hoch | anfällig | T04 | M03 | A | 1A: Umgehend |
| 1kG | HG04152 | 12 | 3 | 17 | 167 | 58 | 156 | moderat | hoch | anfällig | T07 | M03 | A | 2A: Dringend |
| 1kG | HG00266 | 14 | 1 | 17 | 189 | 24 | 168 | hoch | hoch | anfällig | T04 | M03 | A | 1A: Umgehend |
| 1kG | HG01766 | 12 | 3 | 17 | 182 | 36 | 163 | hoch | hoch | anfällig | T04 | M03 | A | 1A: Umgehend |
| 1kG | HG01104 | 13 | 2 | 17 | 182 | 41 | 158 | moderat | hoch | anfällig | T07 | M03 | A | 2A: Dringend |
| 1kG | HG03228 | 13 | 2 | 17 | 161 | 76 | 144 | moderat | hoch | anfällig | T07 | M03 | A | 2A: Dringend |
| 1kG | HG00360 | 12 | 3 | 17 | 190 | 23 | 168 | hoch | hoch | anfällig | T04 | M03 | A | 1A: Umgehend |
| 1kG | HG03781 | 12 | 3 | 17 | 183 | 35 | 163 | hoch | hoch | anfällig | T04 | M03 | A | 1A: Umgehend |
| TMG | GL29428214MW | 12 | 5 | 17 | | | | | | | | M03 | | |
| TMG | GL22872212FX | 12 | 4 | 17 | | | | | | | | M03 | | |
| TMG | GL63427410WK | 13 | 3 | 17 | | | | | | | | M01 | | |
| TMG | GL92376214QU | 15 | 2 | 17 | | | | | | | | M01 | | |
| TMG | GL93878310NA | 13 | 3 | 17 | | | | | | | | M01 | | |
| TMG | GL83593813PK | 12 | 5 | 17 | | | | | | | | M01 | | |
| TMG | GL39895215XP | 16 | | 17 | | | | | | | | M01 | | |
| TMG | GL42337212KT | 14 | 3 | 17 | | | | | | | | M01 | | |
| TMG | GL25352411CK | 12 | 4 | 17 | | | | | | | | M01 | | |
| TMG | GL28829410AN | 14 | 1 | 17 | | | | | | | | M01 | | |
| TMG | GL22743912JF | 15 | 2 | 17 | | | | | | | | M01 | | |

(fortgesetzt)

| Kohorte Proben-ID | 32 p<10⁵ [tmg] | | | 381 [all] | p<10⁵ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | HG02383 | 13 | 2 | 17 | 189 | 26 | 166 | hoch | hoch | anfällig | T04 | M01 | A | 1A: Umgehend |
| 1kG | HG01130 | 14 | 1 | 17 | 187 | 34 | 160 | hoch | hoch | anfällig | T04 | M01 | A | 1A: Umgehend |
| CG69 | GS12880 | 14 | 1 | 17 | 193 | 21 | 167 | hoch | hoch | anfällig | 704 | M01 | A | 1A: Umgehend |
| 1kG | HG01512 | 13 | 2 | 17 | 160 | 81 | 140 | moderat | hoch | anfällig | T07 | M01 | A | 2A: Dringend |
| 1kG | NA19786 | 15 | | 17 | 186 | 38 | 157 | moderat | hoch | anfällig | T11 | M01 | A | 2A: Dringend |
| 1kG | NA12144 | 13 | 2 | 17 | 186 | 29 | 166 | hoch | hoch | anfällig | T04 | M01 | A | 1A: Umgehend |
| 1kG | NA20761 | 12 | 4 | 16 | 81 | 213 | 87 | asymptomatisch | hoch | anfällig | T47 | M02 | A | 5A: Freiwillig |
| 1kG | NA20875 | 14 | 2 | 16 | 148 | 101 | 132 | moderat_gering | hoch | anfällig | T27 | M03 | A | 3A: Baldig |
| 1kG | NA20587 | 14 | 2 | 16 | 191 | 30 | 160 | hoch | hoch | anfällig | T04 | M03 | A | 1A: Umgehend |
| 1kG | HG00403 | 13 | 3 | 16 | 160 | 80 | 141 | moderat | hoch | anfällig | T07 | M03 | A | 2A: Dringend |
| 1kG | HG00662 | 15 | 1 | 16 | 188 | 37 | 156 | moderat | hoch | anfällig | T07 | M03 | A | 2A: Dringend |
| 1kG | HG01624 | 12 | 4 | 16 | 165 | 65 | 151 | moderat | hoch | anfällig | T07 | M03 | A | 2A: Dringend |
| 1kG | NA20805 | 15 | 1 | 16 | 195 | 19 | 167 | hoch | hoch | anfällig | T04 | M03 | A | 1A: Umgehend |
| 1kG | HG00337 | 13 | 3 | 16 | 175 | 42 | 164 | hoch | hoch | anfällig | T04 | M03 | A | 1A: Umgehend |
| 1kG | HG00369 | 13 | 3 | 16 | 175 | 47 | 159 | moderat | hoch | anfällig | T07 | M03 | A | 2A: Dringend |
| 1kG | HG00559 | 13 | 3 | 16 | 155 | 80 | 146 | moderat | hoch | anfällig | T07 | M03 | A | 2A: Dringend |
| 1kG | HG02384 | 13 | 3 | 16 | 156 | 81 | 144 | moderat | hoch | anfällig | T07 | M03 | A | 2A: Dringend |

(fortgesetzt)

| Kohorte Proben-ID | 32 p<10⁵ [tmg] | | | 381 [all] | p<10⁵ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | HG03945 | 13 | 3 | 16 | 172 | 63 | 146 | moderat | hoch | anfällig | T07 | M03 | A | 2A: Dringend |
| 1kG | NA12058 | 12 | 4 | 16 | 178 | 47 | 156 | moderat | hoch | anfällig | T07 | M03 | A | 2A: Dringend |
| 1kG | NA20531 | 11 | 5 | 16 | 147 | 102 | 132 | moderat_gering | hoch | anfällig | T24 | M03 | A | 3A: Baldig |
| 1kG | NA20807 | 11 | 5 | 16 | 142 | 105 | 134 | moderat_gering | hoch | anfällig | T24 | M03 | A | 3A: Baldig |
| CG69 | GS12891 | 12 | 4 | 16 | 171 | 64 | 146 | moderat | hoch | anfällig | T07 | M03 | A | 2A: Dringend |
| 1kG | HG00261 | 13 | 3 | 16 | 188 | 27 | 166 | hoch | hoch | anfällig | T04 | M03 | A | 1A: Umgehend |
| 1kG | NA12341 | 13 | 3 | 16 | 180 | 42 | 159 | moderat | hoch | anfällig | T07 | M03 | A | 2A: Dringend |
| 1kG | NA12778 | 12 | 4 | 16 | 154 | 86 | 141 | moderat | hoch | anfällig | T07 | M03 | A | 2A: Dringend |
| 1kG | HG01465 | 13 | 3 | 16 | 168 | 51 | 162 | hoch | hoch | anfällig | T04 | M03 | A | 1A: Umgehend |
| 1kG | NA18612 | 13 | 3 | 16 | 172 | 53 | 156 | moderat | hoch | anfällig | T07 | M03 | A | 2A: Dringend |
| 1kG | HG00186 | 12 | 4 | 16 | 186 | 32 | 163 | hoch | hoch | anfällig | T04 | M03 | A | 1A: Umgehend |
| 1kG | NA18618 | 14 | 2 | 16 | 187 | 33 | 161 | hoch | hoch | anfällig | T04 | M03 | A | 1A: Umgehend |
| 1kG | NA18982 | 14 | 2 | 16 | 190 | 19 | 172 | hoch | hoch | anfällig | T04 | M03 | A | 1A: Umgehend |
| 1kG | NA07051 | 13 | 3 | 16 | 184 | 38 | 159 | moderat | hoch | anfällig | T07 | M03 | A | 2A: Dringend |
| CG69 | GS12883 | 12 | 4 | 16 | 178 | 48 | 155 | moderat | hoch | anfällig | T07 | M03 | A | 2A: Dringend |
| 1kG | HG02613 | 12 | 4 | 16 | 182 | 45 | 154 | moderat | hoch | anfällig | T11 | M03 | A | 2A: Dringend |
| 1kG | HG03015 | 13 | 3 | 16 | 179 | 61 | 141 | moderat | hoch | anfällig | Til | M03 | A | 2A: Dringend |

(fortgesetzt)

| Kohorte | Proben-ID | 32 p<10^5 [tmg] | | 381 [all] | p<10^5 | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | NA18641 | 14 | 2 | 16 | 197 | 15 | 169 | hoch | hoch | anfällig | T04 | M03 | A | 1A: Umgehend |
| 1kG | NA12282 | 8 | 8 | 16 | 150 | 97 | 134 | moderat_gering | hoch | anfällig | T24 | M03 | A | 3A: Baldig |
| 1kG | NA21123 | 14 | 2 | 16 | 192 | 20 | 169 | hoch | hoch | anfällig | T04 | M03 | A | 1A: Umgehend |
| 1kG | HG03765 | 14 | 2 | 16 | 188 | 28 | 165 | hoch | hoch | anfällig | T04 | M03 | A | 1A: Umgehend |
| 1kG | HG00641 | 12 | 4 | 16 | 175 | 54 | 152 | moderat | hoch | anfällig | T07 | M03 | A | 2A: Dringend |
| 1kG | HG01861 | 13 | 3 | 16 | 176 | 46 | 159 | moderat | hoch | anfällig | T07 | M03 | A | 2A: Dringend |
| 1kG | NA11840 | 14 | 2 | 16 | 186 | 26 | 169 | hoch | hoch | anfällig | T04 | M03 | A | 1A: Umgehend |
| 1kG | NA20767 | 14 | 2 | 16 | 163 | 78 | 140 | moderat | hoch | anfällig | T07 | M03 | A | 2A: Dringend |
| 1kG | HG01846 | 12 | 4 | 16 | 157 | 82 | 142 | moderat | hoch | anfällig | T07 | M03 | A | 2A: Dringend |
| 1kG | HG00255 | 11 | 5 | 16 | 182 | 37 | 162 | hoch | hoch | anfällig | T04 | M03 | A | 1A: Umgehend |
| TMG | GL57228616JA | 13 | 4 | 16 | | | | | | | | M01 | | |
| TMG | GL83348210WQ | 14 | 4 | 16 | | | | | | | | M01 | | |
| TMG | GL64272211PN | 14 | 2 | 16 | | | | | | | | M01 | | |
| TMG | GL45262410DR | 16 | 2 | 16 | | | | | | | | M01 | | |
| TMG | GL28922310AJ | 12 | 4 | 16 | | | | | | | | M01 | | |
| TMG | GL23237910XH | 11 | 7 | 16 | | | | | | | | M01 | | |
| 1kG | HG00851 | 14 | 2 | 16 | 161 | 78 | 142 | moderat | hoch | anfällig | T07 | M01 | A | 2A: Dringend |
| 1kG | HG01277 | 15 | 1 | 16 | 195 | 22 | 164 | hoch | hoch | anfällig | T04 | M01 | A | 1A: Umgehend |
| 1kG | HG01796 | 16 | | 16 | 192 | 28 | 161 | hoch | hoch | gleichrangig | T04 | M01 | A | 1A: Umgehend |
| 1kG | NA18560 | 16 | | 16 | 185 | 33 | 163 | hoch | hoch | gleichrangig | T04 | M01 | A | 1A: Umgehend |

(fortgesetzt)

| Kohorte | Proben-ID | 32 p<10⁵ [tmg] | | 381 [all] | p<10⁵ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | NA20778 | 14 | 2 | 16 | 198 | 17 | 166 | hoch | hoch | anfällig | T04 | M01 | A | 1A: Umgehend |
| 1kG | HG02076 | 15 | 1 | 16 | 171 | 63 | 147 | moderat | hoch | anfällig | T07 | M01 | A | 2A: Dringend |
| 1kG | HG03746 | 13 | 3 | 16 | 166 | 72 | 143 | moderat | hoch | anfällig | T07 | M01 | A | 2A: Dringend |
| 1kG | HG03631 | 13 | 3 | 16 | 151 | 94 | 136 | moderat_gering | hoch | anfällig | T27 | M01 | A | 3A: Baldig |
| CG69 | GS12892 | 14 | 2 | 16 | 189 | 52 | 160 | hoch | hoch | anfällig | T04 | M01 | A | 1A: Umgehend |
| 1kG | HG03241 | 15 | 1 | 16 | 185 | 42 | 154 | moderat | hoch | anfällig | T11 | M01 | A | 2A: Dringend |
| 1kG | HG01348 | 14 | 2 | 16 | 191 | 24 | 166 | hoch | hoch | anfällig | T04 | M01 | A | 1A: Umgehend |
| 1kG | HG02390 | 15 | 1 | 16 | 199 | 18 | 164 | hoch | hoch | anfällig | T04 | M01 | A | 1A: Umgehend |
| 1kG | HG02408 | 15 | 1 | 16 | 192 | 26 | 163 | hoch | hoch | anfällig | T04 | M01 | A | 1A: Umgehend |
| 1kG | NA18565 | 15 | 1 | 16 | 180 | 38 | 163 | hoch | hoch | anfällig | T04 | M01 | A | 1A: Umgehend |
| 1kG | HG03760 | 15 | 1 | 16 | 191 | 21 | 169 | hoch | hoch | anfällig | T04 | M01 | A | 1A: Umgehend |
| 1kG | HG04025 | 15 | 1 | 16 | 192 | 19 | 170 | hoch | hoch | anfällig | T06 | M01 | A | 1A: Umgehend |
| 1kG | HG00640 | 15 | 1 | 16 | 196 | 20 | 165 | hoch | hoch | anfällig | T04 | M01 | A | 1A: Umgehend |
| 1kG | HG04056 | 15 | 1 | 16 | 196 | 22 | 163 | hoch | hoch | anfällig | T03 | M01 | A | 1A: Umgehend |
| 1kG | HG04098 | 13 | 3 | 16 | 152 | 91 | 138 | moderat_gering | hoch | anfällig | T24 | M01 | A | 3A: Baldig |
| 1kG | HG00246 | 14 | 2 | 16 | 182 | 39 | 160 | hoch | hoch | anfällig | iG4 | M01 | A | 1A: Umgehend |
| 1kG | NA12777 | 14 | 2 | 16 | 169 | 58 | 154 | moderat | hoch | anfällig | T07 | M01 | A | 2A: Dringend |

(fortgesetzt)

| Kohorte Proben-ID | 32 $p<10^5$ [tmg] | | | 381 [all] | $p<10^5$ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | NA12763 | 13 | 3 | 16 | 159 | 75 | 147 | moderat | hoch | anfällig | T07 | M01 | A | 2A: Dringend |
| 1kG | H600325 | 14 | 2 | 16 | 191 | 26 | 164 | hoch | hoch | anfällig | T04 | M01 | A | 1A: Umgehend |
| 1kG | HG00189 | 14 | 2 | 16 | 162 | 67 | 152 | moderat | hoch | anfällig | T07 | M01 | A | 2A: Dringend |
| 1kG | HG02694 | 14 | 2 | 16 | 180 | 49 | 152 | moderat | hoch | anfällig | T07 | M01 | A | 2A: Dringend |
| 1kG | HG04200 | 12 | 5 | 15 | 90 | 215 | 76 | asymptomatisch | hoch | anfällig | T47 | M03 | A | 5A: Freiwillig |
| 1kG | HG02943 | 15 | 2 | 15 | 178 | 49 | 154 | moderat | hoch | gleichrangig | T17 | M02 | A | 2A: Dringend |
| 1kG | HG03711 | 12 | 5 | 15 | 123 | 154 | 104 | gering | hoch | anfällig | T40 | M03 | A | 4A: Später |
| 1kG | NA20585 | 12 | 5 | 15 | 164 | 68 | 149 | moderat | hoch | anfällig | T07 | M03 | A | 2A: Dringend |
| 1kG | NA19834 | 13 | 4 | 15 | 185 | 40 | 156 | moderat | hoch | anfällig | T12 | M03 | A | 2A: Dringend |
| CG69 | GS19834 | 13 | 4 | 15 | 186 | 40 | 155 | moderat | hoch | anfällig | T12 | M03 | A | 2A: Dringend |
| 1kG | HG03821 | 13 | 4 | 15 | 167 | 69 | 145 | moderat | hoch | anfällig | T07 | M03 | A | 2A: Dringend |
| 1kG | HG01447 | 15 | 2 | 15 | 193 | 17 | 171 | hoch | hoch | gleichrangig | T05 | M03 | A | 1A: Umgehend |
| 1kG | NA12287 | 11 | 6 | 15 | 177 | 47 | 157 | moderat | hoch | anfällig | T07 | M03 | A | 2A: Dringend |
| 1kG | NA20543 | 12 | 5 | 15 | 172 | 61 | 148 | moderat | hoch | anfällig | T07 | M03 | A | 2A: Dringend |
| 1kG | NA20766 | 13 | 4 | 15 | 178 | 47 | 156 | moderat | hoch | anfällig | T07 | M03 | A | 2A: Dringend |
| 1kG | HG01271 | 11 | 6 | 15 | 184 | 42 | 155 | moderat | hoch | anfällig | T07 | M03 | A | 2A: Dringend |
| 1kG | HG01455 | 13 | 4 | 15 | 178 | 48 | 155 | moderat | hoch | anfällig | T07 | M03 | A | 2A: Dringend |

(fortgesetzt)

| Kohorte Proben-ID | 32 p<10⁵ [tmg] | | | 381 [all] | p<10⁵ | | Schweregrad -1000 | TMG-32 | Allelfrequenz | Indy-Med Gruppe | Covid19 Ind-yMed Klassi-fikation | Indy-Med Score | Impfempfeh-lung | |
|---|---|---|---|---|---|---|---|---|---|---|---|---|---|---|
| 1kG | NA19726 | 13 | 4 | 15 | 187 | 34 | 160 | hoch | hoch | anfällig | T05 | M03 | A | 1A: Umgehend |
| 1kG | HG00533 | 14 | 3 | 15 | 178 | 49 | 154 | moderat | hoch | anfällig | T07 | M03 | A | 2A: Dringend |
| 1kG | HG02219 | 13 | 4 | 15 | 172 | 62 | 147 | moderat | hoch | anfällig | T07 | M03 | A | 2A: Dringend |
| 1kG | HG02380 | 13 | 4 | 15 | 179 | 47 | 155 | moderat | hoch | anfällig | T07 | M03 | A | 2A: Dringend |
| CG69 | HG00733 | 12 | 5 | 15 | 181 | 43 | 157 | moderat | hoch | anfällig | T07 | M03 | A | 2A: Dringend |
| 1kG | HG00419 | 13 | 4 | 15 | 156 | 88 | 137 | moderat_gering | hoch | anfällig | T24 | M03 | A | 3A: Baldig |
| 1kG | NA19773 | 15 | 2 | 15 | 162 | 81 | 138 | moderat_gering | hoch | gleichrangig | T24 | M03 | A | 3A: Baldig |
| 1kG | NA20796 | 14 | 3 | 15 | 154 | 97 | 130 | moderat_gering | hoch | anfällig | T24 | M03 | A | 3A: Baldig |
| 1kG | HG01709 | 12 | 5 | 15 | 149 | 107 | 125 | gering | hoch | anfällig | T35 | M03 | A | 4A: Später |
| 1kG | HG00336 | 14 | 3 | 15 | 133 | 125 | 123 | gering | hoch | anfällig | T40 | M03 | A | 4A: Später |
| 1kG | HG00592 | 13 | 4 | 15 | 174 | 46 | 161 | hoch | hoch | anfällig | T05 | M02 | A | 1A: Umgehend |
| 1kG | HG00281 | 14 | 3 | 15 | 193 | 16 | 172 | hoch | hoch | anfällig | T05 | M03 | A | 1A: Umgehend |
| 1kG | HG01786 | 13 | 4 | 15 | 186 | 32 | 163 | hoch | hoch | anfällig | T06 | M03 | A | 1A: Umgehend |
| 1kG | HG03963 | 14 | 3 | 15 | 152 | 60 | 169 | hoch | hoch | anfällig | T01 | M02 | A | 1A: Umgehend |
| 1kG | HG03624 | 12 | 5 | 15 | 163 | 74 | 144 | moderat | hoch | anfällig | T07 | M03 | A | 2A: Dringend |
| 1kG | HG03941 | 15 | 2 | 15 | 169 | 36 | 176 | hoch | hoch | gleichrangig | T01 | M03 | A | 1A: Umgehend |
| 1kG | NA19119 | 15 | 2 | 15 | 204 | 12 | 165 | hoch | hoch | gleichrangig | T03 | M03 | A | 1A: Umgehend |
| 1kG | HG00864 | 15 | 2 | 15 | 200 | 18 | 163 | hoch | hoch | gleichrangig | T05 | M03 | A | 1A: Umgehend |
| 1kG | HG02224 | 13 | 4 | 15 | 184 | 31 | 166 | hoch | hoch | anfällig | T05 | M03 | A | 1A: Umge-hend |

(fortgesetzt)

| Kohorte | Proben-ID | 32 p<10⁵ [tmg] | | 381 [all] | p<10⁵ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | HG01872 | 14 | 3 | 15 | 183 | 46 | 152 | moderat | hoch | anfällig | T07 | M03 | A | 2A: Dringend |
| 1kG | HG01988 | 10 | 7 | 15 | 162 | 75 | 144 | moderat | hoch | anfällig | T07 | M03 | A | 2A: Dringend |
| 1kG | HG02130 | 12 | 5 | 15 | 188 | 35 | 158 | moderat | hoch | anfällig | T07 | M03 | A | 2A: Dringend |
| 1kG | NA19089 | 13 | 4 | 15 | 176 | 54 | 151 | moderat | hoch | anfällig | T07 | M03 | A | 2A: Dringend |
| 1kG | NA21104 | 16 | 1 | 15 | 173 | 63 | 145 | moderat | hoch | protektiv | T07 | M03 | A | 2A: Dringend |
| 1kG | HG03082 | 11 | 6 | 15 | 186 | 41 | 154 | moderat | hoch | anfällig | T12 | M03 | A | 2A: Dringend |
| 1kG | NA18507 | 15 | 2 | 15 | 181 | 57 | 143 | moderat | hoch | gleichrangig | T15 | M03 | A | 2A: Dringend |
| 1kG | HG04155 | 15 | 2 | 15 | 154 | 65 | 162 | hoch | hoch | gleichrangig | T01 | M02 | A | 1A: Umgehend |
| 1kG | HG02820 | 12 | 5 | 15 | 148 | 107 | 126 | gering | hoch | anfällig | T40 | M03 | A | 4A: Später |
| 1kG | NA18595 | 14 | 3 | 15 | 187 | 32 | 162 | hoch | hoch | anfällig | T05 | M03 | A | 1A: Umgehend |
| 1kG | NA12003 | 12 | 5 | 15 | 182 | 42 | 157 | moderat | hoch | anfällig | T07 | M03 | A | 2A: Dringend |
| 1kG | HG01323 | 13 | 4 | 15 | 160 | 82 | 139 | moderat_gering | hoch | anfällig | T24 | M03 | A | 3A: Baldig |
| 1kG | HG01697 | 12 | 5 | 15 | 156 | 88 | 137 | moderat_gering | hoch | anfällig | T24 | M03 | A | 3A: Baldig |
| 1kG | NA18984 | 12 | 5 | 15 | 132 | 136 | 113 | gering | hoch | anfällig | T40 | M03 | A | 4A: Später |
| 1kG | HG00599 | 14 | 3 | 15 | 187 | 31 | 163 | hoch | hoch | anfällig | T05 | M03 | A | 1A: Umgehend |
| 1kG | HG02253 | 13 | 4 | 15 | 181 | 39 | 161 | hoch | hoch | anfällig | 105 | M03 | A | 1A: Umgehend |
| 1kG | HG04176 | 14 | 3 | 15 | 191 | 23 | 167 | hoch | hoch | anfällig | T05 | M03 | A | 1A: Umgehend |
| 1kG | NA18986 | 15 | 2 | 15 | 188 | 32 | 161 | hoch | hoch | gleichrangig | T05 | M03 | A | 1A: Umgehend |

(fortgesetzt)

| Kohorte | Proben-ID | 32 $p<10^5$ [tmg] | | 381 [all] | $p<10^5$ | | Schweregrad -1000 | TMG-32 | Allelfrequenz | Indy-Med Gruppe | Covid19 IndyMed Klassifikation | Indy-Med Score | Impfempfehlung | |
|---|---|---|---|---|---|---|---|---|---|---|---|---|---|---|
| 1kG | HG00342 | 13 | 4 | 15 | 169 | 65 | 147 | moderat | hoch | anfällig | T07 | M03 | A | 2A: Dringend |
| 1kG | HG00373 | 14 | 3 | 15 | 178 | 53 | 150 | moderat | hoch | anfällig | T07 | M03 | A | 2A: Dringend |
| 1kG | HG01808 | 13 | 4 | 15 | 167 | 67 | 147 | moderat | hoch | anfällig | T07 | M03 | A | 2A: Dringend |
| 1kG | HG03021 | 14 | 3 | 15 | 183 | 39 | 159 | moderat | hoch | anfällig | T07 | M03 | A | 2A: Dringend |
| 1kG | NA18591 | 14 | 3 | 15 | 156 | 84 | 141 | moderat | hoch | anfällig | T07 | M03 | A | 2A: Dringend |
| 1kG | NA18951 | 14 | 3 | 15 | 179 | 50 | 152 | moderat | hoch | anfällig | T07 | M03 | A | 2A: Dringend |
| 1kG | HG01598 | 13 | 4 | 15 | 154 | 93 | 134 | moderat_gering | hoch | anfällig | T24 | M03 | A | 3A: Baldig |
| 1kG | HG03702 | 11 | 6 | 15 | 179 | 39 | 163 | hoch | hoch | anfällig | T05 | M03 | A | 1A: Umgehend |
| 1kG | HG00513 | 12 | S | 15 | 165 | 69 | 147 | moderat | hoch | anfällig | T07 | M03 | A | 2A: Dringend |
| 1kG | HG03736 | 13 | 4 | 15 | 186 | 34 | 161 | hoch | hoch | anfällig | T05 | M03 | A | 1A: Umgehend |
| 1kG | NA19716 | 15 | 2 | 15 | 194 | 13 | 174 | hoch | hoch | gleichrangig | T05 | M03 | A | 1A: Umgehend |
| 1kG | NA20502 | 13 | 4 | 15 | 175 | 56 | 150 | moderat | hoch | anfällig | T07 | M03 | A | 2A: Dringend |
| CG69 | G520502 | 15 | 4 | 15 | 177 | 56 | 148 | moderat | hoch | anfällig | T07 | M03 | A | 2A: Dringend |
| 1kG | NA18623 | 13 | 2 | 15 | 187 | 32 | 162 | hoch | hoch | gleichrangig | T05 | M03 | A | 1A: Umgehend |

**Tabelle 2b: PCA Klassenzuordnung**

| Kohorte | Proben-ID | 32 $p<10^{-5}$ [tmg] | | | 381 $p<10^{-5}$ [all] | | | rs306313 | rs126191 | rs170783 | rs130794 | rs177119 | rs983815 | rs697492 | rs100910 | rs687621 | rs687289 | rs657152 | rs644234 | rs544873 | rs545971 | rs817666 | rs493216 | rs495203 | rs582094 | rs276907 | rs677355 | rs676996 | rs550057 | rs674302 | rs554833 | rs505922 | rs529565 | rs122233 | rs803852 | rs456926 | rs761874 | rs561325 | rs126104 | Gruppe | Muster | PCA | Impfempfehlung |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | | 0 | 1 | 2 | 0 | 1 | 2 | | | | | | | | | | | | | | | | | | | | | | | | | | | | | | | | | | | | |
| CG69 | GS12886 | 12 | 1 | 19 | 197 | 14 | 170 | 0 | 0 | 2 | 0 | 2 | 2 | 2 | 2 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 1 | 2 | T04 | M03 | G01 | 1A |
| TMG | GL36724711RQ | 10 | 5 | 19 | | | | 2 | 0 | 2 | 0 | 2 | 2 | 2 | 1 | 0 | 2 | 2 | 0 | 2 | 2 | 2 | 2 | 2 | 0 | 0 | 1 | 0 | 2 | 2 | 2 | 0 | 0 | 2 | 1 | 0 | 2 | 1 | 1 | | M03 | | |
| TMG | GL23757411XK | 11 | 4 | 19 | | | | 0 | 1 | 2 | 0 | 2 | 2 | 2 | 2 | 0 | 2 | 2 | 0 | 2 | 2 | 2 | 2 | 2 | 0 | 0 | 1 | 0 | 2 | 2 | 2 | 0 | 0 | 1 | 1 | 0 | 2 | 0 | 2 | | M03 | | |
| TMG | GL52779612GN | 13 | 2 | 19 | | | | 1 | 0 | 2 | 0 | 2 | 2 | 2 | 1 | 2 | 0 | 0 | 2 | 2 | 0 | 2 | 2 | 0 | 2 | 2 | 0 | 2 | 0 | 0 | 0 | 2 | 2 | 2 | 2 | 0 | 2 | 2 | 2 | | M01 | | |
| TMG | W2N2T4B5U5U7 | 13 | 3 | 18 | | | | 0 | 0 | 2 | 0 | 2 | 2 | 0 | 1 | 0 | 2 | 2 | 0 | 2 | 2 | 2 | 2 | 2 | 0 | 0 | 2 | 0 | 2 | 2 | 2 | 0 | 0 | 1 | 2 | 0 | 2 | 0 | 1 | | M03 | | |
| 1kG | HG02391 | 14 | | 18 | 201 | 14 | 166 | 0 | 0 | 2 | 0 | 2 | 2 | 0 | 0 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 2 | 2 | T04 | M03 | G01 | 1A |
| 1kG | NA20505 | 13 | 1 | 18 | 191 | 24 | 166 | 0 | 0 | 2 | 0 | 2 | 2 | 2 | 1 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 0 | 2 | T04 | M03 | G01 | 1A |
| 1kG | NA20530 | 12 | 2 | 18 | 188 | 27 | 166 | 0 | 0 | 2 | 0 | 2 | 2 | 2 | 1 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 1 | 2 | T04 | M03 | G01 | 1A |
| 1kG | HG01583 | 13 | 1 | 18 | 191 | 25 | 165 | 0 | 0 | 2 | 0 | 2 | 2 | 1 | 2 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 0 | 2 | T04 | M03 | G01 | 1A |
| 1kG | HG00739 | 12 | 2 | 18 | 164 | 72 | 145 | 0 | 1 | 2 | 0 | 2 | 2 | 0 | 2 | 0 | 2 | 2 | 0 | 2 | 2 | 0 | 2 | 2 | 0 | 0 | 2 | 0 | 2 | 2 | 2 | 0 | 0 | 2 | 2 | 0 | 2 | 1 | 2 | T07 | M03 | G02 | 2A |
| 1kG | HG02185 | 13 | 1 | 18 | 188 | 20 | 173 | 0 | 2 | 2 | 0 | 2 | 2 | 0 | 0 | 0 | 2 | 2 | 0 | 2 | 2 | 0 | 2 | 2 | 0 | 0 | 2 | 0 | 2 | 2 | 2 | 0 | 0 | 1 | 2 | 0 | 2 | 2 | 2 | T04 | M03 | G03 | 1A |
| 1kG | HG00346 | 13 | 1 | 18 | 180 | 34 | 167 | 0 | 0 | 2 | 0 | 2 | 2 | 2 | 2 | 0 | 2 | 2 | 0 | 2 | 2 | 0 | 2 | 2 | 0 | 0 | 2 | 0 | 2 | 2 | 2 | 0 | 0 | 1 | 2 | 0 | 2 | 0 | 2 | T04 | M03 | G01 | 1A |

| | | | | | | | | | | | | |
|---|---|---|---|---|---|---|---|---|---|---|---|---|
| 1kG | HG00356 | 13 | 1 | 18 | 184 | 33 | 164 | 2 2 2 2 2 2 2 2 2 2 2 2 2 2 2 1 2 2 2 | T04 | M03 | G01 | 1A |
| 1kG | NA12046 | 12 | 2 | 18 | 187 | 25 | 169 | 2 2 2 2 2 2 2 2 2 2 2 2 2 2 2 1 2 2 1 2 | T04 | M03 | G01 | 1A |
| TMG | H6E4T8E0H3D1 | 13 | 3 | 18 | | | | 2 2 2 2 2 2 2 2 2 2 2 1 2 2 2 1 2 2 1 | | M03 | | |
| 1kG | NA19190 | 13 | 1 | 18 | 184 | 51 | 146 | 1 2 2 2 2 2 2 2 2 2 2 2 2 2 2 2 2 2 | T15 | M03 | G04 | 2A |
| TMG | GL22377811YA | 10 | 4 | 18 | | | | 2 2 2 1 2 2 2 2 2 2 2 2 1 2 2 2 2 2 1 1 | | M03 | | |
| 1kG | NA10847 | 13 | 1 | 18 | 191 | 22 | 168 | 2 2 2 2 2 2 2 2 2 2 2 2 1 2 2 2 2 2 2 | T04 | M03 | G01 | 1A |
| TMG | GL32292210QG | 11 | 3 | 18 | | | | 2 2 2 2 2 2 2 2 2 2 2 1 1 2 2 1 2 2 2 | | M03 | | |
| TMG | GL64456713ZJ | 13 | 3 | 18 | | | | 2 2 2 2 2 2 2 2 2 2 2 2 1 1 2 2 2 2 1 | | M03 | | |
| TMG | GL39282510FX | 11 | 4 | 18 | | | | 1 2 1 1 1 2 2 2 2 2 2 2 2 2 2 2 2 2 2 2 2 2 | | M01 | | |
| TMG | GL24222510XX | 13 | 2 | 18 | | | | 1 2 2 2 2 2 2 2 2 2 2 2 2 2 2 2 2 2 1 2 | | M01 | | |
| TMG | GL44792211DV | 15 | 1 | 18 | | | | 2 2 2 2 2 2 2 2 2 2 2 2 2 2 2 2 2 2 1 2 | | M01 | | |
| TMG | GL98739411WZ | 14 | 1 | 18 | | | | 2 2 2 2 2 2 2 2 2 2 2 2 2 2 2 2 1 2 | | M01 | | |
| TMG | GL55694410KN | 11 | 6 | 17 | | | | 2 1 1 2 1 2 2 2 2 2 2 2 2 2 2 2 1 2 1 2 1 | | M03 | | |
| 1kG | HG01603 | 13 | 2 | 17 | 184 | 33 | 164 | 2 2 2 1 1 2 2 2 2 2 2 2 2 2 2 2 2 2 2 | T04 | M03 | G01 | 1A |
| 1kG | HG01615 | 13 | 2 | 17 | 190 | 26 | 165 | 2 2 2 1 1 2 2 2 2 2 2 2 2 2 2 2 2 2 2 | T04 | M03 | G01 | 1A |
| 1kG | NA18619 | 14 | 1 | 17 | 171 | 61 | 149 | 1 2 2 2 2 2 2 2 2 2 2 2 2 2 2 2 2 2 | T07 | M03 | G02 | 2A |
| 1kG | HG02067 | 12 | 3 | 17 | 186 | 25 | 170 | 2 2 2 2 1 2 2 2 2 2 2 2 2 2 2 2 2 2 1 1 | T04 | M03 | G03 | 1A |
| 1kG | HG00285 | 11 | 4 | 17 | 186 | 27 | 168 | 1 2 2 2 2 1 2 2 2 2 2 2 2 2 2 2 2 2 2 1 1 | T04 | M03 | G01 | 1A |
| 1kG | NA18625 | 12 | 3 | 17 | 161 | 80 | 140 | 1 2 2 2 2 2 2 2 2 2 2 2 2 2 2 2 1 2 1 2 | T07 | M03 | G02 | 2A |
| 1kG | HG01868 | 12 | 3 | 17 | 158 | 85 | 138 | 1 2 2 2 1 2 2 2 2 2 2 2 2 2 2 1 2 2 2 2 | T24 | M03 | G02 | 3A |
| 1kG | HG01507 | 13 | 2 | 17 | 184 | 28 | 169 | 2 2 2 2 1 2 2 2 2 2 2 2 2 2 2 2 2 1 2 | T04 | M03 | G01 | 1A |
| 1kG | NA12760 | 14 | 1 | 17 | 193 | 27 | 161 | 2 2 2 2 2 2 2 2 2 2 2 2 2 2 1 2 2 2 | T04 | M03 | G01 | 1A |

| | | | | | | | | | | | | |
|---|---|---|---|---|---|---|---|---|---|---|---|---|
| 1kG | NA19722 | 12 | 3 | 17 | 181 | 44 | 156 | 2 2 2 2 1 2 2 2 2 2 2 2 2 2 2 2 1 2 2 1 2 | T07 | M03 | G01 | 2A |
| 1kG | HG01623 | 13 | 2 | 17 | 186 | 24 | 171 | 2 2 2 1 2 1 2 2 2 2 2 2 2 2 2 2 2 2 2 2 | T04 | M03 | G01 | 1A |
| 1kG | HG01437 | 13 | 2 | 17 | 177 | 42 | 162 | 2 2 2 2 2 2 2 2 2 2 2 2 2 2 2 2 2 1 2 2 1 | T04 | M03 | G01 | 1A |
| 1kG | NA18983 | 14 | 1 | 17 | 183 | 18 | 180 | 2 2 2 2 2 1 2 2 2 2 2 2 2 2 2 2 2 2 2 | T04 | M03 | G03 | 1A |
| 1kG | NA19774 | 14 | 1 | 17 | 187 | 19 | 175 | 2 2 2 2 2 2 2 2 2 2 2 2 2 2 2 2 1 2 2 | T04 | M03 | G01 | 1A |
| TMG | GL22283511TB | 12 | 4 | 17 | | | | 2 2 2 1 2 2 2 2 2 2 2 1 2 2 2 1 2 1 2 | | M03 | | |
| TMG | GL48992215KV | 10 | 5 | 17 | | | | 1 1 2 2 2 2 2 2 2 2 2 2 2 2 2 2 1 2 1 1 | | M03 | | |
| 1kG | HG00376 | 13 | 2 | 17 | 190 | 27 | 164 | 2 2 2 2 1 2 2 2 2 2 2 2 1 2 2 2 2 2 2 2 | T04 | M03 | G01 | 1A |
| 1kG | HG03947 | 13 | 2 | 17 | 192 | 25 | 164 | 2 2 2 2 1 2 2 2 2 2 2 2 2 2 2 2 2 1 2 | T04 | M03 | G01 | 1A |
| 1kG | HG04152 | 12 | 3 | 17 | 167 | 58 | 156 | 1 2 2 2 2 1 2 2 2 2 2 2 2 2 2 2 2 2 1 2 | T07 | M03 | G02 | 2A |
| 1kG | HG00266 | 14 | 1 | 17 | 189 | 24 | 168 | 1 2 2 2 2 2 2 2 2 2 2 2 2 2 2 2 2 2 | T04 | M03 | G01 | 1A |
| 1kG | HG01766 | 12 | 3 | 17 | 182 | 36 | 163 | 2 2 2 1 2 2 2 2 2 2 2 2 1 2 2 2 2 2 1 2 | T04 | M03 | G01 | 1A |
| 1kG | HG01104 | 13 | 2 | 17 | 182 | 41 | 158 | 2 2 2 1 2 2 2 2 2 2 2 1 2 2 2 2 2 2 | T07 | M03 | G01 | 2A |
| 1kG | HG03228 | 13 | 2 | 17 | 161 | 76 | 144 | 1 2 2 2 1 2 2 2 2 2 2 2 2 2 2 2 2 2 | T07 | M03 | G02 | 2A |
| 1kG | HG00360 | 12 | 3 | 17 | 190 | 23 | 168 | 2 2 2 2 2 2 2 2 2 2 2 2 1 2 2 2 2 1 1 | T04 | M03 | G01 | 1A |
| 1kG | HG03781 | 12 | 3 | 17 | 183 | 35 | 163 | 2 2 2 2 1 2 2 2 2 2 2 2 1 2 2 1 2 2 2 2 | T04 | M03 | G01 | 1A |
| TMG | GL29428214MW | 12 | 5 | 17 | | | | 1 1 2 2 1 2 2 2 2 2 2 2 2 1 1 2 2 2 2 2 | | M03 | | |
| TMG | GL22872212FX | 12 | 4 | 17 | | | | 2 2 1 2 2 2 2 2 2 2 1 1 2 2 1 2 2 2 | | M03 | | |
| TMG | GL63427410WK | 13 | 3 | 17 | | | | 1 2 2 2 2 2 2 2 2 2 2 2 2 2 2 2 2 1 1 | | M01 | | |
| TMG | GL92376214QU | 15 | 2 | 17 | | | | 2 2 2 2 2 2 2 2 2 2 2 2 2 1 2 1 2 | | M01 | | |
| TMG | GL93878310NA | 13 | 3 | 17 | | | | 1 2 2 2 2 2 2 2 2 2 2 2 2 2 1 1 2 2 | | M01 | | |
| TMG | GL83593813PK | 12 | 5 | 17 | | | | 2 2 2 2 2 2 1 1 2 2 2 2 2 2 2 2 1 2 2 2 | | M01 | | |

| | | | | | | | | | | | | |
|---|---|---|---|---|---|---|---|---|---|---|---|---|
| TMG | GL39895215XP | 16 | | 17 | | | | 2 2 2 2 22 22 22 2 2222 2 2 | | M01 | | |
| TMG | GL42337212KT | 14 | 3 | 17 | | | | 2 21212 22 22 22 2 2222 212 | | M01 | | |
| TMG | GL25352411CK | 12 | 4 | 17 | | | | 11 21222 22 22 22 2 2222 212 | | M01 | | |
| TMG | GL28829410AN | 14 | 1 | 17 | | | | 12 2 222 22 22 22 2 2222 2 | | M01 | | |
| TMG | GL22743912JF | 15 | 2 | 17 | | | | 2 22212 22 22 22 2 2221 2 2 | | M01 | | |
| 1kG | HG02383 | 13 | 2 | 17 | 189 | 26 | 166 | 22 22112 2 2 22 2 2222 222 | T04 | M01 | G03 | 1A |
| 1kG | HG01130 | 14 | 1 | 17 | 187 | 34 | 160 | 2 22222 2 2 22 2 2222 212 | T04 | M01 | G01 | 1A |
| CG69 | GS12880 | 14 | 1 | 17 | 193 | 21 | 167 | 2 22222 2 2 22 2 2222 212 | T04 | M01 | G01 | 1A |
| 1kG | HG01512 | 13 | 2 | 17 | 160 | 81 | 140 | 12 22222 2 2 22 2 2222 212 | T07 | M01 | G02 | 2A |
| 1kG | NA19786 | 15 | | 17 | 186 | 38 | 157 | 2 22222 2 2 22 2 2222 2 2 | T11 | M01 | G01 | 2A |
| 1kG | NA12144 | 13 | 2 | 17 | 186 | 29 | 166 | 22 22222 2 2 22 2 2222 211 | T04 | M01 | G03 | 1A |
| 1kG | NA20761 | 12 | 4 | 16 | 81 | 213 | 87 | 21111 2 22 22 22 2 222 22 2 2 | T47 | M02 | G12 | 5A |
| 1kG | NA20875 | 14 | 2 | 16 | 148 | 101 | 132 | 11 222 22 22 22 2 222 22 2 | T27 | M03 | G02 | 3A |
| 1kG | NA20587 | 14 | 2 | 16 | 191 | 30 | 160 | 2 22 1 22 22 22 2 222 21 2 2 | T04 | M03 | G01 | 1A |
| 1kG | HG00403 | 13 | 3 | 16 | 160 | 80 | 141 | 12 221 22 22 22 2 222 2 212 | T07 | M03 | G02 | 2A |
| 1kG | HG00662 | 15 | 1 | 16 | 188 | 37 | 156 | 2 22 22 22 22 2 222 21 2 2 | T07 | M03 | G01 | 2A |
| 1kG | HG01624 | 12 | 4 | 16 | 165 | 65 | 151 | 12 22 1 22 22 22 2 222 21 212 | T07 | M03 | G01 | 2A |
| 1kG | NA20805 | 15 | 1 | 16 | 195 | 19 | 167 | 1 22 2 22 22 22 2 222 2 2 2 | T04 | M03 | G01 | 1A |
| 1kG | HG00337 | 13 | 3 | 16 | 175 | 42 | 164 | 2 2211 22 22 22 2 222 2 212 | T04 | M03 | G01 | 1A |
| 1kG | HG00369 | 13 | 3 | 16 | 175 | 47 | 159 | 2 2211 22 22 22 2 222 2 212 | T07 | M03 | G01 | 2A |
| 1kG | HG00559 | 13 | 3 | 16 | 155 | 80 | 146 | 12 2211 22 22 22 2 222 2 2 2 | T07 | M03 | G02 | 2A |
| 1kG | HG02384 | 13 | 3 | 16 | 156 | 81 | 144 | 12 2211 22 22 22 2 222 2 2 2 | T07 | M03 | G02 | 2A |

| | | | | | | | | | | | | |
|---|---|---|---|---|---|---|---|---|---|---|---|---|
| 1kG | HG03945 | 13 | 3 | 16 | 172 | 63 | 146 | 1 2 2 2 1 2 2 2 2 2 2 2 2 2 2 1 2 2 2 | T07 | M03 | G01 | 2A |
| 1kG | NA12058 | 12 | 4 | 16 | 178 | 47 | 156 | 2 2 2 1 1 2 2 2 2 2 2 2 2 2 2 1 2 2 1 2 | T07 | M03 | G01 | 2A |
| 1kG | NA20531 | 11 | 5 | 16 | 147 | 102 | 132 | 1 2 2 2 1 1 2 2 2 2 2 2 2 2 2 2 1 2 2 1 2 | T24 | M03 | G02 | 3A |
| 1kG | NA20807 | 11 | 5 | 16 | 142 | 105 | 134 | 1 2 2 2 1 1 2 2 2 2 2 2 2 2 2 2 1 2 2 1 2 | T24 | M03 | G02 | 3A |
| CG69 | GS12891 | 12 | 4 | 16 | 171 | 64 | 146 | 1 2 2 1 2 2 2 2 2 2 2 2 2 2 2 1 2 2 1 2 | T07 | M03 | G01 | 2A |
| 1kG | HG00261 | 13 | 3 | 16 | 188 | 27 | 166 | 2 2 2 2 1 2 2 2 2 2 2 2 2 2 2 2 2 1 1 | T04 | M03 | G01 | 1A |
| 1kG | NA12341 | 13 | 3 | 16 | 180 | 42 | 159 | 2 2 2 1 2 2 2 2 2 2 2 2 2 2 2 1 2 1 2 | T07 | M03 | G01 | 2A |
| 1kG | NA12778 | 12 | 4 | 16 | 154 | 86 | 141 | 1 2 2 2 2 2 2 2 2 2 2 2 2 2 2 2 1 2 1 1 | T07 | M03 | G02 | 2A |
| 1kG | HG01465 | 13 | 3 | 16 | 168 | 51 | 162 | 2 2 2 2 1 2 2 2 2 2 2 2 2 2 2 1 1 2 2 | T04 | M03 | G02 | 1A |
| 1kG | NA18612 | 13 | 3 | 16 | 172 | 53 | 156 | 2 2 2 2 1 2 2 2 2 2 2 2 2 2 2 2 1 2 1 2 | T07 | M03 | G03 | 2A |
| 1kG | HG00186 | 12 | 4 | 16 | 186 | 32 | 163 | 2 2 2 2 1 2 2 2 2 2 2 2 2 2 2 2 1 1 2 1 2 | T04 | M03 | G01 | 1A |
| 1kG | NA18618 | 14 | 2 | 16 | 187 | 33 | 161 | 2 2 2 2 2 2 2 2 2 2 2 2 2 2 2 1 1 2 2 | T04 | M03 | G01 | 1A |
| 1kG | NA18982 | 14 | 2 | 16 | 190 | 19 | 172 | 2 2 2 2 1 2 2 2 2 2 2 2 2 2 2 2 1 2 2 | T04 | M03 | G01 | 1A |
| 1kG | NA07051 | 13 | 3 | 16 | 184 | 38 | 159 | 2 2 2 2 1 2 2 2 2 2 2 2 2 2 2 2 1 1 2 2 | T07 | M03 | G01 | 2A |
| CG69 | GS12883 | 12 | 4 | 16 | 178 | 48 | 155 | 2 2 2 1 2 2 2 2 2 2 2 2 2 2 2 2 1 1 2 1 2 | T07 | M03 | G01 | 2A |
| 1kG | HG02613 | 12 | 4 | 16 | 182 | 45 | 154 | 2 2 2 1 1 2 2 2 2 2 2 2 2 2 2 2 2 2 1 2 1 | T11 | M03 | G01 | 2A |
| 1kG | HG03015 | 13 | 3 | 16 | 179 | 61 | 141 | 1 2 2 1 1 2 2 2 2 2 2 2 2 2 2 2 2 2 2 2 | T11 | M03 | G01 | 2A |
| 1kG | NA18641 | 14 | 2 | 16 | 197 | 15 | 169 | 2 2 2 1 2 2 2 2 2 2 2 2 2 2 2 2 2 1 2 | T04 | M03 | G01 | 1A |
| 1kG | NA12282 | 8 | 8 | 16 | 150 | 97 | 134 | 1 1 2 2 2 1 1 2 2 2 2 2 2 2 1 2 2 1 1 2 2 2 1 2 | T24 | M03 | G02 | 3A |
| 1kG | NA21123 | 14 | 2 | 16 | 192 | 20 | 169 | 2 2 2 2 1 2 2 2 2 2 2 2 2 2 2 2 2 2 1 | T04 | M03 | G03 | 1A |
| 1kG | HG03765 | 14 | 2 | 16 | 188 | 28 | 165 | 2 2 2 2 1 2 2 2 2 2 2 2 2 2 2 1 2 2 | T04 | M03 | G01 | 1A |
| 1kG | HG00641 | 12 | 4 | 16 | 175 | 54 | 152 | 1 2 2 2 1 2 2 2 2 2 2 2 1 2 2 1 2 2 2 2 | T07 | M03 | G01 | 2A |

| | | | | | | | | | | | | |
|---|---|---|---|---|---|---|---|---|---|---|---|---|
| 1kG | HG01861 | 13 | 3 | 16 | 176 | 46 | 159 | 2 2 2 2 1 2 2 2 2 2 2 2 2 2 2 1 2 2 1 2 | T07 | M03 | G03 | 2A |
| 1kG | NA11840 | 14 | 2 | 16 | 186 | 26 | 169 | 1 2 2 2 2 2 2 2 2 2 2 2 1 2 2 2 2 2 | T04 | M03 | G01 | 1A |
| 1kG | NA20767 | 14 | 2 | 16 | 163 | 78 | 140 | 1 2 2 2 2 2 2 2 2 2 2 2 2 2 2 1 2 2 2 | T07 | M03 | G02 | 2A |
| 1kG | HG01846 | 12 | 4 | 16 | 157 | 82 | 142 | 1 2 2 2 2 2 2 2 2 2 2 2 1 2 2 1 1 2 2 2 | T07 | M03 | G02 | 2A |
| 1kG | HG00255 | 11 | 5 | 16 | 182 | 37 | 162 | 2 2 2 2 2 2 2 2 2 2 2 2 1 2 2 1 1 1 2 1 2 | T04 | M03 | G01 | 1A |
| TMG | GL57228616JA | 13 | 4 | 16 | | | | 1 2 2 1 2 2 2 2 2 2 2 2 2 2 2 2 1 2 2 1 | | M01 | | |
| TMG | GL83348210WQ | 14 | 4 | 16 | | | | 2 1 1 1 2 2 2 2 2 2 2 2 2 2 2 2 1 2 2 2 | | M01 | | |
| TMG | GL64272211PN | 14 | 2 | 16 | | | | 2 2 2 2 2 2 2 2 2 2 2 2 2 2 1 2 2 1 | | M01 | | |
| TMG | GL45262410DR | 16 | 2 | 16 | | | | 2 2 1 2 2 2 2 2 2 2 2 2 2 2 2 1 2 2 | | M01 | | |
| TMG | GL28922310AJ | 12 | 4 | 16 | | | | 2 2 2 1 2 1 2 2 2 2 2 2 2 2 2 2 2 2 | | M01 | | |
| TMG | GL23237910XH | 11 | 7 | 16 | | | | 1 2 2 2 2 2 2 1 1 2 2 2 2 2 2 2 2 1 1 2 2 | | M01 | | |
| 1kG | HG00851 | 14 | 2 | 16 | 161 | 78 | 142 | 1 2 2 2 1 2 2 2 2 2 2 2 2 2 2 2 2 2 | T07 | M01 | G02 | 2A |
| 1kG | HG01277 | 15 | 1 | 16 | 195 | 22 | 164 | 2 2 2 2 2 2 2 2 2 2 2 2 2 2 2 2 2 1 | T04 | M01 | G01 | 1A |
| 1kG | HG01796 | 16 | | 16 | 192 | 28 | 161 | 2 2 2 2 2 2 2 2 2 2 2 2 2 2 2 2 2 | T04 | M01 | G01 | 1A |
| 1kG | NA18560 | 16 | | 16 | 185 | 33 | 163 | 2 2 2 2 2 2 2 2 2 2 2 2 2 2 2 2 2 | T04 | M01 | G01 | 1A |
| 1kG | NA20778 | 14 | 2 | 16 | 198 | 17 | 166 | 2 2 2 2 1 2 2 2 2 2 2 2 2 2 2 2 2 1 2 | T04 | M01 | G01 | 1A |
| 1kG | HG02076 | 15 | 1 | 16 | 171 | 63 | 147 | 1 2 2 2 2 2 2 2 2 2 2 2 2 2 2 2 2 | T07 | M01 | G02 | 2A |
| 1kG | HG03746 | 13 | 3 | 16 | 166 | 72 | 143 | 1 2 2 2 2 1 2 2 2 2 2 2 2 2 2 2 2 1 2 | T07 | M01 | G02 | 2A |
| 1kG | HG03631 | 13 | 3 | 16 | 151 | 94 | 136 | 1 2 2 2 2 1 2 2 2 2 2 2 2 2 2 2 1 2 2 | T27 | M01 | G02 | 3A |
| CG69 | GS12892 | 14 | 2 | 16 | 189 | 32 | 160 | 2 2 2 1 2 2 2 2 2 2 2 2 2 2 2 2 1 2 | T04 | M01 | G01 | 1A |
| 1kG | HG03241 | 15 | 1 | 16 | 185 | 42 | 154 | 2 2 2 1 2 2 2 2 2 2 2 2 2 2 2 2 2 | T11 | M01 | G01 | 2A |
| 1kG | HG01348 | 14 | 2 | 16 | 191 | 24 | 166 | 2 2 2 2 1 2 2 2 2 2 2 2 2 2 2 1 2 2 2 | T04 | M01 | G01 | 1A |

| | | | | | | | | | | | | |
|---|---|---|---|---|---|---|---|---|---|---|---|---|
| 1kG | HG02390 | 15 | 1 | 16 | 19<br>9 | 18 | 164 | 2 2 2 2 2 2 2 2 2 2 2 2 2 1 2 2 2 | T04 | M01 | G01 | 1A |
| 1kG | HG02408 | 15 | 1 | 16 | 19<br>2 | 26 | 163 | 2 2 2 2 2 2 2 2 2 2 2 2 2 2 1 2 2 2 | T04 | M01 | G01 | 1A |
| 1kG | NA18565 | 15 | 1 | 16 | 18<br>0 | 38 | 163 | 2 2 2 2 2 2 2 2 2 2 2 2 2 2 1 2 2 | T04 | M01 | G03 | 1A |
| 1kG | HG03760 | 15 | 1 | 16 | 19<br>1 | 21 | 169 | 2 2 2 2 2 2 2 2 2 2 2 2 2 2 1 2 2 | T04 | M01 | G01 | 1A |
| 1kG | HG04025 | 15 | 1 | 16 | 19<br>2 | 19 | 170 | 2 2 2 2 2 2 2 2 2 2 2 2 2 2 1 2 2 | T06 | M01 | G01 | 1A |
| 1kG | HG00640 | 15 | 1 | 16 | 19<br>6 | 20 | 165 | 2 2 2 2 2 2 2 2 2 2 2 2 2 2 2 1 2 | T04 | M01 | G03 | 1A |
| 1kG | HG04056 | 15 | 1 | 16 | 19<br>6 | 22 | 163 | 2 2 2 2 2 2 2 2 2 2 2 2 1 2 2 2 2 | T03 | M01 | G01 | 1A |
| 1kG | HG04098 | 13 | 3 | 16 | 15<br>2 | 91 | 138 | 1 2 2 2 2 1 2 2 2 2 2 2 2 2 1 2 2 2 2 | T24 | M01 | G02 | 3A |
| 1kG | HG00246 | 14 | 2 | 16 | 18<br>2 | 39 | 160 | 2 2 2 2 2 2 2 2 2 2 2 2 2 1 2 2 2 1 | T04 | M01 | G01 | 1A |
| 1kG | NA12777 | 14 | 2 | 16 | 16<br>9 | 58 | 154 | 2 2 2 2 1 2 2 2 2 2 2 2 2 2 2 2 1 2 | T07 | M01 | G03 | 2A |
| 1kG | NA12763 | 13 | 3 | 16 | 15<br>9 | 75 | 147 | 2 1 2 2 2 2 2 2 2 2 2 2 2 2 1 2 2 1 2 | T07 | M01 | G03 | 2A |
| 1kG | HG00325 | 14 | 2 | 16 | 19<br>1 | 26 | 164 | 2 2 2 2 2 2 2 2 2 2 2 2 2 1 2 2 1 2 | T04 | M01 | G01 | 1A |
| 1kG | HG00189 | 14 | 2 | 16 | 16<br>2 | 67 | 152 | 1 2 2 2 2 2 2 2 2 2 2 2 2 2 2 2 1 2 | T07 | M01 | G02 | 2A |
| 1kG | HG02694 | 14 | 2 | 16 | 18<br>0 | 49 | 152 | 2 2 2 2 2 2 1 2 2 2 2 2 2 2 1 2 2 2 | T07 | M01 | G01 | 2A |
| 1kG | HG04200 | 12 | 5 | 15 | 90 | 21<br>5 | 76 | 1 1 1 1 2 1 2 2 2 2 2 2 2 2 2 2 2 2 2 2 | T47 | M03 | G10 | 5A |
| 1kG | HG02943 | 15 | 2 | 15 | 17<br>8 | 49 | 154 | 2 2 2 2 2 2 2 2 2 2 2 2 2 2 1 1 2 | T17 | M02 | G07 | 2A |
| 1kG | HG03711 | 12 | 5 | 15 | 12<br>3 | 15<br>4 | 104 | 2 1 1 2 2 2 2 2 2 2 2 2 2 2 2 2 1 2 1 1 | T40 | M03 | G10 | 4A |
| 1kG | NA20585 | 12 | 5 | 15 | 16<br>4 | 68 | 149 | 1 1 2 2 2 1 2 2 2 2 2 2 2 2 2 2 2 1 2 1 | T07 | M03 | G02 | 2A |
| 1kG | NA19834 | 13 | 4 | 15 | 18<br>5 | 40 | 156 | 2 2 1 2 2 2 2 2 2 2 2 2 2 2 1 2 1 2 1 2 | T12 | M03 | G01 | 2A |
| CG69 | GS19834 | 13 | 4 | 15 | 18<br>6 | 40 | 155 | 2 2 1 2 2 2 2 2 2 2 2 2 2 1 2 1 2 1 2 | T12 | M03 | G01 | 2A |
| 1kG | HG03821 | 13 | 4 | 15 | 16<br>7 | 69 | 145 | 1 2 2 1 1 2 2 2 2 2 2 2 2 2 2 2 1 2 2 2 | T07 | M03 | G01 | 2A |
| 1kG | HG01447 | 15 | 2 | 15 | 19<br>3 | 17 | 171 | 2 2 2 2 2 2 2 2 2 2 2 2 2 2 2 2 1 1 | T05 | M03 | G01 | 1A |

| | | | | | | | | | | | | |
|---|---|---|---|---|---|---|---|---|---|---|---|---|
| 1kG | NA12287 | 11 | 6 | 15 | 177 | 47 | 157 | 1 2 2 2 1 1 2 2 2 2 2 2 2 2 2 2 1 2 2 1 1 | T07 | M03 | G01 | 2A |
| 1kG | NA20543 | 12 | 5 | 15 | 172 | 61 | 148 | 2 2 2 1 1 2 2 2 2 2 2 2 2 2 2 1 2 2 1 1 | T07 | M03 | G01 | 2A |
| 1kG | NA20766 | 13 | 4 | 15 | 178 | 47 | 156 | 2 2 2 1 2 2 2 2 2 2 2 2 2 2 1 2 2 1 1 | T07 | M03 | G01 | 2A |
| 1kG | HG01271 | 11 | 6 | 15 | 184 | 42 | 155 | 2 2 2 1 2 2 2 2 2 2 2 2 2 2 1 1 1 2 1 1 2 | T07 | M03 | G01 | 2A |
| 1kG | HG01455 | 13 | 4 | 15 | 178 | 48 | 155 | 2 2 2 1 1 2 2 2 2 2 2 2 2 2 2 1 2 1 2 | T07 | M03 | G01 | 2A |
| 1kG | NA19726 | 13 | 4 | 15 | 187 | 34 | 160 | 2 2 2 1 1 2 2 2 2 2 2 2 2 2 2 1 2 1 2 | T05 | M03 | G01 | 1A |
| 1kG | HG00533 | 14 | 3 | 15 | 178 | 49 | 154 | 2 2 2 1 2 2 2 2 2 2 2 2 2 2 1 1 2 2 | T07 | M03 | G01 | 2A |
| 1kG | HG02219 | 13 | 4 | 15 | 172 | 62 | 147 | 2 2 2 1 1 2 2 2 2 2 2 2 2 2 2 1 2 1 2 | T07 | M03 | G01 | 2A |
| 1kG | HG02380 | 13 | 4 | 15 | 179 | 47 | 155 | 2 2 2 1 2 2 2 2 2 2 2 2 2 2 1 1 2 1 2 | T07 | M03 | G01 | 2A |
| CG69 | HG00733 | 12 | 5 | 15 | 181 | 43 | 157 | 1 2 2 2 1 2 2 2 2 2 2 2 2 2 2 2 1 1 2 1 2 | T07 | M03 | G01 | 2A |
| 1kG | HG00419 | 13 | 4 | 15 | 156 | 88 | 137 | 1 2 2 2 2 2 2 2 2 2 2 2 2 2 1 1 2 1 2 | T24 | M03 | G02 | 3A |
| 1kG | NA19773 | 15 | 2 | 15 | 162 | 81 | 138 | 1 2 2 2 2 2 2 2 2 2 2 2 2 2 2 1 2 | T24 | M03 | G02 | 3A |
| 1kG | NA20796 | 14 | 3 | 15 | 154 | 97 | 130 | 1 2 2 2 1 2 2 2 2 2 2 2 2 2 2 1 2 2 | T24 | M03 | G02 | 3A |
| 1kG | HG01709 | 12 | 5 | 15 | 149 | 107 | 125 | 1 2 2 2 1 1 2 2 2 2 2 2 2 2 2 2 1 2 1 2 | T35 | M03 | G02 | 4A |
| 1kG | HG00336 | 14 | 3 | 15 | 133 | 125 | 123 | 2 2 1 2 2 2 2 2 2 2 2 2 2 2 1 1 2 2 | T40 | M03 | G07 | 4A |
| 1kG | HG00592 | 13 | 4 | 15 | 174 | 46 | 161 | 2 2 2 2 1 1 2 2 2 2 2 2 2 2 2 2 2 1 2 1 | T05 | M02 | G03 | 1A |
| 1kG | HG00281 | 14 | 3 | 15 | 193 | 16 | 172 | 2 2 2 2 1 2 2 2 2 2 2 2 2 2 2 2 2 1 1 | T05 | M03 | G01 | 1A |
| 1kG | HG01786 | 13 | 4 | 15 | 186 | 32 | 163 | 2 2 1 1 2 2 2 2 2 2 2 1 2 2 2 2 2 2 1 | T06 | M03 | G01 | 1A |
| 1kG | HG03963 | 14 | 3 | 15 | 152 | 60 | 169 | 2 1 2 1 2 2 2 2 2 2 2 2 2 2 2 2 2 1 2 | T01 | M02 | G15 | 1A |
| 1kG | HG03624 | 12 | 5 | 15 | 163 | 74 | 144 | 1 2 2 2 1 2 2 2 2 2 2 2 1 2 2 2 2 2 1 1 | T07 | M03 | G02 | 2A |
| 1kG | HG03941 | 15 | 2 | 15 | 169 | 36 | 176 | 2 2 2 1 2 2 2 2 2 2 2 2 2 2 1 2 2 | T01 | M03 | G13 | 1A |
| 1kG | NA19119 | 15 | 2 | 15 | 204 | 12 | 165 | 2 2 2 2 2 2 2 2 2 2 2 2 1 1 2 2 2 | T03 | M03 | G01 | 1A |

| | | | | | | | | | | | | |
|---|---|---|---|---|---|---|---|---|---|---|---|---|
| 1kG | HG00864 | 15 | 2 | 15 | 200 | 18 | 163 | 2 2 2 2 2 2 2 2 2 1 2 2 2 1 2 2 | T05 | M03 | G01 | 1A |
| 1kG | HG02224 | 13 | 4 | 15 | 184 | 31 | 166 | 2 2 2 1 1 2 2 2 2 2 2 2 1 2 2 2 1 2 2 | T05 | M03 | G01 | 1A |
| 1kG | HG01872 | 14 | 3 | 15 | 183 | 46 | 152 | 2 2 2 1 2 2 2 2 2 2 2 1 2 2 2 1 2 2 | T07 | M03 | G01 | 2A |
| 1kG | HG01988 | 10 | 7 | 15 | 162 | 75 | 144 | 2 2 2 1 1 2 2 2 2 2 2 2 1 2 2 1 1 2 1 2 1 2 | T07 | M03 | G01 | 2A |
| 1kG | HG02130 | 12 | 5 | 15 | 188 | 35 | 158 | 2 2 2 1 1 2 2 2 2 2 2 2 1 2 2 2 1 2 1 2 | T07 | M03 | G01 | 2A |
| 1kG | NA19089 | 13 | 4 | 15 | 176 | 54 | 151 | 1 2 2 2 2 2 2 2 2 2 2 1 2 2 2 1 2 1 2 | T07 | M03 | G01 | 2A |
| 1kG | NA21104 | 16 | 1 | 15 | 173 | 63 | 145 | 2 2 2 1 2 2 2 2 2 2 2 2 2 2 2 2 | T07 | M03 | G01 | 2A |
| 1kG | HG03082 | 11 | 6 | 15 | 186 | 41 | 154 | 1 2 2 2 1 2 2 2 2 2 2 2 2 2 1 1 2 1 2 1 2 | T12 | M03 | G01 | 2A |
| 1kG | NA18507 | 15 | 2 | 15 | 181 | 57 | 143 | 2 2 2 1 2 2 2 2 2 2 2 1 2 2 2 2 2 | T15 | M03 | G04 | 2A |
| 1kG | HG04155 | 15 | 2 | 15 | 154 | 65 | 162 | 1 2 2 2 2 2 2 2 2 2 2 2 2 2 1 2 2 | T01 | M02 | G14 | 1A |
| 1kG | HG02820 | 12 | 5 | 15 | 148 | 107 | 126 | 1 2 2 2 2 2 2 2 2 2 2 1 2 2 1 1 2 1 2 2 | T40 | M03 | G04 | 4A |
| 1kG | NA18595 | 14 | 3 | 15 | 187 | 32 | 162 | 2 2 2 2 1 2 2 2 2 2 2 2 2 2 2 1 2 1 | T05 | M03 | G03 | 1A |
| 1kG | NA12003 | 12 | 5 | 15 | 182 | 42 | 157 | 2 2 2 2 1 2 2 2 2 2 2 2 1 2 2 2 1 1 2 1 | T07 | M03 | G01 | 2A |
| 1kG | HG01323 | 13 | 4 | 15 | 160 | 82 | 139 | 1 2 2 2 2 2 2 2 2 2 2 2 1 2 2 1 2 1 2 | T24 | M03 | G02 | 3A |
| 1kG | HG01697 | 12 | 5 | 15 | 156 | 88 | 137 | 1 2 2 2 1 2 2 2 2 2 2 2 2 2 2 1 2 1 2 1 | T24 | M03 | G02 | 3A |
| 1kG | NA18984 | 12 | 5 | 15 | 132 | 136 | 113 | 2 2 1 1 1 2 2 2 2 2 2 2 1 2 2 1 2 2 2 2 | T40 | M03 | G07 | 4A |
| 1kG | HG00599 | 14 | 3 | 15 | 187 | 31 | 163 | 2 2 2 1 2 2 2 2 2 2 2 1 2 2 1 2 2 2 | T05 | M03 | G01 | 1A |
| 1kG | HG02253 | 13 | 4 | 15 | 181 | 39 | 161 | 2 2 2 1 1 2 2 2 2 2 2 2 1 2 2 1 2 2 2 | T05 | M03 | G01 | 1A |
| 1kG | HG04176 | 14 | 3 | 15 | 191 | 23 | 167 | 2 2 2 1 2 2 2 2 2 2 2 1 2 2 1 2 2 2 | T05 | M03 | G01 | 1A |
| 1kG | NA18986 | 15 | 2 | 15 | 188 | 32 | 161 | 2 2 2 2 2 2 2 2 2 2 1 2 2 1 2 2 2 | T05 | M03 | G01 | 1A |
| 1kG | HG00342 | 13 | 4 | 15 | 169 | 65 | 147 | 2 2 2 1 1 2 2 2 2 2 2 2 2 2 1 2 1 2 2 | T07 | M03 | G01 | 2A |
| 1kG | HG00373 | 14 | 3 | 15 | 178 | 53 | 150 | 2 2 2 1 2 2 2 2 2 2 2 2 2 1 2 2 1 2 | T07 | M03 | G01 | 2A |

| | | | | | | | | | | | | |
|---|---|---|---|---|---|---|---|---|---|---|---|---|
| 1kG | HG01808 | 13 | 4 | 15 | 167 | 67 | 147 | 1 2 2 2 1 2 2 2 2 2 2 2 1 2 2 2 2 1 2 | T07 | M03 | G02 | 2A |
| 1kG | HG03021 | 14 | 3 | 15 | 183 | 39 | 159 | 2 2 2 1 2 2 2 2 2 2 2 1 2 2 1 2 2 2 | T07 | M03 | G01 | 2A |
| 1kG | NA18591 | 14 | 3 | 15 | 156 | 84 | 141 | 1 2 2 2 1 2 2 2 2 2 2 2 1 2 2 2 2 2 | T07 | M03 | G02 | 2A |
| 1kG | NA18951 | 14 | 3 | 15 | 179 | 50 | 152 | 2 2 2 2 2 2 2 2 2 2 1 2 2 1 2 2 1 2 | T07 | M03 | G01 | 2A |
| 1kG | HG01598 | 13 | 4 | 15 | 154 | 93 | 134 | 1 2 2 2 1 2 2 2 2 2 2 2 1 2 2 2 2 1 2 | T24 | M03 | G02 | 3A |
| 1kG | HG03702 | 11 | 6 | 15 | 179 | 39 | 163 | 1 2 2 2 2 1 2 2 2 2 2 2 2 1 2 2 2 1 2 1 1 | T05 | M03 | G01 | 1A |
| 1kG | HG00513 | 12 | 5 | 15 | 165 | 69 | 147 | 1 2 2 2 2 2 2 2 2 2 2 2 1 2 2 1 2 2 1 1 | T07 | M03 | G02 | 2A |
| 1kG | HG03736 | 13 | 4 | 15 | 186 | 34 | 161 | 1 2 2 2 2 2 2 2 2 2 2 2 2 2 1 2 2 1 1 | T05 | M03 | G01 | 1A |
| 1kG | NA19716 | 15 | 2 | 15 | 194 | 13 | 174 | 2 2 2 2 2 2 2 2 2 2 1 2 2 2 2 1 | T05 | M03 | G01 | 1A |
| 1kG | NA20502 | 13 | 4 | 15 | 175 | 56 | 150 | 2 2 2 1 2 2 2 2 2 2 2 2 1 2 2 1 2 2 1 | T07 | M03 | G01 | 2A |
| CG69 | GS20502 | 13 | 4 | 15 | 177 | 56 | 148 | 2 2 2 1 2 2 2 2 2 2 2 2 1 2 2 1 2 2 1 | T07 | M03 | G01 | 2A |
| 1kG | NA18623 | 15 | 2 | 15 | 187 | 32 | 162 | 2 2 2 1 2 2 2 2 2 2 2 2 2 1 2 2 2 | T05 | M03 | G01 | 1A |

**Tabelle 2c: Covid-19 Gruppenzuordnung**

| | 32 TMG Homozygot Effekt (2) | | 381 WGS Homozygot (2) Effekt | | 72 Chr.3 Homozygot kein Effekt | | Heterozygot | | Homozygot Effekt | | 32 TMG Homozygot Effekt | 381 WGS Homozygot Effekt | 72 Chr.3 Homorygot kein Effekt | Heterozygot | Homozygot Effekt |
|---|---|---|---|---|---|---|---|---|---|---|---|---|---|---|---|
| | min | max | min | max | min | max | min | max | min | max | Intervall | Intervall | Intervall | Intervall | Intervall |
| **T01** | 11 | 15 | 161 | 190 | 72 | 72 | 0 | 0 | 0 | 0 | [11;32] | [160;381] | [72;72] | | [0;0] |
| **T02** | 3 | 6 | 161 | 168 | 72 | 72 | 0 | 0 | 0 | 0 | [0;10] | [160;381] | [72;72] | | [0;0] |
| **T03** | 10 | 16 | 160 | 168 | 1 | 44 | 0 | 3 | 28 | 71 | [10;16] | [160;381] | [1;71] | | [1;71] |
| **T04** | 16 | 19 | 160 | 180 | 0 | 0 | 0 | 0 | 72 | 72 | [16;32] | [160;381] | [0;0] | | [72;72] |
| **T05** | 6 | 15 | 160 | 175 | 0 | 0 | 0 | 0 | 72 | 72 | [0;15] | [160;381] | [0;0] | | [72;72] |
| **T06** | 11 | 16 | 160 | 170 | 0 | 0 | 1 | 4 | 68 | 71 | [0;32] | [160;381] | [0;0] | | [1;71] |
| **T07** | 15 | 18 | 140 | 159 | 0 | 0 | 0 | 0 | 72 | 72 | [15;32] | [140;159] | [0;0] | | [72;72] |
| **T08** | 14 | 14 | 140 | 159 | 0 | 0 | 0 | 0 | 72 | 72 | [14;14] | [140;159] | [0;0] | | [72;72] |
| **T09** | 7 | 13 | 140 | 159 | 0 | 0 | 0 | 0 | 72 | 72 | [7;13] | [140;159] | [0;0] | | [72;72] |
| **T10** | 4 | 6 | 140 | 157 | 0 | 0 | 0 | 0 | 72 | 72 | [0;6] | [140;159] | [0;0] | | [72;72] |
| **T11** | 16 | 17 | 141 | 157 | 0 | 1 | 0 | 1 | 71 | 71 | [16;17] | [140;159] | [0;2] | [0;2] | [70;71] |
| **T12** | 14 | 15 | 144 | 159 | 0 | 1 | 0 | 2 | 70 | 71 | [14;15] | [140;159] | [0;2] | [0;2] | [70;71] |
| **T13** | 7 | 13 | 141 | 159 | 0 | 1 | 0 | 2 | 70 | 71 | [7;13] | [140;159] | [0.2] | [0;2] | [70;71] |
| **T14** | **3** | 6 | 140 | 154 | 0 | 1 | 0 | 2 | 70 | 71 | [0;6] | [140;159] | [0;2] | [0;2] | [70;71] |
| **T15** | 10 | 18 | 140 | 157 | 0 | 2 | 3 | 19 | 53 | 69 | [10;32] | [140;159] | [0;2] | [3;72] | [53;70] |
| **T16** | 5 | 6 | 141 | 143 | 0 | 1 | 2 | 3 | 69 | 69 | [0;6] | [140;159] | [0;2] | | [69;69] |
| **T17** | 14 | 15 | 142 | 159 | 18 | 20 | 0 | 2 | 52 | 52 | [14;15] | [140;159] | [10;20] | | [52;53] |
| **T18** | 7 | 13 | 142 | 159 | 16 | 20 | 0 | 4 | 52 | 53 | [7;13] | [140;159] | [10;20] | | [52;53] |
| **T19** | 3 | 6 | 140 | 151 | 18 | 20 | 0 | 2 | 52 | 53 | [0;6] | [140;159] | [10;20] | | [52;53] |
| **T20** | 11 | 14 | 140 | 146 | 1 | 2 | 18 | 19 | 52 | 52 | [10;15] | [140;159] | [1;10] | | [52;52] |
| **T21** | 10 | 15 | 140 | 150 | 0 | 0 | 20 | 26 | 46 | 52 | [10;15] | [140;159] | [0;0] | [20;72] | [0;69] |
| **T22** | 11 | 14 | 140 | 159 | 65 | 72 | 0 | 3 | 0 | 6 | [10;15] | [140;159] | [21;72] | | [0;71] |
| **T23** | 2 | 6 | 141 | 158 | 44 | 72 | 0 | 8 | 0 | 28 | [0;6] | [140;159] | [21;72] | | [0;71] |
| **T24** | 14 | 17 | 130 | 139 | 0 | 0 | 0 | 0 | 72 | 72 | [14;17] | [130;139] | [0;0] | | [72;72] |
| **T25** | 7 | 13 | 130 | 139 | 0 | 0 | 0 | 0 | 72 | 72 | [7;13] | [130;139] | [0;0] | | [72;72] |
| **T26** | 3 | 6 | 130 | 139 | 0 | 0 | 0 | 0 | 72 | 72 | [0;6] | [130;139] | [0;0] | | [72;72] |
| **T27** | 3 | 16 | 131 | 139 | 0 | 1 | 0 | 5 | 67 | 71 | [3;16] | [130;139] | [0;3] | | [65;71] |
| **T28** | 13 | 15 | 130 | 139 | 0 | 2 | 18 | 20 | 52 | 54 | [13;15] | [130;139] | [0;3] | | [50;65] |
| **T29** | 7 | 12 | 130 | 139 | 0 | 2 | 11 | 20 | 52 | 61 | [7;12] | [130;139] | [0;3] | | [50;65] |
| **T30** | 3 | 6 | 130 | 135 | 0 | 3 | 11 | 20 | 52 | 61 | [0;6] | [130;139] | [0;3] | | [50;65] |
| **131** | 8 | 13 | 130 | 136 | 18 | 20 | 0 | 2 | 52 | 52 | [7;13] | [130;139] | [4;72] | | [52;52] |

(fortgesetzt)

| | 32 TMG Homozygot Effekt (2) | | 381 WGS Homozygot (2) Effekt | | 72 Chr.3 Homozygot kein Effekt | | Heterozygot | | Homozygot Effekt | | 32 TMG Homozygot Effekt | 381 WGS Homozygot Effekt | 72 Chr.3 Homorygot kein Effekt | Heterozygot | Homozygot Effekt |
|---|---|---|---|---|---|---|---|---|---|---|---|---|---|---|---|
| | min | max | min | max | min | max | min | max | min | max | Intervall | Intervall | Intervall | Intervall | Intervall |
| **T32** | 3 | 6 | 130 | 139 | 12 | 20 | 0 | 2 | 51 | 60 | [0;6] | [130;139] | [4;72] | | [50;60] |
| **T133** | 11 | 14 | 138 | 139 | 69 | 72 | 0 | 3 | 0 | 0 | [11;14] | [130;139] | [4;72] | | [0;0] |
| **T34** | 2 | 5 | 130 | 139 | 57 | 72 | 0 | 15 | 0 | 0 | [0;6] | [130;139] | [4;72] | | [0;0] |
| **T35** | 13 | 15 | 116 | 129 | 0 | 0 | 0 | 0 | 72 | 72 | [13;15] | [100;129] | [0;0] | | [72;72] |
| **T36** | 7 | 12 | 105 | 129 | 0 | 0 | 0 | 0 | 72 | 72 | [7;12] | [100;129] | [0;0] | | [72;72] |
| **T37** | 3 | 6 | 102 | 129 | 0 | 19 | 0 | 2 | 51 | 72 | [0;6] | [100;129] | [0;19] | [0;2] | [50;72] |
| **T38** | 7 | 15 | 106 | 129 | 0 | 0 | 1 | 3 | 69 | 71 | [7;15] | [100;129] | [0;0] | | [60;71] |
| **139** | 3 | 6 | 107 | 129 | 0 | 0 | 1 | 8 | 64 | 71 | [0;6] | [100,129] | [0;0] | [1;9] | [61;71] |
| **T40** | 13 | 15 | 100 | 129 | 0 | 1 | 15 | 72 | 0 | 57 | [13;15] | [100;129] | [0;5] | | [0;60] |
| **T41** | 7 | 12 | 102 | 129 | 0 | 3 | 15 | 46 | 26 | 57 | [7;12] | [100;129] | [0;5] | | [1;60] |
| **T42** | 1 | 6 | 100 | 129 | 0 | 2 | 14 | 44 | 28 | 58 | [0;6] | [100;129] | [0;5] | [10;50] | [1;60] |
| **T43** | 4 | 15 | 101 | 126 | 15 | 69 | 3 | 57 | 0 | 0 | [0;15] | [100;129] | [10;71] | | [0;0] |
| **T44** | 3 | 5 | 117 | 126 | 20 | 20 | 0 | 0 | 52 | 52 | [0;6] | [100;129] | [20;20] | [0;0] | [52;52] |
| **T45** | 3 | 4 | 125 | 126 | 72 | 72 | 0 | 0 | 0 | 0 | [0;6] | [100:129] | [72;72] | | [0;0] |
| **T46** | 3 | 13 | 81 | 98 | 0 | 0 | 12 | 33 | 39 | 60 | [0;13] | [0;99] | [0;0] | | [30;72] |
| **T47** | 13 | 16 | 52 | 99 | 0 | 0 | 72 | 72 | 0 | 0 | [13;16] | [0;99] | [0;0] | [72;72] | [0;0] |
| **T48** | 7 | 12 | 34 | 96 | 0 | 0 | 72 | 72 | 0 | 0 | [7;12] | [0;99] | [0;0] | [72;72] | [0;0] |
| **T49** | 1 | 6 | 28 | 82 | 0 | 0 | 72 | 72 | 0 | 0 | [0;6] | [0;99] | [0;0] | [72;72] | [0;0] |
| **T50** | 7 | 14 | 49 | 96 | 0 | 20 | 44 | 71 | 0 | 28 | [7;14] | [0;99] | [0;72] | [0;71] | [0;29] |
| **T51** | 1 | 6 | 42 | 98 | 0 | 28 | 44 | 71 | 0 | 28 | [0;6] | [0;99] | [0;72] | [0;71] | [0;29] |

Tabelle 3a: FYCO1 SNPs

| coord | DBSNP.ID | dbsnp gene strand | dbsnp gene symbol | dbsnp Ref | dbsnp Alt | COV EA | COV OA | Pval main | Pval_corr sex_age | Pval min | isTMG | Klasse A/B |
|---|---|---|---|---|---|---|---|---|---|---|---|---|
| 3:45960420 | rs1994491 | - | FYCO1 | G | C | C | G | 5,981E-06 | 4,638E-06 | 4,638E-06 | | A |
| 3:45960700 | rs1994493 | - | FYCO1 | C | T | T | C | 3,364E-06 | 7,644E-06 | 3,364E-06 | | A |
| 3:45970391 | rs35831747 | - | FYCO1 | G | A | A | G | 3,072E-06 | 7,729E-06 | 3,072E-06 | | A |
| 3:45974092 | rs35477280 | - | FYCO1 | G | A | A | G | 2,938E-06 | 7,582E-06 | 2.938E-06 | | A |
| 3:45975443 | rs13066516 | - | FYCO1 | C | T | T | C | 3,285E-06 | 7,688E-06 | 3,285E-06 | | A |
| 3:45976662 | rs77902290 | - | FYCO1 | C | T | T | C | 3,086E-06 | 7.51E-06 | 3,086E-06 | | A |
| 3:45981171 | rs2171531 | +;;- | CXCR6 ;; FYCO1 | C | T | T | C | 4,609E-06 | 1,041E-05 | 4,609E-06 | | A |
| 3:45983476 | rs55920693 | +;;- | CXCR6 ;; FYCO1 | T | A | A | T | 4,661E-06 | 0,0000108 | 4,661E-06 | | A |
| 3:45989873 | rs56332428 | +;;- | CXCR6 ;; FYCO1 | C | T | T | C | 8,317E-06 | 6,324E-06 | 6,314E-06 | | A |
| 3:45989921 | rs71325095 | +;;- | CXCR6 ;; FYCO1 | G | T | T | G | 7,851E-06 | 5,997E-06 | 5,997E-06 | | A |
| 3:45993645 | rs35501575 | - | FYCO1 | C | T | T | C | 4,552E-06 | 0,0000105 | 4,552E-06 | | A |
| 3:46000870 | rs13069079 | - | FYCO1 | G | A | A | G | 4,844E-06 | 1,088E-05 | 4,844E-06 | | A |
| 3:46001227 | rs76597151 | - | FYCO1 | G | A | A | G | 8,018E-06 | 6,065E-06 | 6,065E-06 | | A |
| 3:46001367 | rs34849862 | - | FYCO1 | C | A | A | C | 4,844E-06 | 1.088E-05 | 4,844E-06 | | A |
| 3:46003537 | rs36039366 | - | FYCO1 | G | A | A | G | 7,761E-06 | 5,967E-06 | 5,967E-06 | | A |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | isTMG | Klasse A/B |
|---|---|---|---|---|---|---|---|---|---|---|---|---|
| 3:46006269 | rs35525815 | - | FYCO1 | C | T | T | C | 6,012E-06 | 5,176E-06 | 5,176E-06 | | A |
| 3:46007488 | rs13078739 | - | FYCO1 | G | A | A | G | 5,015E-06 | 4,769E-06 | 4,769E-06 | | A |
| 3:46007823 | rs13079478 | - | FYCO1 | G | T | T | G | 3,01E-06 | 8,545E-06 | 3,01E-06 | X | A |
| 3:46008087 | rs13079869 | - | FYCO1 | G | A | A | G | 3.01E-06 | 8,545E-06 | 3,01E-06 | | A |
| 3:46009487 | rs33910087 | - | FYCO1 | G | A | A | G | 2,967E-06 | 8,514E-06 | 2,967E-06 | | A |
| 3:46013832 | rs71325100 | - | FYCO1 | C | T | T | C | 2.55E-06 | 7,604E-06 | 2,55E-06 | | A |
| 3:46018344 | rs13066062 | - | FYCO1 | G | A | A | G | 2,809E-06 | 8,183E-06 | 2,809E-06 | | A |
| 3:46022833 | rs36122610 | - | FYCO1 | G | A | A | G | 2,175E-06 | 6,059E-06 | 2,175E-06 | | A |
| 3:46024529 | rs34442130 | - | FYCO1 | T | A | A | T | 2,161E-06 | 6,023E-06 | 2,161E-06 | | A |
| 3:46025048 | rs13075758 | - | FYCO1 | G | A | A | G | 2,189E-06 | 6,095E-06 | 2,189E-06 | | A |
| 3:46036521 | rs71327003 | - | FYCO1 | C | T | T | C | 2,026E-06 | 5.715E-06 | 2,026E-06 | | A |
| 3:46039060 | rs36023124 | - | FYCO1 | G | C | C | G | 2,013E-06 | 6,15 E-06 | 2,013E-06 | | A |
| 3:45960646 | rs1994492 | - | FYCO1 | T | C | T | C | 3,364E-06 | 7,802E-06 | 3,364E-06 | | B |
| 3:45962603 | rs75928798 | - | FYCO1 | A | G | A | G | 4,304E-06 | 1,114E-05 | 4,304E-06 | | B |
| 3:45968043 | rs2373087 | - | FYCO1 | T | G | T | G | 3,244E-06 | 7,599E-06 | 3,244E-06 | | B |
| 3:45970944 | rs13099120 | - | FYCO1 | C | G | C | G | 2.822E-06 | 5,982 E-06 | 2,822E-06 | | B |
| | | | CXCR6 ;; | | | | | | | | | |
| 3:45985347 | rs6785091 | +;;- | FYCO1 | C | G | C | G | 4,913E-06 | 6,643E-06 | 4,913E-06 | | B |
| 3:45995748 | rs34381952 | - | FYCO1 | T | C | T | C | 8,108E-06 | 6,101E-06 | 6,101E-06 | | B |

(fortgesetzt)

| coord | DBSNP.ID | dbsnp gene strand | dbsnp gene symbol | dbsnp Ref | dbsnp Alt | COV EA | COV OA | Pval main | Pval_corr sex_age | Pval min | isTMG | Klasse A/B |
|---|---|---|---|---|---|---|---|---|---|---|---|---|
| 3:45996501 | rs4388012 | - | FYCO1 | A | G | A | G | 8,066E-06 | 6,101E-06 | 6,101E-06 | | B |
| 3:45997263 | rs41289616 | - | FYCO1 | T | C | T | C | 8,039E-06 | 6,101E-06 | 6,101E-06 | | B |
| 3:45999209 | rs34000569 | - | FYCO1 | A | G | A | G | 4,819E-06 | 1,083E-05 | 4,819E-06 | | B |
| 3:46000728 | rs34324101 | - | FYCO1 | T | G | T | G | 4,802E-06 | 1.086E-05 | 4, 802 E-06 | | B |
| 3:46001720 | rs35827997 | - | FYCO1 | T | G | T | G | 8,039E-06 | 6,065E-06 | 6,065E-06 | | B |
| 3:46003496 | rs35209528 | - | FYCO1 | T | C | T | C | 4,642E-06 | 1,061E-05 | 4,642E-06 | | B |
| 3:46006239 | rs35855315 | - | FYCO1 | A | G | A | G | 5,036E-06 | 4,71E-06 | 4,71E-06 | | B |
| 3:46006341 | rs17280623 | - | FYCO1 | C | G | C | G | 5,085E-06 | 4,713E-06 | 4,713E-06 | | B |
| 3:46007825 | rs13059238 | - | FYCO1 | T | C | T | C | 5,015E-06 | 4,769E-06 | 4,769E-06 | | B |
| 3:46010007 | rs13071283 | - | FYCO1 | T | C | T | C | 4,923E-06 | 4,739E-06 | 4,739E-06 | | B |
| 3:46011436 | rs17214952 | - | FYCO1 | A | G | A | G | 4,808E-06 | 4,707E-06 | 4,707E-06 | x | B |
| 3:46012279 | rs17215008 | - | FYCO1 | T | C | T | C | 2.884E-06 | 8,335E-06 | 2,884E-06 | | B |
| 3:46012391 | rs71325098 | - | FYCO1 | A | G | A | G | 2,877E-06 | 8,37E-06 | 2,877E-06 | | B |
| 3:46014545 | rs41289622 | - | FYCO1 | T | G | T | G | 2,518E-06 | 7,559E-06 | 2,518E-06 | | B |
| 3:46015569 | rs71325101 | - | FYCO1 | T | C | T | C | 4.524E-06 | 4,335E-06 | 4,335E-06 | | B |
| 3:46015933 | rs71325102 | - | FYCO1 | T | C | T | C | 4,753E-06 | 4,619E-06 | 4,619E-06 | | B |
| 3:46035097 | rs17330872 | - | FYCO1 | A | G | A | G | 1,741E-06 | 4,783E-06 | 1,741E-06 | | B |

**Tabelle 3b: FYCO1 Haplotypen-Verteilung**

| Gesamt: 2504 Genome | | | | |
|---|---|---|---|---|
| | Homozygot (2) | | | Heterozygot(1) |
| SNPs | Klasse A (27 SNPs) | Klasse B (23 SNPs) | Gemischt (A&B) | Alle 50 FYCO1-SNPs |
| alle SNPs | 74 | 1519 | | 349 |
| alle SNPs außer 1 | | 502 | | 4 |
| einige SNPs aus A oder B | 10 | 44 | 2 | |
| TMG | | Homozygot (2) | Herterozygot (1) | Homozygot (0) |
| Marker-SNP Klasse A | rs13079478 | 80 | 367 | 2057 |
| Marker-SNP Klasse B | rs17214952 | 2036 | 387 | 81 |

**Tabelle 4: Ählichkeitsbestimmungen**

| **TMG-Probe** | **Ähnlichkeitsindex** | **32 SNP-basierte Impfempfehlung** | **Ähnlichste(r) Patient(en) aus "1000 Genomes"** |
|---|---|---|---|
| GL35389515EN | 59 | 2E (1) | NA19741 |
| GL36996614FP | 59 | 5B (1) | HG01200 |
| GL38322312TJ | 57 | 1B (1); 2B (1) | HG00120; NA21126 |
| GL63427410WK | 57 | 1A (1); 2A (1); 2B (1) | HG01459; HG01512; NA12144 |
| GL32292210QG | 58 | 1A (3); 2B (2); 3B (1) | HG00346; HG00356; HG02667; HG02676; NA10847; NA20359 |
| GL44792211DV | 58 | 1A (5); 1B (1); 2B (1); 3B (1) | HG01130; HG01550; HG02610; HG03760; HG03870; HG03977; HG04025; NA20815 |
| GL47988212AC | 56 | 2D (2); 4D (1) | HG02562; HG02588: NA12154 |
| GL22377811YA | 59 | 1A (1); 3B (1) | HG00360; HG01883 |
| GL22743912JF | 60 | 1A (1) | HG01205 |
| GL28922310AJ | 57 | 1A (1) | NA07056 |
| GL48693211UV | 59 | 2D (1); 2E (1); 4D (1) | HG02562; HG03028; HG03189 |
| GL54223312YS | 56 | 3E (2); 4E (2); 2D (1); 2E (1); 3D (1); 4D (1) | HG01149; HG02136; HG02465; HG03558; HG04038; NA12748; NA18558; NA18881 |
| GL77222213DY | 58 | 5E (2) | HG03771; NA21135 |
| GL64456713ZJ | 59 | 1A (3) | HG00315; HG01437; NA19716 |
| GL37896210TN | 56 | 5E (1) | HG00100 |
| GL97352610HP | 59 | 3E (1); 4D (1); 4E (1) | HG01813; HG03718; NA18942 |
| GL22366212FG | 57 | 5B (1); 5C (1) | HG03016; HG03629 |
| GL24222510XX | 57 | 1A (2) | HG01130; NA20589 |
| GL39895215XP | 58 | 1A (2); 1B (1) | HG01796; NA18560; NA19323 |
| GL43228510XY | 58 | 3E (1); 5E (1) | HG03558; HG03867 |
| GL55694410KN | 58 | 5B (2) | HG00324; HG00335 |
| GL98739411WZ | 58 | 1A (5); 1B (1); 2B (1); 3B (1) | HG01130; HG01550; HG02610; HG03760; HG03870; HG03977; HG04025; NA20815 |
| GL22652910FJ | 58 | 3B (3); 4B (3); 2B (1); 5B (1) | HG01619; HG02032; HG03476; HG03713; HG03800; NA18970; NA19011; NA19060 |
| GL24722210CV | 57 | 2C (1); 2D (1) | HG02231; HG02588 |
| GL25442710SC | 59 | 5E (4) | HG04153; HG04171; HG04180; NA20770 |

(fortgesetzt)

| TMG-Probe | Ähnlichkeitsindex | 32 SNP-basierte Impfempfehlung | Ähnlichste(r) Patient(en) aus "1000 Genomes" |
|---|---|---|---|
| GL39797615XK | 58 | 2D (4); 3D (1) | HG00176; HG00736; HG01783; NA11894; NA20787 |
| GL39966312AG | 57 | 2B (3); 1B (1); 2A (1) | HG01204; HG01862; HG03382; NA12004; NA20586 |
| GL56525212NV | 59 | 2D (1) | HG01668 |
| GL28252210GR | 56 | 2D (2) | HG02562; HG02588 |
| W2N2T4B5U5U7 | 60 | 2A (2); 1A(1) | HG03021; HG04176; NA20543 |
| GL57285311ND | 58 | 5B (1) | HG02780 |
| GL25943811CN | 57 | 3E (1) | HG03558 |
| GL52343814YM | 59 | 2E (1) | HG00158 |
| GL23935510BT | 57 | 2D (2); 2E (1); 4D (1) | HG02588; HG03028; NA12154; NA20800 |
| GL43247212CJ | 59 | 3E (1) | HG02768 |
| GL43558617QB | 56 | 2D (2); 4D (1); 4E (1) | HG00553; NA12750; NA19669; NA20790 |
| GL48992215KV | 57 | 2B (1) | HG01986 |
| GL98888515CK | 60 | 4E (2); 2E (1) | HG00531; HG01798; NA18646 |
| GL25382311SC | 59 | 2B (1) | HG00131 |
| GL26343213BF | 56 | 2C (1) | HG02231 |
| H6E4T8EOH3D1 | 60 | 1A (1) | NA12760 |
| GL32474216NU | 57 | 1B (1) | HG01137 |
| GL46223518BK | 59 | 3E (1); 4E (1) | HG02852; HG03115 |
| GL83593813PK | 61 | 1A (1) | HG00320 |
| GL29428214MW | 58 | 1A (1); 2B (1); 4B (1) | HG03108; HG03585; NA20805 |
| GL32923811RN | 59 | 5B (3) | HG02494; HG03629; HG04093 |
| GL22868814EU | 59 | 4D (5) | HG00844; HG01303; HG02150; NA18558; NA20753 |
| GL23757411XK | 59 | 1A (3); 2A (1) | HG00346; HG00356; NA07051; NA19774 |
| GL25352411CK | 56 | 2A (1) | HG01512 |
| GL43876211KM | 58 | 5B (2) | NA20877; NA20911 |
| GL55622912CY | 56 | 2D (1) | HG02772 |
| GL22252414QR | 58 | 1B (3); 3B (1) | HG01079; HG03397; NA18498; NA19214 |
| GL22427210MD | 58 | 2B (17); 1B (4); 3B (2) | HG00628; HG01174; HG01378; HG01498; HG01551; HG01628; HG01799; HG01866; HG01944; HG02147; HG02260; HG02272; HG02700; HG02851; HG02981; HG03461; HG03470; HG03556; HG03611; HG03690; NA19355; NA19380; NA19782 |
| GL22872212FX | 59 | 2B (2); 3B (1) | HG02667; HG02676; NA20359 |
| GL46263713AJ | 59 | 2D (4) | HG00327; HG01515; HG01522; NA12878 |
| GL52845911JZ | 59 | 2B (2); 1B (1) | HG01305; HG01513; HG02312 |
| GL65483610PU | 57 | 4E (2); 3D (1); 3E (1); 5E (1) | HG00338; HG01896; HG02379; NA18537; NA19080 |
| GL72235218HK | 58 | 2B (3); 1B (1); 4B (1) | HG01377; HG02445; HG02501; NA20356; NA20806 |
| GL45934911HU | 58 | 5B (1) | NA20905 |
| GL54259511UT | 57 | 3B (1); 4B (1) | HG00353; HG00525 |
| GL83348210WQ | 59 | 5A (1) | NA12829 |
| GL44622710EP | 60 | 2D (1) | HG00311 |

(fortgesetzt)

| TMG-Probe | Ähnlichkeitsindex | 32 SNP-basierte Impfempfehlung | Ähnlichste(r) Patient(en) aus "1000 Genomes" |
|---|---|---|---|
| GL22226417BS | 58 | 2C (1) | NA18519 |
| GL23237910XH | 60 | 3B (1) | NA20539 |
| GL23793210PT | 56 | 4E (4); 2E (3); 3E (2) | HG00693; HG00734; HG02595; HG02759; HG03796; NA18952; NA19041; NA19204; NA19332 |
| GL47878215RR | 56 | 5E (3); 4D (1) | HG00321; HG00524; HG01182; HG02008 |
| GL75538810GR | 57 | 5E (4); 2E (1) | HG00242; HG02654; HG02655; HG02690; HG03644 |
| GL22262417VG | 59 | 3B (1) | HG01619 |
| GL34222210JX | 55 | 2D (1); 4D (1) | HG00178; NA12750 |
| GL52779612GN | 58 | 1A (2) | HG01348: NA20778 |
| GL22283511TB | 58 | 1A (4) | HG00186; HG01507; NA18982; NA19726 |
| GL28829410AN | 55 | 2A (4); 2B (2); 1B (1); 4B (1) | HG00129; HG01512; HG01626; HG02445; NA18868; NA19235; NA19323; NA19786 |
| GL22924211GU | 57 | 4E (2); 2D (1) | HG01113; HG02239; NA18999 |
| GL52353414QM | 59 | 5C (1) | HG04018 |
| GL92376214QU | 59 | 1A (2); 1B (1) | HG01550; HG02922; NA19658 |
| GL27757712E2 | 59 | 2B (2) | HG00323; HG01066 |
| GL42293515CB | 59 | 5B (2); 5C (1) | HG01431; HG01980; HG04018 |
| GL42337212KT | 59 | 1A (1) | NA20778 |
| GL28929912BE | 55 | 1B (2) | HG00141; NA19795 |
| GL52286311FD | 57 | 2B (1) | NA21093 |
| GL89453512XG | 58 | 2B (1); 3B (1) | HG01111; NA19819 |
| GL22386211UA | 59 | 5E (1) | HG03812 |
| GL22762311NN | 58 | 4D (1) | HG03991 |
| GL29282610TQ | 58 | 2D (2); 2E (1); 3D (1) | HG00651; HG02772; HG03388; NA19734 |
| GL39282510FX | 58 | 5B (1) | HG04047 |
| GL42289916GA | 56 | 3D (1) | HG03136 |
| GL45882413XD | 56 | 2E (1); 3E (1) | HG02332; HG02763 |
| GLS7228616JA | 56 | 2A (2); 2B (1); 4B (1) | HG00150; HG00525; HG02367; HG03756 |
| GL98522917RP | 59 | 2D (8); 4D (5); 3D (3) | HG00632; HG00692; HG00844; HG01060; HG01070; HG01080; HG01198; HG01303; HG01474; HG01865; HG02009; HG02150; HG02485; HG03882; NA12044; NA20753 |
| GL26245818ND | 60 | 2D (1) | NA12340 |
| GL62287410VS | 59 | 1B (1); 2B (1); 3B (1); 4B (1) | HG02445; HG03476; NA19206; NA20356 |
| GL64272211PN | 58 | 2B (2); 1A (1) | HG00179; HG01459; HG02445 |
| GL88253311DH | 58 | 5E (2) | HG02455; HG02783 |
| GL93878310NA | 60 | 3A (1) | NA19719 |
| GL45262410DR | 59 | 1A (1); 2B (1) | HG01187; NA19658 |
| GL92232910UJ | 57 | 3B (1); 4B (1) | HG00146: NA20814 |
| GL36724711RQ | 57 | 1A (3); 2A (1) | HG00186; HG00285; NA12287; NA20530 |
| GL24556513MM | 56 | 5E (1) | HG04107 |
| GL45899311XX | 55 | 2A (2); 2B (1); 4B (1) | HG00513; HG02658; HG04152; NA20510 |
| GL25922211YZ | 60 | 4D (1) | NA18558 |

Die obigen Tabellen 2a und 2b werden im Folgenden an einem Beispiel erläutert:

Der erste Eintrag mit der Proben-ID GS12886 weist nach den obigen Angaben in der Spalte "32p<$10^{-5}$ [tmg]" / Unterspalte "2" 19 Covid-19-relevante SNPs und in der Spalte "381 p<$10^{-5}$ [all]" / Unterspalte "2" 170 Covid-19-relevante SNPs auf. Somit ergibt sich nach den obigen Bewertungskriterien (Scoring) mit dem Wert 19 in der Spalte "32p<$10^{-5}$ [tmg]" / Unterspalte "2" in Spalte "Schweregrad TMG-32" der Score "hoch" und mit dem Wert 170 in der Spalte "Schweregrad - 1000" der Score "hoch". Die verwendeten 19 SNPs lassen sich anhand der Referenz-SNP-Codes (rs#) in den Spalten rs# aus Tabelle 2b ablesen (jeweils mit "2" gekennzeichnet).

Die Erfindung ermöglicht eine personalisierte polygenetische Corona-Risiko-Bewertung auf Basis des folgenden Schemas und der darin angegebenen Formel (es sei an dieser Stelle darauf hingewiesen, dass die Formel aber auch für beliebige Infektionskrankheiten anwendbar ist):

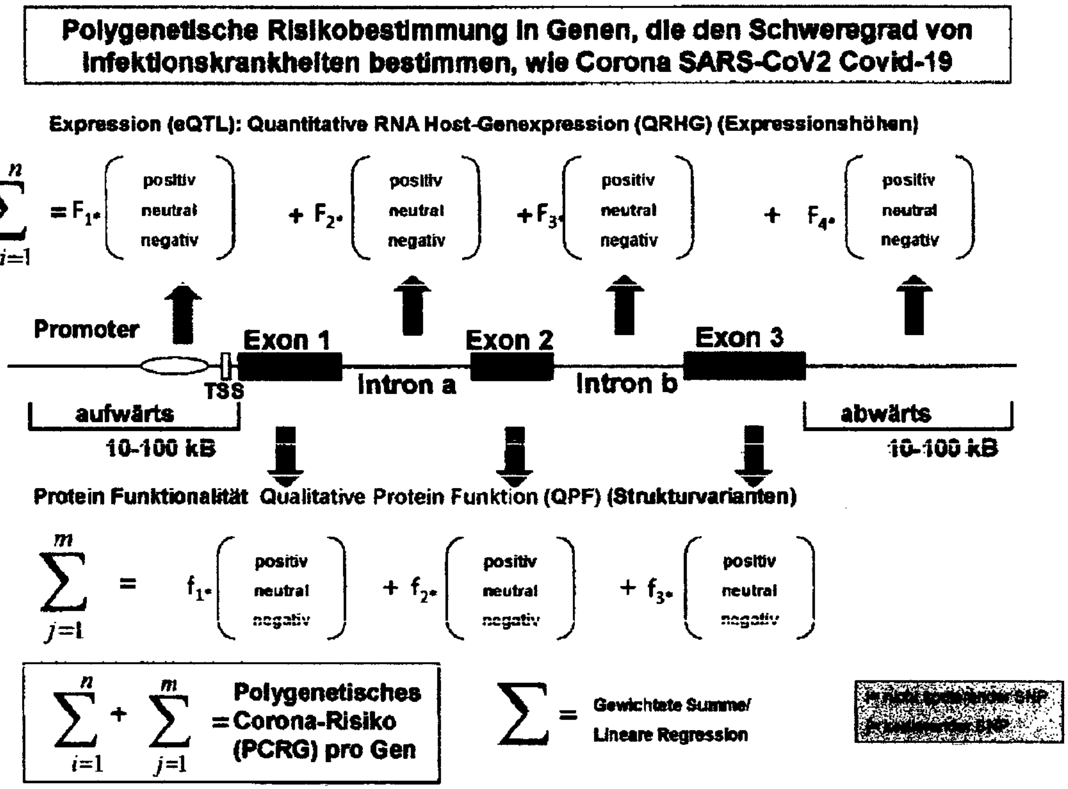


In den Formeln bedeuten:

$\Sigma$ = gewichtete Summe/Lineare Regression
i ... n =Anzahl nicht kodierender SNPs
j ... m = Anzahl kodierender SNPs
$F_1$, $F_2$, $F_3$, ... $F_n$ = individuelle Bewertungsparameter
$f_1$, $f_2$, $f_3$, ... $f_n$ = individuelle Bewertungsparameter
positiv = Anzahl der SNPs, die einen günstigen Effekt (Wert 2) haben
neutral = Anzahl der SNPs, die keinen Effekt (Wert 1) haben
negativ = Anzahl der SNPs, die einen ungünstigen Effekt (Wert 0) haben

Eine individuelle Risikobewertung ist möglich, wenn das Genom eines Probanden vollständig sequenziert wird.

Außerdem sind anamnestische Daten der Person zum Alter, Geschlecht, BMI und zu den Comorbiditäten erforderlich.

Eine Optimierung der Risikobewertung ist möglich, wenn Komplettgenome mit ähnlichen und vergleichbaren Komorbiditäten miteinander verglichen werden, indem diese mit entsprechenden Genomen des "1000 Genome"-Projektes und dessen SNP-Muster verglichen werden.

Neben krankheitsrelevanten SNPs fließt auch die Bestimmung des SNP-Haplotyp-Gegenparts als Haplotyp-Information in die Bewertung ein.

Die Formel erlaubt es, im iterativen Prozess unter Erweiterung der Komplettgenomdatensätze von Covid-Schwer-Erkrankten und von asymptomatisch Corona-infizierten entsprechende Bewertungsfaktoren $F_1$-$F_n$ bzw. $f_1$-$f_n$ individuell zu bestimmen. Gleichzeitig erhalten auch die Pathways der beteiligten Host-Genom-Gene entsprechende Bewertungsfaktoren $P_1$-$P_n$. Die Pathways setzen sich aus unterschiedlichen Genen zusammen. Die aus der Formel gewonnen Einzelgen-SNP-Informationen werden den Pathways über ihre Genbeteiligung zugeordnet. Die Bewertungsfaktoren $P_1$-$P_n$ stratifizieren die Aussagekraft der einzelnen Pathways in der Bestimmung der polygenetischen Risikobewertung.

Die öffentlichen Gen-Datenbanken geben an, an welcher Stelle im Genom die relevanten SNPs vorkommen, so dass genau definiert ist, ob diese SNPs im Bereich eines Genes liegen, bzw. genaufwärts oder genabwärts, bzw. im intergenischen Bereich liegen, bzw. im Exon zu Strukturveränderungen im Protein führen oder die Genexpression der zugehörigen RNA in der Expressionshöhe beeinflussen. Diese Unterteilungen sind in dem obigen Schema graphisch dargestellt. In den Klammern wird dann eingetragen, ob ein SNP einen positiven, einen neutralen oder einen negativen Einfluß auf die Bestimmung des polygenetischen Corona-Risikos pro Gen hat (PCRG). Diese Werte werden für alle Genbereiche der beteiligten Gene, deren SNPs erfindungsgemäß bestimmt werden, berechnet. Diese Werte werden dann den Genen in den bekannten KEGG-Pathways (https://www.genome.jp/kegg/pathway.html) zugerechnet. Wichtig ist, die Kombinationen der SNP-Haplotyp-Varianten (nicht krankheitsverstärkend/protektiv, neutral, krankheitsverstärkend) pro individuelles Genom führen dazu, dass jedes Gen auf das Genom des Covid-Patienten bezogen eine individuelle Bewertung erhält.

Im iterativen Prozess unter Einbindung eigener Komplett-Genom-Analyse-Daten von Covid-Patienten bzw. unter Einbindung der Datensätze des HapMap-Konsortiums (https://www.ncbi.nlm.nih.gov/probe/docs/projhapmap/) und des Host-Genom-Initiative (https://www.covid19hg.org/) werden dann individuelle Bewertungsparameter $F_1$-$F_n$ für die quantitative RNA-Host-Genexpressionsbestimmung (QRHG), bzw. $f_1$-$f_n$ für die Qualitative Protein Funktionsbeschreibung (QPF) der Strukturvarianten in der Berechnung berücksichtigt.

Durch die Lage der relevanten Gene im Verbund mit Nachbargenen unterliegen diese Gene wiederum übergeordneten Regulationseinheiten (Begriff: Super-Enhancer: Diese Elemente steuern u.a. solche Expressionsmuster in Reaktion auf externe Stimuli wie Stress, Infektion, Immunreaktion, Impfung usw.)

Die Genfunktionen werden außerdem in sogenannte Regulationswege (Pathways) zusammengefasst, siehe als Beispiel Abbildung 2 in Hundeshagen et al. Journal of Neuroinflammation 2012, 9:140. Eine Animation unter Einbindung der SNPs des "1000 Genom"-Projektes" ist unter http://indy-med.net/#http://sch197.med.uni-rostock.de/interferon/ visualisiert.

Komplettgenom-Analysen der SNPs einer 108-Jährigen hinsichtlich der assoziierten Corona-Komorbiditäten (die relevanten SNPs sind in Anspruch 5 definiert) haben ergeben, dass die Affektallele, die ein hohes Alter versprechen, in vielen Fällen protektiv gegenüber einer Covid-19 Erkrankung sind, falls deren entgegengesetzte homozygote Varianten als krankheitsbestimmend identifiziert wurden.

Durch zusätzliche Bestimmung der in Anspruch 6 angegebenen mit der Alterung assozierten SNPs läßt sich eine erhebliche Verbesserung der Aussagekraft der personalisierten polygenetischen Corona-Risiko-Bewertung erzielen. Zur Vereinfachung wurde nur in die obige Formel eingegeben, ob es sich um eine homozygotes Affekt-Allel handelt, oder um ein homozygotes protektives Allel, oder um eine heterozygote Konstellation, die hier als neutral (o.k.) bezeichnet wird.

in den folgenden Tabellen bezeichnen:

CEN, DBJ, DCB, DHJ, DHB, FRJ, ATH, MTH Komplettgenome von 8 anonymisierten Patienten. Bei "CEN" handelt sich um das bereits erwähnte Genom einer über 108-Jährigen.

Die SNPs (Polymorphismen), die einen hohen Signifikanzwert für das gesunde Altern versprechen (im Folgenden referenziert mit "Alter"), stammen aus Gierman HJ, Fortney K, Roach JC, Coles NS, Li H, et al. (2014) Whole-Genome Sequencing of the World's Oldest People. PLoS ONE 9(11): e112430. doi:10.1371/journal.pone.0112430.Verwendet wurden SNPs mit einem p-Signifikanz-Wert < 0,01 (T-Test, vom Erfinder durchgeführt). Es handelt sich um folgende SNPs:

| | | | | | |
|---|---|---|---|---|---|
| rs2274789 | rs1801214 | rs10224902 | rs1663564 | rs2591590 | rs6530368 |
| rs228648 | rs1995319 | rs73694648 | rs935241 | rs2591591 | rs1731469 |
| rs56125713 | rs2465570 | rs1800504 | rs935240 | rs2547068 | rs6527818 |
| rs609320 | rs2437323 | rs10250576 | rs278109 | rs2547069 | rs3213451 |
| rs9508 | rs893805 | rs12338 | rs1491893 | rs2547070 | rs5926304 |

(fortgesetzt)

| | | | | | |
|---|---|---|---|---|---|
| rs35672330 | rs11935573 | rs2291219 | rs16919359 | rs2591593 | rs2529541 |
| rs17399810 | rs3810870 | rs16889283 | rs2297224 | rs2547072 | rs2071309 |
| rs1676486 | rs4701997 | rs11779306 | rs2770928 | rs2547073 | rs2071311 |
| rs59123361 | rs1445823 | rs2381409 | rs3751354 | rs953551 | rs2856733 |
| rs749387317 | rs3213936 | rs1138374 | rs2428249 | rs2591594 | rs1800280 |
| rs2266782 | rs7714670 | rs10780682 | rs3742290 | rs2547074 | rs228406 |
| rs11586699 | rs6453022 | rs17062264 | rs34756139 | rs2547075 | rs5917933 |
| rs12120084 | rs34592828 | rs2282683 | rs4883918 | rs2547076 | rs6610447 |
| rs3729547 | rs8654 | rs1057406 | rs147785041 | rs2591595 | rs501128 |
| rs3085 | rs160632 | rs10867826 | rs17852293 | rs2591596 | rs2495783 |
| rs6604561 | rs6874840 | rs10818708 | rs35435463 | rs862461 | rs11545211 |
| rs360057 | rs10479190 | rs35902535 | rs941581 | rs1862462 | rs2516023 |
| rs966365 | rs10055840 | rs35898523 | rs4087943 | rs2591597 | rs5919015 |
| rs3811445 | rs2950844 | rs9886752 | rs1267657 | rs1048290 | rs2296542 |
| rs4148211 | rs2910327 | rs2297145 | rs35270670 | rs1133330 | rs12397326 |
| rs17850223 | rs2910330 | rs2274634 | rs35582838 | rs1054487 | rs12393722 |
| rs11556184 | rs2910005 | rs41284748 | rs8042146 | rs35647251 | rs7884806 |
| rs4129190 | rs3749780 | rs7908745 | rs169068 | rs7249743 | rs7880917 |
| rs6761276 | rs1042719 | rs1227049 | rs4610 | rs36106401 | rs7580 |
| rs61744148 | rs1042720 | rs17109671 | rs7192331 | rs2604901 | rs3012627 |
| rs13422424 | rs1823035 | rs11188225 | rs35583599 | rs3810327 | rs6647476 |
| rs3214503 | rs168726 | rs1048445 | rs117461525 | rs17271272 | rs1113265 |
| rs492594 | rs705441 | rs3740078 | rs12926089 | rs3745521 | rs10126146 |
| rs28630685 | rs416574 | rs11190245 | rs12926669 | rs5030878 | rs363774 |
| rs35813871 | rs8333 | rs2278842 | rs12935737 | rs8113436 | rs1045686 |
| rs918949 | rs2274136 | rs11197835 | rs12923538 | rs12984188 | rs5951328 |
| rs3796028 | rs1042303 | rs2901343 | rs3752426 | rs12984473 | rs111588852 |
| rs1004814 | rs2760118 | rs11813597 | rs2303238 | rs34604090 | rs6622126 |
| rs1364641 | rs4678 | rs484955 | rs8052064 | rs433291 | rs5946294 |
| rs3796031 | rs3131787 | rs6356 | rs3809870 | rs925878 | rs2278954 |
| rs3796032 | rs8084 | rs4758267 | rs2003968 | rs3810340 | rs371749 |
| rs11895915 | rs7192 | rs3213706 | rs2271231 | rs3760849 | rs12390 |
| rs6728818 | rs17882603 | rs61752934 | rs4796555 | rs35914474 | rs5910611 |
| rs1063639 | rs1130034 | rs948847 | rs17853331 | rs145250945 | rs11452643 |
| rs7613447 | rs10093 | rs174535 | rs17856697 | rs12609654 | rs502434 |
| rs2291862 | rs1048372 | rs1800007 | rs7209228 | rs1205194 | rs3747343 |
| rs394558 | rs1049133 | rs2004649 | rs906807 | rs16987712 | rs2498776 |
| rs2245532 | rs1049130 | rs61744384 | rs1484873 | rs6017667 | rs877761 |
| rs62244134 | rs1063323 | rs7116967 | rs56342526 | rs3746493 | rs176037 |

(fortgesetzt)

| | | | | | |
|---|---|---|---|---|---|
| rs2286720 | rs3208181 | rs2848477 | rs56131056 | rs3737137 | rs3765272 |
| rs2276818 | rs3213484 | rs3809018 | rs55659726 | rs434857 | rs237520 |
| rs1044243 | rs1049110 | rs1052313 | rs3752234 | rs2017816 | rs626560 |
| rs9855810 | rs2071551 | rs10891705 | rs3752237 | rs116988298 | rs12116111 |
| rs11708527 | rs762815 | rs2282537 | rs3752246 | rs6586239 | rs5983916 |
| rs3732799 | rs1042187 | rs595986 | rs757292 | rs1980982 | rs561077 |
| rs1381057 | rs1042331 | rs11063529 | rs7250872 | rs1128127 | rs1139916 |
| rs3218649 | rs1042335 | rs7298766 | rs2396359 | rs165734 | rs1432838968 |
| rs9884018 | rs1071597 | rs11612427 | rs3746045 | rs165602 | rs5969919 |
| rs1056523 | rs12528378 | rs35642012 | rs2074789 | rs5997714 | rs6526155 |
| rs4688761 | rs623813 | rs1049112 | rs9973235 | rs56174639 | rs34966902 |
| rs2242353 | rs2297970 | rs1806191 | rs9304915 | rs36125344 | rs1059703 |
| rs9823911 | rs13194610 | rs1805482 | rs778805 | rs55653327 | rs1059702 |
| rs9836841 | rs7767455 | rs10841611 | rs2287915 | rs11705624 | rs2728532 |
| rs1709657 | rs1130656 | rs2720298 | rs2216663 | rs1127000 | rs1126762 |
| rs843334 | rs3823465 | rs2603104 | rs2547064 | rs2306734 | |
| rs11556518 | rs55666134 | rs923829 | rs2252675 | rs11222 | |
| rs2515960 | rs6968741 | rs7955450 | rs1867691 | rs10871864 | |
| rs1024323 | rs3735398 | rs17034524 | rs2121133 | rs41309545 | |

Damit ergibt sich die folgende personalisierte polygenetische Corona-Risiko-Bewertung:

| Alter | CEN | DBJ | DCB | DHJ | DHB | FRJ | ATH | MTH |
|---|---|---|---|---|---|---|---|---|
| Ungünstig 0 | 50 | 58 | 84 | 108 | 84 | 77 | 88 | 98 |
| OK 1 | 146 | 189 | 132 | 85 | 119 | 147 | 119 | 82 |
| Sehr gut 2 | 178 | 127 | 158 | 181 | 171 | 150 | 167 | 194 |

Mit den folgenden in Anspruch 1 angegebenen SNPs (im Folgenden referenziert mit "Patent"):

| | | | | | |
|---|---|---|---|---|---|
| rs306313 | rs505922 | rs12185539 | rs1455664 | rs10747951 | rs12224894 |
| rs12619108 | rs529565 | rs12185540 | rs911360 | rs10506432 | rs7951926 |
| rs6974924 | rs75558547 | rs807610 | rs6930922 | rs11174296 | rs7952569 |
| rs10091098 | rs2080811 | rs34779292 | rs12706520 | rs9738190 | rs7952095 |
| rs4569267 | rs12619874 | rs807607 | rs4506155 | rs11627388 | rs7942564 |
| rs56132597 | rs62120724 | rs807606 | rs4595081 | rs7152677 | rs7948070 |
| rs12610495 | rs2302929 | rs807605 | rs6948273 | rs2059266 | rs7948539 |
| rs12223324 | rs2032778 | rs807604 | rs10156036 | rs7197718 | rs78243302 |
| rs8038527 | rs79808866 | rs807602 | rs6970487 | rs114093749 | rs12814401 |
| rs76187417 | rs41264195 | rs807599 | rs6974918 | rs73606907 | rs9592188 |
| rs17078348 | rs3770462 | rs62122281 | rs6951312 | rs79407403 | rs9598460 |

(fortgesetzt)

| | | | | | |
|---|---|---|---|---|---|
| rs13079478 | rs4668941 | rs807598 | rs2335848 | rs73606932 | rs12904268 |
| rs17214952 | rs2302934 | rs807597 | rs6963124 | rs79289897 | rs2279379 |
| rs9838450 | rs12616567 | rs807596 | rs3965316 | rs7198537 | rs35344266 |
| rs687621 | rs76880770 | rs807595 | rs10276960 | rs79300914 | rs34508855 |
| rs687289 | rs76860840 | rs807594 | rs10273930 | rs60395048 | rs12989715 |
| rs657152 | rs62122279 | rs807592 | rs6949642 | rs16957124 | rs134130 |
| rs644234 | rs4668946 | rs807590 | rs10088118 | rs76668502 | rs9882319 |
| rs544873 | rs12614308 | rs807589 | rs4389947 | rs16957126 | rs35896106 |
| rs545971 | rs807636 | rs807587 | rs10087789 | rs77595609 | rs13071258 |
| rs8176663 | rs807635 | rs807586 | rs13263570 | rs117438562 | rs17763537 |
| rs493246 | rs807628 | rs807585 | rs28730361 | rs13381043 | rs34668658 |
| rs495203 | rs807627 | rs807583 | rs12796811 | rs4797120 | rs17078346 |
| rs582094 | rs807623 | rs807581 | rs10506436 | rs7936322 | rs17763742 |
| rs2769071 | rs807622 | rs807579 | rs10784278 | rs3901233 | rs72893671 |
| rs677355 | rs807620 | rs807578 | rs10784279 | rs7945912 | rs17713054 |
| rs676996 | rs807618 | rs807577 | rs9739956 | rs4963163 | rs13078854 |
| rs550057 | rs807615 | rs807576 | rs10877786 | rs7940065 | rs71325088 |
| rs674302 | rs807613 | rs1455662 | rs11174294 | rs7930569 | rs10490770 |
| rs554833 | rs807611 | rs1455663 | rs10877787 | rs3934992 | rs35624553 |

| | | | | | |
|---|---|---|---|---|---|
| rs67959919 | rs13066516 | rs34442130 | rs60019065 | rs71327014 | rs60328892 |
| rs35508621 | rs77902290 | rs13075758 | rs71327009 | rs71327015 | rs73434199 |
| rs34288077 | rs2171531 | rs17330872 | rs35930050 | rs34127208 | rs73434201 |
| rs35081325 | rs55920693 | rs71327003 | rs34584867 | rs35516580 | rs117184073 |
| rs35731912 | rs6785091 | rs36023124 | rs35161099 | rs34766614 | rs76670680 |
| rs34326463 | rs56332428 | rs71615438 | rs35159820 | rs71327017 | rs17452856 |
| rs76374459 | rs71325095 | rs34754340 | rs71327010 | rs13072267 | rs2440652 |
| rs73064425 | rs35501575 | rs34836513 | rs13090194 | rs36057789 | rs2461037 |
| rs13081482 | rs34381952 | rs34283240 | rs59166269 | rs76647202 | rs643434 |
| rs35652899 | rs4388012 | rs76281521 | rs57437758 | rs71327021 | rs612169 |
| rs35044562 | rs41289616 | rs13433997 | rs34562820 | rs13091868 | rs491626 |
| rs73064431 | rs34000569 | rs67937868 | rs13082697 | rs13092030 | rs492488 |
| rs13092887 | rs34324101 | rs55875328 | rs 13060287 | rs17215981 | rs494242 |
| rs2191031 | rs13069079 | rs67200151 | rs34619093 | rs13089554 | rs582118 |
| rs34901975 | rs76597151 | rs13089855 | rs68087193 | rs13095717 | rs676457 |
| rs17764831 | rs34849862 | rs34493660 | rs2102055 | rs13075528 | rs527210 |
| rs34518147 | rs35827997 | rs3851346 | rs2102056 | rs13095602 | rs660340 |
| rs74586549 | rs35209528 | rs35883205 | rs2088690 | rs71327023 | rs581107 |

(fortgesetzt)

| | | | | | |
|---|---|---|---|---|---|
| rs34338823 | rs36039366 | rs61650989 | rs34774687 | rs13063527 | rs659104 |
| rs71325091 | rs35855315 | rs35669129 | rs35814488 | rs13068570 | rs647800 |
| rs3774641 | rs35525815 | rs13081151 | rs6790866 | rs1491950 | rs473533 |
| rs17764980 | rs17280623 | rs13081213 | rs6791016 | rs4373103 | rs475419 |
| rs17714101 | rs13078739 | rs34168660 | rs6780028 | rs34492478 | rs476410 |
| rs17714228 | rs13059238 | rs35751180 | rs2088692 | rs71327024 | rs645982 |
| rs71325092 | rs13079869 | rs4362758 | rs34438204 | rs1491951 | rs500498 |
| rs35280891 | rs33910087 | rs73833520 | rs13063033 | rs34460587 | rs633862 |
| rs34068335 | rs13071283 | rs71327006 | rs34679077 | rs34452002 | |
| rs1994491 | rs17215008 | rs71327007 | rs34863575 | rs34093271 | |
| rs1994492 | rs71325098 | rs35454877 | rs35772789 | rs13093179 | |
| rs1994493 | rs71325100 | rs35334665 | rs34340587 | rs71327025 | |
| rs75928798 | rs41289622 | rs36040135 | rs77399277 | rs35539222 | |
| rs2373087 | rs71325101 | rs13074382 | rs34718164 | rs35110864 | |
| rs35831747 | rs71325102 | rs13097556 | rs13096741 | rs35581648 | |
| rs13099120 | rs13066062 | rs2230322 | rs35420565 | rs13190738 | |
| rs35477280 | rs36122610 | rs876668 | rs34047915 | rs55839388 | |

ergibt sich die folgende personalisierte polygenetische Corona-Risiko-Bewertung:

| Patent | CEN | DBJ | DCB | DHJ | DHB | FRJ | ATH | MTH |
|---|---|---|---|---|---|---|---|---|
| Sehr gut 0 | 38 | 32 | 39 | 141 | 130 | 125 | 140 | 126 |
| OK 1 | 238 | 246 | 236 | 38 | 75 | 80 | 50 | 81 |
| Ungünstig 2 | 33 | 31 | 34 | 130 | 104 | 104 | 119 | 102 |

Mit den folgenden weiteren in Anspurch 1 angegeben SNPs (im Folgenden referenziert mit "HGI" (Quelle: "Mapping the human genetic architecture of COVID-19 by worldwide meta-analysis", The COVID-19 Host Genetics Initiative, Andrea Ganna doi: https://doi.org/10.1101/2021.03.10.21252820; https://www.covid19hg.org; verwendet wurden die SNPs mit einem p-Wert < $10^{-10}$):

| | | | | | |
|---|---|---|---|---|---|
| rs11385942 | rs9877748 | rs34414382 | rs4766665 | rs1859333 | rs3937434 |
| rs35482426 | rs9845382 | rs34194160 | rs4988618 | rs1981554 | rs2016831 |
| rs7653372 | rs9845542 | rs34745455 | rs7958379 | rs9971885 | rs757405 |
| rs6782814 | rs13069742 | rs71327041 | rs6489866 | rs57484342 | rs2010604 |
| rs71619614 | rs35951367 | rs71327042 | rs6489867 | rs61266553 | rs4767045 |
| rs147246298 | rs35464221 | rs114115904 | rs6489868 | rs7966314 | rs10744791 |
| rs143443576 | rs13085367 | rs34677490 | rs6489869 | rs4767037 | rs12462642 |
| rs35257780 | rs13063635 | rs1542756 | rs6489870 | rs1981557 | rs732631 |
| rs147316571 | rs71327027 | rs1542755 | rs4766666 | rs1981556 | rs2109069 |
| rs71325096 | rs78380307 | rs13060713 | rs4767033 | rs1981555 | rs2109070 |
| rs139901862 | rs4443214 | rs78919248 | rs4767034 | rs3759376 | rs2277732 |

(fortgesetzt)

| | | | | | |
|---|---|---|---|---|---|
| rs34044394 | rs4493469 | rs72856706 | rs10850099 | rs3759375 | rs7255545 |
| rs71619620 | rs17282391 | rs111837807 | rs10850100 | rs4767040 | rs12627039 |
| rs61556067 | rs13083881 | rs112785163 | rs4766667 | rs10774679 | rs17860115 |
| rs2088693 | rs17216717 | rs143334143 | rs4766668 | rs7132797 | rs12482556 |
| rs58552652 | rs13075836 | rs72856713 | rs4766671 | rs1156361 | rs11088247 |
| rs13087776 | rs13096325 | rs10774671 | rs4766672 | rs3815178 | rs2300370 |
| rs13088143 | rs13097340 | rs1131476 | rs4766673 | rs1859330 | rs2248420 |
| rs13068410 | rs3091314 | rs2660 | rs4766674 | rs7299132 | rs2834154 |
| rs13094262 | rs34558763 | rs7135577 | rs4766675 | rs6489879 | rs6517153 |
| rs34805664 | rs35178150 | rs4767024 | rs4766676 | rs4238033 | rs17860142 |
| rs35028116 | rs34693386 | rs4767026 | rs7304898 | rs4767041 | rs9636867 |
| rs34610609 | rs79948053 | rs4767027 | rs7315441 | rs7955267 | rs12482193 |
| rs13086534 | rs34523728 | rs4767029 | rs7305035 | rs7311182 | rs12482060 |
| rs13064632 | rs34570200 | rs4767030 | rs7316586 | rs10735079 | rs17860169 |
| rs34254987 | rs35560301 | rs6489865 | rs916972 | rs6489880 | rs9976829 |
| rs4683162 | rs34134191 | rs10850093 | rs7134391 | rs7980275 | rs13050728 |
| rs4683163 | rs115102354 | rs10850094 | rs7306205 | rs7977345 | rs2834157 |
| rs4683164 | rs13069750 | rs10850095 | rs1859336 | rs6489881 | rs2236756 |
| rs34155121 | rs34340501 | rs10774672 | rs2384071 | rs6489882 | rs2252639 |
| rs4234453 | rs71327038 | rs10850096 | rs2384072 | rs7131998 | rs2252650 |
| rs72901034 | rs36010446 | rs10850097 | rs2384073 | rs7135260 | rs2284549 |
| rs12108042 | rs13069845 | rs10774673 | rs6489874 | rs2269899 | rs2284550 |
| rs34867672 | rs17282797 | rs10774674 | rs6489875 | rs2384074 | rs2284551 |
| rs34924300 | rs13080979 | rs10850098 | rs6489876 | rs10850103 | rs12053666 |
| rs4683166 | rs13098911 | rs10774676 | rs6489877 | rs2285932 | rs2073361 |
| rs7652478 | rs34378541 | rs10774677 | rs6489878 | rs7310667 | rs2834161 |
| rs7642229 | rs17282922 | rs4767031 | rs7298184 | rs10744789 | rs2834163 |
| rs7642320 | rs71327040 | rs4766663 | rs12322160 | rs4238034 | rs2834164 |
| rs9824651 | rs34289272 | rs4766664 | rs7132404 | rs4767044 | rs2834165 |
| rs9825081 | rs34005848 | rs4767032 | rs4767036 | rs1557866 | rs2236757 |

ergibt sich die folgende personalisierte polygenetische Corona-Risiko-Bewertung:

| HGI | CEN | DBJ | DCB | DHJ | DHB | FRJ | ATH | MTH |
|---|---|---|---|---|---|---|---|---|
| Sehr gut 0 | 157 | 56 | 32 | 96 | 97 | 81 | 210 | 211 |
| OK 1 | | 157 | 181 | 117 | 0 | 144 | 4 | 2 |
| Ungünstig 2 | 12 | 12 | 12 | 12 | 128 | 0 | 11 | 12 |

Für die Kombination der SNPs "Patent" + "HGI" ergibt sich die folgende personalisierte polygenetische Corona-Risiko-Bewertung:

| Patent+HGI | CEN | DBJ | DCB | DHJ | DHB | FRJ | ATH | MTH |
|---|---|---|---|---|---|---|---|---|
| Sehr gut 0 | 195 | 88 | 71 | 237 | 227 | 206 | 350 | 337 |
| OK 1 | 294 | 403 | 417 | 155 | 75 | 224 | 54 | 83 |
| Ungünstig 2 | 45 | 43 | 46 | 142 | 232 | 104 | 130 | 114 |

Diese Berechnungen dienen dazu, die krankheitsassoziierten SNPs nach der Erfindung mit assoziierten Corona-Marker im individuellen Genom verknüpfen zu können. Der Vorteil des hier beschriebenen Verfahrens besteht darin, dass jedes individuelle Genom eine gesonderte Bewertung erfährt, siehe die Faktoren $F_1$-$F_n$ und $f_1$-$f_n$, die auf Grundlage des Vorkommens der einzelnen homozygoten SNPs unter 0 als protektiv bzw. unter 2 als ungünstig beschrieben werden. Je nachdem welche SNPs in welchen Proteinen und Genen vorkommen, bzw. wie oft Mehrfachnennungen in den zugeordneten Pathways erfolgen, werden diese Faktoren $F_1$-$F_n$ und $f_1$-$f_n$ iterativ bestimmt.

Werden zusätzlich noch die SNPs "Alter" berücksichtigt, dann ergibt sich folgendes Bild:

| Alter+P+HGI | CEN | DBJ | DCB | DHJ | DHB | FRJ | ATH | MTH |
|---|---|---|---|---|---|---|---|---|
| Sehr gut 0 | 373 | 215 | 229 | 418 | 398 | 356 | 517 | 531 |
| OK 1 | 440 | 592 | 549 | 240 | 194 | 371 | 173 | 165 |
| Ungünstig 2 | 95 | 101 | | 250 | 316 | 181 | 218 | 212 |
| | | | | | | | | |
| rel. Gesamtscore | 278 | 114 | 99 | 168 | 82 | 175 | 299 | 319 |
| | | | | | | | | |

Final ergibt sich dann ein relativer Gesamt-Wert, wenn die Werte "sehr gut 0" von "ungünstig 2" abgezogen werden.

Zur Verdeutlichung der Analyse-Verfahren wurde hier nur berücksichtig, ob ein Affekt-Allel homozygot oder heterozygot auftritt, bzw. wie oft das Nicht-Affektallel bei den Probanden auftritt. Zur Verfeinerung der Analyse können p-Signifikanz-Werte verwendet werden, die mit statischen Verfahren wie dem T-Test aus den vorliegenden SNPs der Komplettgenom-Daten ermittelt werden können.

Falls die SNP Haplotypen für ein hohes Alter sprechen und gleichsinnig die SNP-Haplotypen die Schwere einer Covid-Erkrankung beschreiben, vermindert sich der Signifikanz-Wert als negativ dekadischer Logarithmus für die Schwere der Erkrankung, weil diese Alterungs-SNPs einen protektiven Effekt haben. Im einfachsten Falle ergibt sich ein verminderter Signifikanz-Wert durch Subtraktion beider P-Werte. Sprechen beide Signifikanz-Werte für ein langes Leben und für eine protektive Wirkung, dann erhöht sich der Signifikanzwert als negativ dekadischer Logarithmus. Hier könnte im einfachsten Falle eine Addition der Signifikanz-Werte erfolgen.

Die Signifikanz-Werte können noch mit einem Faktor n größer, gleich oder kleiner 1 unterschiedlich stark bewertet und angepasst werden. Dies gilt insbesondere für die Integration der heterozygoten Werte. Die genbasierten Faktoren $F_1$-$F_n$ bzw. $f_1$-$f_n$ werden während der weiteren bioinformatischen Analyse durch iterative Verfahren optimiert. Gleiches gilt für die Bewertung von Pathways (siehe KEGG-Pathways) mit den Faktoren $P_1$-$P_n$.

Beispiel:

Ein SNP-Haplotyp, der nicht dem Haplotyp für Covid-19-Erkrankungen entspricht, somit auch nicht mit der Schwere einer Covid-19 Erkrankung assoziiert ist, verstärkt sich, wenn gleichzeitig dieser Haplotyp mit seinem SNP-Signifikanzwert für ein langes Leben assoziiert ist. Dies ist ein überraschendes Ergebnis.

Als Beispiel wird das komplette Genom einer 108- Jährigen exemplarisch verwandt, siehe obige Berechnungen. Ihr hohes Alter beruht u.a. darauf, dass ihre SNP-Komorbiditätsgen-Haplotypen größtenteils nicht mit den Krankheitsvarianten der in Anspruch 5 definierten SNPs korrelieren.

Mit großer Signifikanz ergab die im Rahmen der Erfindung durchgeführte Analyse, dass insbesondere die homozygoten SNP-Varianten einen hohen protektiven Beitrag zur Vorhersage einer schweren Covid-19-Erkrankung liefern, wenn die homozygoten Varianten als Minor bzw. Major Allele positiv mit dem Erreichen eines hohen Lebensalters assoziiert sind, bzw. krankheitsassoziierte nicht gleichzeitig als positiv korrelierende Aging/Alterungs-Gen-Varianten bezeichnet werden.

Generell zeigen umfangreichen Analysen unter Einbindung publizierter Daten, dass viele publizierte Daten zu weniger signifikanten P-Werten führen, wenn diese p-Werte mittels GWAS-Studien ermittelt wurden, aber nicht die assoziierten Genvarianten komplettsequenzierter Genome einschließen, die mit dem individuellen Genom des jeweiligen Patienten bzw. Probanden assoziiert sind. Erst die Integration und Kombination individueller SNPs ermöglicht eine individuelle Risiko-Bestimmung.

Die obigen Daten ergeben sich aus folgendem Studien-Design:

1. Zuerst sind die Anamnese der Covid-19-Patienten auf den Intensiv-Stationen inklusive klinischer Parameter und bekannter Komorbiditäten vom Arzt unter Beachtung der Ethikvoten zu erfassen.
2. Mit schriftlicher Einverständnis-Erklärung des Patienten werden anamnestische und klinische Daten erhoben und eine Ganzgenomsequenzierung durchgeführt, ergänzend wird das Verhältnis der Antikörpertiter gegen das Spike- und das nCapsid-Anti-SARS-CoV2 bestimmt.
3. Es werden die SNPs bestimmt, die in den Patentansprüchen definiert sind. Es werden für jeden SNP bestimmt, welcher Haplotyp vorliegt (homozygot Minor oder Major Allel). Es wird die Haplotyp-Verteilung der jeweiligen Volksgruppe (Allelfrequenz) für die weitere bioinformatische Analysen werden Komplettgenome aus dem "1000 Human Genome"-Projekt integriert.
4. Es wird dann ein persönlicher individueller Corona-Risiko-Score ermittelt, wie oben beschrieben. Dieser Score bezieht sich auf die SNPs, die in den Ansprüchen 1-5 definiert sind, erweitert um die in Anspruch 6 definierten zusätzlichen SNPs.
5. Hierzu wird die Anzahl der in den Ansprüchen definierten homozygoten SNPs in den individuellen Genomen ermittelt und entsprechend den anamnestischen Daten vergleichend analysiert.
6. Eine 1. Priorisierung dieser Homozygoten-SNP-Daten erfolgt anfangs über die Anzahl der homozygoten SNPs, die entweder protektiv oder krankheitsfördernd sind und getrennt erfasst werden. Eine 2. Priorisierung erfolgt, indem diese homozygoten SNPs krankheitsassoziiert oder protektiv den einzelnen KEGG-Pathways zu geordnet werden. Hierbei wird unterschieden, ob es sich um strukturelle SNPs handelt, die als nonsynonyme SNPs bezeichnet werden und damit Proteinvarianten mit unterschiedlichen funktionellen und physikochemischen Eigenschaften definieren, oder ob es sich um synonyme oder nicht in der Struktur modifizierte Struktur-Varianten handelt. Handelt es sich um enzymatisch wirksame oder funktionell-aktive Domänen, werden hier die Bewertungsfaktorn "$f_1$-$f_n$" als Verstärkungs- oder Abschwächungsfaktoren iterativ ermittelt und für die finale Ermittlung des individuellen poygenetischen Risikos, an Covid-19 schwer zu erkranken, genutzt.
7. Jeder mit seinen Genen über assoziierte SNPs beteiligte Pathway wird somit über einen theoretisch erreichbaren Faktor $P_1$-$P_n$ beschrieben, der sich aus den einzelnen Signifikanz-Werten in der SNP-Liste bestimmen läßt.
8. Der maximale Pathway-Wert errechnet sich aus der Anzahl der p-Werte der protektiven und der Anzahl der nicht protektiven krankheitsrelevanten p-Werte pro Pathway. Die p-Werte werden miteinander verrechnet wie hier beschrieben, z.B. wie bei der obigen Analyse einer 108-jährigen mit 7 anderen Genomen.

Das folgende Beispiel zeigt die SNP-Risiko-Berechnungen für die 2509 Genome des "1000 Genome"-Projektes. Histogramme und "Heatmaps" dazu sind in den Figuren 7 und 8 wiedergegeben.

| Gender | Atter_ 0 | Alter_ 1 | Alter_ 2 | CoV_ Eli_0 | CoV_ Eli_1 | CoV_ Eli_2 | CoV_ HGI_0 | CoV_ HGI_1 | CoV_ NGI_2 |
|---|---|---|---|---|---|---|---|---|---|
| Max | 167 | 153 | 207 | 169 | 282 | 161 | 206 | 218 | 198 |
| Mean | 122,4 | 95,2 | 156,4 | 120,8 | 85,8 | 102,4 | 72,5 | 67,7 | 84,8 |
| Min | 92 | 51 | 103 | 10 | 8 | 14 | 6 | 4 | 1 |

Bis zum 28.08.2021 gab es 215 Millionen Corona-Infizierte und 4,48 Millionen Covid-19-Tote, somit sterben nach einer Corona-Infektion weltweit ohne Impfung 2,08 % der Infizierten. Allerdings ergeben sich auf der ganzen Welt vergleichbare Verhältnisse, so dass von einer starken epi/-genetischen Beteiligung auszugehen ist. Final sollten insbesondere die Individuen, die einen niedrigen polygenetischen Corona-Risiko-Wert ausweisen, zu einem großen Prozentsatz zu den Schwererkrankten zählen, bzw. versterben, wenn sie über 75 Jahre alt sein sollten.

Die weitere Analyse beschränket sich auf die 503 europäischen Genome des "1000 Genome"-Projek-tes aus Großbritannien (GBR), Finnland (Fin), Spanien (IBS), Toskana in Italien (TSI), Europäer in Utah (CEU). In den Histogrammen wird die Anzahl der in dieser Population vorkommenden SNP-Gruppen mit den Werten an 0, 1 und 2 graphisch dargestellt. Die 2 repräsentiert die Affektallele für das Alter, für die Patent-SNPs und für die Allele des Host-Genome-Initiative.

Diese Histogramme erlauben es nun, dass die SNP-Muster der Covid-Genome in diese Histogramme integriert werden können, so dass unterschieden werden kann, ob die schweren Erkrankungen der Covid-Patienten polygenetische Ursachen haben, dann würden diese in die Kategorien kleine Anzahl an SNPs in Gruppe Alter_0, hohe Werte in CoV-Patent_2 und hohe Werte in CoV_2 fallen. In den anderen Fällen, wenn CoV2-Patienten mit schwerer Erkrankung hohe Werte in der Gruppe Alter_2, bzw. niedrige Werte in CoV2_0 und in HGI_0 haben sollten, dann ist davon auszugehen, dass es sich bei diesen Patienten um Covid-19-Patienten handelt, deren Erkrankung eher bestimmten Proteinvarianten zuzurechnen sind, die auf eine monogenetische Ursache hindeuten.

Die Genome von schwer an Covid-19 erkrankten Patienten werden zusammen mit den Genomen der 503 Kaukasier-Genome analysiert, so dass definiert werden kann, welche Genome diesem Patienten am ähnlichsten sind. Es kann somit entschieden werden, ob es sich bei dem Patienten um einen Patienten handelt, der aufgrund eines monogenetischen Defekts eher erkrankt, bzw. ob eine schwere Covid-Erkrankung durch das Zusammenspiel von vielen SNP-Genvarianten (Strukturproteinen) mit Ihren assoziierten regulatorischen SNP-Elementem verursacht wird.

Weitere unter Anwendung des erfindungsgemäßen Verfahrens gewonnene Ergebnisse sind in den Figuren 1 bis 8 dargestellt.

In den Figuren 1 und 2 bedeuten:

"Principal Component 1": Die Verteilung der Genome auf der x-Achse, die größte Varianz zeigend "Principal Component 2": Die Verteilung der Genome auf der y-Achse mit zweit-größter Varianz Sektoren G01 - G15: Verteilung von 2573 Genomen aufgrund der Principal Component Analyse (PCA) in 15 Sektoren

Die Figuren 1 und 2 zeigen folgendes:

In den Sektoren G01, G03, G07 und G13 zeigen die dunkelgrauen Punkte zu erwartende schwere Covid-19-Krankheitsverläufe und die hellgrauen Punkte asymptomatische Covid-19-Krankheitsverläufe, vgl. Erläuterungen zu Tabelle 2a/2b, Punkte 5 (Wert X in Spalte "381 p<10-5 [all]", Unterspalte "2" $\geq$ 160 = hoher Schweregrad bzw. schwerer Verlauf, Wert X < 100 = asymptomatischer Schweregrad bzw. Verlauf. Die Abbildung zeigt eindeutig, dass sich die Genome aufgrund der hier vorgenommen Klassifikation unterscheiden, so dass sich asymptomatische und schwere Covid-19 Fälle trennen lassen. Mit dem erfindungsgemäßen Verfahren kann nachgewiesen werden, ob genetische Komponenten an der Pathogenese der Virusverbreitung und an der Schwere der Covid-19-Erkrankung beteiligt sind. Hierzu bedarf es der PCA-Analyse in Figur 1 und Figur 2. Demnach können die menschlichen Genome in 15 Gruppen G1-G15 unterteilt werden. Wenn ausschließlich schwererkrankteCovid-19-Patienten getestet werden, und diese würden sich auf die Klassen G1 bis G15 der PCA-Analyse (Figur 1 und Figur 2) entsprechend der Verteilung von Allelfrequenzen in der getesteten Bevölkerung verteilen, dann wüßte man, dass es keine individualgenetischen Komponenten geben wird, die die Schwere einer SARS-CoV2 Infektion herleiten lassen. Dem Fachmann ist klar, dass mit diesem Verfahren sogar generell geprüft werden kann, welche genetischen SNP-Muster für das Auftreten bzw. Nicht-Auftreten von Zivilisationserkrankungen in einer Bevölkerung verantwortlich sind. Das erfindungsgemäße Verfahren kann somit auch generell zur Klärung dienen, ob und welchen Anteil genetische SNP-Kombinationen eines Individual-Genoms an einer Erkrankung bzw. einer Infektion haben (wie hier dargestellt am Beispiel der SARS-Cov2 bedingten Covid-19-Erkrankung).

Figur 3:

Es werden jeweils SNP-Daten von 4 Probanden (32 SNPs) den entsprechenden SNPs der 2504 Genomen zugeordnet und dann entsprechend 4 Beispielsätze gezeigt.

Figur 4:

Zeigt eine "Heatmap" der oben mit "Patent" referenzierten SNPs.

Quelle:

Genomewide Association Study of Severe Covid-19 with Respiratory Failure, D. Ellinghaus et al, N Engl J Med 2020; 383:1522-1534 DOI: 10.1056/NEJMoa2020283
Verwendet wurden SNPs mit einem p-Wert < $10^{-6}$ (siehe Publikation).

Figur 5:

Zeigt eine "Heatmap" der oben mit "HGI" referenzierten SNPs.

Quelle:

Mapping the human genetic architecture of COVID-19 by worldwide meta-analysis

The COVID-19 Host Genetics Initiative, Andrea Ganna
doi: https://doi.org/10.1101/2021.03.10.21252820
https://www.covid19hg.org/

Verwendet wurden SNPs mit einem p-Wert < $10^{-10}$.

Figur 6:

Zeigt eine "Heatmap" der oben mit "Alter" referenzierten SNPs.

Quelle:

Gierman HJ, Fortney K, Roach JC, Coles NS, Li H, et al. (2014) Whole-Genome Sequencing of the World's Oldest People. PLoS ONE 9(11): e112430. doi:10.1371/journal.pone.0112430.
Verwendet wurden SNPs mit einem p-Wert < 0,01 (T-Test, vom Erfinder durchgeführt).
Mit "2" homozygot wird das Affektallei ausgewiesen, mit "0" homozygot die Variante, die keinen bzw. sogar einen negativen Effekt auf ein langes Leben haben könnte. "1" bezeichnet eine heterozygote Konstellation. Bei "CEN" handelt sich um das Genom einer über 108-Jährigen. Die Heatmap visualisiert, welche homozygoten, heterozygoten SNPs von Individuen mit gleichen anamnestischen Daten wie gleiches Geschlecht, BMI, Alter, vergleichbare Komorbiditäten mit der über 108-Jährigen gleichsinnig sind. Es ist bekannt, dass über 100-Jährige eine Covid-19-Erkrankung mit leichter Symptomatik überlebt haben, bzw. es ist auch bekannt, dass etwa 10-15 % der Corona-infizierten der betagteren Personen an Covid-19 erkrankt und verstorben sind.

Figuren 7a-7i:

Histogramme der SNP-Risiko-Berechnungen für 503 europäische Genome des "1000 Genome"-Pro-jektes.

Exemplarisch werden hier ausgewiesen:

Figur 7a:

Alter_0: Die Personen mit hohen SNP Werten sollten für Komorbiditäten anfällig sein und sollten nicht vor allgemeinen Komorbiditäten besonders geschützt sein und damit auch einen geringeren Schutz vor schweren Covid-Erkrankungen haben im Gegensatz zu Personen mit niedrigen SNP-Werten gelistet unter Alter_0.

Figur 7b:

Alter_1: Die Personen mit diesen SNPs der Kategorie 1 sind heterozygot und erhalten eine neutrale Bewertung.

Figur 7c:

Alter_2: Die Personen mit geringen Werten sollten für Komorbiditäten anfällig sein, die Personen mit hohen SNP Werten sollten protektiv gegen allgemeine Komorbiditäten sein und damit auch in bestimmten Fällen einen gewissen Schutz vor schweren Covid-Erkrankungen haben.

Figur 7d:

CoV-Patent_SNP_0: Die Personen mit geringen Werten sollten weniger schwer an Covid-19 erkranken, die Personen mit hohen SNP Werten sollten leicht erkranken können und somit kaum einen Schutz vor schweren Covid-Erkrankungen haben.

Figur 7e:

CoV-Patent_1: Die Personen mit diesen SNPs der Kategorie 1 sind heterozygot und erhalten eine neutrale Bewertung.

Figur 7f:

CoV-Patent_2: Die Personen mit geringen Werten sollten weniger schwer an Covid-19 erkranken, die Personen mit hohen SNP Werten sollten leicht erkranken können und somit kaum einen Schutz vor schweren Covid-Erkrankungen haben.

Figur 7g:

CoV-HGI_0: CoV-Patent_HGI_0: Die Personen mit geringen Werten sollten weniger schwer an Covid-19 erkranken, die Personen mit hohen SNP Werten sollten leicht erkranken können und somit kaum einen Schutz vor schweren Covid-Erkrankungen haben.

Figur 7h:

CoV-HGI_ 1: Die Personen mit diesen SNPs der Kategorie 1 sind heterozygot und erhalten eine neutrale Bewertung.

Figur 7i:

CoV-HGI_2: Die Personen mit hohen Werten sollten weniger schwer an Covid-19 erkranken, die Personen mit niedrigen SNP Werten sollten kaum geschützt sein und somit schwer an Covid-19 erkranken können.

Figur 8a:

Diese Heatmap analysiert das Vorhandensein der Haplotyp-Muster der oben mit "Patent" referenzierten SNPs. Diese Heatmap zeigt oben, welche Genome der 503 Kaukasier sich ähnlich sind, bzw. an der linken Seite welche oben mit "Patent" referenzierten SNPs ähnliche Haplotypen 0, 1, und 2 in den Genomen aufweisen.

Figur 8b:

Diese Heatmap analysiert das Vorhandensein der Haplotyp-Muster der SNPs im HGI-SNP-Datensatz. Diese Heatmap zeigt oben, welche Genome der 503 Kaukasier sich ähnlich sind, bzw. an der linken Seite welche oben mit "Patent" referenzierten SNPs ähnliche Haplotypen 0, 1, und 2 in den Genomen aufweisen.

## Patentansprüche

**1.** Verfahren zur Prognose der Schwere des Verlaufs einer Covid-19-Erkrankung bei einem menschlichen Patienten, wobei man bei dem Verfahren:

das Vorhandensein von Einzelnukleotidpolymorphismen (SNPs), die mit einer Covid-19-Erkrankung assoziiert sind, in einer Patientenprobe bestimmt, wobei die SNPs mit den folgenden Referenzcodes in der *Single Nucleotide Polymorphismen Database (dbSNP)* des *National Center for Biotechnology Information (NCBI)* verwendet werden:

| | | | | | |
|---|---|---|---|---|---|
| rs306313 | rs505922 | rs12185539 | rs1455664 | rs10747951 | rs12224894 |
| rs12619108 | rs529565 | rs12185540 | rs911360 | rs10506432 | rs7951926 |
| rs6974924 | rs75558547 | rs807610 | rs6930922 | rs11174296 | rs7952569 |
| rs10091098 | rs2080811 | rs34779292 | rs12706520 | rs9738190 | rs7952095 |
| rs4569267 | rs12619874 | rs807607 | rs4506155 | rs11627388 | rs7942564 |
| rs56132597 | rs62120724 | rs807606 | rs4595081 | rs7152677 | rs7948070 |
| rs12610495 | rs2302929 | rs807605 | rs6948273 | rs2059266 | rs7948539 |

(fortgesetzt)

| | | | | | |
|---|---|---|---|---|---|
| rs12223324 | rs2032778 | rs807604 | rs10156036 | rs7197718 | rs78243302 |
| rs8038527 | rs79808866 | rs807602 | rs6970487 | rs114093749 | rs12814401 |
| rs76187417 | rs41264195 | rs807599 | rs6974918 | rs73606907 | rs9592188 |
| rs17078348 | rs3770462 | rs62122281 | rs6951312 | rs79407403 | rs9598460 |
| rs13079478 | rs4668941 | rs807598 | rs2335848 | rs73606932 | rs12904268 |
| rs17214952 | rs2302934 | rs807597 | rs6963124 | rs79289897 | rs2279379 |
| rs9838450 | rs12616567 | rs807596 | rs3965316 | rs7198537 | rs35344266 |
| rs687621 | rs76880770 | rs807595 | rs10276960 | rs79300914 | rs34508855 |
| rs687289 | rs76860840 | rs807594 | rs10273930 | rs60395048 | rs12989715 |
| rs657152 | rs62122279 | rs807592 | rs6949642 | rs16957124 | rs134130 |
| rs644234 | rs4668946 | rs807590 | rs10088118 | rs76668502 | rs9882319 |
| rs544873 | rs12614308 | rs807589 | rs4389947 | rs16957126 | rs35896106 |
| rs545971 | rs807636 | rs807587 | rs10087789 | rs77595609 | rs13071258 |
| rs8176663 | rs807635 | rs807586 | rs13263570 | rs117438562 | rs17763537 |
| rs493246 | rs807628 | rs807585 | rs28730361 | rs13381043 | rs34668658 |
| rs495203 | rs807627 | rs807583 | rs12796811 | rs4797120 | rs17078346 |
| rs582094 | rs807623 | rs807581 | rs10506436 | rs7936322 | rs17763742 |
| rs2769071 | rs807622 | rs807579 | rs10784278 | rs3901233 | rs72893671 |
| rs677355 | rs807620 | rs807578 | rs10784279 | rs7945912 | rs17713054 |
| rs676996 | rs807618 | rs807577 | rs9739956 | rs4963163 | rs13078854 |
| rs550057 | rs807615 | rs807576 | rs10877786 | rs7940065 | rs71325088 |
| rs674302 | rs807613 | rs1455662 | rs11174294 | rs7930569 | rs10490770 |
| rs554833 | rs807611 | rs1455663 | rs10877787 | rs3934992 | rs35624553 |

| | | | | | |
|---|---|---|---|---|---|
| rs67959919 | rs13066516 | rs34442130 | rs60019065 | rs71327014 | rs60328892 |
| rs35508621 | rs77902290 | rs13075758 | rs71327009 | rs71327015 | rs73434199 |
| rs34288077 | rs2171531 | rs17330872 | rs35930050 | rs34127208 | rs73434201 |
| rs35081325 | rs55920693 | rs71327003 | rs34584867 | rs35516580 | rs117184073 |
| rs35731912 | rs6785091 | rs36023124 | rs35161099 | rs34766614 | rs76670680 |
| rs34326463 | rs56332428 | rs71615438 | rs35159820 | rs71327017 | rs17452856 |
| es76374459 | rs71325095 | rs3475340 | rs71327010 | rs13072267 | rs2440652 |
| rs73064425 | rs35501575 | rs34836513 | rs13090194 | rs36057789 | rs2461037 |
| rs13081482 | rs34381952 | rs34283240 | rs59166269 | rs76647202 | rs643434 |
| rs35652899 | rs4388012 | rs76281521 | rs57437758 | rs71327021 | rs612169 |
| rs35044562 | rs41289616 | rs13433997 | rs34562820 | rs13091868 | rs491626 |
| rs73064431 | rs34000569 | rs67937868 | rs13082697 | rs13092030 | rs492488 |
| rs13092887 | rs34324101 | rs55875328 | rs13060287 | rs17215981 | rs494242 |
| rs2191031 | rs13069079 | rs67200151 | rs34619093 | rs13089554 | rs582118 |

(fortgesetzt)

| | | | | | |
|---|---|---|---|---|---|
| rs34901975 | rs76597151 | rs13089855 | rs68087193 | rs13095717 | rs676457 |
| rs17764831 | rs34849862 | rs34493660 | rs2102055 | rs13075528 | rs527210 |
| rs34518147 | rs35827997 | rs3851346 | rs2102056 | rs13095602 | rs660340 |
| rs74586549 | rs35209528 | rs35883205 | rs2088690 | rs71327023 | rs581107 |
| rs34338823 | rs36039366 | rs61650989 | rs34774687 | rs13063527 | rs659104 |
| rs71325091 | rs35855315 | rs35669129 | rs35814488 | rs13068570 | rs647800 |
| rs3774641 | rs35525815 | rs13081151 | rs6790866 | rs1491950 | rs473533 |
| rs17764980 | rs17280623 | rs13081213 | rs6791016 | rs4373103 | rs475419 |
| rs17714101 | rs13078739 | rs34168660 | rs6780028 | rs34492478 | rs476410 |
| rs17714228 | rs13059238 | rs35751180 | rs2088692 | rs71327024 | rs645982 |
| rs71325092 | rs13079869 | rs4362758 | rs34438204 | rs1491951 | rs500498 |
| rs35280891 | rs33910087 | rs73833520 | rs13063033 | rs34460587 | rs633862 |
| rs34068335 | rs13071283 | rs71327006 | rs34679077 | rs34452002 | |
| rs1994491 | rs17215008 | rs71327007 | rs34863575 | rs34093271 | |
| rs1994492 | rs71325098 | rs35454877 | rs35772789 | rs13093179 | |
| rs1994493 | rs71325100 | rs35334665 | rs34340587 | rs71327025 | |
| rs75928798 | rs41289622 | rs36040135 | rs77399277 | rs35539222 | |
| rs2373087 | rs71325101 | rs13074382 | rs34718164 | rs35110864 | |
| rs35831747 | rs71325102 | rs13097556 | rs13096741 | rs35581648 | |
| rs13099120 | rs13066062 | rs2230322 | rs35420565 | rs13190738 | |
| rs35477280 | rs36122610 | rs876668 | rs34047915 | rs55839388 | |

| | | | | | |
|---|---|---|---|---|---|
| rs11385942 | rs9877748 | rs34414382 | rs4766665 | rs1859333 | rs3937434 |
| rs35482426 | rs9845382 | rs34194160 | rs4988618 | rs1981554 | rs2016831 |
| rs7653372 | rs9845542 | rs34745455 | rs7958379 | rs9971885 | rs757405 |
| rs6782814 | rs13069742 | rs71327041 | rs6489866 | rs57484342 | rs2010604 |
| rs71619614 | rs35951367 | rs71327042 | rs6489867 | rs61266553 | rs4767045 |
| rs147246298 | rs35464221 | rs114115904 | rs6489868 | rs7966314 | rs10744791 |
| rs143443576 | rs13085367 | rs34677490 | rs6489869 | rs4767037 | rs12462642 |
| rs35257780 | rs13063635 | rs1542756 | rs6489870 | rs1981557 | rs732631 |
| rs147316571 | rs71327027 | rs1542755 | rs4766666 | rs1981556 | rs2109069 |
| rs71325096 | rs78380307 | rs13060713 | rs4767033 | rs1981555 | rs2109070 |
| rs139901862 | rs4443214 | rs78919248 | rs4767034 | rs3759376 | rs2277732 |
| rs34044394 | rs4493469 | rs72856706 | rs10850099 | rs3759375 | rs7255545 |
| rs71619620 | rs17282391 | rs111837807 | rs10850100 | rs4767040 | rs12627039 |
| rs61556067 | rs13083881 | rs112785163 | rs4766667 | rs10774679 | rs17860115 |
| rs2088693 | rs17216717 | rs143334143 | rs4766668 | rs7132797 | rs12482556 |
| rs58552652 | rs13075836 | rs72856713 | rs4766671 | rs1156361 | rs11088247 |

(fortgesetzt)

| | | | | | |
|---|---|---|---|---|---|
| rs13087776 | rs13096325 | rs10774671 | rs4766672 | rs3815178 | rs2300370 |
| rs13088143 | rs13097340 | rs1131476 | rs4766673 | rs1859330 | rs2248420 |
| rs13068410 | rs3091314 | rs2660 | rs4766674 | rs7299132 | rs2834154 |
| rs13094262 | rs34558763 | rs7135577 | rs4766675 | rs6489879 | rs6517153 |
| rs34805664 | rs35178150 | rs4767024 | rs4766676 | rs4238033 | rs17860142 |
| rs35028116 | rs34693386 | rs4767026 | rs7304898 | rs4767041 | rs9636867 |
| rs34610609 | rs79948053 | rs4767027 | rs7315441 | rs7955267 | rs12482193 |
| rs13086534 | rs34523728 | rs4767029 | rs7305035 | rs7311182 | rs12482060 |
| rs13064632 | rs34570200 | rs4767030 | rs7316586 | rs10735079 | rs17860169 |
| rs34254987 | rs35560301 | rs6489865 | rs916972 | rs6489880 | rs9976829 |
| rs4683162 | rs34134191 | rs10850093 | rs7134391 | rs7980275 | rs13050728 |
| rs4683163 | rs115102354 | rs10850094 | rs7306205 | rs7977345 | rs2834157 |
| rs4683164 | rs13069750 | rs10850095 | rs1859336 | rs6489881 | rs2236756 |
| rs34155121 | rs34340501 | rs10774672 | rs2384071 | rs6489882 | rs2252639 |
| rs4234453 | rs71327038 | rs10850096 | rs2384072 | rs7131998 | rs2252650 |
| rs72901034 | rs36010446 | rs10850097 | rs2384073 | rs7135260 | rs2284549 |
| rs12108042 | rs13069845 | rs10774673 | rs6489874 | rs2269899 | rs2284550 |
| rs34867672 | rs17282797 | rs10774674 | rs6489875 | rs2384074 | rs2284551 |
| rs34924300 | rs13080979 | rs10850098 | rs6489876 | rs10850103 | rs12053666 |
| rs4683166 | rs13098911 | rs10774676 | rs6489877 | rs2285932 | rs2073361 |
| rs7652478 | rs34378541 | rs10774677 | rs6489878 | rs7310667 | rs2834161 |
| rs7642229 | rs17282922 | rs4767031 | rs7298184 | rs10744789 | rs2834163 |
| rs7642320 | rs71327040 | rs4766663 | rs12322160 | rs4238034 | rs2834164 |
| rs9824651 | rs34289272 | rs4766664 | rs7132404 | rs4767044 | rs2834165 |
| rs9825081 | rs34005848 | rs4767032 | rs4767036 | rs1557866 | rs2236757 |

die Anzahl der bestimmten vorstehenden SNPs in dem Patienten mit einer Referenzanzahl vergleicht, wobei die Referenzanzahl mit einem bekannten Schweregrad der Covid-19-Erkrankung assoziiert ist, und
wobei das Vorliegen einer Anzahl von SNPs in dem Patienten, die mit einem Schweregrad der Covid-19-Erkrankung assoziiert sind, prognostisch dafür ist, zu welchem Schweregrad sich bei dem Patienten eine Covid-19-Erkrankung entwickelt.

2. Verfahren nach Anspruch 1, wobei die folgenden SNPs bestimmt werden:

| | | | | | |
|---|---|---|---|---|---|
| rs306313 | rs12610495 | rs17214952 | rs544873 | rs2769071 | rs505922 |
| rs12619108 | rs12223324 | rs9838450 | rs545971 | rs677355 | rs529565 |
| rs6974924 | rs8038527 | rs687621 | rs8176663 | rs676996 | |
| rs10091098 | rs7618417 | rs687289 | rs493246 | rs550057 | |
| rs4569267 | rs17078348 | rs657152 | rs495203 | rs674302 | |
| rs56132597 | rs13079478 | rs644234 | rs582094 | rs554833 | |

**3.** Verfahren nach einem der vorstehenden Ansprüche, wobei zusätzlich zu dem SNP rs13079478 folgende SNPs bestimmt werden:

| | | | | | |
|---|---|---|---|---|---|
| rs1994491 | rs77902290 | rs35501575 | rs35525815 | rs71325100 | rs71327003 |
| rs1994493 | rs2171531 | rs13069079 | rs13078739 | rs13066062 | rs36023124 |
| rs35831747 | rs55920693 | rs76597151 | rs13079478 | rs36122610 | |
| rs35477280 | rs56332428 | rs34849862 | rs13079869 | rs34442130 | |
| rs13066516 | rs71325095 | rs36039366 | rs33910087 | rs13075758 | |

und zusätzlich zu dem SNP rs17214952 folgende SNPs bestimmt werden:

| | | | | | |
|---|---|---|---|---|---|
| rs1994492 | rs34381952 | rs35827997 | rs13071283 | rs71325101 | |
| rs75928798 | rs4388012 | rs35209528 | rs17214952 | rs71325102 | |
| rs2373087 | rs41289616 | rs35855315 | rs17215008 | rs17330872 | |
| rs13099120 | rs34000569 | rs17280623 | rs71325098 | | |
| rs6785091 | rs34324101 | rs13059238 | rs41289622 | | |

bei denen es sich um SNPs des Gens FYC01 handelt, die kausale Marker für die Pathogenese und die Schwere einerCovid-19-Erkrankung sind.

**4.** Verfahren nach einem der vorstehenden Ansprüche, wobei zusätzlich mindestens einer der folgenden SNPs, die die Replikationsrate des SARS-CoV2-Virus beeinflussen, bestimmt wird:

| | | | | | |
|---|---|---|---|---|---|
| rs10907185 | rs1061508 | rs11684157 | rs6720483 | rs34037363 | rs17027526 |
| rs3855951 | rs1061508 | rs3099559 | rs12469386 | rs7647812 | rs116034902 |
| rs3131660 | rs12079454 | rs3099559 | rs74453786 | rs11706052 | rs78248692 |
| rs34517448 | rs3736757 | rs3112916 | rs2305138 | rs11716445 | rs6831 |
| rs4233338 | rs67337751 | rs3112916 | rs3738904 | rs80073654 | rs3209157 |
| rs4233338 | rs60400761 | rs116686919 | rs7652331 | rs114691754 | rs6889892 |
| rs4610985 | rs73093505 | rs4666170 | rs7652331 | rs16838298 | rs72647244 |
| rs4610985 | rs76309804 | rs4666170 | rs1545985 | rs114453087 | rs62364494 |
| rs191000784 | rs10495150 | rs34656234 | rs13079478 | rs12510574 | rs28460815 |
| rs273885 | rs6428948 | rs2723088 | rs33910087 | rs77591659 | rs72787387 |
| rs76861341 | rs79779531 | rs2723089 | rs33910087 | rs10015634 | rs2291628 |
| rs67008022 | rs9287719 | rs10195328 | rs4683158 | rs6532203 | rs12523609 |
| rs12116970 | rs6714413 | rs10195328 | rs4683158 | rs6532208 | rs374748 |
| rs12116970 | rs6714413 | rs62140404 | rs4683158 | rs10003496 | rs28763954 |
| rs7521948 | rs72777326 | rs12714145 | rs17214952 | rs7655622 | rs331079 |
| rs6692394 | rs11684157 | rs2346553 | rs6791542 | rs10516428 | rs7701116 |
| rs7724108 | rs12547746 | rs117341758 | rs78925317 | rs10774517 | rs8182064 |
| rs11951084 | rs4738406 | rs74924161 | rs10770437 | rs280590 | rs2703580 |
| rs1549203 | rs73264571 | rs61917258 | rs1514831 | rs57620361 | rs116915857 |
| rs11958425 | rs116990828 | rs61917258 | rs1514831 | rs73168527 | rs76089422 |

(fortgesetzt)

| | | | | | |
|---|---|---|---|---|---|
| rs72800300 | rs72722967 | rs7311276 | rs7133858 | rs9507502 | rs118031436 |
| rs116200464 | rs7865343 | rs7311276 | rs7133858 | rs9507502 | rs8042654 |
| rs116200464 | rs7028704 | rs11061620 | rs10841162 | rs7335635 | rs8033595 |
| rs17061784 | rs16918878 | rs12812965 | rs10841162 | rs117936226 | rs76029207 |
| rs12652763 | rs117351867 | rs2887533 | rs10770446 | rs117936226 | rs76029207 |
| rs72814105 | rs34680486 | rs3924361 | rs7962879 | rs9544446 | rs62021260 |
| rs79931282 | rs10985215 | rs117086722 | rs4497470 | rs11617489 | rs1058260 |
| rs9392453 | rs2296793 | rs73041063 | rs16915248 | rs78768204 | rs1135911 |
| rs17513153 | rs148203698 | rs73041063 | rs11044463 | rs12863903 | rs2277589 |
| rs17169592 | rs148203698 | rs11061660 | rs7965006 | rs12863903 | rs3784471 |
| rs1459558 | rs7077992 | rs73029107 | rs7965006 | rs222670 | rs1982906 |
| rs1649229 | rs6560859 | rs73029190 | rs10841210 | rs222671 | rs12901252 |
| rs328902 | rs6560859 | rs56262248 | rs79072570 | rs568318446 | rs7168655 |
| rs34949146 | rs6560859 | rs73034919 | rs10843330 | rs10137084 | rs215095 |
| rs79787993 | rs10829268 | rs144000701 | rs1496218 | rs10137084 | rs215059 |
| rs117718957 | rs10829268 | rs10848459 | rs10843346 | rs7150942 | rs215059 |
| rs117718957 | rs10829268 | rs73038672 | rs10843346 | rs12717411 | rs215066 |
| rs78906654 | rs11101684 | rs77082078 | rs116789485 | rs12717411 | rs119774 |
| rs2868177 | rs11538725 | rs6489283 | rs1454934 | rs11158820 | rs215069 |
| rs2868180 | rs11538725 | rs6489283 | rs7967264 | rs117146868 | rs6498595 |
| rs7804806 | rs7937799 | rs118186282 | rs11052270 | rs2498827 | rs6498595 |
| rs56295910 | rs2957865 | rs73597943 | rs7979825 | rs2498825 | rs8187843 |
| rs56295910 | rs10897453 | rs111449649 | rs701006 | rs2474030 | rs8187843 |
| rs3757508 | rs10897453 | rs75321406 | rs1633360 | rs77743349 | rs8187843 |
| rs2395833 | rs928948 | rs76653618 | rs41292019 | rs12885234 | rs4780589 |
| rs257376 | rs17825668 | rs12313774 | rs78948244 | rs62011404 | rs4780589 |
| rs16930092 | rs548907 | rs7975502 | rs61068064 | rs78141811 | rs1967120 |
| rs7830335 | rs548907 | rs79833769 | rs77411677 | rs76249401 | rs1967120 |
| rs12547746 | rs117341758 | rs78925317 | rs55707601 | rs8182064 | rs3784862 |
| rs3784862 | rs212079 | rs4782377 | rs75584356 | rs3865452 | rs2839217 |
| rs3784863 | rs2299670 | rs11646212 | rs3931634 | rs17857107 | rs2073382 |
| rs152033 | rs212080 | rs3751675 | rs3931634 | rs17857107 | rs35940413 |
| rs2230669 | rs246141 | rs4782307 | rs3931634 | rs17857107 | rs2839245 |
| rs35599 | rs12932079 | rs2879097 | rs7253253 | rs431579 | rs2839245 |
| rs35621 | rs9935402 | rs9892443 | rs17001450 | rs431579 | rs1044998 |
| rs35626 | rs4782505 | rs7215873 | rs75973396 | rs6115218 | rs35346764 |
| rs11861085 | rs8053676 | rs117859736 | rs2079014 | rs4811837 | rs2839246 |
| rs11861115 | rs8053676 | rs746628 | rs2079014 | rs34500739 | rs7277175 |
| rs3887893 | rs7199004 | rs77584326 | rs3760884 | rs2839217 | rs2839256 |

(fortgesetzt)

| | | | | | |
|---|---|---|---|---|---|
| rs212079 | rs7199004 | rs74917169 | rs7507651 | rs2839217 | rs2839256 |
| rs743346 | rs4646316 | rs4646316 | rs2072798 | rs11704825 | |

**5.** Verfahren nach einem der vorstehenden Ansprüche, wobei zusätzlich mindestens einer der folgenden SNPs, die mit folgenden Erkrankungen assoziiert sind, bestimmt wird, um den Einfluß von krankheitsassoziierten SNPs in die Covid-19-Risikobetrachtung zu integrieren:

| | | | |
|---|---|---|---|
| rs63750847 | Alzheimer Erkrankung | rs28930068 | Hypokalemische Periodische Lähmung |
| rs1801131 | Anaphylaxie nach Impfungen | rs28930069 | Hypokalemische Periodische Lähmung |
| rs1801133 | Anaphylaxie nach Impfungen | rs80338779 | Hypokalemische Periodische Lähmung |
| rs949963 | Anaphylaxie nach Impfungen | rs80338777 | Hypokalemische Periodische Lähmung |
| rs2228139 | Anaphylaxie nach Impfungen | rs121908555 | Hypokalemische Periodische Lähmung |
| rs4848300 | Anaphylaxie nach Impfungen | rs80338788 | Hypokalemische Periodische Lähmung |
| rs17561 | Anaphylaxie nach Impfungen | rs80338784 | Hypokalemische Periodische Lähmung |
| rs1800587 | Anaphylaxie nach Impfungen | rs121918007 | Hypophosphatasie |
| rs1801278 | Antidiabeticum Sulphonylharnstoff | rs121918002 | Hypophosphatasie |
| rs1801282 | Antidiabeticum Sulphonylharnstoff | rs2476601 | Hypothyroidismus |
| rs7903146 | Antidiabeticum Sulphonylharnstoff | rs7850258 | Hypothyroidismus |
| rs12255372 | Antidiabeticum Sulphonylharnstoff | rs3184504 | Hypothyroidismus |
| rs5215 | Antidiabeticum Sulphonylharnstoff | rs2517532 | Hypothyroidismus |
| rs5219 | Antidiabeticum Sulphonylharnstoff | rs12610495 | Idiopathische Lungenfibrose |
| rs757110 | Antidiabeticum Sulphonylharnstoff | rs1278769 | Idiopathische Lungenfibrose |
| rs1799859 | Antidiabeticum Sulphonylharnstoff | rs1980653 | Idiopathische Lungenfibrose |
| rs6196 | Antipsychotika und Gewichtszunahme | rs1981997 | Idiopathische Lungenfibrose |
| rs17782313 | Antipsychotika und Gewichtszunahme | rs2034650 | Idiopathische Lungenfibrose |
| rs489693 | Antipsychotika und Gewichtszunahme | rs6793295 | Idiopathische Lungenfibrose |
| rs1801131 | Antipsychotika und Gewichtszunahme | rs7934606 | Idiopathische Lungenfibrose |
| rs5067 | Asthma | rs2187668 | Idiopathische membranöse Nephroapthie |
| rs689465 | Asthma | rs4664308 | Idiopathische membranöse Nephroapthie |
| rs4950928 | Asthma | rs9275596 | IgA Nephropathie |
| rs1042713 | Asthma | rs363039 | Intelligenz Messung |
| rs1695 | Asthma | rs363050 | Intelligenz Messung |
| rs8069176 | Asthma | rs1511412 | Keloidbildung |
| rs7216389 | Asthma | rs8032158 | Keloidbildung |
| rs13153971 | Asthma | rs873549 | Keloidbildung |
| rs37973 | Asthma Inhalation Corticosteroide | rs940187 | Keloidbildung |
| rs2240017 | Asthma Inhalation Corticosteroide | rs7639618 | Knie Osteoarthrose |
| rs11204971 | Atopische Dermatitis | rs4140564 | Knie Osteoarthrose bei Frauen |
| rs4950928 | Atopisches Asthma | rs5751876 | Koffein Genuss |
| rs1143634 | Belastungsbedingte Muskelschäden | rs6968865 | Koffein Genuss |
| rs28497577 | Belastungsbedingte Muskelschäden | rs2472297 | Koffein Genuss |
| rs1800629 | Belastungsbedingte Muskelschäden | rs6511720 | Koronare Herz-Erkrankung |

(fortgesetzt)

| | | | |
|---|---|---|---|
| rs4880 | Belastungsbedingte Muskelschäden | rs6903956 | Koronare Herz-Erkrankung |
| rs1800795 | Belastungsbedingte Muskelschäden | rs9834312 | Körpergröße |
| rs13266634 | Belastungsbedingte Muskelschäden | rs2179922 | Körpergröße |
| rs1815739 | Belastungsbedingte Muskelschäden | rs1042725 | Körpergröße |
| rs1801282 | Belastungsglukose Test | rs6060369 | Körpergröße |
| rs10936599 | Biologisches Altern | rs8038652 | Körpergröße |
| rs2736100 | Biologisches Altern | rs2802288 | Langlebigkeit |
| rs1042522 | Biologisches Altern | rs2802292 | Langlebigkeit |
| rs1064395 | Bipolare Störung | rs2764264 | Langlebigkeit |
| rs2820037 | Bluthochdruck | rs2811712 | Langlebigkeit |
| rs5186 | Bluthochdruck | rs34516635 | Langlebigkeit |
| rs4961 | Bluthochdruck | rs2073711 | Lumbaler Bandscheibenvorfall |
| rs5335 | Bluthochdruck | rs2736100 | Lungenfibrose |
| rs5370 | Bluthochdruck | rs2076295 | Lungenfibrose |
| rs6997709 | Bluthochdruck | rs13277113 | Lupus (Systemic Lupus Erythematosus) |
| rs7961152 | Bluthochdruck | rs2187668 | Lupus (Systemic Lupus Erythematosus) |
| rs11110912 | Bluthochdruck | rs2205960 | Lupus (Systemic Lupus Erythematosus) |
| rs1937506 | Bluthochdruck | rs9888739 | Lupus (Systemic Lupus Erythematosus) |
| rs2398162 | Bluthochdruck | rs9287638 | Männliche Glatze |
| rs13333226 | Bluthochdruck | rs9479482 | Männliche Glatze |
| rs5065 | Bluthochdrucktherapie | rs2073963 | Männliche Glatze |
| rs13130484 | Body Mass Index | rs6945541 | Männliche Glatze |
| rs6971091 | Body Mass Index | rs10502861 | Männliche Glatze |
| rs2272383 | Body Mass Index | rs8085664 | Männliche Glatze |
| rs1528133 | Body Mass Index | rs2180439 | Männliche Glatze |
| rs1121980 | Body Mass Index | rs6047844 | Männliche Glatze |
| rs10871777 | Body Mass Index | rs1160312 | Männliche Glatze |
| rs12970134 | Body Mass Index | rs1385699 | Männliche Glatze |
| rs2229616 | Body Mass Index | rs2223841 | Männliche Glatze |
| rs2272382 | Body Mass Index | rs6152 | Männliche Glatze |
| rs1892534 | C-reactives Protein | rs1801131 | Metfromin Wirkung |
| rs8034191 | COPD | rs1801133 | Metfromin Wirkung |
| rs7671167 | COPD | rs683369 | Metfromin Wirkung |
| rs2653349 | Cluster Kopfschmerz | rs34059508 | Metfromin Wirkung |
| rs10883365 | Colitis ulcerosa | rs11212617 | Metfromin Wirkung |
| rs2395185 | Colitis ulcerosa | rs1801133 | Methotrexat und Rheumatoide Arthritis |
| rs9858542 | Colitis ulcerosa | rs2651899 | Migräne |
| rs11209026 | Colitis Ulcerosa | rs10761659 | Morbus Crohn |
| rs646776 | Coronare Herzerkrankung | rs11190140 | Morbus Crohn |
| rs599839 | Coronare Herzerkrankung | rs17234657 | Morbus Crohn |
| rs3900940 | Coronare Herzerkrankung | rs1893217 | Morbus Crohn |
| rs7439293 | Coronare Herzerkrankung | rs2066844 | Morbus Crohn |
| rs383830 | Coronare Herzerkrankung | rs2066845 | Morbus Crohn |
| rs6922269 | Coronare Herzerkrankung | rs2066847 | Morbus Crohn |
| rs3798220 | Coronare Herzerkrankung | rs2241880 | Morbus Crohn |
| rs2070744 | Coronare Herzerkrankung | rs3197999 | Morbus Crohn |
| rs4977574 | Coronare Herzerkrankung | rs10513789 | Morbus Parkinson |

(fortgesetzt)

| | | | |
|---|---|---|---|
| rs1333048 | Coronare Herzerkrankung | rs12487066 | Multiple Sclerose |
| rs1333049 | Coronare Herzerkrankung | rs6897932 | Multiple Sclerose |
| rs501120 | Coronare Herzerkrankung | rs3129934 | Multiple Sclerose |
| rs964184 | Coronare Herzerkrankung | rs3135388 | Multiple Sclerose |
| rs2298566 | Coronare Herzerkrankung | rs7326018 | Multiple Sclerose |
| rs3184504 | Coronare Herzerkrankung | rs725613 | Multiple Sclerose |
| rs17228212 | Coronare Herzerkrankung | rs1154155 | Narcolepsie |
| rs8055236 | Coronare Herzerkrankung | rs2305795 | Narcolepsie |
| rs7250581 | Coronare Herzerkrankung | rs16944 | Osteoarthrose der Hüfte |
| rs4420638 | Coronare Herzerkrankung | rs10947262 | Osteoarthrose der Knie |
| rs688034 | Coronare Herzerkrankung | rs3815148 | Osteoarthrose der Knie |
| rs1360780 | Depression | rs11842874 | Osteoarthrose der Knie |
| rs1545843 | Depression | rs143383 | Osteoarthrose der Knie |
| rs1031681 | Depression | rs7776725 | Osteoporose |
| rs4655595 | Diabetes Mellitus, Type 2 | rs3736228 | Osteoporose |
| rs6718526 | Diabetes Mellitus, Type 2 | rs1544410 | Osteoporose |
| rs1801282 | Diabetes Mellitus, Type 2 | rs174230 | Plötzlicher Herztod |
| rs358806 | Diabetes Mellitus, Type 2 | rs1800312 | Pompe Erkankung 1 & 2 |
| rs7659604 | Diabetes Mellitus, Type 2 | rs28940868 | Pompe Erkrankung 1 & 2 |
| rs9465871 | Diabetes Mellitus, Type 2 | rs2856683 | Primäre biliäre Zirrhose |
| rs1799884 | Diabetes Mellitus, Type 2 | rs7530511 | Psoriasis |
| rs13266634 | Diabetes Mellitus, Type 2 | rs11209026 | Psoriasis |
| rs10811661 | Diabetes Mellitus, Type 2 | rs3212227 | Psoriasis |
| rs9326506 | Diabetes Mellitus, Type 2 | rs6887695 | Psoriasis |
| rs4506565 | Diabetes Mellitus, Type 2 | rs10484554 | Psoriasis |
| rs7903146 | Diabetes Mellitus, Type 2 | rs11203366 | Rheumatoide Arthritis |
| rs12255372 | Diabetes Mellitus, Type 2 | rs13031237 | Rheumatoide Arthritis |
| rs5219 | Diabetes Mellitus, Type 2 | rs1569723 | Rheumatoide Arthritis |
| rs9300039 | Diabetes Mellitus, Type 2 | rs2327832 | Rheumatoide Arthritis |
| rs10830963 | Diabetes Mellitus, Type 2 | rs2201841 | Rheumatoide Arthritis |
| rs12304921 | Diabetes Mellitus, Type 2 | rs10889677 | Rheumatoide Arthritis |
| rs1495377 | Diabetes Mellitus, Type 2 | rs11162922 | Rheumatoide Arthritis |
| rs2930291 | Diabetes Mellitus, Type 2 | rs6679677 | Rheumatoide Arthritis |
| rs2903265 | Diabetes Mellitus, Type 2 | rs3738919 | Rheumatoide Arthritis |
| rs8050136 | Diabetes Mellitus, Type 2 | rs7574865 | Rheumatoide Arthritis |
| rs11868035 | Diabetes Mellitus, Type 2 | rs3087243 | Rheumatoide Arthritis |
| rs1889018 | Diabetes Mellitus, Type 2 | rs3816587 | Rheumatoide Arthritis |
| rs2295490 | Diabetes Mellitus, Type 2 | rs2269475 | Rheumatoide Arthritis |
| rs104893919 | Diastophische Dysplasia | rs6910071 | Rheumatoide Arthritis |
| rs104893915 | Diastophische Dysplasia | rs6457617 | Rheumatoide Arthritis |
| rs104893924 | Diastophische Dysplasia | rs10499194 | Rheumatoide Arthritis |
| rs5082 | Diätwirkung | rs6920220 | Rheumatoide Arthritis |
| rs1801282 | Diätwirkung | rs729302 | Rheumatoide Arthritis |
| rs17300539 | Diätwirkung | rs2004640 | Rheumatoide Arthritis |
| rs1800497 | Diätwirkung | rs10488631 | Rheumatoide Arthritis |
| rs662799 | Diätwirkung | rs3761847 | Rheumatoide Arthritis |
| rs16879765 | Dupuytren’sche Erkrankung | rs10818488 | Rheumatoide Arthritis |
| rs16946160 | Erworbenes nephrotisches Syndrom | rs2104286 | Rheumatoide Arthritis |
| rs12713559 | Familiäre Hypercholesterolämie Type B | rs9550642 | Rheumatoide Arthritis |

(fortgesetzt)

| | | | |
|---|---|---|---|
| rs5742904 | Familiäre Hypercholesterolämie Type B | rs2837960 | Rheumatoide Arthritis |
| rs144467873 | Familiäre Hypercholesterolämie Type B | rs1953126 | Rheumatoide Arthritis |
| rs28933405 | Familiäre Hypertroph. Kardiomyopathie | rs6684865 | Rheumatoide Arthritis |
| rs28938173 | Familiäre Hypertroph. Kardiomyopathie | rs3890745 | Rheumatoide Arthritis |
| rs104894369 | Familiäre Hypertroph. Kardiomyopathie | rs1049550 | Sarcoidose |
| rs104894370 | Familiäre Hypertroph. Kardiomyopathie | rs12425791 | Schalganfall |
| rs3218714 | Familiäre Hypertroph. Kardiomyopathie | rs10830962 | Schangerschaftsdiabetes (GDM) |
| rs3218713 | Familiäre Hypertroph. Kardiomyopathie | rs4402960 | Schwangerschaftsdiabetis |
| rs104894503 | Familiäre Hypertroph. Kardiomyopathie | rs1799884 | Schwangerschaftsdiabetis |
| rs104894502 | Familiäre Hypertroph. Kardiomyopathie | rs7903146 | Schwangerschaftsdiabetis |
| rs1801282 | Fettgewebebildung | rs12255372 | Schwangerschaftsdiabetis |
| rs4788102 | Fettsucht | rs10830963 | Schwangerschaftsdiabetis |
| rs6548238 | Fettsucht | rs2187668 | Selective IgA Mangel |
| rs7647305 | Fettsucht | rs1990760 | Selective IgA Mangel |
| rs925946 | Fettsucht | rs9271366 | Selective IgA Mangel |
| rs5082 | Fettsucht | rs1004819 | Spondylitis ankylosans |
| rs17300539 | Fettsucht | rs10489629 | Spondylitis ankylosans |
| rs35859249 | Fettsucht | rs2201841 | Spondylitis ankylosans |
| rs6232 | Fettsucht | rs11465804 | Spondylitis ankylosans |
| rs6971091 | Fettsucht | rs11209026 | Spondylitis ankylosans |
| rs662799 | Fettsucht | rs1343151 | Spondylitis ankylosans |
| rs17817449 | Fettsucht | rs10889677 | Spondylitis ankylosans |
| rs17782313 | Fettsucht | rs11209032 | Spondylitis ankylosans |
| rs2229616 | Fettsucht | rs10865331 | Spondylitis ankylosans |
| rs1799884 | Geburtsgewicht | rs17482078 | Spondylitis ankylosans |
| rs7903146 | Geburtsgewicht | rs10050860 | Spondylitis ankylosans |
| rs1051730 | Geburtsgewicht | rs2287987 | Spondylitis ankylosans |
| rs12565727 | Glatze | rs2303138 | Spondylitis ankylosans |
| rs76723693 | Glucose-6-phosphate-dehydrogenase-mangel | rs1800972 | Tendinopathien obere Extremität |
| rs137852318 | Glucose-6-phosphate-dehydrogenase-mangel | rs1138545 | Tendinopathien obere Extremität |
| rs5030868 | Glucose-6-phosphate-dehydrogenase-mangel | rs1800012 | Tendinopathien obere Extremität |
| rs1050828 | Glucose-6-phosphate-dehydrogenase-mangel | rs2104772 | Tendinopathien untere Extremität |
| rs80356491 | Glycogen Speicher Krankheit 1B | rs12722 | Tendinopathien untere Extremität |
| rs80356490 | Glycogen Speicher Krankheit 1B | rs4747096 | Tendinopathien untere Extremität |
| rs80356489 | Glycogen Speicher Krankheit 1B | rs679620 | Tendinopathien untere Extremität |
| rs113994129 | Glycogen Speicher Krankheit Typ 3 | rs1800012 | Tendinopathien untere Extremität |
| rs113994130 | Glycogen Speicher Krankheit Typ 3 | rs4789932 | Tendinopathien untere Extremität |
| rs113994131 | Glycogen Speicher Krankheit Typ 3 | rs143383 | Tendinopathien untere Extremität |

(fortgesetzt)

| | | | |
|---|---|---|---|
| rs119103251 | Glycogen Speicherkrankheit Typ 5 | rs6025 | Tiefe Venenthrombose |
| rs116987552 | Glycogen Speicherkrankheit Typ 5 | rs5361 | Tiefe Venenthrombose |
| rs2155219 | Graspollen-Allergie und Heufieber | rs268 | Tiefe Venenthrombose |
| rs646776 | Herzinfarkt | rs1893217 | Typ 1 Diabetes |
| rs 17465637 | Herzinfarkt | rs 1990760 | Typ 1 Diabetes |
| rs8089 | Herzinfarkt | rs3741208 | Typ 1 Diabetes |
| rs1333040 | Herzinfarkt | rs9273363 | Typ 1 Diabetes |
| rs2383206 | Herzinfarkt | rs10012946 | Typ 2 Diabetes |
| rs2383207 | Herzinfarkt | rs 1111875 | Typ 2 Diabetes |
| rs 10757278 | Herzinfarkt | rs 1387153 | Typ 2 Diabetes |
| rs1927911 | Herzinfarkt | rs2237892 | Typ 2 Diabetes |
| rs501120 | Herzinfarkt | rs2383208 | Typ 2 Diabetes |
| rs662799 | Herzinfarkt | rs4712523 | Typ 2 Diabetes |
| rs 17228212 | Herzinfarkt | i3002432 | Venöse Thromboembolie |
| rs17576 | Herzinfarkt | rs2282679 | Vitamin D Spiegel |
| rs3782886 | Herzinfarkt | rs10741657 | Vitamin D Spiegel |
| rs7291467 | Herzinfarkt | rs1801175 | Von Gierke Erkrankung |
| rs5742905 | Homocystinurie, Pyridoxin abhängig | rs1801176 | Von Gierke Erkrankung |
| rs12413409 | Hypertension | rs80356484 | Von Gierke Erkrankung |
| rs1378942 | Hypertension | rs80356487 | Von Gierke Erkrankung |
| rs 16998073 | Hypertension | rs80356479 | Von Gierke Erkrankung |
| rs17367504 | Hypertension | rs2200733 | Vorhof Flimmern |
| rs3754777 | Hypertension | rs10033464 | Vorhof Flimmern |
| rs964184 | Hypertriglyceridemie | rs 1801253 | Wirkung von β-Blockern |

**6.** Verfahren nach einem der vorstehenden Ansprüche, wobei zusätzlich die folgenden SNPs, die mit gesundem Altern assoziiert sind, bestimmt werden:

| | | | | | |
|---|---|---|---|---|---|
| rs2274789 | rs1801214 | rs10224902 | rs1663564 | rs2591590 | rs6530368 |
| rs228648 | rs1995319 | rs73694648 | rs935241 | rs2591591 | rs1731469 |
| rs56125713 | rs2465570 | rs1800504 | rs935240 | rs2547068 | rs6527818 |
| rs609320 | rs2437323 | rs10250576 | rs278109 | rs2547069 | rs3213451 |
| rs9508 | rs893805 | rs12338 | rs1491893 | rs2547070 | rs5926304 |
| rs35672330 | rs11935573 | rs2291219 | rs16919359 | rs2591593 | rs2529541 |
| rs17399810 | rs3810870 | rs16889283 | rs2297224 | rs2547072 | rs2071309 |
| rs1676486 | rs4701997 | rs11779306 | rs2770928 | rs2547073 | rs2071311 |
| rs59123361 | rs1445823 | rs2381409 | rs3751354 | rs953551 | rs2856733 |
| rs749387317 | rs3213936 | rs1138374 | rs2428249 | rs2591594 | rs1800280 |
| rs2266782 | rs7714670 | rs10780682 | rs3742290 | rs2547074 | rs228406 |
| rs11586699 | rs6453022 | rs17062264 | rs34756139 | rs2547075 | rs5917933 |
| rs12120084 | rs34592828 | rs2282683 | rs4883918 | rs2547076 | rs6610447 |
| rs3729547 | rs8654 | rs1057406 | rs147785041 | rs2591595 | rs501128 |
| rs3085 | rs160632 | rs10867826 | rs17852293 | rs2591596 | rs2495783 |
| rs6604561 | rs6874840 | rs10818708 | rs35435463 | rs1862461 | rs11545211 |
| rs360057 | rs10479190 | rs35902535 | rs941581 | rs1862462 | rs2516023 |

(fortgesetzt)

| | | | | | |
|---|---|---|---|---|---|
| rs966365 | rs10055840 | rs35898523 | rs4087943 | rs2591597 | rs5919015 |
| rs3811445 | rs2950844 | rs9886752 | rs1267657 | rs1048290 | rs2296542 |
| rs4148211 | rs2910327 | rs2297145 | rs35270670 | rs1133330 | rs12397326 |
| rs17850223 | rs2910330 | rs2274634 | rs35582838 | rs1054487 | rs12393722 |
| rs11556184 | rs2910005 | rs41284748 | rs8042146 | rs35647251 | rs7884806 |
| rs4129190 | rs3749780 | rs7908745 | rs169068 | rs7249743 | rs7880917 |
| rs6761276 | rs1042719 | rs1227049 | rs4610 | rs36106401 | rs7580 |
| rs61744148 | rs1042720 | rs17109671 | rs7192331 | rs2604901 | rs3012627 |
| rs13422424 | rs1823035 | rs11188225 | rs35583599 | rs3810327 | rs6647476 |
| rs3214503 | rs168726 | rs1048445 | rs117461525 | rs17271272 | rs1113265 |
| rs492594 | rs705441 | rs3740078 | rs12926089 | rs3745521 | rs10126146 |
| rs28630685 | rs416574 | rs11190245 | rs12926669 | rs5030878 | rs363774 |
| rs35813871 | rs8333 | rs2278842 | rs12935737 | rs8113436 | rs1045686 |
| rs918949 | rs2274136 | rs11197835 | rs12923538 | rs12984188 | rs5951328 |
| rs3796028 | rs1042303 | rs2901343 | rs3752426 | rs12984473 | rs111588852 |
| rs1004814 | rs2760118 | rs11813597 | rs2303238 | rs34604090 | rs6622126 |
| rs1364641 | rs4678 | rs484955 | rs8052064 | rs433291 | rs5946294 |
| rs3796031 | rs3131787 | rs6356 | rs3809870 | rs925878 | rs2278954 |
| rs3796032 | rs8084 | rs4758267 | rs2003968 | rs3810340 | rs371749 |
| rs11895915 | rs7192 | rs3213706 | rs2271231 | rs3760849 | rs12390 |
| rs6728818 | rs17882603 | rs61752934 | rs4796555 | rs35914474 | rs5910611 |
| rs1063639 | rs1130034 | rs948847 | rs17853331 | rs145250945 | rs11452643 |
| rs7613447 | rs10093 | rs174535 | rs17856697 | rs12609654 | rs502434 |
| rs2291862 | rs1048372 | rs1800007 | rs7209228 | rs1205194 | rs3747343 |
| rs394558 | rs1049133 | rs2004649 | rs906807 | rs16987712 | rs2498776 |
| rs2245532 | rs1049130 | rs61744384 | rs1484873 | rs6017667 | rs877761 |
| rs62244134 | rs1063323 | rs7116967 | rs56342526 | rs3746493 | rs176037 |
| rs2286720 | rs3208181 | rs2848477 | rs56131056 | rs3737137 | rs3765272 |
| rs2276818 | rs3213484 | rs3809018 | rs55659726 | rs434857 | rs237520 |
| rs1044243 | rs1049110 | rs1052313 | rs3752234 | rs2017816 | rs626560 |
| rs9855810 | rs2071551 | rs10891705 | rs3752237 | rs116988298 | rs12116111 |
| rs11708527 | rs762815 | rs2282537 | rs3752246 | rs6586239 | rs5983916 |
| rs3732799 | rs1042187 | rs595986 | rs757292 | rs1980982 | rs561077 |
| rs1381057 | rs1042331 | rs11063529 | rs7250872 | rs1128127 | rs1139916 |
| rs3218649 | rs1042335 | rs7298766 | rs2396359 | rs165734 | rs1432838968 |
| rs9884018 | rs1071597 | rs11612427 | rs3746045 | rs165602 | rs5969919 |
| rs1056523 | rs12528378 | rs35642012 | rs2074789 | rs5997714 | rs6526155 |
| rs4688761 | rs623813 | rs1049112 | rs9973235 | rs56174639 | rs34966902 |
| rs2242353 | rs2297970 | rs1806191 | rs9304915 | rs36125344 | rs1059703 |

(fortgesetzt)

| | | | | | |
|---|---|---|---|---|---|
| rs9823911 | rs1319410 | rs1805482 | rs778805 | rs55653327 | rs1059702 rs |
| rs9836841 | rs7767455 | rs10841611 | rs2287915 | rs11705624 | rs2728532 |
| rs1709657 | rs1130656 | rs2720298 | rs2216663 | rs1127000 | rs1126762 |
| rs843334 | rs3823465 | rs2603104 | rs2547064 | rs2306734 | |
| rs11556518 | rs55666134 | rs923829 | rs2252675 | rs11222 | |
| rs2515960 | rs6968741 | rs7955450 | rs1867691 | rs10871864 | |
| rs1024323 | rs3735398 | rs17034524 | rs2121133 | rs41309545 | |

**7.** Verfahren nach einem der vorstehenden Ansprüche, wobei zur Prognose der Schwere des Verlaufs einer Covid-19-Erkrankung bei einem menschlichen Patienten

a) die quantitaive RNA-Host-Genexpression nach folgender Formel bestimmt wird:

$$\sum_{i=1}^{n} = F_{1*}\begin{pmatrix}\text{positiv}\\ \text{neutral}\\ \text{negativ}\end{pmatrix} + F_{2*}\begin{pmatrix}\text{positiv}\\ \text{neutral}\\ \text{negativ}\end{pmatrix} + F_{3*}\begin{pmatrix}\text{positiv}\\ \text{neutral}\\ \text{negativ}\end{pmatrix} + F_{4*}\begin{pmatrix}\text{positiv}\\ \text{neutral}\\ \text{negativ}\end{pmatrix}$$

b) die qualitative Protein-Funktionalität nach folgender Formel bestimmt wird:

$$\sum_{j=1}^{m} = f_{1*}\begin{pmatrix}\text{positiv}\\ \text{neutral}\\ \text{negativ}\end{pmatrix} + f_{2*}\begin{pmatrix}\text{positiv}\\ \text{neutral}\\ \text{negativ}\end{pmatrix} + f_{3*}\begin{pmatrix}\text{positiv}\\ \text{neutral}\\ \text{negativ}\end{pmatrix}$$

womit sich das polygenetische Corona-Risiko pro Gen ergibt:

$$\boxed{\sum_{i=1}^{n} + \sum_{j=1}^{m} = \textbf{Polygenetisches Corona-Risiko (PCRG) pro Gen}}$$

wobei in den Formeln bedeuten:

Σ = gewichtete Summe/Lineare Regression
i ... n = Anzahl nicht kodierender SNPs
j ... m = Anzahl kodierender SNPs
$F_1$, $F_2$, $F_3$, ... $F_n$ = individuelle Bewertungsparameter
$f_1$, $f_2$, $f_3$, ... $f_n$ = individuelle Bewertungsparameter
positiv = Anzahl der SNPs, die einen günstigen Effekt (Wert 2) haben
neutral = Anzahl der SNPs, die keinen Effekt (Wert 1) haben
negativ = Anzahl der SNPs, die einen ungünstigen Effekt (Wert 0) haben

**8.** Verfahren nach einem der vorstehenden Ansprüche, wobei differenziert wird, ob die Covid-19-Erkrankung durch einen monogenetischen Defekt oder durch einen polygenetischen Defekt durch das Zusammenspiel von vielen SNP-Varianten (Strukturproteinen) und regulatorischen SNP-Elementen hervorgerufen wird.

## Claims

1. Method for predicting the severity of the progression of a Covid-19 infection in a human patient, the method comprising

determining the presence of single nucleotide polymorphisms (SNPs) associated with Covid-19 infection in a patient sample using the SNPs with the following reference codes in the *National Center for Biotechnology Information (NCBI) Single Nucleotide Polymorphisms Database (dbSNP):*

| | | | | | |
|---|---|---|---|---|---|
| rs306313 | rs505922 | rs12185539 | rs1455664 | rs10747951 | rs12224894 |
| rs12619108 | rs529565 | rs12185540 | rs911360 | rs10506432 | rs7951926 |
| rs6974924 | rs75558547 | rs807610 | rs6930922 | rs11174296 | rs7952569 |
| rs10091098 | rs2080811 | rs34779292 | rs12706520 | rs9738190 | rs7952095 |
| rs4569267 | rs12619874 | rs807607 | rs4506155 | rs11627388 | rs7942564 |
| rs56132597 | rs62120724 | rs807606 | rs4595081 | rs7152677 | rs7948070 |
| rs12610495 | rs2302929 | rs807605 | rs6948273 | rs2059266 | rs7948539 |
| rs12223324 | rs2032778 | rs807604 | rs10156036 | rs7197718 | rs78243302 |
| rs8038527 | rs79808866 | rs807602 | rs6970487 | rs114093749 | rs12814401 |
| rs76187417 | rs41264195 | rs807599 | rs6974918 | rs73606907 | rs9592188 |
| rs17078348 | rs3770462 | rs62122281 | rs6951312 | rs79407403 | rs9598460 |
| rs13079478 | rs4668941 | rs807598 | rs2335848 | rs73606932 | rs12904268 |
| rs17214952 | rs2302934 | rs807597 | rs6963124 | rs79289897 | rs2279379 |
| rs9838450 | rs12616567 | rs807596 | rs3965316 | rs7198537 | rs35344266 |
| rs687621 | rs76880770 | rs807595 | rs10276960 | rs79300914 | rs34508855 |
| rs687289 | rs76860840 | rs807594 | rs10273930 | rs60395048 | rs12989715 |
| rs657152 | rs62122279 | rs807592 | rs6949642 | rs16957124 | rs134130 |
| rs644234 | rs4668946 | rs807590 | rs10088118 | rs76668502 | rs9882319 |
| rs544873 | rs12614308 | rs807589 | rs4389947 | rs16957126 | rs35896106 |
| rs545971 | rs807636 | rs807587 | rs10087789 | rs77595609 | rs13071258 |
| rs8176663 | rs807635 | rs807586 | rs13263570 | rs117438562 | rs17763537 |
| rs493246 | rs807628 | rs807585 | rs28730361 | rs13381043 | rs34668658 |
| rs495203 | rs807627 | rs807583 | rs12796811 | rs4797120 | rs17078346 |
| rs582094 | rs807623 | rs807581 | rs10506436 | rs7936322 | rs17763742 |
| rs2769071 | rs807622 | rs807579 | rs10784278 | rs3901233 | rs72893671 |
| rs677355 | rs807620 | rs807578 | rs10784279 | rs7945912 | rs17713054 |
| rs676996 | rs807618 | rs807577 | rs9739956 | rs4963163 | rs13078854 |
| rs550057 | rs807615 | rs807576 | rs10877786 | rs7940065 | rs71325088 |
| rs674302 | rs807613 | rs1455662 | rs11174294 | rs7930569 | rs10490770 |
| rs554833 | rs807611 | rs 1455663 | rs10877787 | rs3934992 | rs35624553 |
| rs67959919 | rs13066516 | rs34442130 | rs60019065 | rs71327014 | rs60328892 |
| rs35508621 | rs77902290 | rs13075758 | rs71327009 | rs71327015 | rs73434199 |
| rs34288077 | rs2171531 | rs17330872 | rs35930050 | rs34127208 | rs73434201 |

(continued)

| | | | | | |
|---|---|---|---|---|---|
| rs35081325 | rs55920693 | rs71327003 | rs34584867 | rs35516580 | rs117184073 |
| rs35731912 | rs6785091 | rs36023124 | rs35161099 | rs34766614 | rs76670680 |
| rs34326463 | rs56332428 | rs71615438 | rs35159820 | rs71327017 | rs17452856 |
| rs76374459 | rs71325095 | rs34754340 | rs71327010 | rs13072267 | rs2440652 |
| rs73064425 | rs35501575 | rs34836513 | rs13090194 | rs36057789 | rs2461037 |
| rs13081482 | rs34381952 | rs34283240 | rs59166269 | rs76647202 | rs643434 |
| rs35652899 | rs4388012 | rs76281521 | rs57437758 | rs71327021 | rs612169 |
| rs35044562 | rs41289616 | rs 13433997 | rs34562820 | rs13091868 | rs491626 |
| rs73064431 | rs34000569 | rs67937868 | rs13082697 | rs13092030 | rs492488 |
| rs13092887 | rs34324101 | rs55875328 | rs13060287 | rs17215981 | rs494242 |
| rs2191031 | rs13069079 | rs67200151 | rs34619093 | rs13089554 | rs582118 |
| rs34901975 | rs76597151 | rs13089855 | rs68087193 | rs13095717 | rs676457 |
| rs17764831 | rs34849862 | rs34493660 | rs2102055 | rs13075528 | rs527210 |
| rs34518147 | rs35827997 | rs3851346 | rs2102056 | rs13095602 | rs660340 |
| rs74586549 | rs35209528 | rs35883205 | rs2088690 | rs71327023 | rs581107 |
| rs34338823 | rs36039366 | rs61650989 | rs34774687 | rs13063527 | rs659104 |
| rs71325091 | rs35855315 | rs35669129 | rs35814488 | rs13068570 | rs647800 |
| rs3774641 | rs35525815 | rs13081151 | rs6790866 | rs1491950 | rs473533 |
| rs17764980 | rs17280623 | rs13081213 | rs6791016 | rs4373103 | rs475419 |
| rs17714101 | rs13078739 | rs34168660 | rs6780028 | rs34492478 | rs476410 |
| rs17714228 | rs13059238 | rs35751180 | rs2088692 | rs71327024 | rs645982 |
| rs71325092 | rs13079869 | rs4362758 | rs34438204 | rs1491951 | rs500498 |
| rs35280891 | rs33910087 | rs73833520 | rs13063033 | rs34460587 | rs633862 |
| rs34068335 | rs13071283 | rs71327006 | rs34679077 | rs34452002 | |
| rs1994491 | rs17215008 | rs71327007 | rs34863575 | rs34093271 | |
| rs1994492 | rs71325098 | rs35454877 | rs35772789 | rs13093179 | |
| rs1994493 | rs71325100 | rs35334665 | rs34340587 | rs71327025 | |
| rs75928798 | rs41289622 | rs36040135 | rs77399277 | rs35539222 | |
| rs2373087 | rs71325101 | rs 13074382 | rs34718164 | rs35110864 | |
| rs35831747 | rs71325102 | rs13097556 | rs13096741 | rs35581648 | |
| rs13099120 | rs13066062 | rs2230322 | rs35420565 | rs13190738 | |
| rs35477280 | rs36122610 | rs876668 | rs34047915 | rs55839388 | |

| | | | | | |
|---|---|---|---|---|---|
| rs11385942 | rs9877748 | rs34414382 | rs4766665 | rs1859333 | rs3937434 |
| rs35482426 | rs9845382 | rs34194160 | rs4988618 | rs1981554 | rs2016831 |
| rs7653372 | rs9845542 | rs34745455 | rs7958379 | rs9971885 | rs757405 |
| rs6782814 | rs13069742 | rs71327041 | rs6489866 | rs57484342 | rs2010604 |
| rs71619614 | rs35951367 | rs71327042 | rs6489867 | rs61266553 | rs4767045 |

(continued)

| | | | | | |
|---|---|---|---|---|---|
| rs147246298 | rs35464221 | rs114115904 | rs6489868 | rs7966314 | rs10744791 |
| rs143443576 | rs13085367 | rs34677490 | rs6489869 | rs4767037 | rs12462642 |
| rs35257780 | rs13063635 | rs1542756 | rs6489870 | rs1981557 | rs732631 |
| rs147316571 | rs71327027 | rs1542755 | rs4766666 | rs1981556 | rs2109069 |
| rs71325096 | rs78380307 | rs13060713 | rs4767033 | rs1981555 | rs2109070 |
| rs139901862 | rs4443214 | rs78919248 | rs4767034 | rs3759376 | rs2277732 |
| rs34044394 | rs4493469 | rs72856706 | rs10850099 | rs3759375 | rs7255545 |
| rs71619620 | rs17282391 | rs111837807 | rs10850100 | rs4767040 | rs12627039 |
| rs61556067 | rs13083881 | rs112785163 | rs4766667 | rs10774679 | rs17860115 |
| rs2088693 | rs17216717 | rs143334143 | rs4766668 | rs7132797 | rs12482556 |
| rs58552652 | rs13075836 | rs72856713 | rs4766671 | rs1156361 | rs11088247 |
| rs13087776 | rs13096325 | rs10774671 | rs4766672 | rs3815178 | rs2300370 |
| rs13088143 | rs13097340 | rs1131476 | rs4766673 | rs1859330 | rs2248420 |
| rs13068410 | rs3091314 | rs2660 | rs4766674 | rs7299132 | rs2834154 |
| rs13094262 | rs34558763 | rs7135577 | rs4766675 | rs6489879 | rs6517153 |
| rs34805664 | rs35178150 | rs4767024 | rs4766676 | rs4238033 | rs17860142 |
| rs35028116 | rs34693386 | rs4767026 | rs7304898 | rs4767041 | rs9636867 |
| rs34610609 | rs79948053 | rs4767027 | rs7315441 | rs7955267 | rs12482193 |
| rs13086534 | rs34523728 | rs4767029 | rs7305035 | rs7311182 | rs12482060 |
| rs13064632 | rs34570200 | rs4767030 | rs7316586 | rs10735079 | rs17860169 |
| rs34254987 | rs35560301 | rs6489865 | rs916972 | rs6489880 | rs9976829 |
| rs4683162 | rs34134191 | rs10850093 | rs7134391 | rs7980275 | rs13050728 |
| rs4683163 | rs115102354 | rs10850094 | rs7306205 | rs7977345 | rs2834157 |
| rs4683164 | rs13069750 | rs10850095 | rs1859336 | rs6489881 | rs2236756 |
| rs34155121 | rs34340501 | rs10774672 | rs2384071 | rs6489882 | rs2252639 |
| rs4234453 | rs71327038 | rs10850096 | rs2384072 | rs7131998 | rs2252650 |
| rs72901034 | rs36010446 | rs10850097 | rs2384073 | rs7135260 | rs2284549 |
| rs12108042 | rs13069845 | rs10774673 | rs6489874 | rs2269899 | rs2284550 |
| rs34867672 | rs17282797 | rs10774674 | rs6489875 | rs2384074 | rs2284551 |
| rs34924300 | rs13080979 | rs10850098 | rs6489876 | rs10850103 | rs12053666 |
| rs4683166 | rs13098911 | rs10774676 | rs6489877 | rs2285932 | rs2073361 |
| rs7652478 | rs34378541 | rs10774677 | rs6489878 | rs7310667 | rs2834161 |
| rs7642229 | rs17282922 | rs4767031 | rs7298184 | rs10744789 | rs2834163 |
| rs7642320 | rs71327040 | rs4766663 | rs12322160 | rs4238034 | rs2834164 |
| rs9824651 | rs34289272 | rs4766664 | rs7132404 | rs4767044 | rs2834165 |
| rs9825081 | rs34005848 | rs4767032 | rs4767036 | rs1557866 | rs2236757 |

comparing the number of the determined above SNPs in the patient to a reference number, wherein the reference number is associated with a known severity of the Covid-19 infection, and
wherein the presence of a number of SNPs in the patient associated with a severity of Covid-19 infection is prognostic of the severity to which Covid-19 infection will develop in the patient.

**2.** The method according to claim 1, wherein the following SNPs are determined:

| | | | | | |
|---|---|---|---|---|---|
| rs306313 | rs12610495 | rs17214952 | rs544873 | rs2769071 | rs505922 |
| rs12619108 | rs12223324 | rs9838450 | rs545971 | rs677355 | rs529565 |
| rs6974924 | rs8038527 | rs687621 | rs8176663 | rs676996 | |
| rs10091098 | rs76187417 | rs687289 | rs493246 | rs550057 | |
| rs4569267 | rs17078348 | rs657152 | rs495203 | rs674302 | |
| rs56132597 | rs13079478 | rs644234 | rs582094 | rs554833 | |

**3.** Method according to one of the preceding claims, wherein the following SNPs are determined in addition to the SNP rs13079478:

| | | | | | |
|---|---|---|---|---|---|
| rs1994491 | rs77902290 | rs35501575 | rs35525815 | rs71325100 | rs71327003 |
| rs1994493 | rs2171531 | rs13069079 | rs13078739 | rs13066062 | rs36023124 |
| rs35831747 | rs55920693 | rs76597151 | rs13079478 | rs36122610 | |
| rs35477280 | rs56332428 | rs34849862 | rs13079869 | rs34442130 | |
| rs13066516 | rs71325095 | rs36039366 | rs33910087 | rs13075758 | |

and, in addition to the SNP rs17214952, the following SNPs were determined:

| | | | | | |
|---|---|---|---|---|---|
| rs1994492 | rs34381952 | rs35827997 | rs13071283 | rs71325101 | |
| rs75928798 | rs4388012 | rs35209528 | rs17214952 | rs71325102 | |
| rs2373087 | rs41289616 | rs35855315 | rs17215008 | rs17330872 | |
| rs13099120 | rs34000569 | rs17280623 | rs71325098 | | |
| rs6785091 | rs34324101 | rs13059238 | rs41289622 | | |

which are SNPs of the FYC01 gene that are causal markers for the pathogenesis and severity of Covid-19 infection.

**4.** The method according to any one of the preceding claims, wherein additionally at least one of the following SNPs influencing the replication rate of the SARS-CoV2 virus is determined:

| | | | | | |
|---|---|---|---|---|---|
| rs10907185 | rs1061508 | rs11684157 | rs6720483 | rs34037363 | rs17027526 |
| rs3855951 | rs1061508 | rs3099559 | rs12469386 | rs7647812 | rs116034902 |
| rs3131660 | rs12079454 | rs3099559 | rs74453786 | rs11706052 | rs78248692 |
| rs34517448 | rs3736757 | rs3112916 | rs2305138 | rs11716445 | rs6831 |
| rs4233338 | rs67337751 | rs3112916 | rs3738904 | rs80073654 | rs3209157 |
| rs4233338 | rs60400761 | rs116686919 | rs7652331 | rs114691754 | rs6889892 |
| rs4610985 | rs73093505 | rs4666170 | rs7652331 | rs16838298 | rs72647244 |
| rs4610985 | rs76309804 | rs4666170 | rs1545985 | rs114453087 | rs62364494 |
| rs191000784 | rs10495150 | rs34656234 | rs13079478 | rs12510574 | rs28460815 |
| rs273885 | rs6428948 | rs2723088 | rs33910087 | rs77591659 | rs72787387 |

(continued)

| | | | | | |
|---|---|---|---|---|---|
| rs76861341 | rs79779531 | rs2723089 | rs33910087 | rs10015634 | rs2291628 |
| rs67008022 | rs9287719 | rs10195328 | rs4683158 | rs6532203 | rs12523609 |
| rs12116970 | rs6714413 | rs10195328 | rs4683158 | rs6532208 | rs374748 |
| rs12116970 | rs6714413 | rs62140404 | rs4683158 | rs10003496 | rs28763954 |
| rs7521948 | rs72777326 | rs12714145 | rs17214952 | rs7655622 | rs331079 |
| rs6692394 | rs11684157 | rs2346553 | rs6791542 | rs10516428 | rs7701116 |
| rs7724108 | rs12547746 | rs117341758 | rs78925317 | rs10774517 | rs8182064 |
| rs11951084 | rs4738406 | rs74924161 | rs10770437 | rs280590 | rs2703580 |
| rs1549203 | rs73264571 | rs61917258 | rs1514831 | rs57620361 | rs116915857 |
| rs11958425 | rs116990828 | rs61917258 | rs1514831 | rs73168527 | rs76089422 |
| rs72800300 | rs72722967 | rs7311276 | rs7133858 | rs9507502 | rs118031436 |
| rs116200464 | rs7865343 | rs7311276 | rs7133858 | rs9507502 | rs8042654 |
| rs116200464 | rs7028704 | rs11061620 | rs10841162 | rs7335635 | rs8033595 |
| rs17061784 | rs16918878 | rs12812965 | rs10841162 | rs117936226 | rs76029207 |
| rs12652763 | rs117351867 | rs2887533 | rs10770446 | rs117936226 | rs76029207 |
| rs72814105 | rs34680486 | rs3924361 | rs7962879 | rs9544446 | rs62021260 |
| rs79931282 | rs10985215 | rs117086722 | rs4497470 | rs11617489 | rs1058260 |
| rs9392453 | rs2296793 | rs73041063 | rs16915248 | rs78768204 | rs1135911 |
| rs17513153 | rs148203698 | rs73041063 | rs11044463 | rs12863903 | rs2277589 |
| rs17169592 | rs148203698 | rs11061660 | rs7965006 | rs12863903 | rs3784471 |
| rs1459558 | rs7077992 | rs73029107 | rs7965006 | rs222670 | rs1982906 |
| rs1649229 | rs6560859 | rs73029190 | rs10841210 | rs222671 | rs12901252 |
| rs328902 | rs6560859 | rs56262248 | rs79072570 | rs568318446 | rs7168655 |
| rs34949146 | rs6560859 | rs73034919 | rs10843330 | rs10137084 | rs215095 |
| rs79787993 | rs10829268 | rs144000701 | rs1496218 | rs10137084 | rs215059 |
| rs117718957 | rs10829268 | rs10848459 | rs10843346 | rs7150942 | rs215059 |
| rs117718957 | rs10829268 | rs73038672 | rs10843346 | rs12717411 | rs215066 |
| rs78906654 | rs11101684 | rs77082078 | rs116789485 | rs12717411 | rs119774 |
| rs2868177 | rs11538725 | rs6489283 | rs1454934 | rs11158820 | rs215069 |
| rs2868180 | rs11538725 | rs6489283 | rs7967264 | rs117146868 | rs6498595 |
| rs7804806 | rs7937799 | rs118186282 | rs11052270 | rs2498827 | rs6498595 |
| rs56295910 | rs2957865 | rs73597943 | rs7979825 | rs2498825 | rs8187843 |
| rs56295910 | rs10897453 | rs111449649 | rs701006 | rs2474030 | rs8187843 |
| rs3757508 | rs10897453 | rs75321406 | rs1633360 | rs77743349 | rs8187843 |
| rs2395833 | rs928948 | rs76653618 | rs41292019 | rs12885234 | rs4780589 |
| rs257376 | rs17825668 | rs12313774 | rs78948244 | rs62011404 | rs4780589 |
| rs16930092 | rs548907 | rs7975502 | rs61068064 | rs78141811 | rs1967120 |
| rs7830335 | rs548907 | rs79833769 | rs77411677 | rs76249401 | rs1967120 |
| rs12547746 | rs117341758 | rs78925317 | rs55707601 | rs8182064 | rs3784862 |

(continued)

| | | | | | |
|---|---|---|---|---|---|
| rs3784862 | rs212079 | rs4782377 | rs75584356 | rs3865452 | rs2839217 |
| rs3784863 | rs2299670 | rs11646212 | rs3931634 | rs17857107 | rs2073382 |
| rs152033 | rs212080 | rs3751675 | rs3931634 | rs17857107 | rs35940413 |
| rs2230669 | rs246141 | rs4782307 | rs3931634 | rs17857107 | rs2839245 |
| rs35599 | rs12932079 | rs2879097 | rs7253253 | rs431579 | rs2839245 |
| rs35621 | rs9935402 | rs9892443 | rs17001450 | rs431579 | rs1044998 |
| rs35626 | rs4782505 | rs7215873 | rs75973396 | rs6115218 | rs35346764 |
| rs11861085 | rs8053676 | rs117859736 | rs2079014 | rs4811837 | rs2839246 |
| rs11861115 | rs8053676 | rs746628 | rs2079014 | rs34500739 | rs7277175 |
| rs3887893 | rs7199004 | rs77584326 | rs3760884 | rs2839217 | rs2839256 |
| rs212079 | rs7199004 | rs74917169 | rs7507651 | rs2839217 | rs2839256 |
| rs743346 | rs4646316 | rs4646316 | rs2072798 | rs11704825 | |

**5.** Method according to one of the preceding claims, wherein additionally at least one of the following SNPs associated with the following diseases is determined in order to integrate the influence of disease-associated SNPs into the Covid-19 risk assessment:

| | | | |
|---|---|---|---|
| rs63750847 | Alzheimer's disease | rs28930068 | Hypokalemic periodic paralysis |
| rs1801131 | Anaphylaxis after vaccinations | rs28930069 | Hypokalemic periodic paralysis |
| rs1801133 | Anaphylaxis after vaccinations | rs80338779 | Hypokalemic periodic paralysis |
| rs949963 | Anaphylaxis after vaccinations | rs80338777 | Hypokalemic periodic paralysis |
| rs2228139 | Anaphylaxis after vaccinations | rs121908555 | Hypokalemic periodic paralysis |
| rs4848300 | Anaphylaxis after vaccinations | rs80338788 | Hypokalemic periodic paralysis |
| rs17561 | Anaphylaxis after vaccinations | rs80338784 | Hypokatemic periodic paralysis |
| rs1800587 | Anaphylaxis after vaccinations | rs121918007 | Hypophosphatasia |
| rs1801278 | Antidiabetic sulphonylurea | rs121918002 | Hypophosphatasia |
| rs1801282 | Antidiabetic sulphonylurea | rs2476601 | Hypothyroidism |
| rs7903146 | Antidiabetic sulphonylurea | rs7850258 | Hypothyroidism |
| rs12255372 | Antidiabetic sulphonylurea | rs3184504 | Hypothyroidism |
| rs5215 | Antidiabetic sulphonylurea | rs2517532 | Hypothyroidism |
| rs5219 | Antidiabetic sulphonylurea | rs12610495 | Idiopathic pulmonary fibrosis |
| rs757110 | Antidiabetic sulphonylurea | rs1278769 | Idiopathic pulmonary fibrosis |
| rs1799859 | Antidiabetic sulphonylurea | rs1980653 | Idiopathic pulmonary fibrosis |
| rs6196 | Antipsychotics and weight gain | rs1981997 | Idiopathic pulmonary fibrosis |
| rs17782313 | Antipsychotics and weight gain | rs2034650 | Idiopathic pulmonary fibrosis |
| rs489693 | Antipsychotics and weight gain | rs6793295 | Idiopathic pulmonary fibrosis |
| rs1801131 | Antipsychotics and weight gain | rs7934606 | Idiopathic pulmonary fibrosis |
| rs5067 | Asthma | rs2187668 | Idiopathic membranous nephroapthia |
| rs689465 | Asthma | rs4664308 | Idiopathic membranous nephroapthia |
| rs4950928 | Asthma | rs9275596 | IgA nephropathy |
| rs1042713 | Asthma | rs363039 | Intelligence measurement |
| rs1695 | Asthma | rs363050 | Intelligence measurement |
| rs8069176 | Asthma | rs1511412 | Keloid formation |
| rs7216389 | Asthma | rs8032158 | Keloid formation |
| rs13153971 | Asthma | rs873549 | Keloid formation |

(continued)

| | | | |
|---|---|---|---|
| rs37973 | Asthma inhalation corticosteroids | rs940187 | Keloid formation |
| rs2240017 | Asthma inhalation corticosteroids | rs7639618 | Knee osteoarthritis |
| rs11204971 | Atopic dermatitis | rs4140564 | Knee osteoarthritis in women |
| rs4950928 | Atopischic asthma | rs5751876 | Caffeine enjoyment |
| rs1143634 | Exercise-induced muscle damage | rs6968865 | Caffeine enjoyment |
| rs28497577 | Exercise-induced muscle damage | rs2472297 | Caffeine enjoyment |
| rs1800629 | Exercise-induced muscle damage | rs6511720 | Coronary heart disease |
| rs4880 | Exercise-induced muscle damage | rs6903956 | Coronary heart disease |
| rs1800795 | Exercise-induced muscle damage | rs9834312 | Body size |
| rs13266634 | Exercise-induced muscle damage | rs2179922 | Body size |
| rs1815739 | Exercise-induced muscle damage | rs1042725 | Body size |
| rs1801282 | Stress glucose test | rs6060369 | Body size |
| rs10936599 | Biological ageing | rs8038652 | Body size |
| rs2736100 | Biological ageing | rs2802288 | Longevity |
| rs1042522 | Biological ageing | rs2802292 | Longevity |
| rs1064395 | Bipolar disorder | rs2764264 | Longevity |
| rs2820037 | High blood pressure | rs2811712 | Longevity |
| rs5186 | High blood pressure | rs34516635 | Longevity |
| rs4961 | High blood pressure | rs2073711 | Lumbar disc herniation |
| rs5335 | High blood pressure | rs2736100 | Pulmonary fibrosis |
| rs5370 | High blood pressure | rs2076295 | Pulmonary fibrosis |
| rs6997709 | High blood pressure | rs13277113 | Lupus (Systemic Lupus Erythematosus) |
| rs7961152 | High blood pressure | rs2187668 | Lupus (Systemic Lupus Erythematosus) |
| rs11110912 | High blood pressure | rs2205960 | Lupus (Systemic Lupus Erythematosus) |
| rs1937506 | High blood pressure | rs9888739 | Lupus (Systemic Lupus Erythematosus) |
| rs2398162 | High blood pressure | rs9287638 | Male baldness |
| rs13333226 | High blood pressure | rs9479482 | Male baldness |
| rs5065 | Hypertension therapy | rs2073963 | Male baldness |
| rs13130484 | Body Mass Index | rs6945541 | Male baldness |
| rs6971091 | Body Mass Index | rs10502861 | Male baldness |
| rs2272383 | Body Mass Index | rs8085664 | Male baldness |
| rs1528133 | Body Mass Index | rs2180439 | Male baldness |
| rs1121980 | Body Mass Index | rs6047844 | Male baldness |
| rs10871777 | Body Mass Index | rs1160312 | Male baldness |
| rs12970134 | Body Mass Index | rs1385699 | Male baldness |
| rs2229616 | Body Mass Index | rs2223841 | Male baldness |
| rs2272382 | Body Mass Index | rs6152 | Male baldness |
| rs1892534 | C-reactive protein | rs1801131 | Metfromin effect |
| rs8034191 | COPD | rs1801133 | Metfromin effect |
| rs7671167 | COPD | rs683369 | Metfromin effect |
| rs2653349 | Cluster headache | rs34059508 | Metfromin effect |
| rs10883365 | Colitis ulcerosa | rs11212617 | Metfromin effect |
| rs2395185 | Colitis ulcerosa | rs1801133 | Methotrexate and rheumatoid arthritis |
| rs9858542 | Colitis ulcerosa | rs2651899 | Migraine |
| rs11209026 | Colitis Ulcerosa | rs10761659 | Morbus Crohn |
| rs646776 | Coronary heart disease | rs11190140 | Morbus Crohn |

(continued)

| | | | |
|---|---|---|---|
| rs599839 | Coronary heart disease | rs17234657 | Morbus Crohn |
| rs3900940 | Coronary heart disease | rs1893217 | Morbus Crohn |
| rs7439293 | Coronary heart disease | rs2066844 | Morbus Crohn |
| rs383830 | Coronary heart disease | rs2066845 | Morbus Crohn |
| rs6922269 | Coronary heart disease | rs2066847 | Morbus Crohn |
| rs3798220 | Coronary heart disease | rs2241880 | Morbus Crohn |
| rs2070744 | Coronary heart disease | rs3197999 | Morbus Crohn |
| rs4977574 | Coronary heart disease | rs10513789 | Morbus Parkinson |
| rs1333048 | Coronary heart disease | rs12487066 | Multiple sclerosis |
| rs1333049 | Coronary heart disease | rs6897932 | Multiple sclerosis |
| rs501120 | Coronary heart disease | rs3129934 | Multiple sclerosis |
| rs964184 | Coronary heart disease | rs3135388 | Multiple sclerosis |
| rs2298566 | Coronary heart disease | rs7326018 | Multiple sclerosis |
| rs3184504 | Coronary heart disease | rs725613 | Multiple sclerosis |
| rs17228212 | Coronary heart disease | rs1154155 | Narcolepsy |
| rs8055236 | Coronary heart disease | rs2305795 | Narcolepsy |
| rs7250581 | Coronary heart disease | rs 16944 | Osteoarthritis of the hip |
| rs4420638 | Coronary heart disease | rs10947262 | Osteoarthritis of the knees |
| rs688034 | Coronary heart disease | rs3815148 | Osteoarthritis of the knees |
| rs1360780 | Depression | rs11842874 | Osteoarthritis of the knees |
| rs1545843 | Depression | rs143383 | Osteoarthritis of the knees |
| rs1031681 | Depression | rs7776725 | Osteoporosis |
| rs4655595 | Diabetes Mellitus, Type 2 | rs3736228 | Osteoporosis |
| rs6718526 | Diabetes Mellitus, Type 2 | rs1544410 | Osteoporosis |
| rs1801282 | Diabetes Mellitus, Type 2 | rs174230 | Sudden cardiac death |
| rs358806 | Diabetes Mellitus, Type 2 | rs1800312 | Pompe disease 1 & 2 |
| rs7659604 | Diabetes Mellitus, Type 2 | rs28940868 | Pompe disease 1 & 2 |
| rs9465871 | Diabetes Mellitus, Type 2 | rs2856683 | Primary biliary cirrhosis |
| rs1799884 | Diabetes Mellitus, Type 2 | rs7530511 | Psoriasis |
| rs13266634 | Diabetes Mellitus, Type 2 | rs11209026 | Psoriasis |
| rs10811661 | Diabetes Mellitus, Type 2 | rs3212227 | Psoriasis |
| rs9326506 | Diabetes Mellitus, Type 2 | rs6887695 | Psoriasis |
| rs4506565 | Diabetes Mellitus, Type 2 | rs10484554 | Psoriasis |
| rs7903146 | Diabetes Mellitus, Type 2 | rs11203366 | Rheumatoid arthritis |
| rs12255372 | Diabetes Mellitus, Type 2 | rs13031237 | Rheumatoid arthritis |
| rs5219 | Diabetes Mellitus, Type 2 | rs1569723 | Rheumatoid arthritis |
| rs9300039 | Diabetes Mellitus, Type 2 | rs2327832 | Rheumatoid arthritis |
| rs10830963 | Diabetes Mellitus, Type 2 | rs2201841 | Rheumatoid arthritis |
| rs12304921 | Diabetes Mellitus, Type 2 | rs10889677 | Rheumatoid arthritis |
| rs1495377 | Diabetes Mellitus, Type 2 | rs11162922 | Rheumatoid arthritis |
| rs2930291 | Diabetes Mellitus, Type 2 | rs6679677 | Rheumatoid arthritis |
| rs2903265 | Diabetes Mellitus, Type 2 | rs3738919 | Rheumatoid arthritis |
| rs8050136 | Diabetes Mellitus, Type 2 | rs7574865 | Rheumatoid arthritis |
| rs11868035 | Diabetes Mellitus, Type 2 | rs3087243 | Rheumatoid arthritis |
| rs1889018 | Diabetes Mellitus, Type 2 | rs3816587 | Rheumatoid arthritis |
| rs2295490 | Diabetes Mellitus, Type 2 | rs2269475 | Rheumatoid arthritis |
| rs104893919 | Diastophic dysplasia | rs6910071 | Rheumatoid arthritis |
| rs104893915 | Diastophic dysplasia | rs6457617 | Rheumatoid arthritis |
| rs104893924 | Diastophic dysplasia | rs10499194 | Rheumatoid arthritis |
| rs5082 | Dietary effect | rs6920220 | Rheumatoid arthritis |
| rs1801282 | Dietary effect | rs729302 | Rheumatoid arthritis |

(continued)

| | | | |
|---|---|---|---|
| rs17300539 | Dietary effect | rs2004640 | Rheumatoid arthritis |
| rs1800497 | Dietary effect | rs10488631 | Rheumatoid arthritis |
| rs662799 | Dietary effect | rs3761847 | Rheumatoid arthritis |
| rs16879765 | Dupuytren's disease | rs10818488 | Rheumatoid arthritis |
| rs16946160 | Acquired nephrotic syndrome | rs2104286 | Rheumatoid arthritis |
| rs12713559 | Familial hypercholesterolemia type B | rs9550642 | Rheumatoid arthritis |
| rs5742904 | Familial hypercholesterolemia type B | rs2837960 | Rheumatoid arthritis |
| rs144467873 | Familial hypercholesterolemia type B | rs1953126 | Rheumatoid arthritis |
| rs28933405 | Familial hypertroph. Cardiomyopathy | rs6684865 | Rheumatoid arthritis |
| rs28938173 | Familial hypertroph. Cardiomyopathy | rs3890745 | Rheumatoid arthritis |
| rs104894369 | Familial hypertroph. Cardiomyopathy | rs1049550 | Sarcoidosis |
| rs104894370 | Familial hypertroph. Cardiomyopathy | rs12425791 | Stroke |
| rs3218714 | Familial hypertroph. Cardiomyopathy | rs10830962 | Gestational diabetes (GDM) |
| rs3218713 | Familial hypertroph. Cardiomyopathy | rs4402960 | Gestational diabetes |
| rs104894503 | Familial hypertroph. Cardiomyopathy | rs1799884 | Gestational diabetes |
| rs104894502 | Familial hypertroph. Cardiomyopathy | rs7903146 | Gestational diabetes |
| rs1801282 | Fatty tissue formation | rs12255372 | Gestational diabetes |
| rs4788102 | Obesity | rs10830963 | Gestational diabetes |
| rs6548238 | Obesity | rs2187668 | Selective IgA deficiency |
| rs7647305 | Obesity | rs1990760 | Selective IgA deficiency |
| rs925946 | Obesity | rs9271366 | Selective IgA deficiency |
| rs5082 | Obesity | rs1004819 | Spondylitis ankylosans |
| rs17300539 | Obesity | rs10489629 | Spondylitis ankylosans |
| rs35859249 | Obesity | rs2201841 | Spondylitis ankylosans |
| rs6232 | Obesity | rs11465804 | Spondylitis ankylosans |
| rs6971091 | Obesity | rs11209026 | Spondylitis ankylosans |
| rs662799 | Obesity | rs1343151 | Spondylitis ankylosans |
| rs17817449 | Obesity | rs10889677 | Spondylitis ankylosans |
| rs17782313 | Obesity | rs11209032 | Spondylitis ankylosans |
| rs2229616 | Obesity | rs10865331 | Spondylitis ankylosans |
| rs1799884 | Birth weight | rs17482078 | Spondylitis ankylosans |
| rs7903146 | Birth weight | rs10050860 | Spondylitis ankylosans |
| rs1051730 | Birth weight | rs2287987 | Spondylitis ankylosans |
| rs12565727 | Bald head | rs2303138 | Spondylitis ankylosans |
| rs76723693 | Glucose-6-phosphate dehydrogenase deficiency | rs1800972 | Tendinopathies of the upper extremity |
| rs137852318 | Glucose-6-phosphate dehydrogenase deficiency | rs1138545 | Tendinopathies of the upper extremity |
| rs5030868 | Glucose-6-phosphate dehydrogenase deficiency | rs1800012 | Tendinopathies of the upper extremity |
| rs1050828 | Glucose-6-phosphate dehydrogenase deficiency | rs2104772 | Tendinopathies of the lower extremity |
| rs80356491 | Glycogen storage disease 1B | rs12722 | Tendinopathies of the lower extremity |
| rs80356490 | Glycogen storage disease 1B | rs4747096 | Tendinopathies of the lower extremity |
| rs80356489 | Glycogen storage disease 1B | rs679620 | Tendinopathies of the lower extremity |
| rs113994129 | Glycogen storage disease type 3 | rs1800012 | Tendinopathies of the lower extremity |

(continued)

| | | | |
|---|---|---|---|
| rs113994130 | Glycogen storage disease type 3 | rs4789932 | Tendinopathies of the lower extremity |
| rs113994131 | Glycogen storage disease type 3 | rs143383 | Tendinopathies of the lower extremity |
| rs119103251 | Glycogen storage disease type 5 | rs6025 | Deep vein thrombosis |
| rs116987552 | Glycogen storage disease type 5 | rs5361 | Deep vein thrombosis |
| rs2155219 | Grass pollen allergy and hay fever | rs268 | Deep vein thrombosis |
| rs646776 | Heart attack | rs1893217 | Type 1 diabetes |
| rs17465637 | Heart attack | rs1990760 | Type 1 diabetes |
| rs8089 | Heart attack | rs3741208 | Type 1 diabetes |
| rs1333040 | Heart attack | rs9273363 | Type 1 diabetes |
| rs2383206 | Heart attack | rs10012946 | Type 2 diabetes |
| rs2383207 | Heart attack | rs1111875 | Type 2 diabetes |
| rs10757278 | Heart attack | rs1387153 | Type 2 diabetes |
| rs1927911 | Heart attack | rs2237892 | Type 2 diabetes |
| rs501120 | Heart attack | rs2383208 | Type 2 diabetes |
| rs662799 | Heart attack | rs4712523 | Type 2 diabetes |
| rs17228212 | Heart attack | i3002432 | Venous thromboembolism |
| rs17576 | Heart attack | rs2282679 | Vitamin D level |
| rs3782886 | Heart attack | rs10741657 | Vitamin D level |
| rs7291467 | Heart attack | rs1801175 | Von Gierke disease |
| rs5742905 | Homocystinuria, pyridoxine dependent | rs1801176 | Von Gierke disease |
| rs12413409 | Hypertension | rs80356484 | Von Gierke disease |
| rs1378942 | Hypertension | rs80356487 | Von Gierke disease |
| rs16998073 | Hypertension | rs80356479 | Von Gierke disease |
| rs17367504 | Hypertension | rs2200733 | Atrial fibrillation |
| rs3754777 | Hypertension | rs10033464 | Atrial fibrillation |
| rs964184 | Hypertriglyceridemie | rs1801253 | Effect of β-blockers |

**6.** The method according to any one of the preceding claims, wherein the following SNPs associated with healthy aging are additionally determined:

| | | | | | |
|---|---|---|---|---|---|
| rs2274789 | rs1801214 | rs10224902 | rs1663564 | rs2591590 | rs6530368 |
| rs228648 | rs1995319 | rs73694648 | rs935241 | rs2591591 | rs1731469 |
| rs56125713 | rs2465570 | rs 1800504 | rs935240 | rs2547068 | rs6527818 |
| rs609320 | rs2437323 | rs10250576 | rs278109 | rs2547069 | rs3213451 |
| rs9508 | rs893805 | rs12338 | rs1491893 | rs2547070 | rs5926304 |
| rs35672330 | rs11935573 | rs2291219 | rs16919359 | rs2591593 | rs2529541 |
| rs17399810 | rs3810870 | rs16889283 | rs2297224 | rs2547072 | rs2071309 |
| rs1676486 | rs4701997 | rs11779306 | rs2770928 | rs2547073 | rs2071311 |
| rs59123361 | rs1445823 | rs2381409 | rs3751354 | rs953551 | rs2856733 |
| rs749387317 | rs3213936 | rs1138374 | rs2428249 | rs2591594 | rs1800280 |
| rs2266782 | rs7714670 | rs10780682 | rs3742290 | rs2547074 | rs228406 |
| rs11586699 | rs6453022 | rs17062264 | rs34756139 | rs2547075 | rs5917933 |
| rs12120084 | rs34592828 | rs2282683 | rs4883918 | rs2547076 | rs6610447 |
| rs3729547 | rs8654 | rs1057406 | rs147785041 | rs2591595 | rs501128 |

(continued)

| | | | | | |
|---|---|---|---|---|---|
| rs3085 | rs160632 | rs10867826 | rs17852293 | rs2591596 | rs2495783 |
| rs6604561 | rs6874840 | rs10818708 | rs35435463 | rs1862461 | rs11545211 |
| rs360057 | rs10479190 | rs35902535 | rs941581 | rs1862462 | rs2516023 |
| rs966365 | rs10055840 | rs35898523 | rs4087943 | rs2591597 | rs5919015 |
| rs3811445 | rs2950844 | rs9886752 | rs1267657 | rs1048290 | rs2296542 |
| rs4148211 | rs2910327 | rs2297145 | rs35270670 | rs1133330 | rs12397326 |
| rs17850223 | rs2910330 | rs2274634 | rs35582838 | rs1054487 | rs12393722 |
| rs11556184 | rs2910005 | rs41284748 | rs8042146 | rs35647251 | rs7884806 |
| rs4129190 | rs3749780 | rs7908745 | rs169068 | rs7249743 | rs7880917 |
| rs6761276 | rs1042719 | rs1227049 | rs4610 | rs36106401 | rs7580 |
| rs61744148 | rs1042720 | rs17109671 | rs7192331 | rs2604901 | rs3012627 |
| rs13422424 | rs1823035 | rs11188225 | rs35583599 | rs3810327 | rs6647476 |
| rs3214503 | rs168726 | rs1048445 | rs117461525 | rs17271272 | rs 1113265 |
| rs492594 | rs705441 | rs3740078 | rs12926089 | rs3745521 | rs10126146 |
| rs28630685 | rs416574 | rs11190245 | rs12926669 | rs5030878 | rs363774 |
| rs35813871 | rs8333 | rs2278842 | rs12935737 | rs8113436 | rs1045686 |
| rs918949 | rs2274136 | rs11197835 | rs12923538 | rs12984188 | rs5951328 |
| rs3796028 | rs1042303 | rs2901343 | rs3752426 | rs12984473 | rs111588852 |
| rs1004814 | rs2760118 | rs11813597 | rs2303238 | rs34604090 | rs6622126 |
| rs1364641 | rs4678 | rs484955 | rs8052064 | rs433291 | rs5946294 |
| rs3796031 | rs3131787 | rs6356 | rs3809870 | rs925878 | rs2278954 |
| rs3796032 | rs8084 | rs4758267 | rs2003968 | rs3810340 | rs371749 |
| rs11895915 | rs7192 | rs3213706 | rs2271231 | rs3760849 | rs12390 |
| rs6728818 | rs17882603 | rs61752934 | rs4796555 | rs35914474 | rs5910611 |
| rs1063639 | rs1130034 | rs948847 | rs17853331 | rs145250945 | rs11452643 |
| rs7613447 | rs10093 | rs174535 | rs17856697 | rs12609654 | rs502434 |
| rs2291862 | rs1048372 | rs1800007 | rs7209228 | rs1205194 | rs3747343 |
| rs394558 | rs1049133 | rs2004649 | rs906807 | rs16987712 | rs2498776 |
| rs2245532 | rs1049130 | rs61744384 | rs1484873 | rs6017667 | rs877761 |
| rs62244134 | rs1063323 | rs7116967 | rs56342526 | rs3746493 | rs176037 |
| rs2286720 | rs3208181 | rs2848477 | rs56131056 | rs3737137 | rs3765272 |
| rs2276818 | rs3213484 | rs3809018 | rs55659726 | rs434857 | rs237520 |
| rs1044243 | rs1049110 | rs1052313 | rs3752234 | rs2017816 | rs626560 |
| rs9855810 | rs2071551 | rs10891705 | rs3752237 | rs116988298 | rs12116111 |
| rs11708527 | rs762815 | rs2282537 | rs3752246 | rs6586239 | rs5983916 |
| rs3732799 | rs1042187 | rs595986 | rs757292 | rs1980982 | rs561077 |
| rs1381057 | rs1042331 | rs11063529 | rs7250872 | rs1128127 | rs1139916 |
| rs3218649 | rs1042335 | rs7298766 | rs2396359 | rs165734 | rs1432838968 |
| rs9884018 | rs1071597 | rs11612427 | rs3746045 | rs165602 | rs5969919 |

(continued)

| | | | | | |
|---|---|---|---|---|---|
| rs1056523 | rs12528378 | rs35642012 | rs2074789 | rs5997714 | rs6526155 |
| rs4688761 | rs623813 | rs1049112 | rs9973235 | rs56174639 | rs34966902 |
| rs2242353 | rs2297970 | rs1806191 | rs9304915 | rs36125344 | rs1059703 |
| rs9823911 | rs13194610 | rs1805482 | rs778805 | rs55653327 | rs1059702 |
| rs9836841 | rs7767455 | rs10841611 | rs2287915 | rs11705624 | rs2728532 |
| rs1709657 | rs1130656 | rs2720298 | rs2216663 | rs1127000 | rs1126762 |
| rs843334 | rs3823465 | rs2603104 | rs2547064 | rs2306734 | |
| rs11556518 | rs55666134 | rs923829 | rs2252675 | rs11222 | |
| rs2515960 | rs6968741 | rs7955450 | rs1867691 | rs10871864 | |
| rs1024323 | rs3735398 | rs17034524 | rs2121133 | rs41309545 | |

**7.** Method according to one of the preceding claims, wherein for predicting the severity of the course of a Covid-19 infection in a human patient

a) the quantitative RNA host gene expression is determined according to the following formula:

$$\sum_{i=1}^{n} = F_{1*}\begin{pmatrix} positive \\ neutral \\ negative \end{pmatrix} + F_{2*}\begin{pmatrix} positive \\ neutral \\ negative \end{pmatrix} + F_{3*}\begin{pmatrix} positive \\ neutral \\ negative \end{pmatrix} + F_{4*}\begin{pmatrix} positive \\ neutral \\ negative \end{pmatrix}$$

b) the qualitative protein functionality is determined according to the following formula:

$$\sum_{j=1}^{m} = f_{1*}\begin{pmatrix} positive \\ neutral \\ negative \end{pmatrix} + f_{2*}\begin{pmatrix} positive \\ neutral \\ negative \end{pmatrix} + f_{3*}\begin{pmatrix} positive \\ neutral \\ negative \end{pmatrix}$$

which results in the polygenetic corona risk per gene:

$$\sum_{i=1}^{n} + \sum_{j=1}^{m} = \begin{matrix} \text{Polygenetic} \\ \text{corona risk} \\ \text{(PCRG) per gene} \end{matrix}$$

where in the formulas mean:

$\Sigma$= weighted sum/linear regression
i ... n = number of non-coding SNPs
j ... m = number of coding SNPs
$F_1$, $F_2$, $F_3$, ... $F_n$ = individual evaluation parameters
$f_1$, $f_2$, $f_3$, ... $f_n$ = individual evaluation parameters
positive = number of SNPs that have a favorable effect (value 2)
neutral = number of SNPs that have no effect (value 1)
negative = number of SNPs that have an unfavorable effect (value 0)

**8.** Method according to one of the preceding claims, wherein a distinction is made as to whether the Covid-19 infection is caused by a monogenetic defect or by a polygenetic defect due to the interaction of many SNP variants (structural proteins) and regulatory SNP elements.

## Revendications

**1.** Procédé pour prédire la gravité de l'évolution d'une maladie de Covid-19 chez un patient humain, ledit procédé comprenant :

déterminer la présence de polymorphismes nucléotidiques uniques (SNP) associés à une maladie Covid-19 dans un échantillon de patient, les SNP étant utilisés avec les codes de référence suivants dans la *Base de Données des Polymorphismes Nucléotidiques Uniques (dbSNP)* du *National Center for Biotechnology Information (NCBI)* :

| | | | | | |
|---|---|---|---|---|---|
| rs306313 | rs505922 | rs12185539 | rs1455664 | rs10747951 | rs12224894 |
| rs12619108 | rs529565 | rs12185540 | rs911360 | rs10506432 | rs7951926 |
| rs6974924 | rs75558547 | rs807610 | rs6930922 | rs11174296 | rs7952569 |
| rs10091098 | rs2080811 | rs34779292 | rs12706520 | rs9738190 | rs7952095 |
| rs4569267 | rs 12619874 | rs807607 | rs4506155 | rs11627388 | rs7942564 |
| rs56132597 | rs62120724 | rs807606 | rs4595081 | rs7152677 | rs7948070 |
| rs12610495 | rs2302929 | rs807605 | rs6948273 | rs2059266 | rs7948539 |
| rs12223324 | rs2032778 | rs807604 | rs10156036 | rs7197718 | rs78243302 |
| rs8038527 | rs79808866 | rs807602 | rs6970487 | rs114093749 | rs12814401 |
| rs76187417 | rs41264195 | rs807599 | rs6974918 | rs73606907 | rs9592188 |
| rs17078348 | rs3770462 | rs62122281 | rs6951312 | rs79407403 | rs9598460 |
| rs13079478 | rs4668941 | rs807598 | rs2335848 | rs73606932 | rs12904268 |
| rs17214952 | rs2302934 | rs807597 | rs6963124 | rs79289897 | rs2279379 |
| rs9838450 | rs12616567 | rs807596 | rs3965316 | rs7198537 | rs35344266 |
| rs687621 | rs76880770 | rs807595 | rs10276960 | rs79300914 | rs34508855 |
| rs687289 | rs76860840 | rs807594 | rs10273930 | rs60395048 | rs12989715 |
| rs657152 | rs62122279 | rs807592 | rs6949642 | rs16957124 | rs134130 |
| rs644234 | rs4668946 | rs807590 | rs10088118 | rs76668502 | rs9882319 |
| rs544873 | rs12614308 | rs807589 | rs4389947 | rs16957126 | rs35896106 |
| rs545971 | rs807636 | rs807587 | rs10087789 | rs77595609 | rs13071258 |
| rs8176663 | rs807635 | rs807586 | rs13263570 | rs117438562 | rs17763537 |
| rs493246 | rs807628 | rs807585 | rs28730361 | rs13381043 | rs34668658 |
| rs495203 | rs807627 | rs807583 | rs12796811 | rs4797120 | rs17078346 |
| rs582094 | rs807623 | rs807581 | rs10506436 | rs7936322 | rs17763742 |
| rs2769071 | rs807622 | rs807579 | rs10784278 | rs3901233 | rs72893671 |
| rs677355 | rs807620 | rs807578 | rs10784279 | rs7945912 | rs17713054 |
| rs676996 | rs807618 | rs807577 | rs9739956 | rs4963163 | rs13078854 |
| rs550057 | rs807615 | rs807576 | rs10877786 | rs7940065 | rs71325088 |
| rs674302 | rs807613 | rs1455662 | rs11174294 | rs7930569 | rs10490770 |
| rs554833 | rs807611 | rs1455663 | rs10877787 | rs3934992 | rs35624553 |
| rs67959919 | rs13066516 | rs34442130 | rs60019065 | rs71327014 | rs60328892 |

(suite)

| | | | | | |
|---|---|---|---|---|---|
| rs35508621 | rs77902290 | rs13075758 | rs71327009 | rs71327015 | rs73434199 |
| rs34288077 | rs2171531 | rs17330872 | rs35930050 | rs34127208 | rs73434201 |
| rs35081325 | rs55920693 | rs71327003 | rs34584867 | rs35516580 | rs117184073 |
| rs35731912 | rs6785091 | rs36023124 | rs35161099 | rs34766614 | rs76670680 |
| rs34326463 | rs56332428 | rs71615438 | rs35159820 | rs71327017 | rs17452856 |
| rs76374459 | rs71325095 | rs34754340 | rs71327010 | rs13072267 | rs2440652 |
| rs73064425 | rs35501575 | rs34836513 | rs13090194 | rs36057789 | rs2461037 |
| rs13081482 | rs34381952 | rs34283240 | rs59166269 | rs76647202 | rs643434 |
| rs35652899 | rs4388012 | rs76281521 | rs57437758 | rs71327021 | rs612169 |
| rs35044562 | rs41289616 | rs13433997 | rs34562820 | rs13091868 | rs491626 |
| rs73064431 | rs34000569 | rs67937868 | rs13082697 | rs13092030 | rs492488 |
| rs13092887 | rs34324101 | rs55875328 | rs13060287 | rs17215981 | rs494242 |
| rs2191031 | rs13069079 | rs67200151 | rs34619093 | rs13089554 | rs582118 |
| rs34901975 | rs76597151 | rs13089855 | rs68087193 | rs13095717 | rs676457 |
| rs17764831 | rs34849862 | rs34493660 | rs2102055 | rs13075528 | rs527210 |
| rs34518147 | rs35827997 | rs3851346 | rs2102056 | rs13095602 | rs660340 |
| rs74586549 | rs35209528 | rs35883205 | rs2088690 | rs71327023 | rs581107 |
| rs34338823 | rs36039366 | rs61650989 | rs34774687 | rs13063527 | rs659104 |
| rs71325091 | rs35855315 | rs35669129 | rs35814488 | rs13068570 | rs647800 |
| rs3774641 | rs35525815 | rs13081151 | rs6790866 | rs1491950 | rs473533 |
| rs17764980 | rs17280623 | rs13081213 | rs6791016 | rs4373103 | rs475419 |
| rs17714101 | rs13078739 | rs34168660 | rs6780028 | rs34492478 | rs476410 |
| rs17714228 | rs13059238 | rs35751180 | rs2088692 | rs71327024 | rs645982 |
| rs71325092 | rs13079869 | rs4362758 | rs34438204 | rs1491951 | rs500498 |
| rs35280891 | rs33910087 | rs73833520 | rs13063033 | rs34460587 | rs633862 |
| rs34068335 | rs13071283 | rs71327006 | rs34679077 | rs34452002 | |
| rs1994491 | rs 17215008 | rs71327007 | rs34863575 | rs34093271 | |
| rs1994492 | rs71325098 | rs35454877 | rs35772789 | rs13093179 | |
| rs1994493 | rs71325100 | rs35334665 | rs34340587 | rs71327025 | |
| rs75928798 | rs41289622 | rs36040135 | rs77399277 | rs35539222 | |
| rs2373087 | rs71325101 | rs13074382 | rs34718164 | rs35110864 | |
| rs35831747 | rs71325102 | rs13097556 | rs13096741 | rs35581648 | |
| rs13099120 | rs13066062 | rs2230322 | rs35420565 | rs13190738 | |
| rs35477280 | rs36122610 | rs876668 | rs34047915 | rs55839388 | |

| | | | | | |
|---|---|---|---|---|---|
| rs11385942 | rs9877748 | rs34414382 | rs4766665 | rs1859333 | rs3937434 |
| rs35482426 | rs9845382 | rs34194160 | rs4988618 | rs1981554 | rs2016831 |
| rs7653372 | rs9845542 | rs34745455 | rs7958379 | rs9971885 | rs757405 |

(suite)

| | | | | | |
|---|---|---|---|---|---|
| rs6782814 | rs13069742 | rs71327041 | rs6489866 | rs57484342 | rs2010604 |
| rs71619614 | rs35951367 | rs71327042 | rs6489867 | rs61266553 | rs4767045 |
| rs147246298 | rs35464221 | rs114115904 | rs6489868 | rs7966314 | rs10744791 |
| rs143443576 | rs13085367 | rs34677490 | rs6489869 | rs4767037 | rs12462642 |
| rs35257780 | rs13063635 | rs1542756 | rs6489870 | rs1981557 | rs732631 |
| rs147316571 | rs71327027 | rs1542755 | rs4766666 | rs1981556 | rs2109069 |
| rs71325096 | rs78380307 | rs13060713 | rs4767033 | rs1981555 | rs2109070 |
| rs139901862 | rs4443214 | rs78919248 | rs4767034 | rs3759376 | rs2277732 |
| rs34044394 | rs4493469 | rs72856706 | rs10850099 | rs3759375 | rs7255545 |
| rs71619620 | rs17282391 | rs111837807 | rs10850100 | rs4767040 | rs12627039 |
| rs61556067 | rs13083881 | rs112785163 | rs4766667 | rs10774679 | rs17860115 |
| rs2088693 | rs17216717 | rs143334143 | rs4766668 | rs7132797 | rs12482556 |
| rs58552652 | rs13075836 | rs72856713 | rs4766671 | rs1156361 | rs11088247 |
| rs13087776 | rs13096325 | rs10774671 | rs4766672 | rs3815178 | rs2300370 |
| rs13088143 | rs13097340 | rs1131476 | rs4766673 | rs1859330 | rs2248420 |
| rs13068410 | rs3091314 | rs2660 | rs4766674 | rs7299132 | rs2834154 |
| rs13094262 | rs34558763 | rs7135577 | rs4766675 | rs6489879 | rs6517153 |
| rs34805664 | rs35178150 | rs4767024 | rs4766676 | rs4238033 | rs17860142 |
| rs35028116 | rs34693386 | rs4767026 | rs7304898 | rs4767041 | rs9636867 |
| rs34610609 | rs79948053 | rs4767027 | rs7315441 | rs7955267 | rs12482193 |
| rs13086534 | rs34523728 | rs4767029 | rs7305035 | rs7311182 | rs12482060 |
| rs13064632 | rs34570200 | rs4767030 | rs7316586 | rs10735079 | rs17860169 |
| rs34254987 | rs35560301 | rs6489865 | rs916972 | rs6489880 | rs9976829 |
| rs4683162 | rs34134191 | rs10850093 | rs7134391 | rs7980275 | rs13050728 |
| rs4683163 | rs115102354 | rs10850094 | rs7306205 | rs7977345 | rs2834157 |
| rs4683164 | rs13069750 | rs10850095 | rs1859336 | rs6489881 | rs2236756 |
| rs34155121 | rs34340501 | rs10774672 | rs2384071 | rs6489882 | rs2252639 |
| rs4234453 | rs71327038 | rs10850096 | rs2384072 | rs7131998 | rs2252650 |
| rs72901034 | rs36010446 | rs10850097 | rs2384073 | rs7135260 | rs2284549 |
| rs12108042 | rs13069845 | rs10774673 | rs6489874 | rs2269899 | rs2284550 |
| rs34867672 | rs17282797 | rs10774674 | rs6489875 | rs23 84074 | rs2284551 |
| rs34924300 | rs13080979 | rs10850098 | rs6489876 | rs10850103 | rs12053666 |
| rs4683166 | rs13098911 | rs10774676 | rs6489877 | rs2285932 | rs2073361 |
| rs7652478 | rs34378541 | rs10774677 | rs6489878 | rs7310667 | rs2834161 |
| rs7642229 | rs17282922 | rs4767031 | rs7298184 | rs10744789 | rs2834163 |
| rs7642320 | rs71327040 | rs4766663 | rs12322160 | rs4238034 | rs2834164 |
| rs9824651 | rs34289272 | rs4766664 | rs7132404 | rs4767044 | rs2834165 |
| rs9825081 | rs34005848 | rs4767032 | rs4767036 | rs1557866 | rs2236757 |

compare le nombre de SNP déterminés en saillie chez le patient à un nombre de référence, le nombre de référence étant associé à un degré de gravité connu de la maladie de Covid 19, et
dans lequel la présence d'un nombre de SNP chez le patient associé à un degré de gravité de la maladie de Covid-19 est pronostique pour le degré de gravité auquel la maladie de Covid-19 se développe chez le patient.

**2.** Procédé selon la revendication 1, dans lequel les SNP suivants sont déterminés :

| | | | | | |
|---|---|---|---|---|---|
| rs306313 | rs12610495 | rs17214952 | rs544873 | rs2769071 | rs505922 |
| rs12619108 | rs12223324 | rs9838450 | rs545971 | rs677355 | rs529565 |
| rs6974924 | rs8038527 | rs687621 | rs8176663 | rs676996 | |
| rs10091098 | rs76187417 | rs687289 | rs493246 | rs550057 | |
| rs4569267 | rs17078348 | rs657152 | rs495203 | rs674302 | |
| rs56132597 | rs13079478 | rs644234 | rs582094 | rs554833 | |

**3.** Procédé selon l'une quelconque des revendications précédentes, dans lequel, en plus du SNP rs13079478, les SNP suivants sont déterminés :

| | | | | | |
|---|---|---|---|---|---|
| rs1994491 | rs77902290 | rs35501575 | rs35525815 | rs71325100 | rs71327003 |
| rs1994493 | rs2171531 | rs13069079 | rs13078739 | rs13066062 | rs36023124 |
| rs35831747 | rs55920693 | rs76597151 | rs13079478 | rs36122610 | |
| rs35477280 | rs56332428 | rs34849862 | rs13079869 | rs34442130 | |
| rs13066516 | rs71325095 | rs36039366 | rs33910087 | rs13075758 | |

et en plus du SNP rs17214952, les SNP suivants ont été déterminés :

| | | | | | |
|---|---|---|---|---|---|
| rs1994492 | rs34381952 | rs35827997 | rs13071283 | rs71325101 | |
| rs75928798 | rs4388012 | rs35209528 | rs17214952 | rs71325102 | |
| rs2373087 | rs41289616 | rs35855315 | rs17215008 | rs17330872 | |
| rs13099120 | rs34000569 | rs17280623 | rs71325098 | | |
| rs6785091 | rs34324101 | rs13059238 | rs41289622 | | |

qui sont des SNP du gène FYC01, qui sont des marqueurs causaux de la pathogenèse et de la gravité d'une maladie Covid-19.

**4.** Procédé selon l'une quelconque des revendications précédentes, dans lequel on détermine en outre au moins l'un des SNP suivants, qui influencent le taux de réplication du virus SARS-CoV2 :

| | | | | | |
|---|---|---|---|---|---|
| rs10907185 | rs1061508 | rs11684157 | rs6720483 | rs34037363 | rs17027526 |
| rs3855951 | rs1061508 | rs3099559 | rs12469386 | rs7647812 | rs116034902 |
| rs3131660 | rs12079454 | rs3099559 | rs74453786 | rs11706052 | rs78248692 |
| rs34517448 | rs3736757 | rs3112916 | rs2305138 | rs11716445 | rs6831 |
| rs4233338 | rs67337751 | rs3112916 | rs3738904 | rs80073654 | rs3209157 |
| rs4233338 | rs60400761 | rs116686919 | rs7652331 | rs114691754 | rs6889892 |
| rs4610985 | rs73093505 | rs4666170 | rs7652331 | rs16838298 | rs72647244 |

(suite)

| | | | | | |
|---|---|---|---|---|---|
| rs4610985 | rs76309804 | rs4666170 | rs1545985 | rs114453087 | rs62364494 |
| rs191000784 | rs10495150 | rs34656234 | rs13079478 | rs12510574 | rs28460815 |
| rs273885 | rs6428948 | rs2723088 | rs33910087 | rs77591659 | rs72787387 |
| rs76861341 | rs79779531 | rs2723089 | rs33910087 | rs10015634 | rs2291628 |
| rs67008022 | rs9287719 | rs10195328 | rs4683158 | rs6532203 | rs12523609 |
| rs12116970 | rs6714413 | rs10195328 | rs4683158 | rs6532208 | rs374748 |
| rs12116970 | rs6714413 | rs62140404 | rs4683158 | rs10003496 | rs28763954 |
| rs7521948 | rs72777326 | rs12714145 | rs17214952 | rs7655622 | rs331079 |
| rs6692394 | rs11684157 | rs2346553 | rs6791542 | rs10516428 | rs7701116 |
| rs7724108 | rs 12547746 | rs117341758 | rs78925317 | rs10774517 | rs8182064 |
| rs11951084 | rs4738406 | rs74924161 | rs10770437 | rs280590 | rs2703580 |
| rs1549203 | rs73264571 | rs61917258 | rs1514831 | rs57620361 | rs116915857 |
| rs11958425 | rs116990828 | rs61917258 | rs1514831 | rs73168527 | rs76089422 |
| rs72800300 | rs72722967 | rs7311276 | rs7133858 | rs9507502 | rs118031436 |
| rs116200464 | rs7865343 | rs7311276 | rs7133858 | rs9507502 | rs8042654 |
| rs116200464 | rs7028704 | rs11061620 | rs10841162 | rs7335635 | rs8033595 |
| rs17061784 | rs16918878 | rs12812965 | rs10841162 | rs117936226 | rs76029207 |
| rs12652763 | rs117351867 | rs2887533 | rs10770446 | rs117936226 | rs76029207 |
| rs72814105 | rs34680486 | rs3924361 | rs7962879 | rs9544446 | rs62021260 |
| rs79931282 | rs10985215 | rs117086722 | rs4497470 | rs11617489 | rs1058260 |
| rs9392453 | rs2296793 | rs73041063 | rs16915248 | rs78768204 | rs1135911 |
| rs17513153 | rs148203698 | rs73041063 | rs11044463 | rs12863903 | rs2277589 |
| rs17169592 | rs148203698 | rs11061660 | rs7965006 | rs12863903 | rs3784471 |
| rs1459558 | rs7077992 | rs73029107 | rs7965006 | rs222670 | rs1982906 |
| rs1649229 | rs6560859 | rs73029190 | rs10841210 | rs222671 | rs12901252 |
| rs328902 | rs6560859 | rs56262248 | rs79072570 | rs568318446 | rs7168655 |
| rs34949146 | rs6560859 | rs73034919 | rs10843330 | rs10137084 | rs215095 |
| rs79787993 | rs10829268 | rs144000701 | rs1496218 | rs10137084 | rs215059 |
| rs117718957 | rs10829268 | rs10848459 | rs10843346 | rs7150942 | rs215059 |
| rs117718957 | rs10829268 | rs73038672 | rs10843346 | rs12717411 | rs215066 |
| rs78906654 | rs11101684 | rs77082078 | rs116789485 | rs12717411 | rs119774 |
| rs2868177 | rs11538725 | rs6489283 | rs1454934 | rs11158820 | rs215069 |
| rs2868180 | rs11538725 | rs6489283 | rs7967264 | rs117146868 | rs6498595 |
| rs7804806 | rs7937799 | rs118186282 | rs11052270 | rs2498827 | rs6498595 |
| rs56295910 | rs2957865 | rs73597943 | rs7979825 | rs2498825 | rs8187843 |
| rs56295910 | rs10897453 | rs111449649 | rs701006 | rs2474030 | rs8187843 |
| rs3757508 | rs10897453 | rs75321406 | rs1633360 | rs77743349 | rs8187843 |
| rs2395833 | rs928948 | rs76653618 | rs41292019 | rs12885234 | rs4780589 |
| rs257376 | rs17825668 | rs12313774 | rs78948244 | rs62011404 | rs4780589 |

(suite)

| | | | | | |
|---|---|---|---|---|---|
| rs16930092 | rs548907 | rs7975502 | rs61068064 | rs78141811 | rs1967120 |
| rs7830335 | rs548907 | rs79833769 | rs77411677 | rs76249401 | rs1967120 |
| rs12547746 | rs117341758 | rs78925317 | rs55707601 | rs8182064 | rs3784862 |
| rs3784862 | rs212079 | rs4782377 | rs75584356 | rs3865452 | rs2839217 |
| rs3784863 | rs2299670 | rs11646212 | rs3931634 | rs17857107 | rs2073382 |
| rs152033 | rs212080 | rs3751675 | rs3931634 | rs17857107 | rs35940413 |
| rs2230669 | rs246141 | rs4782307 | rs3931634 | rs17857107 | rs2839245 |
| rs35599 | rs12932079 | rs2879097 | rs7253253 | rs431579 | rs2839245 |
| rs35621 | rs9935402 | rs9892443 | rs17001450 | rs431579 | rs1044998 |
| rs35626 | rs4782505 | rs7215873 | rs75973396 | rs6115218 | rs35346764 |
| rs11861085 | rs8053676 | rs117859736 | rs2079014 | rs4811837 | rs2839246 |
| rs11861115 | rs8053676 | rs746628 | rs2079014 | rs34500739 | rs7277175 |
| rs3887893 | rs7199004 | rs77584326 | rs3760884 | rs2839217 | rs2839256 |
| rs212079 | rs7199004 | rs74917169 | rs7507651 | rs2839217 | rs2839256 |
| rs743346 | rs4646316 | rs4646316 | rs2072798 | rs11704825 | |

**5.** Procédé selon l'une des revendications précédentes, dans lequel on détermine en outre au moins l'un des SNP suivants, qui sont associés aux maladies suivantes, afin d'intégrer l'influence des SNP associés aux maladies dans l'évaluation du risque Covid-19 :

| | | | |
|---|---|---|---|
| rs63750847 | Maladie d'Alzheimer | rs28930068 | Paralysie périodique hypocalcémique |
| rs1801131 | Anaphylaxie post-vaccinale | rs28930069 | Paralysie périodique hypocalcémique |
| rs1801133 | Anaphylaxie post-vaccinale | rs80338779 | Paralysie périodique hypocalcémique |
| rs949963 | Anaphylaxie post-vaccinale | rs80338777 | Paralysie périodique hypocalcémique |
| rs2228139 | Anaphylaxie post-vaccinale | rs121908555 | Paralysie périodique hypocalcémique |
| rs4848300 | Anaphylaxie post-vaccinale | rs80338788 | Paralysie périodique hypocalcémique |
| rs17561 | Anaphylaxie post-vaccinale | rs80338784 | Paralysie périodique hypocalcémique |
| rs1800587 | Anaphylaxie post-vaccinale | rs121918007 | Hypophosphatasie |
| rs1801278 | Antidiabétique Sulphonylurée | rs121918002 | Hypophosphatasie |
| rs1801282 | Antidiabétique Sulphonylurée | rs2476601 | Hypothyroïdie |
| rs7903146 | Antidiabétique Sulphonylurée | rs7850258 | Hypothyroïdie |
| rs12255372 | Antidiabétique Sulphonylurée | rs3184504 | Hypothyroïdie |
| rs5215 | Antidiabétique Sulphonylurée | rs2517532 | Hypothyroïdie |
| rs5219 | Antidiabétique Sulphonylurée | rs12610495 | Fibrose pulmonaire idiopathique |
| rs757110 | Antidiabétique Sulphonylurée | rs1278769 | Fibrose pulmonaire idiopathique |
| rs1799859 | Antidiabétique Sulphonylurée | rs1980653 | Fibrose pulmonaire idiopathique |
| rs6196 | Antipsychotiques et prise de poids | rs1981997 | Fibrose pulmonaire idiopathique |
| rs17782313 | Antipsychotiques et prise de poids | rs2034650 | Fibrose pulmonaire idiopathique |
| rs489693 | Antipsychotiques et prise de poids | rs6793295 | Fibrose pulmonaire idiopathique |

(suite)

| | | | |
|---|---|---|---|
| rs1801131 | Antipsychotiques et prise de poids | rs7934606 | Fibrose pulmonaire idiopathique |
| rs5067 | Asthme | rs2187668 | Néphroapthie membraneuse idiopathique |
| rs689465 | Asthme | rs4664308 | Néphroapthie membraneuse idiopathique |
| rs4950928 | Asthme | rs9275596 | Néphropathie à IgA |
| rs1042713 | Asthme | rs363039 | Mesure de l'intelligence |
| rs1695 | Asthme | rs363050 | Mesure de l'intelligence |
| rs8069176 | Asthme | rs1511412 | Formation de chéloïdes |
| rs7216389 | Asthme | rs8032158 | Formation de chéloïdes |
| rs13153971 | Asthme | rs873549 | Formation de chéloïdes |
| rs37973 | Asthme Inhalation de corticostéroïdes | rs940187 | Formation de chéloïdes |
| rs2240017 | Asthme Inhalation de corticostéroïdes | rs7639618 | Ostéoarthrose du genou |
| rs11204971 | Dermatite atopique | rs4140564 | Ostéoarthrose du genou chez les femmes |
| rs4950928 | Dermatite atopique | rs5751876 | Le plaisir de la caféine |
| rs1143634 | Lésions musculaires dues à l'effort | rs6968865 | Le plaisir de la caféine |
| rs28497577 | Lésions musculaires dues à l'effort | rs2472297 | Le plaisir de la caféine |
| rs1800629 | Lésions musculaires dues à l'effort | rs6511720 | Maladie coronarienne |
| rs4880 | Lésions musculaires dues à l'effort | rs6903956 | Maladie coronarienne |
| rs 1800795 | Lésions musculaires dues à l'effort | rs9834312 | Taille du corps |
| rs 13266634 | Lésions musculaires dues à l'effort | rs2179922 | Taille du corps |
| rs1815739 | Lésions musculaires dues à l'effort | rs 1042725 | Taille du corps |
| rs1801282 | Test de glucose à l'effort | rs6060369 | Taille du corps |
| rs10936599 | Vieillissement biologique | rs8038652 | Taille du corps |
| rs2736100 | Vieillissement biologique | rs2802288 | Longévité |
| rs1042522 | Vieillissement biologique | rs2802292 | Longévité |
| rs1064395 | Trouble bipolaire | rs2764264 | Longévité |
| rs2820037 | Hypertension artérielle | rs2811712 | Longévité |
| rs5186 | Hypertension artérielle | rs34516635 | Longévité |
| rs4961 | Hypertension artérielle | rs2073711 | Hernie discale lombaire |
| rs5335 | Hypertension artérielle | rs2736100 | Fibrose pulmonaire |
| rs5370 | Hypertension artérielle | rs2076295 | Fibrose pulmonaire |
| rs6997709 | Hypertension artérielle | rs13277113 | Lupus (lupus érythémateux systémique) |
| rs7961152 | Hypertension artérielle | rs2187668 | Lupus (lupus érythémateux systémique) |
| rs11110912 | Hypertension artérielle | rs2205960 | Lupus (lupus érythémateux systémique) |
| rs1937506 | Hypertension artérielle | rs9888739 | Lupus (lupus érythémateux systémique) |
| rs2398162 | Hypertension artérielle | rs9287638 | Calvitie masculine |
| rs13333226 | Hypertension artérielle | rs9479482 | Calvitie masculine |
| rs5065 | Traitement de l'hypertension | rs2073963 | Calvitie masculine |
| rs13130484 | Indice de masse corporelle | rs6945541 | Calvitie masculine |
| rs6971091 | Indice de masse corporelle | rs10502861 | Calvitie masculine |
| rs2272383 | Indice de masse corporelle | rs8085664 | Calvitie masculine |
| rs1528133 | Indice de masse corporelle | rs2180439 | Calvitie masculine |
| rs1121980 | Indice de masse corporelle | rs6047844 | Calvitie masculine |
| rs10871777 | Indice de masse corporelle | rs1160312 | Calvitie masculine |
| rs12970134 | Indice de masse corporelle | rs1385699 | Calvitie masculine |

(suite)

| | | | |
|---|---|---|---|
| rs2229616 | Indice de masse corporelle | rs2223841 | Calvitie masculine |
| rs2272382 | Indice de masse corporelle | rs6152 | Calvitie masculine |
| rs1892534 | Protéine C-réactive | rs 180 1131 | Effet de la metfromine |
| rs8034191 | COPD | rs1801133 | Effet de la metfromine |
| rs7671167 | COPD | rs683369 | Effet de la metfromine |
| rs2653349 | Céphalées en grappe | rs34059508 | Effet de la metfromine |
| rs10883365 | Colitis ulcerosa | rs11212617 | Effet de la metfromine |
| rs2395185 | Colitis ulcerosa | rs1801133 | Méthotrexate et polyarthrite rhumatoïde |
| rs9858542 | Colitis ulcerosa | rs2651899 | Migraine |
| rs11209026 | Colitis Ulcerosa | rs10761659 | Morbus Crohn |
| rs646776 | Maladie cardiaque coronarienne | rs11190140 | Morbus Crohn |
| rs599839 | Maladie cardiaque coronarienne | rs17234657 | Morbus Crohn |
| rs3900940 | Maladie cardiaque coronarienne | rs1893217 | Morbus Crohn |
| rs7439293 | Maladie cardiaque coronarienne | rs2066844 | Morbus Crohn |
| rs383830 | Maladie cardiaque coronarienne | rs2066845 | Morbus Crohn |
| rs6922269 | Maladie cardiaque coronarienne | rs2066847 | Morbus Crohn |
| rs3798220 | Maladie cardiaque coronarienne | rs2241880 | Morbus Crohn |
| rs2070744 | Maladie cardiaque coronarienne | rs3197999 | Morbus Crohn |
| rs4977574 | Maladie cardiaque coronarienne | rs10513789 | Morbus Parkinson |
| rs1333048 | Maladie cardiaque coronarienne | rs12487066 | Sclérose en plaques |
| rs1333049 | Maladie cardiaque coronarienne | rs6897932 | Sclérose en plaques |
| rs501120 | Maladie cardiaque coronarienne | rs3129934 | Sclérose en plaques |
| rs964184 | Maladie cardiaque coronarienne | rs3135388 | Sclérose en plaques |
| rs2298566 | Maladie cardiaque coronarienne | rs7326018 | Sclérose en plaques |
| rs3184504 | Maladie cardiaque coronarienne | rs725613 | Sclérose en plaques |
| rs17228212 | Maladie cardiaque coronarienne | rs1154155 | Narcolepsie |
| rs8055236 | Maladie cardiaque coronarienne | rs2305795 | Narcolepsie |
| rs7250581 | Maladie cardiaque coronarienne | rs16944 | Ostéoarthrose de la hanche |
| rs4420638 | Maladie cardiaque coronarienne | rs10947262 | Ostéoarthrose du genou |
| rs688034 | Maladie cardiaque coronarienne | rs3815148 | Ostéoarthrose du genou |
| rs1360780 | Dépression | rs11842874 | Ostéoarthrose du genou |
| rs1545843 | Dépression | rs143383 | Ostéoarthrose du genou |
| rs1031681 | Dépression | rs7776725 | Ostéoporose |
| rs4655595 | Diabète sucré, type 2 | rs3736228 | Ostéoporose |
| rs6718526 | Diabète sucré, type 2 | rs1544410 | Ostéoporose |
| rs1801282 | Diabète sucré, type 2 | rs174230 | Mort subite d’origine cardiaque |
| rs358806 | Diabète sucré, type 2 | rs1800312 | Maladie de Pompe 1 et 2 |
| rs7659604 | Diabète sucré, type 2 | rs28940868 | Maladie de Pompe 1 et 2 |
| rs9465871 | Diabète sucré, type 2 | rs2856683 | Cirrhose biliaire primaire |
| rs1799884 | Diabète sucré, type 2 | rs7530511 | Psoriasis |
| rs13266634 | Diabète sucré, type 2 | rs11209026 | Psoriasis |
| rs10811661 | Diabète sucré, type 2 | rs3212227 | Psoriasis |
| rs9326506 | Diabète sucré, type 2 | rs6887695 | Psoriasis |
| rs4506565 | Diabète sucré, type 2 | rs10484554 | Psoriasis |
| rs7903146 | Diabète sucré, type 2 | rs11203366 | Arthrite rhumatoïde |
| rs12255372 | Diabète sucré, type 2 | rs13031237 | Arthrite rhumatoïde |
| rs5219 | Diabète sucré, type 2 | rs 1569723 | Arthrite rhumatoïde |
| rs9300039 | Diabète sucré, type 2 | rs2327832 | Arthrite rhumatoïde |
| rs10830963 | Diabète sucré, type 2 | rs2201841 | Arthrite rhumatoïde |
| rs 12304921 | Diabète sucré, type 2 | rs10889677 | Arthrite rhumatoïde |

(suite)

| | | | |
|---|---|---|---|
| rs 1495377 | Diabète sucré, type 2 | rs11162922 | Arthrite rhumatoïde |
| rs2930291 | Diabète sucré, type 2 | rs6679677 | Arthrite rhumatoïde |
| rs2903265 | Diabète sucré, type 2 | rs3738919 | Arthrite rhumatoïde |
| rs8050136 | Diabète sucré, type 2 | rs7574865 | Arthrite rhumatoïde |
| rs11868035 | Diabète sucré, type 2 | rs3087243 | Arthrite rhumatoïde |
| rs1889018 | Diabète sucré, type 2 | rs3816587 | Arthrite rhumatoïde |
| rs2295490 | Diabète sucré, type 2 | rs2269475 | Arthrite rhumatoïde |
| rs104893919 | Dysplasie diastophique | rs6910071 | Arthrite rhumatoïde |
| rs104893915 | Dysplasie diastophique | rs6457617 | Arthrite rhumatoïde |
| rs104893924 | Dysplasie diastophique | rs10499194 | Arthrite rhumatoïde |
| rs5082 | Effet diététique | rs6920220 | Arthrite rhumatoïde |
| rs1801282 | Effet diététique | rs729302 | Arthrite rhumatoïde |
| rs17300539 | Effet diététique | rs2004640 | Arthrite rhumatoïde |
| rs1800497 | Effet diététique | rs10488631 | Arthrite rhumatoïde |
| rs662799 | Effet diététique | rs3761847 | Arthrite rhumatoïde |
| rs16879765 | Maladie de Dupuytren | rs10818488 | Arthrite rhumatoïde |
| rs16946160 | Syndrome néphrotique acquis | rs2104286 | Arthrite rhumatoïde |
| rs12713559 | Hypercholestérolémie familiale de type B | rs9550642 | Arthrite rhumatoïde |
| rs5742904 | Hypercholestérolémie familiale de type B | rs2837960 | Arthrite rhumatoïde |
| rs 144467873 | Hypercholestérolémie familiale de type B | rs1953126 | Arthrite rhumatoïde |
| rs28933405 | Hypertrophique familiale Cardiomyopathie | rs6684865 | Arthrite rhumatoïde |
| rs28938173 | Hypertrophique familiale Cardiomyopathie | rs3890745 | Arthrite rhumatoïde |
| rs104894369 | Hypertrophique familiale Cardiomyopathie | rs1049550 | Sarcoïdose |
| rs104894370 | Hypertrophique familiale Cardiomyopathie | rs12425791 | accident vasculaire cérébral |
| rs3218714 | Hypertrophique familiale Cardiomyopathie | rs10830962 | Diabète gestationnel (GDM) |
| rs3218713 | Hypertrophique familiale Cardiomyopathie | rs4402960 | Diabète gestationnel |
| rs104894503 | Hypertrophique familiale Cardiomyopathie | rs1799884 | Diabète gestationnel |
| rs104894502 | Hypertrophique familiale Cardiomyopathie | rs7903146 | Diabète gestationnel |
| rs1801282 | Formation de tissu adipeux | rs12255372 | Diabète gestationnel |
| rs4788102 | Obésité | rs10830963 | Diabète gestationnel |
| rs6548238 | Obésité | rs2187668 | Déficit sélectif en IgA |
| rs7647305 | Obésité | rs1990760 | Déficit sélectif en IgA |
| rs925946 | Obésité | rs9271366 | Déficit sélectif en IgA |
| rs5082 | Obésité | rs1004819 | Spondylitis ankylosans |
| rs17300539 | Obésité | rs10489629 | Spondylitis ankylosans |
| rs35859249 | Obésité | rs2201841 | Spondylitis ankylosans |
| rs6232 | Obésité | rs11465804 | Spondylitis ankylosans |
| rs6971091 | Obésité | rs11209026 | Spondylitis ankylosans |
| rs662799 | Obésité | rs1343151 | Spondylitis ankylosans |
| rs17817449 | Obésité | rs10889677 | Spondylitis ankylosans |
| rs17782313 | Obésité | rs11209032 | Spondylitis ankylosans |
| rs2229616 | Obésité | rs10865331 | Spondylitis ankylosans |
| rs1799884 | Poids à la naissance | rs17482078 | Spondylitis ankylosans |
| rs7903146 | Poids à la naissance | rs10050860 | Spondylitis ankylosans |
| rs1051730 | Poids à la naissance | rs2287987 | Spondylitis ankylosans |
| rs12565727 | Chauve | rs2303138 | Spondylitis ankylosans |
| rs76723693 | Déficit en glucose-6-phosphate déshydrogénase | rs1800972 | Tendinopathies du membre supérieur |
| rs137852318 | Déficit en glucose-6-phosphate déshydrogénase | rs1138545 | Tendinopathies du membre supérieur |

(suite)

| | | | |
|---|---|---|---|
| rs5030868 | Déficit en glucose-6-phosphate déshydrogénase | rs1800012 | Tendinopathies du membre supérieur |
| rs1050828 | Déficit en glucose-6-phosphate déshydrogénase | rs2104772 | Tendinopathies du membre inférieur |
| rs80356491 | Stockage de glycogène Maladie 1B | rs 12722 | Tendinopathies du membre inférieur |
| rs80356490 | Stockage de glycogène Maladie 1B | rs4747096 | Tendinopathies du membre inférieur |
| rs80356489 | Stockage de glycogène Maladie 1B | rs679620 | Tendinopathies du membre inférieur |
| rs113994129 | Maladie de stockage du glycogène de type 3 | rs1800012 | Tendinopathies du membre inférieur |
| rs113994130 | Maladie de stockage du glycogène de type 3 | rs4789932 | Tendinopathies du membre inférieur |
| rs113994131 | Maladie de stockage du glycogène de type 3 | rs 143383 | Tendinopathies du membre inférieur |
| rs119103251 | Maladie de stockage du glycogène de type 5 | rs6025 | Thrombose veineuse profonde |
| rs116987552 | Maladie de stockage du glycogène de type 5 | rs5361 | Thrombose veineuse profonde |
| rs2155219 | Allergie au pollen de graminées et fièvre des foins | rs268 | Thrombose veineuse profonde |
| rs646776 | Infarctus du myocarde | rs1893217 | Diabète de type 1 |
| rs17465637 | Infarctus du myocarde | rs1990760 | Diabète de type 1 |
| rs8089 | Infarctus du myocarde | rs3741208 | Diabète de type 1 |
| rs1333040 | Infarctus du myocarde | rs9273363 | Diabète de type 1 |
| rs2383206 | Infarctus du myocarde | rs10012946 | Diabète de type 2 |
| rs2383207 | Infarctus du myocarde | rs1111875 | Diabète de type 2 |
| rs10757278 | Infarctus du myocarde | rs1387153 | Diabète de type 2 |
| rs1927911 | Infarctus du myocarde | rs2237892 | Diabète de type 2 |
| rs501120 | Infarctus du myocarde | rs2383208 | Diabète de type 2 |
| rs662799 | Infarctus du myocarde | rs4712523 | Diabète de type 2 |
| rs17228212 | Infarctus du myocarde | i3002432 | Thromboembolie veineuse |
| rs 17576 | Infarctus du myocarde | rs2282679 | Taux de vitamine D |
| rs3782886 | Infarctus du myocarde | rs10741657 | Taux de vitamine D |
| rs7291467 | Infarctus du myocarde | rs1801175 | La maladie de Von Gierke |
| rs5742905 | Homocystinurie, dépendance à la pyridoxine | rs1801176 | La maladie de Von Gierke |
| rs12413409 | Hypertension | rs80356484 | La maladie de Von Gierke |
| rs1378942 | Hypertension | rs80356487 | La maladie de Von Gierke |
| rs16998073 | Hypertension | rs80356479 | La maladie de Von Gierke |
| rs17367504 | Hypertension | rs2200733 | Fibrillation auriculaire |
| rs3754777 | Hypertension | rs10033464 | Fibrillation auriculaire |
| rs964184 | Hypertriglycéridémie | rs1801253 | Effet des β-bloquants |

**6.** Procédé selon l'une quelconque des revendications précédentes, dans lequel on détermine en outre les SNP suivants, qui sont associés à un vieillissement sain :

| | | | | | |
|---|---|---|---|---|---|
| rs2274789 | rs1801214 | rs10224902 | rs1663564 | rs2591590 | rs6530368 |
| rs228648 | rs1995319 | rs73694648 | rs935241 | rs2591591 | rs1731469 |
| rs56125713 | rs2465570 | rs1800504 | rs935240 | rs2547068 | rs6527818 |
| rs609320 | rs2437323 | rs10250576 | rs278109 | rs2547069 | rs3213451 |

(suite)

| | | | | | |
|---|---|---|---|---|---|
| rs9508 | rs893805 | rs12338 | rs1491893 | rs2547070 | rs5926304 |
| rs35672330 | rs11935573 | rs2291219 | rs16919359 | rs2591593 | rs2529541 |
| rs17399810 | rs3810870 | rs16889283 | rs2297224 | rs2547072 | rs2071309 |
| rs1676486 | rs4701997 | rs11779306 | rs2770928 | rs2547073 | rs2071311 |
| rs59123361 | rs1445823 | rs2381409 | rs3751354 | rs953551 | rs2856733 |
| rs749387317 | rs3213936 | rs1138374 | rs2428249 | rs2591594 | rs1800280 |
| rs2266782 | rs7714670 | rs10780682 | rs3742290 | rs2547074 | rs228406 |
| rs11586699 | rs6453022 | rs17062264 | rs34756139 | rs2547075 | rs5917933 |
| rs12120084 | rs34592828 | rs2282683 | rs4883918 | rs2547076 | rs6610447 |
| rs3729547 | rs8654 | rs1057406 | rs147785041 | rs2591595 | rs501128 |
| rs3085 | rs160632 | rs10867826 | rs17852293 | rs2591596 | rs2495783 |
| rs6604561 | rs6874840 | rs10818708 | rs35435463 | rs1862461 | rs11545211 |
| rs360057 | rs10479190 | rs35902535 | rs941581 | rs1862462 | rs2516023 |
| rs966365 | rs10055840 | rs35898523 | rs4087943 | rs2591597 | rs5919015 |
| rs3811445 | rs2950844 | rs9886752 | rs1267657 | rs1048290 | rs2296542 |
| rs4148211 | rs2910327 | rs2297145 | rs35270670 | rs1133330 | rs12397326 |
| rs17850223 | rs2910330 | rs2274634 | rs35582838 | rs1054487 | rs12393722 |
| rs11556184 | rs2910005 | rs41284748 | rs8042146 | rs35647251 | rs7884806 |
| rs4129190 | rs3749780 | rs7908745 | rs169068 | rs7249743 | rs7880917 |
| rs6761276 | rs1042719 | rs1227049 | rs4610 | rs36106401 | rs7580 |
| rs61744148 | rs1042720 | rs17109671 | rs7192331 | rs2604901 | rs3012627 |
| rs13422424 | rs1823035 | rs11188225 | rs35583599 | rs3810327 | rs6647476 |
| rs3214503 | rs168726 | rs1048445 | rs117461525 | rs17271272 | rs1113265 |
| rs492594 | rs705441 | rs3740078 | rs12926089 | rs3745521 | rs10126146 |
| rs28630685 | rs416574 | rs11190245 | rs12926669 | rs5030878 | rs363774 |
| rs35813871 | rs8333 | rs2278842 | rs12935737 | rs8113436 | rs1045686 |
| rs918949 | rs2274136 | rs11197835 | rs12923538 | rs12984188 | rs5951328 |
| rs3796028 | rs1042303 | rs2901343 | rs3752426 | rs12984473 | rs111588852 |
| rs1004814 | rs2760118 | rs11813597 | rs2303238 | rs34604090 | rs6622126 |
| rs1364641 | rs4678 | rs484955 | rs8052064 | rs433291 | rs5946294 |
| rs3796031 | rs3131787 | rs6356 | rs3809870 | rs925878 | rs2278954 |
| rs3796032 | rs8084 | rs4758267 | rs2003968 | rs3810340 | rs371749 |
| rs11895915 | rs7192 | rs3213706 | rs2271231 | rs3760849 | rs12390 |
| rs6728818 | rs17882603 | rs61752934 | rs4796555 | rs35914474 | rs5910611 |
| rs1063639 | rs1130034 | rs948847 | rs17853331 | rs145250945 | rs11452643 |
| rs7613447 | rs10093 | rs174535 | rs 17856697 | rs12609654 | rs502434 |
| rs2291862 | rs1048372 | rs1800007 | rs7209228 | rs 1205194 | rs3747343 |
| rs394558 | rs1049133 | rs2004649 | rs906807 | rs16987712 | rs2498776 |
| rs2245532 | rs1049130 | rs61744384 | rs1484873 | rs6017667 | rs877761 |

(suite)

| | | | | | |
|---|---|---|---|---|---|
| rs62244134 | rs1063323 | rs7116967 | rs56342526 | rs3746493 | rs176037 |
| rs2286720 | rs3208181 | rs2848477 | rs56131056 | rs3737137 | rs3765272 |
| rs2276818 | rs3213484 | rs3809018 | rs55659726 | rs434857 | rs237520 |
| rs1044243 | rs1049110 | rs1052313 | rs3752234 | rs2017816 | rs626560 |
| rs9855810 | rs2071551 | rs10891705 | rs3752237 | rs116988298 | rs12116111 |
| rs11708527 | rs762815 | rs2282537 | rs3752246 | rs6586239 | rs5983916 |
| rs3732799 | rs1042187 | rs595986 | rs757292 | rs1980982 | rs561077 |
| rs1381057 | rs1042331 | rs11063529 | rs7250872 | rs1128127 | rs1139916 |
| rs3218649 | rs1042335 | rs7298766 | rs2396359 | rs165734 | rs1432838968 |
| rs9884018 | rs1071597 | rs11612427 | rs3746045 | rs165602 | rs5969919 |
| rs1056523 | rs12528378 | rs35642012 | rs2074789 | rs5997714 | rs6526155 |
| rs4688761 | rs623813 | rs1049112 | rs9973235 | rs56174639 | rs34966902 |
| rs2242353 | rs2297970 | rs1806191 | rs9304915 | rs36125344 | rs1059703 |
| rs9823911 | rs13194610 | rs1805482 | rs778805 | rs55653327 | rs1059702 |
| rs9836841 | rs7767455 | rs10841611 | rs2287915 | rs11705624 | rs2728532 |
| rs1709657 | rs1130656 | rs2720298 | rs2216663 | rs1127000 | rs1126762 |
| rs843334 | rs3823465 | rs2603104 | rs2547064 | rs2306734 | |
| rs11556518 | rs55666134 | rs923829 | rs2252675 | rs11222 | |
| rs2515960 | rs6968741 | rs7955450 | rs1867691 | rs10871864 | |
| rs1024323 | rs3735398 | rs17034524 | rs2121133 | rs41309545 | |

**7.** Procédé selon l'une quelconque des revendications précédentes, dans lequel, pour prédire la gravité de l'évolution d'une maladie de Covid-19 chez un patient humain

a) l'expression quantitative du gène ARN hôte est déterminée selon la formule suivante :

$$\sum_{i=1}^{n} = F_{1*}\begin{pmatrix} positif \\ neutre \\ négatif \end{pmatrix} + F_{2*}\begin{pmatrix} positif \\ neutre \\ négatif \end{pmatrix} + F_{3*}\begin{pmatrix} positif \\ neutre \\ négatif \end{pmatrix} + F_{4*}\begin{pmatrix} positif \\ neutre \\ négatif \end{pmatrix}$$

b) la fonctionnalité qualitative des protéines est déterminée selon la formule suivante :

$$\sum_{j=1}^{m} = f_{1*}\begin{pmatrix} positif \\ neutre \\ négatif \end{pmatrix} + f_{2*}\begin{pmatrix} positif \\ neutre \\ négatif \end{pmatrix} + f_{3*}\begin{pmatrix} positif \\ neutre \\ négatif \end{pmatrix}$$

ce qui donne le risque de couronne polygénique par gène :

$$\sum_{i=1}^{n} + \sum_{j=1}^{m} = \begin{matrix} \text{Risque de couronne} \\ \text{polygénique} \\ \text{(PCRG) par gène} \end{matrix}$$

où dans les formules signifient :

Σ= somme pondérée/régression linéaire
i ... n = nombre de SNP non codants
j ... m = nombre de SNP codants
$F_1$, $F_2$, $F_3$, ... $F_n$ = paramètres d'évaluation individuels
$f_1$, $f_2$, $f_3$, ... $f_n$ = paramètres d'évaluation individuels
positif = nombre de SNPs ayant un effet favorable (valeur 2)
neutre = nombre de SNPs qui n'ont pas d'effet (valeur 1)
négatif = nombre de SNPs qui ont un effet défavorable (valeur 0)

**8.** Procédé selon l'une des revendications précédentes, dans lequel on différencie si la maladie de Covid-19 est provoquée par un défaut monogénique ou par un défaut polygénique dû à l'interaction de nombreux variants SNP (protéines structurelles) et d'éléments SNP régulateurs.

Figur 1

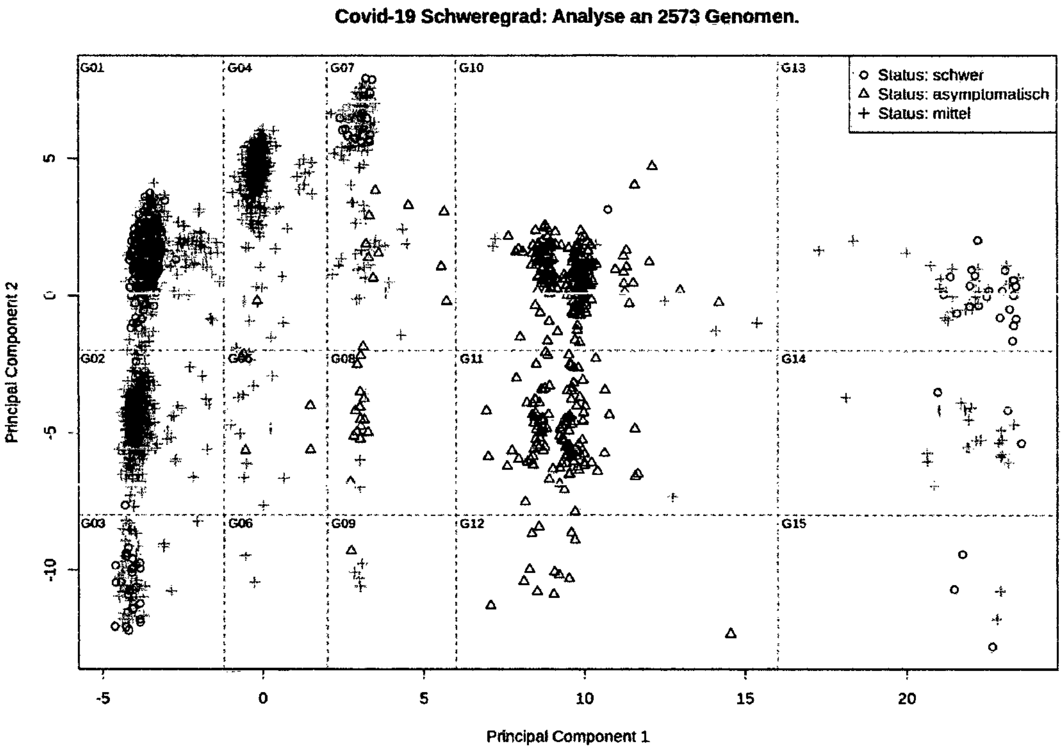


Figur 2

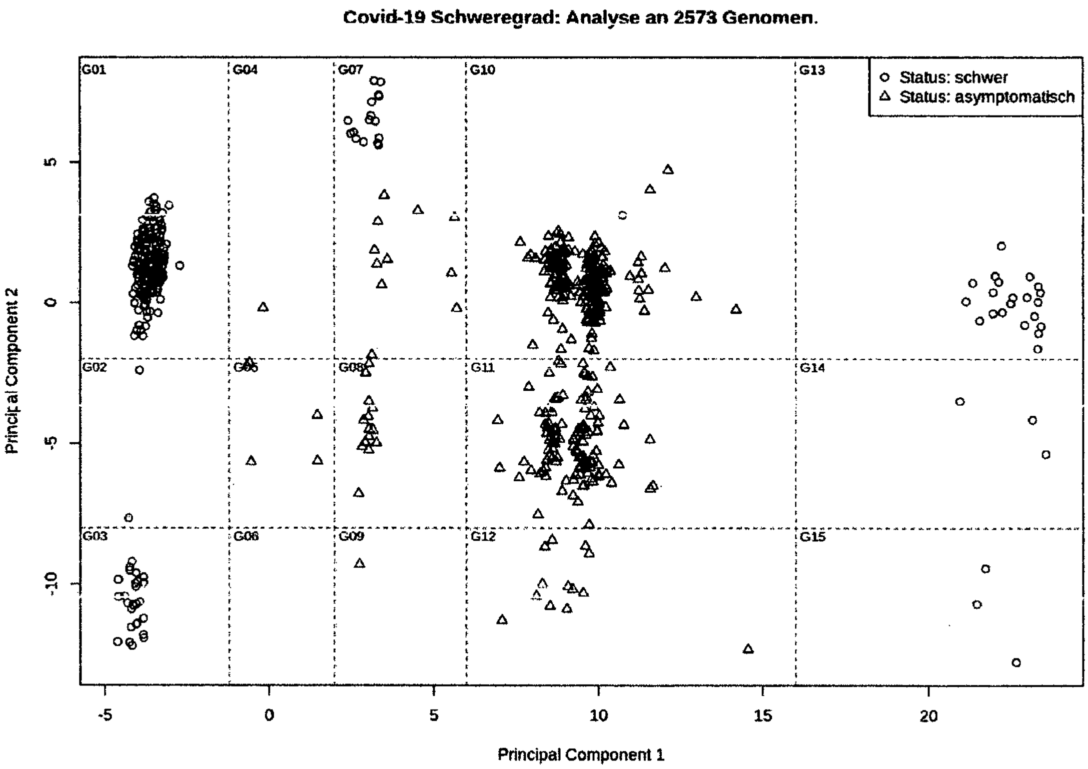

Figur 3

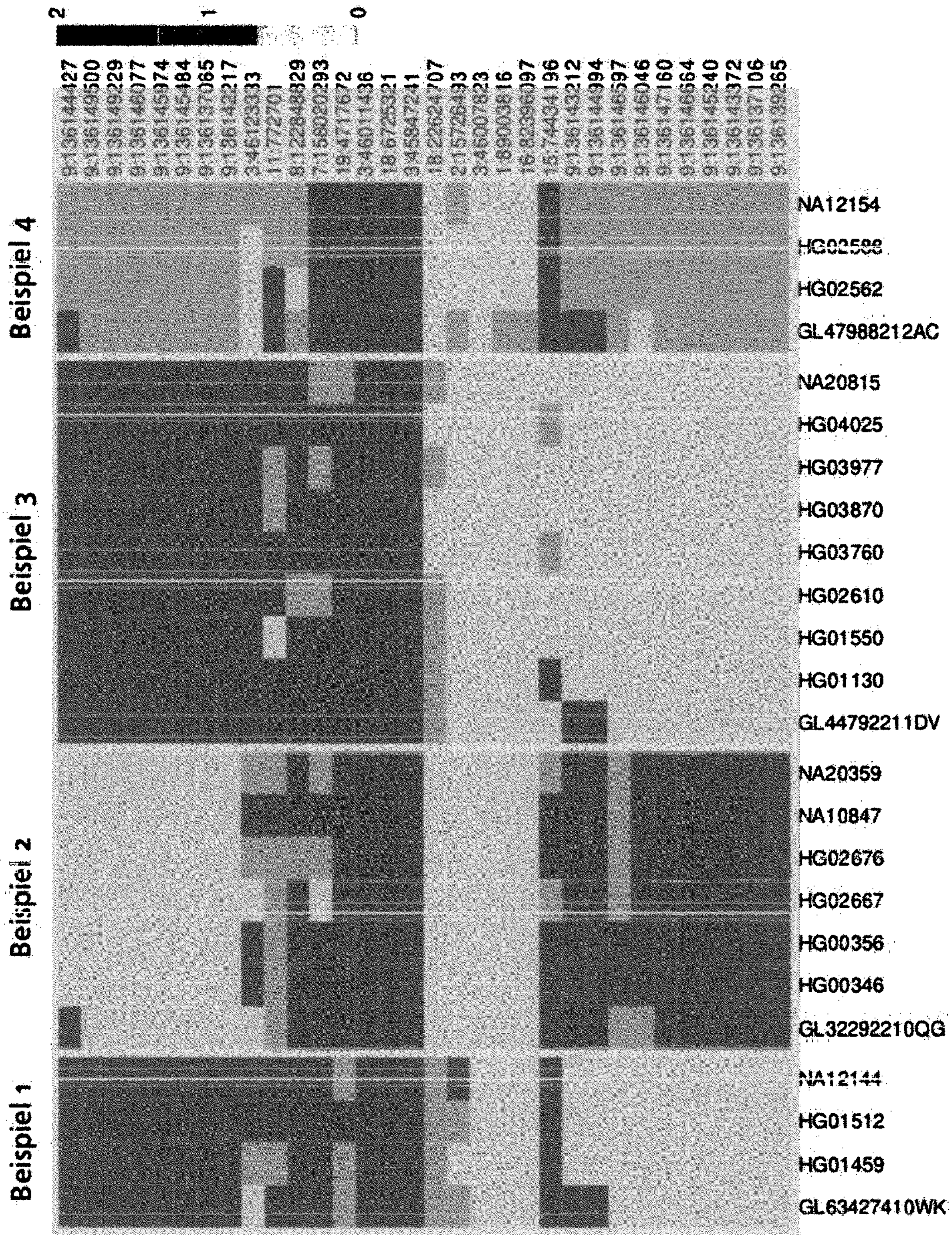
2
1
0
9:136144427
9:136149500
9:136149229
9:136146077
9:136145974
9:136145484
9:136137065
9:136142217
3:46123333
11:772701
8:122848329
7:158020293
19:4717672
3:46011436
18:6725321
3:45847241
18:22624707
2:15726493
3:46007823
1:89003816
16:82396097
15:74434196
9:136143212
9:136144994
9:136146597
9:136146046
9:136147160
9:136146664
9:136145240
9:136143372
9:136137106
9:136139265
Beispiel 4
NA12154
HG02588
HG02562
GL47988212AC
Beispiel 3
NA20815
HG04025
HG03977
HG03870
HG03760
HG02610
HG01550
HG01130
GL44792211DV
Beispiel 2
NA20359
NA10847
HG02676
HG02667
HG00356
HG00346
GL32292210QG
Beispiel 1
NA12144
HG01512
HG01459
GL63427410WK

Figur 4

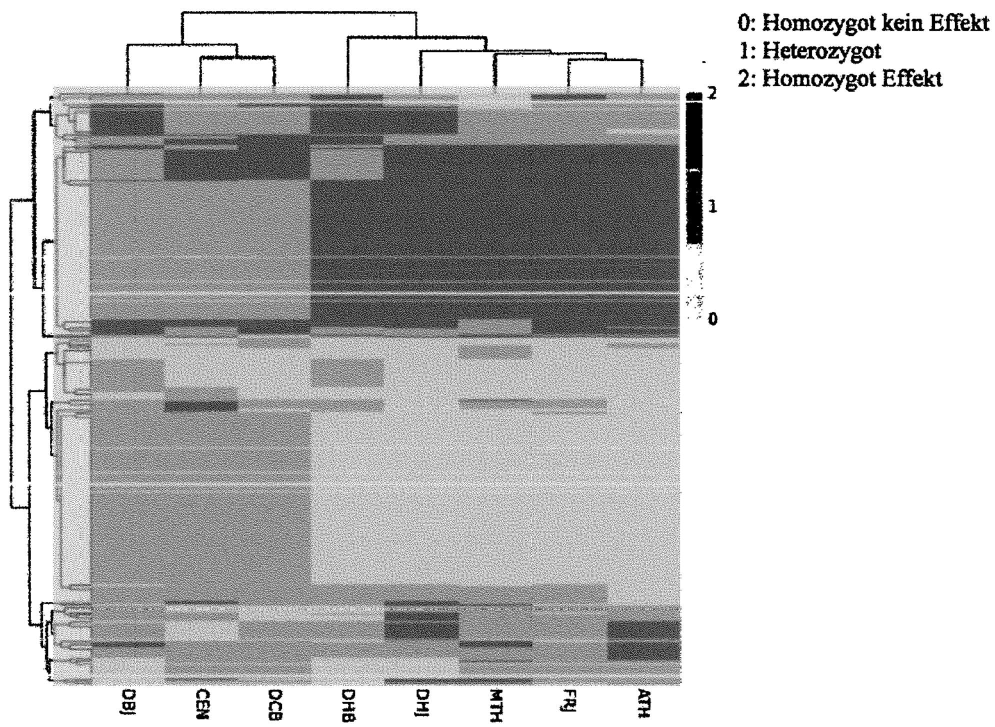


Figur 5

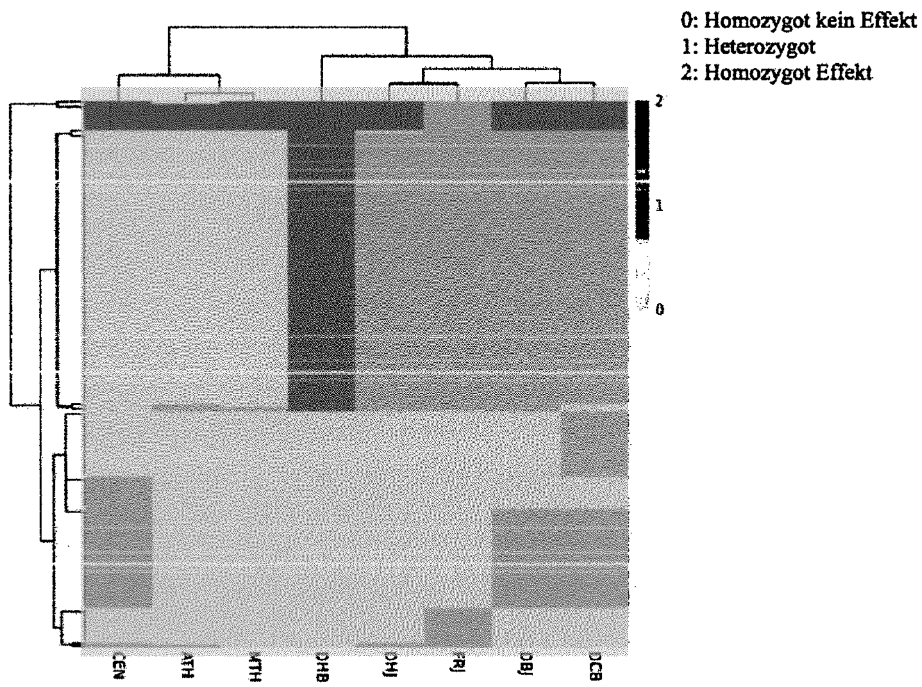

Figur 6

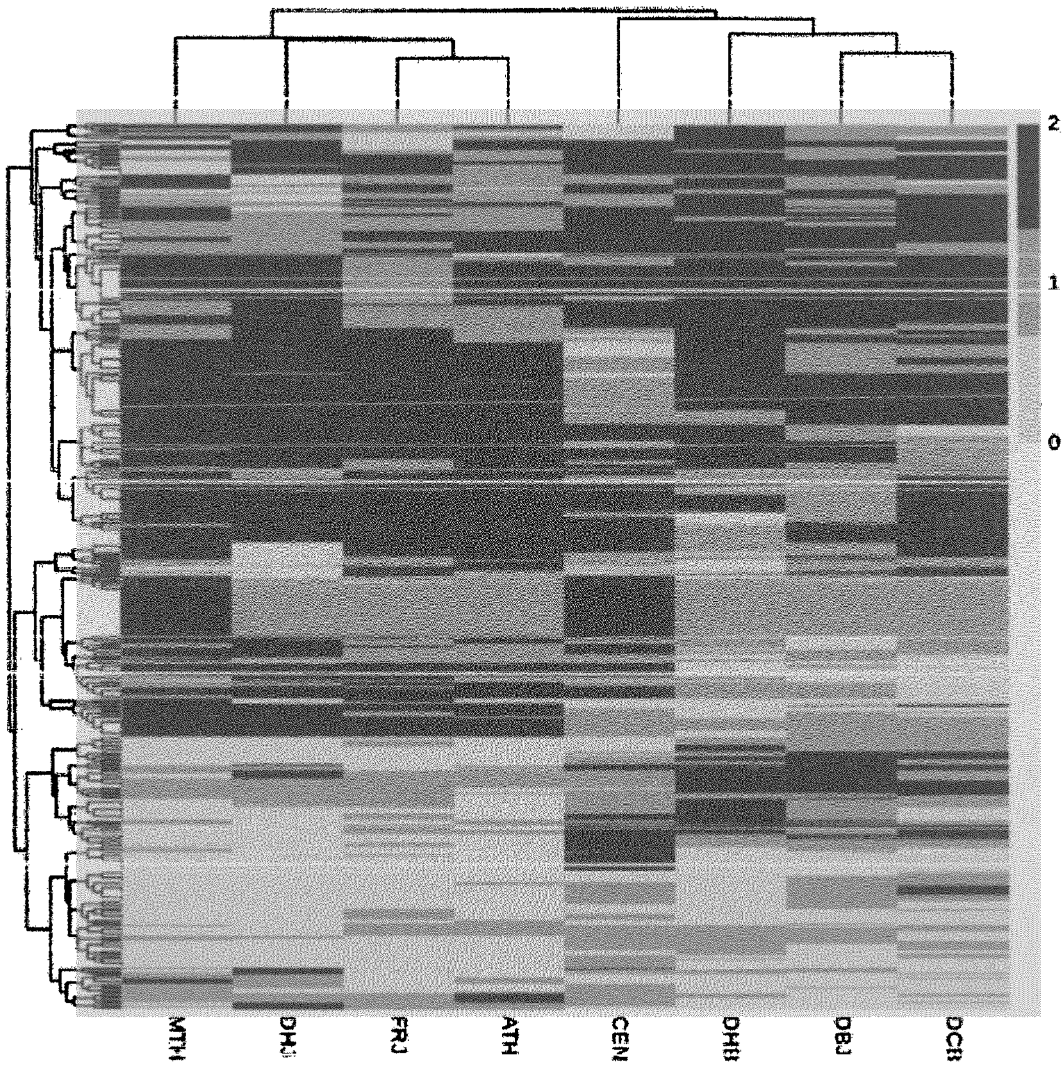


0: Homozygot kein Effekt
1: Heterozygot
2: Homozygot Effekt

Figur 7a

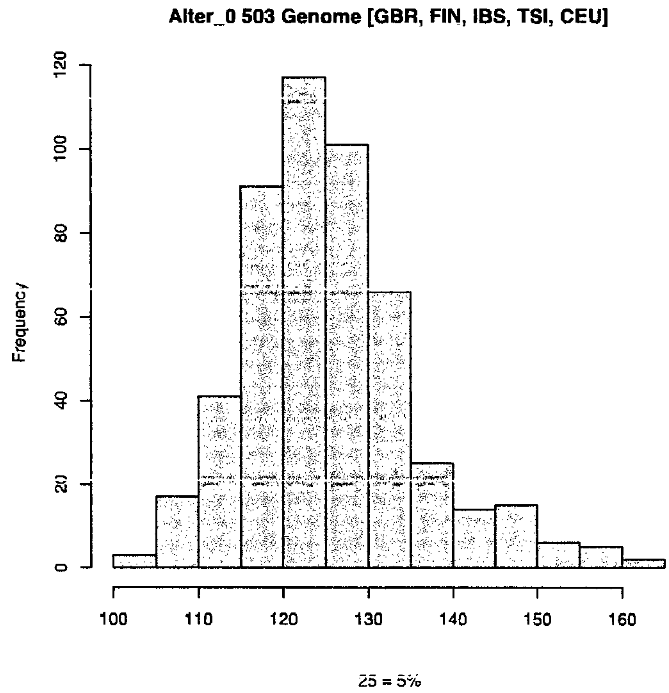


Figur 7b

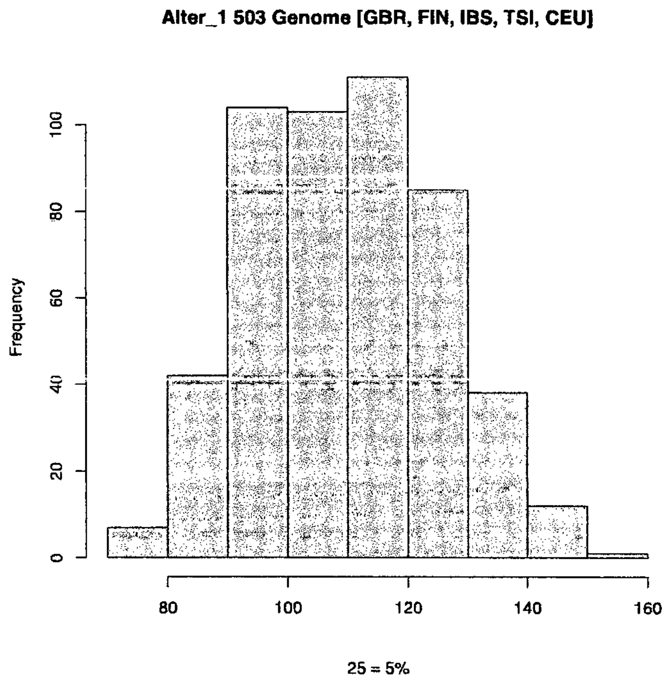

**Figur 7c**

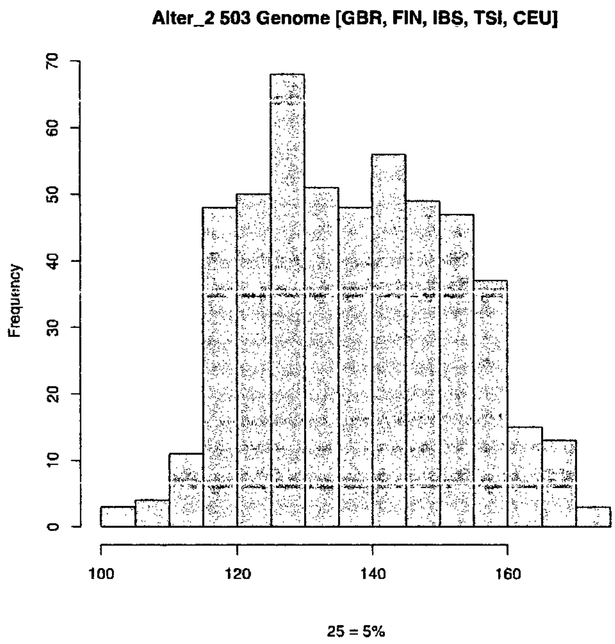


**Figur 7d** CoV_Patent SNP_0:

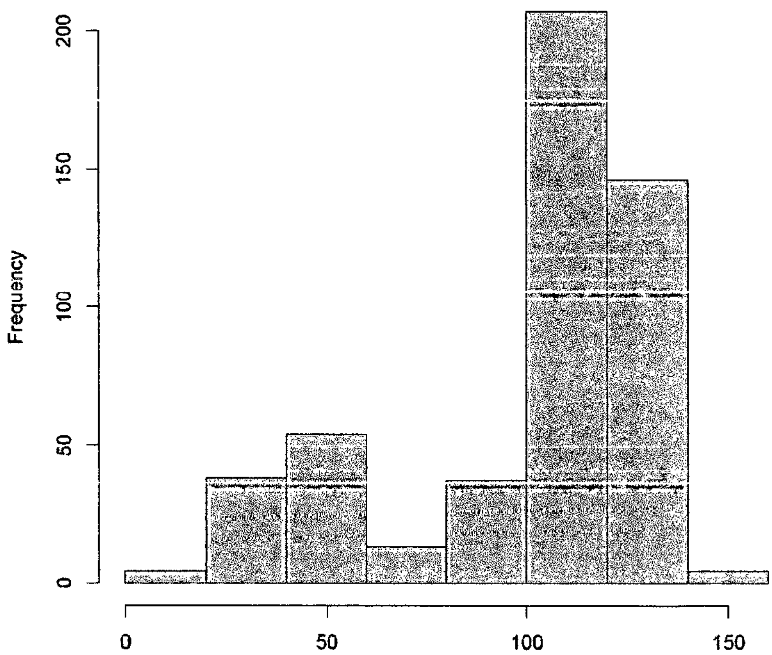

Figur 7e

CoV-Patent_1: 503 Genomes

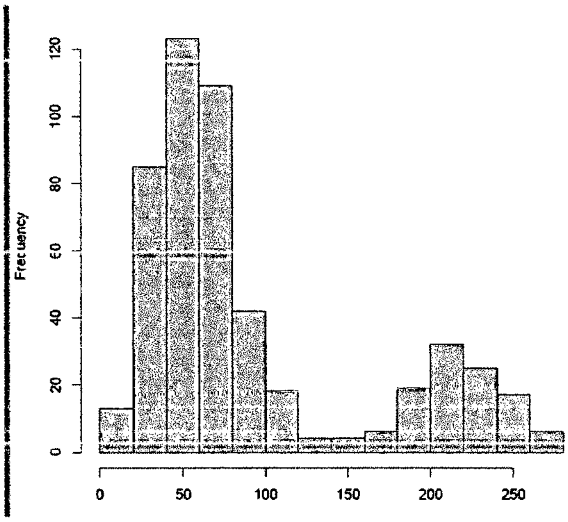


Figur 7f

CoV-Patent_2: 503 Genomes

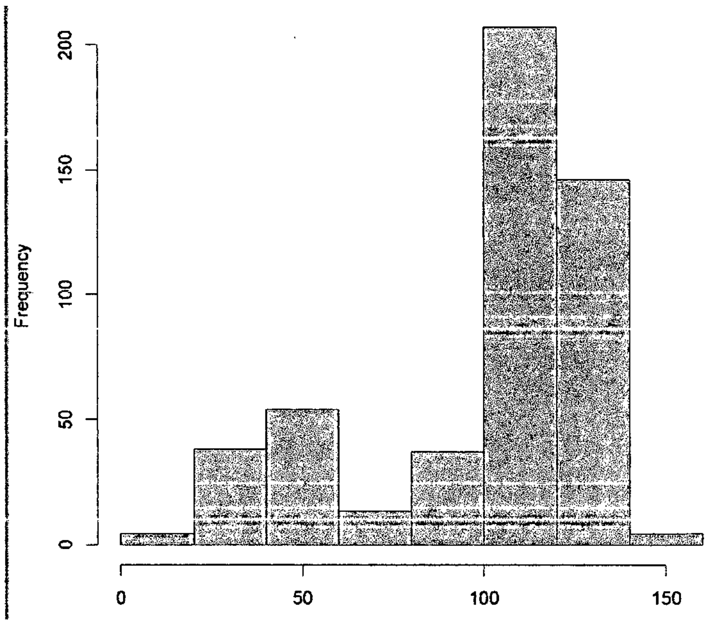

Figur 7g

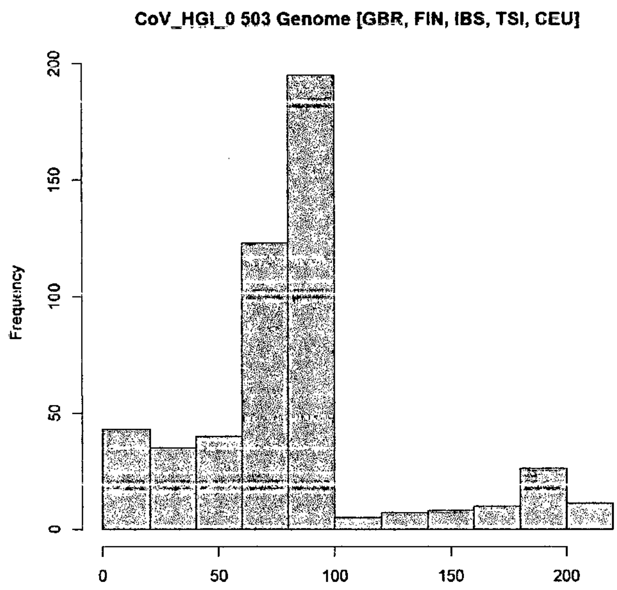


Figur 7h

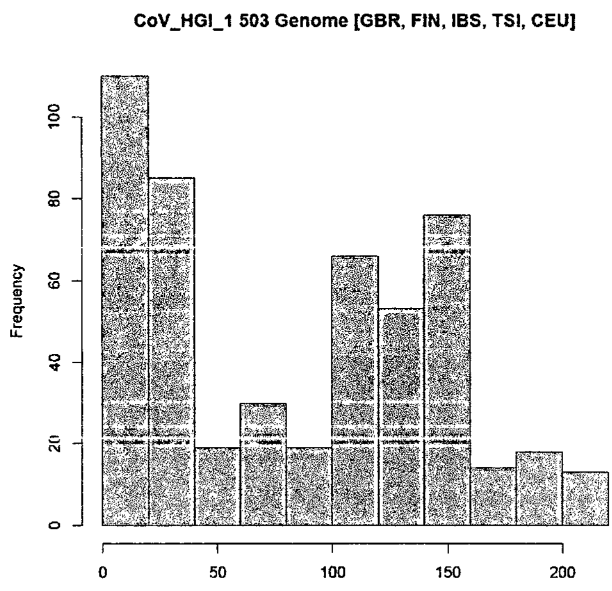

**Figur 7i**

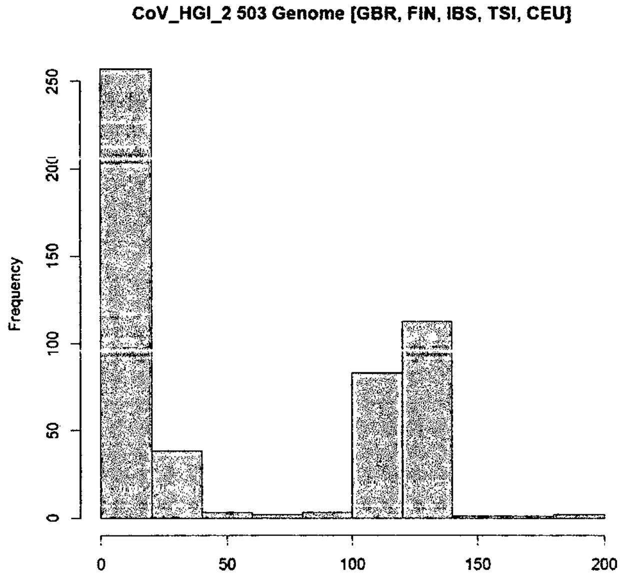
CoV_HGI_2 503 Genome [GBR, FIN, IBS, TSI, CEU]
Frequency
0
50
100
150
200
250
0
50
100
150
200

Figur 8a

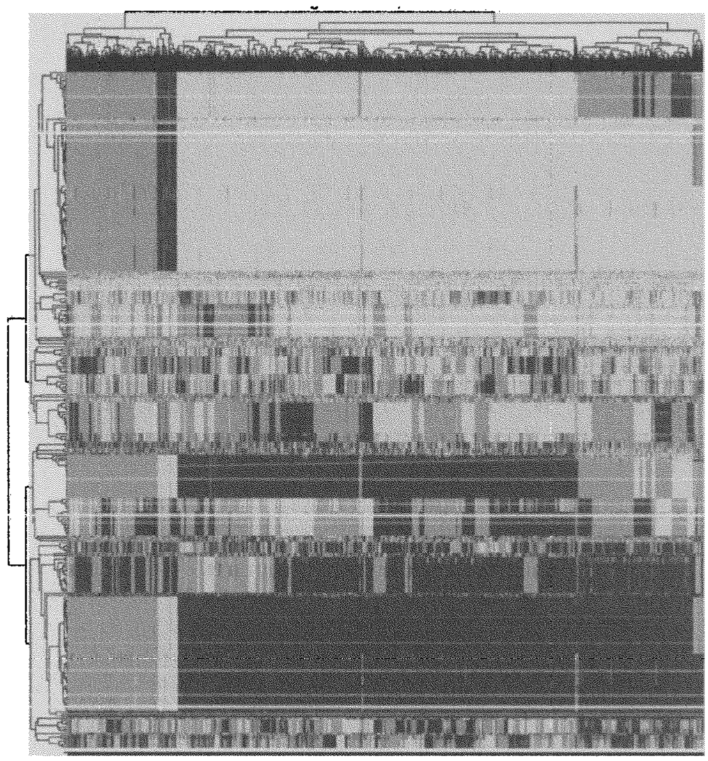

Figur 8b

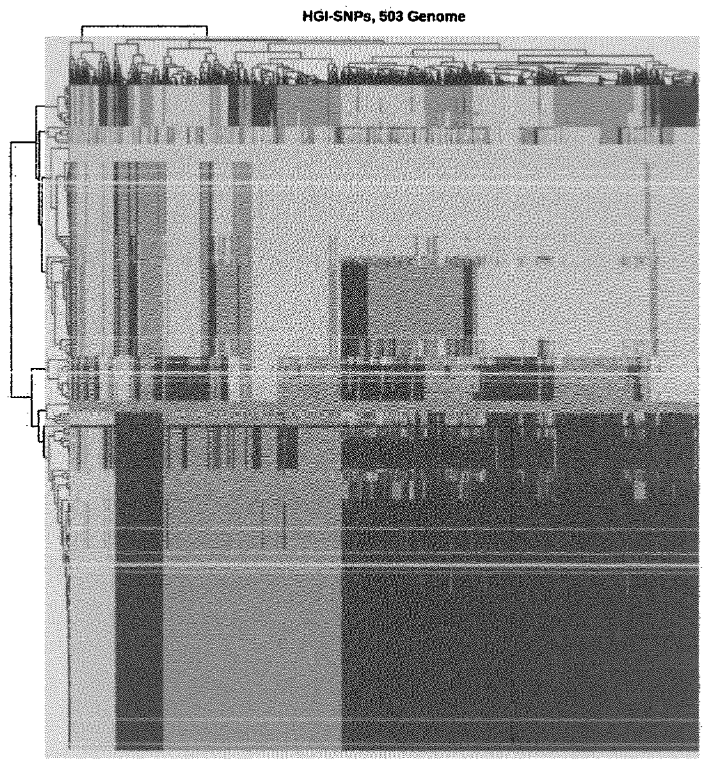

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2423329 A1
- EP 2423330 A1
- EP 2331709 B1

### In der Beschreibung aufgeführte Nicht-Patentliteratur


- **ELLINGHAUS D ; DEGENHARDT F ; BUJANDA L et al.** Genomewide Association Study of Severe Covid-19 with Respiratory Failure. *N Engl J Med,* 17. Juni 2020
- **ZHAO J ; YANG Y ; HUANG H et al.** Relationship between the ABO Blood Group and the Covid-19 Susceptibility. *Clin Infect Dis.,* 04. August 2020
- **GORDON DE ; JANG GM ; BOUHADDOU M et al.** A SARS-CoV-2 protein interaction map reveals targets for drug repurposing. *Nature,* 2020, vol. 583 (7816), 459-468
- **HUNDESHAGEN et al.** *Journal of Neuroinflammation,* 2012, vol. 9, 140
- **GIERMAN HJ ; FORTNEY K ; ROACH JC ; COLES NS ; LI H et al.** Whole-Genome Sequencing of the World's Oldest People. *PLoS ONE,* 2014, vol. 9 (11), e112430
- **QUELLE ; ANDREA GANNA.** Mapping the human genetic architecture of COVID-19 by worldwide meta-analysis. *The COVID-19 Host Genetics Initiative, https://doi.org/10.1101/2021.03.10.21252820*
- **D. ELLINGHAUS et al.** *N Engl J Med,* 2020, vol. 383, 1522-1534